(19) Europäisches Patentamt
European Patent Office
Office européen des brevets

(11) EP 3 418 386 B1

(12) EUROPEAN PATENT SPECIFICATION

(45) Date of publication and mention of the grant of the patent:
**07.04.2021 Bulletin 2021/14**

(51) Int Cl.:
***C12N 15/113*** *(2010.01)* ***A61K 31/713*** *(2006.01)*
*C07H 21/02* *(2006.01)*

(21) Application number: **18187709.3**

(22) Date of filing: **28.06.2012**

# (54) COMPOSITIONS AND METHODS FOR INHIBITING GENE EXPRESSION OF HEPATITIS B VIRUS

ZUSAMMENSETZUNGEN UND VERFAHREN ZUR HEMMUNG DER GENEXPRESSION DES HEPATITIS-B-VIRUS

COMPOSITIONS ET MÉTHODES PERMETTANT D'INHIBER L'EXPRESSION D'UN GÈNE DU VIRUS DE L'HÉPATITE B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2011 EP 11172235**

(43) Date of publication of application:
**26.12.2018 Bulletin 2018/52**

(62) Document number(s) of the earlier application(s) in accordance with Art. 76 EPC:
**12804876.6 / 2 726 613**

(73) Proprietor: **Arrowhead Pharmaceuticals, Inc.**
**Pasadena, CA 91101 (US)**

(72) Inventors:
- **CHIN, Daniel**
  **Bloomfield, NJ 07003 (US)**
- **DECKERT, Jochen**
  **95444 Bayreuth (DE)**
- **HOSSBACH, Markus**
  **95326 Kulmbach (DE)**
- **JOHN, Matthias**
  **96103 Hallstadt (DE)**

(74) Representative: **Cornish, Kristina Victoria Joy**
**Kilburn & Strode LLP**
**Lacon London**
**84 Theobalds Road**
**London WC1X 8NL (GB)**

(56) References cited:
**WO-A1-96/39502** **WO-A1-2011/073218**
**US-A1- 2003 206 887** **US-A1- 2006 292 691**

- **ZHANG, Y.-L. ET AL.: "RNA Interference inhibits Hepatitis B Virus of different genotypes in Vitro and in Vivo.", BMC MICROBIOLOGY, vol. 10, no. 1, 10 August 2010 (2010-08-10), pages 214/1-214/10, XP021073092, ISSN: 1471-2180, DOI: 10.1186/1471-2180-10-214**
- **CHEN, Z. ET AL.: "Combination of small interfering RNAs mediates greater inhibition of human hepatitis B virus replication and antigen expression", JOURNAL OF ZHEJIANG UNIVERSITY, vol. 6B, no. 4, 1 April 2005 (2005-04-01), pages 236-241, XP055241567, CN ISSN: 1009-3095, DOI: 10.1631/jzus.2005.B0236**
- **CHRISTINE I WOODDELL ET AL: "Hepatocyte-targeted RNAi Therapeutics for the Treatment of Chronic Hepatitis B Virus Infection", MOLECULAR THERAPY, vol. 21, no. 5, 1 May 2013 (2013-05-01), pages 973-985, XP055149314, ISSN: 1525-0016, DOI: 10.1038/mt.2013.31**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 418 386 B1

SEQUENCE LISTING

<110> Arrowhead Pharmaceuticals, Inc.
Chin, Daniel
Deckert, Jochen
Hossbach, Markus
John, Matthias

<120> Compositions and Methods for Inhibiting Gene Expression of Hepatitis B Virus

<130> 4/2XN72/26A

<141> 2012-06-08

<150> EP11172235.1
<151> 2011-06-30

<150> US13/535,454
<151> 2012-06-08

<160> 648

<170> Patent In version 3.5

<210> 1
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 1
caagguaugu ugcccguuu 19

<210> 2
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 2
cuguaggcau aaauuggua 19

<210> 3
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 3
ucugcggcgu uuuaucaua 19

<210> 4
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 4
accucugccu aaucaucuc 19

<210> 5
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 5
uuuacuagug ccauuugua 19

<210> 6
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 6
accucugccu aaucaucua 19

<210> 7
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 7
cuguaggcau aaauugguc 19

<210> 8
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 8
ugucugcggc guuuuauca 19

<210> 9
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 9
uacuagugcc auuuguuca 19

<210> 10
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 10
caacuuuuuc accucugca 19

<210> 11
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 11
ccauuuguuc agugguucg 19

<210> 12
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 12
ccaaguguuu gcugacgca 19

<210> 13
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 13
ccauuuguuc agugguuca 19

<210> 14
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 14
uuuacuagug ccauuuguu 19

<210> 15
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 15
caccucugcc uaaucauca 19

<210> 16
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 16
cuggcucagu uuacuagug 19

<210> 17
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 17
caagguaugu ugcccguua 19

<210> 18
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 18
cuggcucagu uuacuagua 19

<210> 19
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 19
gaggcuguag gcauaaauu 19

<210> 20
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 20
caguuuacua gugccauuu 19

<210> 21
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 21
agguauguug cccguuugu 19

<210> 22
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 22
uauguugccc guuugucca 19

<210> 23
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 23
gaggcuguag gcauaaaua 19

<210> 24
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 24
gucugcggcg uuuuaucau 19

<210> 25
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 25
caacuuuuuc accucugcc 19

<210> 26
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 26
ccgugugcac uucgcuuca 19

<210> 27
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 27
ucaagguaug uugcccgua 19

<210> 28
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 28
caguuuacua gugccauua 19

<210> 29
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 29
ugguggacuu cucucaauu 19

<210> 30
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 30
agguauguug cccguuuga 19

<210> 31
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 31
cugcucgugu uacaggcgg 19

<210> 32
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 32
uauguugccc guuuguccu 19

<210> 33
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 33
ucaagguaug uugcccguu 19

<210> 34
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 34
ucuuaucaac acuuccgga 19

<210> 35
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 35
caccucugcc uaaucaucu 19

<210> 36
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 36
auaagaggac ucuuggacu 19

<210> 37
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 37
gucugcggcg uuuuaucaa 19

<210> 38
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 38
ggcgcugaau cccgcggac 19

<210> 39
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 39
cgcgucgcag aagaucuca 19

<210> 40
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 40
aaugucaacg accgaccuu 19

<210> 41
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 41
gcucaguuua cuagugcca 19

<210> 42
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 42
ugguggacuu cucucaaua 19

<210> 43
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 43
aucgccgcgu cgcagaaga 19

<210> 44
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 44
gccauuuguu cagugguuc 19

<210> 45
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 45
cgauccauac ugcggaacu 19

<210> 46
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 46
ucaccucugc cuaaucauc 19

<210> 47
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 47
guggacuucu cucaauuuu 19

<210> 48
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 48
gggucaccau auucuuggg 19

<210> 49
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 49
gccgcgucgc agaagaucu 19

<210> 50
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 50
ucaaucgccg cgucgcaga 19

<210> 51
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 51
uggauguguc ugcggcguu 19

<210> 52
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 52
uacuguucaa gccuccaag 19

<210> 53
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 53
guuuacuagu gccauuugu 19

<210> 54
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 54
acuagugcca uuuguucag 19

<210> 55
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 55
ccgcgucgca gaagaucuc 19

<210> 56
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 56
uaucuuauca acacuuccg 19

<210> 57
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 57
ggccaaaauu cgcagucccc 19

<210> 58
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 58
uucaccucug ccuaaucau 19

<210> 59
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 59
cucaguuuac uagugccau 19

<210> 60
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 60
uguugcccgu uuguccucu 19

<210> 61
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 61
uagugccauu uguucagug 19

<210> 62
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 62
aggcuguagg cauaaauug 19

<210> 63
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 63
augugucugc ggcguuuua 19

<210> 64
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 64
acuucgcuuc accucugca 19

<210> 65
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 65
cgugugcacu ucgcuucac 19

<210> 66
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 66
gugguggacu ucucucaau 19

<210> 67
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 67
ugugucugcg gcguuuuau 19

<210> 68
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 68
aagguauguu gcccguuug 19

<210> 69
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 69
ucaacgaccg accuugagg 19

<210> 70
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 70
cauaagagga cucuuggac 19

<210> 71
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 71
gucaacgacc gaccuugag 19

<210> 72
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 72
auauucuugg gaacaagag 19

<210> 73
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 73
ugcucguguu acaggcggg 19

<210> 74
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 74
caaucgccgc gucgcagaa 19

<210> 75
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 75
acuguucaag ccuccaagc 19

<210> 76
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 76
cgccgcgucg cagaagauc 19

<210> 77
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 77
cauuuguuca gugguucgu 19

<210> 78
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 78
cgcugaaucc cgcggacga 19

<210> 79
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 79
ugggucacca uauucuugg 19

<210> 80
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 80
uccucugccg auccauacu 19

<210> 81
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 81
augucaacga ccgaccuug 19

<210> 82
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 82
ccucugccua aucaucuca 19

<210> 83
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 83
accgugugca cuucgcuuc 19

<210> 84
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 84
ugccgaucca uacugcgga 19

<210> 85
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 85
cagagucuag acucguggu 19

<210> 86
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 86
cuguucaagc cuccaagcu 19

<210> 87
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 87
ggaggcugua ggcauaaau 19

<210> 88
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 88
aggaggcugu aggcauaaa 19

<210> 89
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 89
gguggacuuc ucucaauuu 19

<210> 90
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<400> 90
gcaacuuuuu caccucugc 19

<210> 91
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 91
cugcucgugu uacaggcga 19

<210> 92
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 92
cuagugccau uuguucagu 19

<210> 93
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 93
cugccgaucc auacugcgg 19

<210> 94
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 94
gugugcacuu cgcuucacc 19

<210> 95
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 95
gcucguguua caggcgggc 19

<210> 96
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 96
ccuaucuuau caacacuuc 19

<210> 97
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 97
ucucaaucgc cgcgucgca 19

<210> 98
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 98
gcccgucugu gccuucuca 19

<210> 99
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 99
cuaucuuauc aacacuucc 19

<210> 100
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 100
auguugcccg uuuguccuc 19

<210> 101
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 101
guauguugcc cguuugucc 19

<210> 102
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 102
cuucgcuuca ccucugcac 19

<210> 103
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 103
ugugcacuuc gcuucaccu 19

<210> 104
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 104
gccaaaauuc gcagucccg 19

<210> 105
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 105
ccugcucgug uuacaggcg 19

<210> 106
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 106
uggagugugg auucgcacu 19

<210> 107
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 107
aacgaccgac cuugaggca 19

<210> 108
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 108
acagagucua gacucgugg 19

<210> 109
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 109
aaucgccgcg ucgcagaag 19

<210> 110
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 110
gguauguugc ccguuuguc 19

<210> 111
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 111
gccgauccau acugcggaa 19

<210> 112
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 112
gcccuaucuu aucaacacu 19

<210> 113
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 113
aguuuacuag ugccauuug 19

<210> 114
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 114
ugucaacgac cgaccuuga 19

<210> 115
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 115
acuucucuca auuuucuag 19

<210> 116
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 116
gcgcgggacg uccuuuguc 19

<210> 117
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 117
ucuagacucg ugguggacu 19

<210> 118
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 118
gauccauacu gcggaacuc 19

<210> 119
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 119
cucugccgau ccauacugc 19

<210> 120
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 120
ucugccgauc cauacugcg 19

<210> 121
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 121
ccucugccga uccauacug 19

<210> 122
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 122
gcaccucucu uuacgcggu 19

<210> 123
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 123
aagaacuccc ucgccucgc 19

<210> 124
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 124
gaacucccuc gccucgcag 19

<210> 125
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 125
ucucucaauu uucuagggc 19

<210> 126
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 126
gggcgcaccu cucuuuacg 19

<210> 127
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 127
ccgauccaua cugcggaac 19

<210> 128
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 128
aacucccucg ccucgcaga 19

<210> 129
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 129
cuccucugcc gauccauac 19

<210> 130
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 130
ggagugugga uucgcacuc 19

<210> 131
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 131
cgggcgcacc ucucuuuac 19

<210> 132
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 132
gucucaaucg ccgcgucgc 19

<210> 133
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 133
auccauacug cggaacucc 19

<210> 134
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 134
cgcaccucuc uuuacgcgg 19

<210> 135
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 135
caacgaccga ccuugaggc 19

<210> 136
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 136
ccauacugcg gaacuccua 19

<210> 137
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 137
ugaaucccgc ggacgaccc 19

<210> 138
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 138
agaacucccu cgccucgca 19

<210> 139
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 139
ggcgcaccuc ucuuuacgc 19

<210> 140
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 140
gcgcaccucu cuuuacgcg 19

<210> 141
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 141
gcugaauccc gcggacgac 19

<210> 142
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 142
cacuucgcuu caccucugc 19

<210> 143
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 143
cucaaucgcc gcgucgcag 19

<210> 144
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 144
ucccgucggc gcugaaucc 19

<210> 145
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 145
cugaaucccg cggacgacc 19

<210> 146
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 146
agagucuaga cucguggug 19

<210> 147
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 147
uccauacugc ggaacuccu 19

<210> 148
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 148
gcgcugaauc ccgcggacg 19

<210> 149
<211> 19
<212> RNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<400> 149
aguguggauu cgcacuccu 19

<210> 150
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 150
cccugcucgu guuacaggc 19

<210> 151
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 151
gaaucccgcg gacgacccg 19

<210> 152
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 152
aagcugugcc uugggguggc 19

<210> 153
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 153
gcccugcucg uguuacagg 19

<210> 154
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 154
gucccgucgg cgcugaauc 19

<210> 155
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 155
aucuuaucaa cacuuccgg 19

<210> 156
<211> 19
<212> RNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<400> 156
cuuaucaaca cuuccggaa 19

<210> 157
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 157
aaacgggcaa cauaccuug 19

<210> 158
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 158
taccaauuua ugccuacag 19

<210> 159
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 159
uaugauaaaa cgccgcaga 19

<210> 160
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 160
taugauaaaa cgccgcaga 19

<210> 161
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 161
gagaugauua ggcagaggu 19

<210> 162
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 162
tacaaauggc acuaguaaa 19

<210> 163
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 2..19
<223> /mod_base = "2’-hydroxy corresponding nucleoside"

<400> 163
tagaugauua ggcagaggu 19

<210> 164
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 164
gaccaauuua ugccuacag 19

<210> 165
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 165
ugauaaaacg ccgcagaca 19

<210> 166
<211> 19
<212> DNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<220>
<221> modified_base
<222> 2..19
<223> /mod_base = "2'-hydroxy corresponding nucleoside"

<400> 166
tgauaaaacg ccgcagaca 19

<210> 167
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 167
ugaacaaaug gcacuagua 19

<210> 168
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 168
tgaacaaaug gcacuagua 19

<210> 169
<211> 19
<212> DNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<220>
<221> modified_base
<222> 2..19
<223> /mod_base = "2'-hydroxy corresponding nucleoside"

<400> 169
tgcagaggug aaaaaguug 19

<210> 170
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 170
cgaaccacug aacaaaugg 19

<210> 171
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 171
ugcgucagca aacacuugg 19

<210> 172
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base

<222> 2..19
<223> /mod_base = "2'-hydroxy corresponding nucleoside"

<400> 172
tgcgucagca aacacuugg 19

<210> 173
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 173
tgaaccacug aacaaaugg 19

<210> 174
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 174
aacaaauggc acuaguaaa 19

<210> 175
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base

<222> 2..19
<223> /mod_base = "2'-hydroxy corresponding nucleoside"

<400> 175
tgaugauuag gcagaggug 19

<210> 176
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 176
cacuaguaaa cugagccag 19

<210> 177
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 177
taacgggcaa cauaccuug 19

<210> 178
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base

<222> 2..19
<223> /mod_base = "2'-hydroxy corresponding nucleoside"

<400> 178
tacuaguaaa cugagccag 19

<210> 179
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 179
aauuuaugcc uacagccuc 19

<210> 180
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 180
aaauggcacu aguaaacug 19

<210> 181
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 181
acaaacgggc aacauaccu 19

<210> 182
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 182
uggacaaacg ggcaacaua 19

<210> 183
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 183
tauuuaugcc uacagccuc 19

<210> 184
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 184
augauaaaac gccgcagac 19

<210> 185
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 185
ggcagaggug aaaaaguug 19

<210> 186
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 186
ugaagcgaag ugcacacgg 19

<210> 187
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 187
tgaagcgaag ugcacacgg 19

<210> 188
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base

<222> 2..19
<223> /mod_base = "2'-hydroxy corresponding nucleoside"

<400> 188
tacgggcaac auaccuuga 19

<210> 189
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 189
taauggcacu aguaaacug 19

<210> 190
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 190
aauugagaga aguccacca 19

<210> 191
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base

<222> 2..19
<223> /mod_base = "2'-hydroxy corresponding nucleoside"

<400> 191
tcaaacgggc aacauaccu 19

<210> 192
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 192
ccgccuguaa cacgagcag 19

<210> 193
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 193
aggacaaacg ggcaacaua 19

<210> 194
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 194
aacgggcaac auaccuuga 19

<210> 195
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 195
uccggaagug uugauaaga 19

<210> 196
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 196
tccggaagug uugauaaga 19

<210> 197
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 197
agaugauuag gcagaggug 19

<210> 198
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 198
aguccaagag uccucuuau 19

<210> 199
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 199
tugauaaaac gccgcagac 19

<210> 200
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 200
guccgcggga uucagcgcc 19

<210> 201
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 201
ugagaucuuc ugcgacgcg 19

<210> 202
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 202
aaggucgguc guugacauu 19

<210> 203
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 203
uggcacuagu aaacugagc 19

<210> 204
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 204
tauugagaga aguccacca 19

<210> 205
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 205
ucuucugcga cgcggcgau 19

<210> 206
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 206
gaaccacuga acaaauggc 19

<210> 207
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 207
aguuccgcag uauggaucg 19

<210> 208
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 208
gaugauuagg cagagguga 19

<210> 209
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 209
aaaauugaga gaaguccac 19

<210> 210
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 210
cccaagaaua uggugaccc 19

<210> 211
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 211
agaucuucug cgacgcggc 19

<210> 212
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 212
ucugcgacgc ggcgauuga 19

<210> 213
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 213
aacgccgcag acacaucca 19

<210> 214
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 214
cuuggaggcu ugaacagua 19

<210> 215
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 215
acaaauggca cuaguaaac 19

<210> 216
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 216
cugaacaaau ggcacuagu 19

<210> 217
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 217
gagaucuucu gcgacgcgg 19

<210> 218
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 218
cggaaguguu gauaagaua 19

<210> 219
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 219
gggacugcga auuuuggcc 19

<210> 220
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 220
augauuaggc agaggugaa 19

<210> 221
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 221
auggcacuag uaaacugag 19

<210> 222
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 222
agaggacaaa cgggcaaca 19

<210> 223
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 223
cacugaacaa auggcacua 19

<210> 224
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 224
caauuuaugc cuacagccu 19

<210> 225
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 225
uaaaacgccg cagacacau 19

<210> 226
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 226
taaaacgccg cagacacau 19

<210> 227
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 227
ugcagaggug aagcgaagu 19

<210> 228
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 228
gugaagcgaa gugcacacg 19

<210> 229
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 229
auugagagaa guccaccac 19

<210> 230
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5’-phosphate group"

<400> 230
auaaaacgcc gcagacaca 19

<210> 231
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 231
caaacgggca acauaccuu 19

<210> 232
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 232
ccucaagguc ggucguuga 19

<210> 233
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 233
guccaagagu ccucuuaug 19

<210> 234
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 234
cucaaggucg gucguugac 19

<210> 235
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 235
cucuuguucc caagaauau 19

<210> 236
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 236
cccgccugua acacgagca 19

<210> 237
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 237
uucugcgacg cggcgauug 19

<210> 238
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 238
gcuuggaggc uugaacagu 19

<210> 239
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 239
gaucuucugc gacgcggcg 19

<210> 240
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 240
acgaaccacu gaacaaaug 19

<210> 241
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 241
ucguccgcgg gauucagcg 19

<210> 242
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 242
ccaagaauau ggugaccca 19

<210> 243
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 243
aguauggauc ggcagagga 19

<210> 244
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 244
caaggucggu cguugacau 19

<210> 245
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 245
ugagaugauu aggcagagg 19

<210> 246
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 246
gaagcgaagu gcacacggu 19

<210> 247
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 247
uccgcaguau ggaucggca 19

<210> 248
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 248
accacgaguc uagacucug 19

<210> 249
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 249
agcuuggagg cuugaacag 19

<210> 250
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 250
auuuaugccu acagccucc 19

<210> 251
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 251
uuuaugccua cagccuccu 19

<210> 252
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 252
aaauugagag aaguccacc 19

<210> 253
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 253
gcagagguga aaaaguugc 19

<210> 254
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 254
tcgccuguaa cacgagcag 19

<210> 255
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 255
acugaacaaa uggcacuag 19

<210> 256
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 256
ccgcaguaug gaucggcag 19

<210> 257
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 257
ggugaagcga agugcacac 19

<210> 258
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 258
gcccgccugu aacacgagc 19

<210> 259
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 259
gaaguguuga uaagauagg 19

<210> 260
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 260
ugcgacgcgg cgauugaga 19

<210> 261
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 261
ugagaaggca cagacgggc 19

<210> 262
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 262
ggaaguguug auaagauag 19

<210> 263
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 263
gaggacaaac gggcaacau 19

<210> 264
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 264
ggacaaacgg gcaacauac 19

<210> 265
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 265
gugcagaggu gaagcgaag 19

<210> 266
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 266
aggugaagcg aagugcaca 19

<210> 267
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 267
cgggacugcg aauuuuggc 19

<210> 268
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 268
cgccuguaac acgagcagg 19

<210> 269
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 269
agugcgaauc cacacucca 19

<210> 270
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 270
ugccucaagg ucggucguu 19

<210> 271
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 271
ccacgagucu agacucugu 19

<210> 272
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 272
cuucugcgac gcggcgauu 19

<210> 273
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 273
gacaaacggg caacauacc 19

<210> 274
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 274
uuccgcagua uggaucggc 19

<210> 275
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 275
aguguugaua agauagggc 19

<210> 276
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 276
caaauggcac uaguaaacu 19

<210> 277
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 277
ucaaggucgg ucguugaca 19

<210> 278
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 278
cuagaaaauu gagagaagu 19

<210> 279
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 279
gacaaaggac gucccgcgc 19

<210> 280
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 280
aguccaccac gagucuaga 19

<210> 281
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 281
gaguuccgca guauggauc 19

<210> 282
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 282
gcaguaugga ucggcagag 19

<210> 283
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 283
cgcaguaugg aucggcaga 19

<210> 284
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 284
caguauggau cggcagagg 19

<210> 285
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 285
accgcguaaa gagaggugc 19

<210> 286
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 286
gcgaggcgag ggaguucuu 19

<210> 287
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 287
cugcgaggcg agggaguuc 19

<210> 288
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 288
gcccuagaaa auugagaga 19

<210> 289
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 289
cguaaagaga ggugcgccc 19

<210> 290
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 290
guuccgcagu auggaucgg 19

<210> 291
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<400> 291
ucugcgaggc gagggaguu 19

<210> 292
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 292
guauggaucg gcagaggag 19

<210> 293
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 293
gagugcgaau ccacacucc 19

<210> 294
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 294
guaaagagag gugcgcccg 19

<210> 295
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 295
gcgacgcggc gauugagac 19

<210> 296
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 296
ggaguuccgc aguauggau 19

<210> 297
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 297
ccgcguaaag agaggugcg 19

<210> 298
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 298
gccucaaggu cggucguug 19

<210> 299
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 299
uaggaguucc gcaguaugg 19

<210> 300
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 300
gggucguccg cgggauuca 19

<210> 301
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 301
ugcgaggcga gggaguucu 19

<210> 302
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 302
gcguaaagag aggugcgcc 19

<210> 303
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 303
cgcguaaaga gaggugcgc 19

<210> 304
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 304
gucguccgcg ggauucagc 19

<210> 305
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 305
gcagagguga agcgaagug 19

<210> 306
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 306
cugcgacgcg gcgauugag 19

<210> 307
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 307
ggauucagcg ccgacggga 19

<210> 308
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 308
ggucguccgc gggauucag 19

<210> 309
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 309
caccacgagu cuagacucu 19

<210> 310
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 310
aggaguuccg caguaugga 19

<210> 311
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 311
cguccgcggg auucagcgc 19

<210> 312
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 312
aggagugcga auccacacu 19

<210> 313
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 313
gccuguaaca cgagcaggg 19

<210> 314
<211> 19
<212> RNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<400> 314
cgggucgucc gcgggauuc 19

<210> 315
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 315
gccacccaag gcacagcuu 19

<210> 316
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 316
ccuguaacac gagcagggc 19

<210> 317
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 317
gauucagcgc cgacgggac 19

<210> 318
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 318
ccggaagugu ugauaagau 19

<210> 319
<211> 19
<212> RNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<400> 319
uuccggaagu guugauaag 19

<210> 320
<211> 19
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2..19
<223> /mod_base = "2'-hydroxy corresponding nucleoside"

<400> 320
tuccggaagu guugauaag 19

<210> 321
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 6, 8, 10, 11, 13, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 7, 9, 12, 16
<223> /mod_base = "2'-hydroxy corresponding nucleoside"

<400> 321
caagguaugu ugcccguuut t 21

<210> 322
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 322
cuguaggcau aaauugguat 20

<210> 323
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 8, 10, 11, 12, 13, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 7, 9, 14, 17, 19
<223> /mod_base = "2'-hydroxy corresponding nucleoside"

<400> 323
ucugcggcgu uuuaucauat t 21

<210> 324
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 324
ucugcggcgu uuuaucauat 20

<210> 325
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 8, 9, 10, 13, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 11, 12, 15
<223> /mod_base = "2'-hydroxy corresponding nucleoside"

<400> 325
accucugccu aaucaucuct t 21

<210> 326
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 326

uuuacuagug ccauuuguat 20

<210> 327
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 327
accucugccu aaucaucuat 20

<210> 328
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 8, 10, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 3, 5, 6, 7, 9, 11, 12, 13, 16, 17
<223> /mod_base = "2'-hydroxy corresponding nucleoside"

<400> 328
cuguaggcau aaauugguct t 21

<210> 329
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 10, 12, 13, 14, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 8, 9, 11, 16, 19
<223> /mod_base = "2'-hydroxy corresponding nucleoside"

<400> 329
ugucugcggc guuuuaucat t 21

<210> 330
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19

<223> /mod_base = "2'-deoxy-2'-fluoro corresponding nucleoside"

<220>
<221> modified_base
<222> 20
<223> /mod_base = "3'-3'-linked desoxythymidine"

<400> 330
ugucugcggc guuuuaucat 20

<210> 331
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 7, 9, 10, 12, 13, 14, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 8, 11, 15, 19
<223> /mod_base = "2'-hydroxy corresponding nucleoside"

<400> 331
uacuagugcc auuuguucat t 21

<210> 332
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 332
uacuagugcc auuuguucat 20

<210> 333
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 333
caacuuuuuc accucugcat 20

<210> 334
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 4, 5, 6, 8, 9, 10, 13, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 11, 12, 14, 15, 19
<223> /mod_base = "2'-hydroxy corresponding nucleoside"

<400> 334
ccauuuguuc agugguucgt t 21

<210> 335
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 8, 9, 10, 12, 13, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 7, 11, 14, 15, 17, 19
<223> /mod_base = "2'-hydroxy corresponding nucleoside"

<400> 335
ccaaguguuu gcugacgcat t 21

<210> 336
<211> 20
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 336
ccaaguguuu gcugacgcat 20

<210> 337
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 337
ccauuuguuc agugguucat 20

<210> 338
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 9, 11, 12, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 10, 13, 17
<223> /mod_base = "2'-hydroxy corresponding nucleoside"

<400> 338
uuuacuagug ccauuuguut t 21

<210> 339
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 339
caccucugcc uaaucaucat 20

<210> 340
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 10, 11, 12, 14, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 8, 9, 13, 16, 17, 19
<223> /mod_base = "2'-hydroxy corresponding nucleoside"

<400> 340
cuggcucagu uuacuagugt t 21

<210> 341
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 341

caagguaugu ugcccguuat 20

<210> 342
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 342
cuggcucagu uuacuaguat 20

<210> 343
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 12, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 3, 4, 7, 9, 10, 11, 13, 15, 16, 17
<223> /mod_base = "2'-hydroxy corresponding nucleoside"

<400> 343
gaggcuguag gcauaaauut t 21

<210> 344
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 8, 9, 12, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 10, 11, 13, 16
<223> /mod_base = "2'-hydroxy corresponding nucleoside"

<400> 344
caguuuacua gugccauuut t 21

<210> 345
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 8, 9, 11, 12, 13, 15, 16, 17, 19
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 3, 5, 7, 10, 14, 18
<223> /mod_base = "2'-hydroxy corresponding nucleoside"

<400> 345
agguauguug cccguuugut t 21

<210> 346
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 346
uauguugccc guuuguccat 20

<210> 347
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 347
gaggcuguag gcauaaauat 20

<210> 348
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 9, 11, 12, 13, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 7, 8, 10, 15, 18
<223> /mod_base = "2'-hydroxy corresponding nucleoside"

<400> 348
gucugcggcg uuuuaucaut t 21

<210> 349
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 4, 5, 6, 7, 8, 9, 10, 12, 13, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 11, 17
<223> /mod_base = "2'-hydroxy corresponding nucleoside"

<400> 349
caacuuuuuc accucugcct t 21

<210> 350
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 8, 10, 11, 12, 13, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 9, 14, 19
<223> /mod_base = "2'-hydroxy corresponding nucleoside"

<400> 350
ccgugugcac uucgcuucat t 21

<210> 351
<211> 20
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 351
ccgugugcac uucgcuucat 20

<210> 352
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 352
ucaagguaug uugcccguat 20

<210> 353
<211> 20
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 353
caguuuacua gugccauuat 20

<210> 354
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 8, 9, 10, 11, 12, 13, 14, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 7, 16, 17
<223> /mod_base = "2'-hydroxy corresponding nucleoside"

<400> 354
ugguggacuu cucucaauut t 21

<210> 355
<211> 20

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 355
agguauguug cccguuugat 20

<210> 356
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 8, 10, 11, 13, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 9, 12, 14, 15, 16, 18, 19
<223> /mod_base = "2'-hydroxy corresponding nucleoside"

<400> 356

cugcucgugu uacaggcggt t 21

<210> 357
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 8, 9, 10, 12, 13, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 11, 15
<223> /mod_base = "2'-hydroxy corresponding nucleoside"

<400> 357
uauguugccc guuuguccut t 21

<210> 358
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 7, 9, 11, 12, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 3, 4, 5, 6, 8, 10, 13, 17
<223> /mod_base = "2'-hydroxy corresponding nucleoside"

<400> 358
ucaagguaug uugcccguut t 21

<210> 359
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 10, 12, 13, 14, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 8, 9, 11, 17, 18, 19
<223> /mod_base = "2'-hydroxy corresponding nucleoside"

<400> 359
ucuuaucaac acuuccggat t 21

<210> 360
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19

<223> /mod_base = "2'-deoxy-2'-fluoro corresponding nucleoside"

<220>
<221> modified_base
<222> 20
<223> /mod_base = "3'-3'-linked desoxythymidine"

<400> 360
ucuuaucaac acuuccggat 20

<210> 361
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 7, 9, 10, 11, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 8, 12, 13, 16
<223> /mod_base = "2'-hydroxy corresponding nucleoside"

<400> 361
caccucugcc uaaucaucut t 21

<210> 362
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 10, 11, 12, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 7, 8, 9, 15, 16, 17
<223> /mod_base = "2'-hydroxy corresponding nucleoside"

<400> 362
auaagaggac ucuuggacut t 21

<210> 363
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 363
gucugcggcg uuuuaucaat 20

<210> 364
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 5, 6, 10, 11, 12, 13, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 9, 14, 16, 17, 18
<223> /mod_base = "2'-hydroxy corresponding nucleoside"

<400> 364
ggcgcugaau cccgcggact t 21

<210> 365
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 8, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 9, 10, 11, 12, 13, 14, 19
<223> /mod_base = "2'-hydroxy corresponding nucleoside"

<400> 365
cgcgucgcag aagaucucat t 21

<210> 366
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 3, 5, 6, 9, 12, 13, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 10, 11, 14, 15
<223> /mod_base = "2'-hydroxy corresponding nucleoside"

<400> 366
aaugucaacg accgaccuut t 21

<210> 367
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 7, 8, 9, 11, 12, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 10, 13, 14, 16, 19
<223> /mod_base = "2'-hydroxy corresponding nucleoside"

<400> 367
gcucaguuua cuagugccat t 21

<210> 368
<211> 20
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 368
ugguggacuu cucucaauat 20

<210> 369
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 8, 10, 11, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 9, 12, 14, 15, 16, 17, 18, 19
<223> /mod_base = "2'-hydroxy corresponding nucleoside"

<400> 369
aucgccgcgu cgcagaagat t 21

<210> 370
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 9, 10, 11, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 8, 12, 13, 15, 16
<223> /mod_base = "2'-hydroxy corresponding nucleoside"

<400> 370
gccauuuguu cagugguuct t 21

<210> 371
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 8, 10, 11, 13, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 9, 12, 14, 15, 16, 17
<223> /mod_base = "2'-hydroxy corresponding nucleoside"

<400> 371

cgauccauac ugcggaacut t 21

<210> 372
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 5, 6, 7, 8, 10, 11, 12, 15, 16, 18, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 9, 13, 14, 17
<223> /mod_base = "2’-hydroxy corresponding nucleoside"

<400> 372
ucaccucugc cuaaucauct t 21

<210> 373
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 6, 7, 8, 9, 10, 11, 12, 13, 16, 17, 18, 19
<223> /mod_base = "2’-O-methyl corresponding nucleoside"

<220>
<221> modified_base
<222> 21
<223> /mod_base = "5’-phosphorothioate thymidine"

<220>
<221> modified_base

<222> 3, 4, 5, 14, 15
<223> /mod_base = "2'-hydroxy corresponding nucleoside"

<400> 373
guggacuucu cucaauuuut t 21

<210> 374
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 8, 10, 12, 13, 14, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 9, 11, 17, 18, 19
<223> /mod_base = "2'-hydroxy corresponding nucleoside"

<400> 374
gggucaccau auucuugggt t 21

<210> 375
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 8, 10, 17, 18, 19
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 4, 6, 9, 11, 12, 13, 14, 15, 16
<223> /mod_base = "2'-hydroxy corresponding nucleoside"

<400> 375
gccgcgucgc agaagaucut t 21

<210> 376
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 9, 11, 13, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 7, 10, 12, 15, 17, 18, 19
<223> /mod_base = "2'-hydroxy corresponding nucleoside"

<400> 376
ucaaucgccg cgucgcagat t 21

<210> 377
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 9, 10, 11, 13, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 8, 12, 14, 15, 17
<223> /mod_base = "2'-hydroxy corresponding nucleoside"

<400> 377
uggauguguc ugcggcguut t 21

<210> 378
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 12, 13, 14, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 10, 11, 17, 18, 19
<223> /mod_base = "2'-hydroxy corresponding nucleoside"

<400> 378
uacuguucaa gccuccaagt t 21

<210> 379
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 4, 6, 7, 10, 12, 13, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 8, 9, 11, 14, 18
<223> /mod_base = "2'-hydroxy corresponding nucleoside"

<400> 379
guuuacuagu gccauuugut t 21

<210> 380
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 8, 9, 11, 12, 13, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 10, 14, 18, 19
<223> /mod_base = "2'-hydroxy corresponding nucleoside"

<400> 380
acuagugcca uuuguucagt t 21

<210> 381
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 4, 6, 7, 9, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 8, 10, 11, 12, 13, 14, 15
<223> /mod_base = "2'-hydroxy corresponding nucleoside"

<400> 381
ccgcgucgca gaagaucuct t 21

<210> 382
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 8, 9, 12, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 10, 11, 13, 19
<223> /mod_base = "2'-hydroxy corresponding nucleoside"

<400> 382
uaucuuauca acacuuccgt t 21

<210> 383
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 9, 10, 11, 13, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 7, 8, 12, 14, 15
<223> /mod_base = "2'-hydroxy corresponding nucleoside"

<400> 383
ggccaaaauu cgcaguccct t 21

<210> 384
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 8, 9, 11, 12, 13, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 10, 14, 15, 18
<223> /mod_base = "2'-hydroxy corresponding nucleoside"

<400> 384
uucaccucug ccuaaucaut t 21

<210> 385
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 7, 8, 10, 11, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 9, 12, 13, 15, 18
<223> /mod_base = "2'-hydroxy corresponding nucleoside"

<400> 385
cucaguuuac uagugccaut t 21

<210> 386
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 10, 11, 12, 14, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 13
<223> /mod_base = "2'-hydroxy corresponding nucleoside"

<400> 386

uguugcccgu uuguccucut t 21

<210> 387
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 9, 10, 11, 13, 14, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 8, 12, 16, 17, 19
<223> /mod_base = "2'-hydroxy corresponding nucleoside"

<400> 387
uagugccauu uguucagugt t 21

<210> 388
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 11, 13, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 3, 6, 8, 9, 10, 12, 14, 15, 16, 19
<223> /mod_base = "2'-hydroxy corresponding nucleoside"

<400> 388
aggcuguagg cauaaauugt t 21

<210> 389
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 8, 10, 13, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 9, 11, 12, 14, 19
<223> /mod_base = "2'-hydroxy corresponding nucleoside"

<400> 389
augugucugc ggcguuuuat t 21

<210> 390
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19

<223> /mod_base = "2'-deoxy-2'-fluoro corresponding nucleoside"

<220>
<221> modified_base
<222> 20
<223> /mod_base = "3'-3'-linked desoxythymidine"

<400> 390
augugucugc ggcguuuuat 20

<210> 391
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 9, 10, 12, 13, 14, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 11, 17, 19
<223> /mod_base = "2'-hydroxy corresponding nucleoside"

<400> 391
acuucgcuuc accucugcat t 21

<210> 392
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 9, 10, 11, 12, 14, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 8, 13, 18
<223> /mod_base = "2'-hydroxy corresponding nucleoside"

<400> 392
cgugugcacu ucgcuucact t 21

<210> 393
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 9, 10, 11, 12, 13, 14, 15, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 7, 8, 17, 18
<223> /mod_base = "2'-hydroxy corresponding nucleoside"

<400> 393
gugguggacu ucucucaaut t 21

<210> 394
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 5, 6, 7, 9, 12, 14, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 8, 10, 11, 13, 18
<223> /mod_base = "2'-hydroxy corresponding nucleoside"

<400> 394
ugugucugcg gcguuuuaut t 21

<210> 395
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 9, 10, 12, 13, 14, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 8, 11, 15, 19
<223> /mod_base = "2'-hydroxy corresponding nucleoside"

<400> 395
aagguauguu gcccguuugt t 21

<210> 396
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 5, 8, 9, 12, 13, 14, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 7, 10, 11, 16, 17, 18, 19
<223> /mod_base = "2'-hydroxy corresponding nucleoside"

<400> 396
ucaacgaccg accuugaggt t 21

<210> 397
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 11, 12, 13, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 7, 8, 9, 10, 16, 17, 18
<223> /mod_base = "2'-hydroxy corresponding nucleoside"

<400> 397
cauaagagga cucuuggact t 21

<210> 398
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 9, 10, 13, 14, 15, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 7, 8, 11, 12, 17, 18, 19
<223> /mod_base = "2'-hydroxy corresponding nucleoside"

<400> 398
gucaacgacc gaccuugagt t 21

<210> 399
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 8, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 9, 10, 11, 12, 13, 15, 16, 17, 18, 19
<223> /mod_base = "2'-hydroxy corresponding nucleoside"

<400> 399
auauucuugg gaacaagagt t 21

<210> 400
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 9, 10, 12, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 8, 11, 13, 14, 15, 17, 18, 19
<223> /mod_base = "2'-hydroxy corresponding nucleoside"

<400> 400
ugcucguguu acaggcgggt t 21

<210> 401
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 8, 10, 12, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 9, 11, 14, 16, 17, 18, 19
<223> /mod_base = "2'-hydroxy corresponding nucleoside"

<400> 401

caaucgccgc gucgcagaat t 21

<210> 402
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 11, 12, 13, 14, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 8, 9, 10, 16, 17, 18
<223> /mod_base = "2'-hydroxy corresponding nucleoside"

<400> 402
acuguucaag ccuccaagct t 21

<210> 403
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 8, 9, 11, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 5, 7, 10, 12, 13, 14, 15, 16, 17
<223> /mod_base = "2'-hydroxy corresponding nucleoside"

<400> 403
cgccgcgucg cagaagauct t 21

<210> 404
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 9, 12, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 10, 11, 13, 14, 18
<223> /mod_base = "2'-hydroxy corresponding nucleoside"

<400> 404
cauuuguuca gugguucgut t 21

<210> 405
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 8, 9, 10, 11, 13, 17
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 5, 6, 7, 12, 14, 15, 16, 18, 19
<223> /mod_base = "2'-hydroxy corresponding nucleoside"

<400> 405
cgcugaaucc cgcggacgat t 21

<210> 406
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 8, 9, 11, 13, 14, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 7, 10, 12, 18, 19
<223> /mod_base = "2'-hydroxy corresponding nucleoside"

<400> 406
ugggucacca uauucuuggt t 21

<210> 407
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 8, 9, 12, 13, 14, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 10, 11, 15, 17
<223> /mod_base = "2'-hydroxy corresponding nucleoside"

<400> 407
uccucugccg auccauacut t 21

<210> 408
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 8, 11, 12, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 7, 9, 10, 13, 14, 19
<223> /mod_base = "2'-hydroxy corresponding nucleoside"

<400> 408
augucaacga ccgaccuugt t 21

<210> 409
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 4, 5, 7, 8, 9, 12, 13, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 10, 11, 14, 19
<223> /mod_base = "2'-hydroxy corresponding nucleoside"

<400> 409
ccucugccua aucaucucat t 21

<210> 410
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 9, 11, 12, 13, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 8, 10, 15
<223> /mod_base = "2'-hydroxy corresponding nucleoside"

<400> 410
accgugugca cuucgcuuct t 21

<210> 411
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 3, 4, 7, 8, 9, 11, 13, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 10, 12, 15, 17, 18, 19
<223> /mod_base = "2'-hydroxy corresponding nucleoside"

<400> 411
ugccgaucca uacugcggat t 21

<210> 412
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 8, 12, 13, 14, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 9, 10, 11, 15, 17, 18
<223> /mod_base = "2'-hydroxy corresponding nucleoside"

<400> 412
cagagucuag acucguggut t 21

<210> 413
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 10, 11, 12, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 7, 8, 9, 15, 16, 17
<223> /mod_base = "2'-hydroxy corresponding nucleoside"

<400> 413
cuguucaagc cuccaagcut t 21

<210> 414
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 9, 13, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 8, 10, 11, 12, 14, 16, 17, 18
<223> /mod_base = "2'-hydroxy corresponding nucleoside"

<400> 414
ggaggcugua ggcauaaaut t 21

<210> 415
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 7, 8, 10, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 6, 9, 11, 12, 13, 15, 17, 18, 19
<223> /mod_base = "2'-hydroxy corresponding nucleoside"

<400> 415
aggaggcugu aggcauaaat t 21

<210> 416
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 8, 9, 10, 11, 12, 13, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 15, 16
<223> /mod_base = "2'-hydroxy corresponding nucleoside"

<400> 416

gguggacuuc ucucaauuut t 21

<210> 417
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 5, 6, 7, 8, 9, 10, 11, 13, 14, 15, 16, 17, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 4, 12, 18
<223> /mod_base = "2’-hydroxy corresponding nucleoside"

<400> 417
gcaacuuuuu caccucugct t 21

<210> 418
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 2, 4, 6, 8, 10, 12, 14, 16, 18
<223> /mod_base = "2’-O-methyl corresponding nucleoside"

<220>
<221> modified_base
<222> 1, 3, 5, 7, 9, 11, 13, 15, 17, 19
<223> /mod_base = "2’-deoxy-2’-fluoro corresponding nucleoside"

<220>
<221> modified_base

<222> 20
<223> /mod_base = "3’-3’-linked desoxythymidine"

<400> 418
cugcucgugu uacaggcgat 20

<210> 419
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 5, 7, 8, 10, 11, 12, 14, 15, 16, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 4, 6, 9, 13, 17, 18
<223> /mod_base = "2’-hydroxy corresponding nucleoside"

<400> 419
cuagugccau uuguucagut t 21

<210> 420
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 5, 8, 9, 10, 12, 14, 15, 17
<223> /mod_base = "2’-O-methyl corresponding nucleoside"

<220>
<221> modified_base
<222> 21

<223> /mod_base = "5'-phosphorothioate thymidine"

<220>
<221> modified_base
<222> 3, 6, 7, 11, 13, 16, 18, 19
<223> /mod_base = "2'-hydroxy corresponding nucleoside"

<400> 420
cugccgaucc auacugcggt t 21

<210> 421
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 8, 9, 10, 11, 13, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 12, 17
<223> /mod_base = "2'-hydroxy corresponding nucleoside"

<400> 421
gugugcacuu cgcuucacct t 21

<210> 422
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 8, 9, 11, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 7, 10, 12, 13, 14, 16, 17, 18
<223> /mod_base = "2'-hydroxy corresponding nucleoside"

<400> 422
gcucguguua caggcgggct t 21

<210> 423
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 8, 10, 11, 14, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 9, 12, 13, 15
<223> /mod_base = "2'-hydroxy corresponding nucleoside"

<400> 423
ccuaucuuau caacacuuct t 21

<210> 424
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 4, 7, 8, 10, 11, 13, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 9, 12, 14, 17, 19
<223> /mod_base = "2'-hydroxy corresponding nucleoside"

<400> 424
ucucaaucgc cgcgucgcat t 21

<210> 425
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 10, 12, 13, 14, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 11, 19
<223> /mod_base = "2'-hydroxy corresponding nucleoside"

<400> 425
gcccgucugu gccuucucat t 21

<210> 426
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 4, 5, 6, 7, 9, 10, 13, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 8, 11, 12, 14
<223> /mod_base = "2'-hydroxy corresponding nucleoside"

<400> 426
cuaucuuauc aacacuucct t 21

<210> 427
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 8, 9, 11, 12, 13, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 10, 14
<223> /mod_base = "2'-hydroxy corresponding nucleoside"

<400> 427
auguugcccg uuuguccuct t 21

<210> 428
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 6, 7, 9, 10, 11, 13, 14, 15, 17, 18, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 5, 8, 12, 16
<223> /mod_base = "2’-hydroxy corresponding nucleoside"

<400> 428
guauguugcc cguuugucct t 21

<210> 429
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 3, 4, 6, 7, 8, 9, 11, 12, 13, 14, 15, 17, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 5, 10, 16, 18
<223> /mod_base = "2’-hydroxy corresponding nucleoside"

<400> 429
cuucgcuuca ccucugcact t 21

<210> 430
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 8, 9, 10, 12, 13, 14, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 11, 16
<223> /mod_base = "2'-hydroxy corresponding nucleoside"

<400> 430
ugugcacuuc gcuucaccut t 21

<210> 431
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 8, 9, 10, 12, 15, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 7, 11, 13, 14, 19
<223> /mod_base = "2'-hydroxy corresponding nucleoside"

<400> 431

gccaaaauuc gcaguccgt t 21

<210> 432
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 3, 5, 6, 7, 9, 11, 12, 14, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 4, 8, 10, 13, 15, 16, 17, 19
<223> /mod_base = "2’-hydroxy corresponding nucleoside"

<400> 432
ccugcucgug uuacaggcgt t 21

<210> 433
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 6, 8, 12, 13, 14, 16, 18, 19
<223> /mod_base = "2’-O-methyl corresponding nucleoside"

<220>
<221> modified_base
<222> 21
<223> /mod_base = "5’-phosphorothioate thymidine"

<220>
<221> modified_base

<222> 3, 4, 5, 7, 9, 10, 11, 15, 17
<223> /mod_base = "2'-hydroxy corresponding nucleoside"

<400> 433
uggagugugg auucgcacut t 21

<210> 434
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 7, 10, 11, 12, 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 8, 9, 14, 15, 16, 17, 19
<223> /mod_base = "2'-hydroxy corresponding nucleoside"

<400> 434
aacgaccgac cuugaggcat t 21

<210> 435
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 7, 8, 9, 13, 14, 15, 17
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 3, 4, 5, 6, 10, 11, 12, 16, 18, 19
<223> /mod_base = "2'-hydroxy corresponding nucleoside"

<400> 435
acagagucua gacucguggt t 21

<210> 436
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 9, 11, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 8, 10, 13, 15, 16, 17, 18, 19
<223> /mod_base = "2'-hydroxy corresponding nucleoside"

<400> 436
aaucgccgcg ucgcagaagt t 21

<210> 437
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 8, 10, 11, 12, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 9, 13, 17
<223> /mod_base = "2'-hydroxy corresponding nucleoside"

<400> 437
gguauguugc ccguuuguct t 21

<210> 438
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 6, 7, 8, 10, 12, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 9, 11, 14, 16, 17, 18, 19
<223> /mod_base = "2'-hydroxy corresponding nucleoside"

<400> 438
gccgauccau acugcggaat t 21

<210> 439
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 4, 5, 7, 8, 9, 10, 12, 13, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 11, 14, 15, 17
<223> /mod_base = "2'-hydroxy corresponding nucleoside"

<400> 439
gcccuaucuu aucaacacut t 21

<210> 440
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 11, 13, 14, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 9, 10, 12, 15, 19
<223> /mod_base = "2'-hydroxy corresponding nucleoside"

<400> 440
aguuuacuag ugccauuugt t 21

<210> 441
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 3, 4, 7, 10, 11, 14, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 6, 8, 9, 12, 13, 18, 19
<223> /mod_base = "2'-hydroxy corresponding nucleoside"

<400> 441
ugucaacgac cgaccuugat t 21

<210> 442
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 7, 8, 9, 12, 13, 14, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 10, 11, 18, 19
<223> /mod_base = "2'-hydroxy corresponding nucleoside"

<400> 442
acuucucuca auuuucuagt t 21

<210> 443
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 9, 11, 12, 13, 14, 15, 16, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 6, 7, 8, 10, 17
<223> /mod_base = "2'-hydroxy corresponding nucleoside"

<400> 443
gcgcgggacg uccuuuguct t 21

<210> 444
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 8, 9, 11, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 10, 12, 13, 15, 16, 17
<223> /mod_base = "2'-hydroxy corresponding nucleoside"

<400> 444
ucuagacucg ugguggacut t 21

<210> 445
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 9, 10, 12, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 8, 11, 13, 14, 15, 16
<223> /mod_base = "2'-hydroxy corresponding nucleoside"

<400> 445
gauccauacu gcggaacuct t 21

<210> 446
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 10, 11, 12, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 8, 9, 13, 15, 18
<223> /mod_base = "2'-hydroxy corresponding nucleoside"

<400> 446

cucugccgau ccauacugct t 21

<210> 447
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 9, 10, 11, 13, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 12, 14, 17, 19
<223> /mod_base = "2'-hydroxy corresponding nucleoside"

<400> 447
ucugccgauc cauacugcgt t 21

<210> 448
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 11, 12, 13, 15, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 6, 9, 10, 14, 16, 19
<223> /mod_base = "2'-hydroxy corresponding nucleoside"

<400> 448
ccucugccga uccauacugt t 21

<210> 449
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 8, 9, 10, 11, 12, 14, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 13, 15, 17, 18
<223> /mod_base = "2'-hydroxy corresponding nucleoside"

<400> 449
gcaccucucu uuacgcggut t 21

<210> 450
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 8, 9, 10, 11, 12, 14, 15, 16, 17, 19
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 3, 4, 5, 13, 18
<223> /mod_base = "2'-hydroxy corresponding nucleoside"

<400> 450
aagaacuccc ucgccucgct t 21

<210> 451
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 8, 9, 10, 12, 13, 14, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 11, 16, 18, 19
<223> /mod_base = "2'-hydroxy corresponding nucleoside"

<400> 451
gaacucccuc gccucgcagt t 21

<210> 452
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 9, 10, 11, 12, 13, 14, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 7, 8, 15, 16, 17, 18
<223> /mod_base = "2'-hydroxy corresponding nucleoside"

<400> 452
ucucucaauu uucuagggct t 21

<210> 453
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 8, 9, 10, 11, 12, 13, 14, 15, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 7, 17, 19
<223> /mod_base = "2'-hydroxy corresponding nucleoside"

<400> 453
gggcgcaccu cucuuuacgt t 21

<210> 454
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 5, 6, 7, 9, 11, 12, 14, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 8, 10, 13, 15, 16, 17, 18
<223> /mod_base = "2'-hydroxy corresponding nucleoside"

<400> 454
ccgauccaua cugcggaact t 21

<210> 455
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 6, 7, 8, 9, 11, 12, 13, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 10, 15, 17, 18, 19
<223> /mod_base = "2'-hydroxy corresponding nucleoside"

<400> 455
aacucccucg ccucgcagat t 21

<210> 456
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 3, 4, 5, 6, 7, 9, 10, 13, 14, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 8, 11, 12, 16, 18
<223> /mod_base = "2'-hydroxy corresponding nucleoside"

<400> 456
cuccucugcc gauccauact t 21

<210> 457
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 11, 12, 13, 15, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 8, 9, 10, 14, 16
<223> /mod_base = "2'-hydroxy corresponding nucleoside"

<400> 457
ggagugugga uucgcacuct t 21

<210> 458
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 7, 9, 10, 11, 12, 13, 14, 15, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 6, 8, 18
<223> /mod_base = "2'-hydroxy corresponding nucleoside"

<400> 458
cgggcgcacc ucucuuuact t 21

<210> 459
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 8, 9, 11, 12, 14, 16, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 7, 10, 13, 15, 18
<223> /mod_base = "2'-hydroxy corresponding nucleoside"

<400> 459
gucucaaucg ccgcgucgct t 21

<210> 460
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 8, 9, 11, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 7, 10, 12, 13, 14, 15
<223> /mod_base = "2'-hydroxy corresponding nucleoside"

<400> 460
auccauacug cggaacucct t 21

<210> 461
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 7, 8, 9, 10, 11, 12, 13, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 14, 16, 18, 19
<223> /mod_base = "2'-hydroxy corresponding nucleoside"

<400> 461

cgcaccucuc uuuacgcggt t 21

<210> 462
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 7, 8, 11, 12, 13, 14, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 5, 6, 9, 10, 15, 16, 17, 18
<223> /mod_base = "2’-hydroxy corresponding nucleoside"

<400> 462
caacgaccga ccuugaggct t 21

<210> 463
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 6, 7, 9, 14, 15, 16, 17, 18
<223> /mod_base = "2’-O-methyl corresponding nucleoside"

<220>
<221> modified_base
<222> 21
<223> /mod_base = "5’-phosphorothioate thymidine"

<220>
<221> modified_base

<222> 3, 5, 8, 10, 11, 12, 13, 19
<223> /mod_base = "2'-hydroxy corresponding nucleoside"

<400> 463
ccauacugcg gaacuccuat t 21

<210> 464
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 8, 10, 14, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 9, 11, 12, 13, 15, 16
<223> /mod_base = "2'-hydroxy corresponding nucleoside"

<400> 464
ugaaucccgc ggacgaccct t 21

<210> 465
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 8, 9, 10, 11, 13, 14, 15, 16, 18
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 3, 4, 12, 17, 19
<223> /mod_base = "2'-hydroxy corresponding nucleoside"

<400> 465
agaacucccu cgccucgcat t 21

<210> 466
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 8, 9, 10, 11, 12, 13, 14, 15, 17, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 16, 18
<223> /mod_base = "2'-hydroxy corresponding nucleoside"

<400> 466
ggcgcaccuc ucuuuacgct t 21

<210> 467
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 6, 7, 8, 9, 10, 11, 12, 13, 14, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 5, 15, 17, 19
<223> /mod_base = "2'-hydroxy corresponding nucleoside"

<400> 467
gcgcaccucu cuuuacgcgt t 21

<210> 468
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 7, 8, 9, 10, 12, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 5, 6, 11, 13, 14, 15, 17, 18
<223> /mod_base = "2'-hydroxy corresponding nucleoside"

<400> 468
gcugaauccc gcggacgact t 21

<210> 469
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 1, 2, 3, 4, 5, 6, 8, 9, 10, 11, 13, 14, 15, 16, 17, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 7, 12, 18
<223> /mod_base = "2’-hydroxy corresponding nucleoside"

<400> 469
cacuucgcuu caccucugct t 21

<210> 470
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 3, 6, 7, 9, 10, 12, 14, 15, 17
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 4, 5, 8, 11, 13, 16, 18, 19
<223> /mod_base = "2’-hydroxy corresponding nucleoside"

<400> 470
cucaaucgcc gcgucgcagt t 21

<210> 471
<211> 21
<212> DNA
<213> Artificial sequence

<220>
<223> Description of the artificial sequence: sense strand of dsRNA

<220>
<221> modified_base

<222> 1
<223> /mod_base = "nucleoside: lacks 5'-phosphate group"

<220>
<221> modified_base
<222> 1, 2, 3, 4, 6, 7, 10, 12, 13, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 8, 9, 11, 14, 15, 16
<223> /mod_base = "2'-hydroxy corresponding nucleoside"

<400> 471
ucccgucggc gcugaaucct t 21

<210> 472
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 6, 7, 8, 9, 11, 15, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 5, 10, 12, 13, 14, 16, 17
<223> /mod_base = "2'-hydroxy corresponding nucleoside"

<400> 472
cugaaucccg cggacgacct t 21

<210> 473
<211> 21
<212> DNA
<213> Artificial sequence

<220>

<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 5, 6, 7, 11, 12, 13, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 4, 8, 9, 10, 14, 16, 17, 19
<223> /mod_base = "2'-hydroxy corresponding nucleoside"

<400> 473
agagucuaga cucgguggugt t 21

<210> 474
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 7, 8, 10, 15, 16, 17, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 6, 9, 11, 12, 13, 14
<223> /mod_base = "2'-hydroxy corresponding nucleoside"

<400> 474
uccauacugc ggaacuccut t 21

<210> 475
<211> 21

<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 4, 5, 9, 10, 11, 12, 14, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 3, 6, 7, 8, 13, 15, 16, 17, 19
<223> /mod_base = "2’-hydroxy corresponding nucleoside"

<400> 475
gcgcugaauc ccgcggacgt t 21

<210> 476
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5’-phosphate group"

<220>
<221> modified_base
<222> 1, 2, 3, 5, 9, 10, 11, 13, 15, 16, 17, 18, 19
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 4, 6, 7, 8, 12, 14
<223> /mod_base = "2’-hydroxy corresponding nucleoside"

<400> 476

aguguggauu cgcacuccut t 21

<210> 477
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 6, 7, 8, 10, 12, 13, 15, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 5, 9, 11, 14, 16, 17, 18
<223> /mod_base = "2'-hydroxy corresponding nucleoside"

<400> 477
cccugcucgu guuacaggct t 21

<210> 478
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 6, 7, 9, 13, 16, 17, 18
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 3, 8, 10, 11, 12, 14, 15, 19
<223> /mod_base = "2'-hydroxy corresponding nucleoside"

<400> 478
gaaucccgcg gacgacccgt t 21

<210> 479
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 4, 5, 7, 9, 10, 11, 12, 16, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 3, 6, 8, 13, 14, 15, 17, 18
<223> /mod_base = "2'-hydroxy corresponding nucleoside"

<400> 479
aagcugugcc uugggugggct t 21

<210> 480
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 9, 11, 13, 14, 16
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 6, 10, 12, 15, 17, 18, 19
<223> /mod_base = "2'-hydroxy corresponding nucleoside"

<400> 480
gcccugcucg uguuacaggt t 21

<210> 481
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 11, 13, 14, 18, 19
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 9, 10, 12, 15, 16, 17
<223> /mod_base = "2'-hydroxy corresponding nucleoside"

<400> 481
gucccgucgg cgcugaauct t 21

<210> 482
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 4, 5, 7, 8, 11, 13, 14, 15, 16, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 6, 9, 10, 12, 18, 19
<223> /mod_base = "2'-hydroxy corresponding nucleoside"

<400> 482
aucuuaucaa cacuuccggt t 21

<210> 483
<211> 21
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>
<221> modified_base
<222> 1, 2, 3, 5, 6, 9, 11, 12, 13, 14, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 4, 7, 8, 10, 16, 17, 18, 19
<223> /mod_base = "2'-hydroxy corresponding nucleoside"

<400> 483
cuuaucaaca cuuccggaat t 21

<210> 484
<211> 20
<212> DNA
<213> Artificial sequence

<220>
<223> Description of the artificial sequence: sense strand of dsRNA

<220>
<221> modified_base
<222> 1
<223> /mod_base = "nucleoside: lacks 5'-phosphate group"

<220>

<221> modified_base
<222> 2, 4, 6, 8, 10, 12, 14, 16, 18
<223> /mod_base = "2'-O-methyl corresponding nucleoside"

<220>
<221> modified_base
<222> 1, 3, 5, 7, 9, 11, 13, 15, 17, 19
<223> /mod_base = "2'-deoxy-2'-fluoro corresponding nucleoside"

<220>
<221> modified_base
<222> 20
<223> /mod_base = "3'-3'-linked desoxythymidine"

<400> 484
cuuaucaaca cuuccggaat 20

<210> 485
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 8, 11, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 14, 15, 16, 17, 18, 19
<223> /mod_base = "2'-hydroxy corresponding nucleoside"

<400> 485
aaacgggcaa cauaccuugt t 21

<210> 486
<211> 21
<212> DNA
<213> Artificial sequence

<220>
<223> Description of the artificial sequence: antisense strand of dsRNA

<220>
<221> modified_base

<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 486
taccaauuua ugccuacagt t 21

<210> 487
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 6, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 7, 8, 9, 10, 11, 12, 13, 14, 15, 17, 18, 19
<223> /mod_base = "2'-hydroxy corresponding nucleoside"

<400> 487
uaugauaaaa cgccgcagat t 21

<210> 488
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19

<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 488
taugauaaaa cgccgcagat t 21

<210> 489
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 14, 15, 16, 17, 18, 19
<223> /mod_base = "2'-hydroxy corresponding nucleoside"

<400> 489
gagaugauua ggcagaggut t 21

<210> 490
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 490
tacaaauggc acuaguaaat t 21

<210> 491
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 491
tagaugauua ggcagaggut t 21

<210> 492
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 9, 15, 17
<223> /mod_base = "2'-O-methyl corresponding nucleoside"

<220>

<221> modified_base
<222> 21
<223> /mod_base = "5'-phosphorothioate thymidine"

<220>
<221> modified_base
<222> 1, 2, 3, 5, 6, 7, 8, 10, 11, 12, 13, 14, 16, 18, 19
<223> /mod_base = "2'-hydroxy corresponding nucleoside"

<400> 492
gaccaauuua ugccuacagt t 21

<210> 493
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 14, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 13, 15, 16, 17, 19
<223> /mod_base = "2'-hydroxy corresponding nucleoside"

<400> 493
ugauaaaacg ccgcagacat t 21

<210> 494
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base

<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 494
tgauaaaacg ccgcagacat t 21

<210> 495
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 12, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 13, 14, 16, 17, 19
<223> /mod_base = "2'-hydroxy corresponding nucleoside"

<400> 495
ugaacaaaug gcacuaguat t 21

<210> 496
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18

<223> /mod_base = "2'-deoxy-2'-fluoro corresponding nucleoside"

<220>
<221> modified_base
<222> 21
<223> /mod_base = "5'-phosphorothioate thymidine"

<400> 496
tgaacaaaug gcacuaguat t 21

<210> 497
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 497
tgcagaggug aaaaaguugt t 21

<210> 498
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 14, 15, 16, 17, 18, 19
<223> /mod_base = "2'-hydroxy corresponding nucleoside"

<400> 498
cgaaccacug aacaaauggt t 21

<210> 499
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 9, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 10, 11, 12, 14, 15, 16, 17, 18, 19
<223> /mod_base = "2'-hydroxy corresponding nucleoside"

<400> 499
ugcgucagca aacacuuggt t 21

<210> 500
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>

<221> modified_base
<222> 21
<223> /mod_base = "5'-phosphorothioate thymidine"

<400> 500
tgcgucagca aacacuuggt t 21

<210> 501
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 501
tgaaccacug aacaaauggt t 21

<210> 502
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 10, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 1, 2, 4, 5, 6, 7, 8, 9, 11, 12, 14, 15, 17, 18, 19
<223> /mod_base = "2'-hydroxy corresponding nucleoside"

<400> 502
aacaaauggc acuaguaaat t 21

<210> 503
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 503
tgaugauuag gcagaggugt t 21

<210> 504
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 4, 7, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 5, 6, 8, 9, 10, 11, 12, 13, 14, 15, 16, 18, 19

<223> /mod_base = "2'-hydroxy corresponding nucleoside"

<400> 504
cacuaguaaa cugagccagt t 21

<210> 505
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 505
taacgggcaa cauaccuugt t 21

<210> 506
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 506
tacuaguaaa cugagccagt t 21

<210> 507

<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 11, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 12, 14, 15, 16, 17, 18, 19
<223> /mod_base = "2'-hydroxy corresponding nucleoside"

<400> 507
aauuuaugcc uacagccuct t 21

<210> 508
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 14, 15, 16, 17, 18, 19
<223> /mod_base = "2'-hydroxy corresponding nucleoside"

<400> 508
aaauggcacu aguaaacugt t 21

<210> 509
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 10, 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 11, 12, 14, 16, 17, 18, 19
<223> /mod_base = "2'-hydroxy corresponding nucleoside"

<400> 509
acaaacgggc aacauaccut t 21

<210> 510
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 510

tggacaaacg ggcaacauat t 21

<210> 511
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 511
tauuuaugcc uacagccuct t 21

<210> 512
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 16, 17, 18, 19
<223> /mod_base = "2'-hydroxy corresponding nucleoside"

<400> 512
augauaaaac gccgcagact t 21

<210> 513
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 16, 17, 18, 19
<223> /mod_base = "2'-hydroxy corresponding nucleoside"

<400> 513
ggcagaggug aaaaaguugt t 21

<210> 514
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 13, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 14, 16, 17, 18, 19
<223> /mod_base = "2'-hydroxy corresponding nucleoside"

<400> 514
ugaagcgaag ugcacacggt t 21

<210> 515
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 515
tgaagcgaag ugcacacggt t 21

<210> 516
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 516
tacgggcaac auaccuugat t 21

<210> 517
<211> 21
<212> DNA

<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 517
taauggcacu aguaaacugt t 21

<210> 518
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 17, 19
<223> /mod_base = "2'-hydroxy corresponding nucleoside"

<400> 518
aauugagaga aguccaccat t 21

<210> 519
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 519
tcaaacgggc aacauaccut t 21

<210> 520
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 8, 11, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 14, 15, 16, 18, 19
<223> /mod_base = "2'-hydroxy corresponding nucleoside"

<400> 520
ccgccuguaa cacgagcagt t 21

<210> 521
<211> 21
<212> DNA
<213> Artificial sequence

<220>

<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 13, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 14, 15, 17, 19
<223> /mod_base = "2'-hydroxy corresponding nucleoside"

<400> 521
aggacaaacg ggcaacauat t 21

<210> 522
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 10, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 13, 14, 15, 16, 17, 18, 19
<223> /mod_base = "2'-hydroxy corresponding nucleoside"

<400> 522
aacgggcaac auaccuugat t 21

<210> 523
<211> 21

<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 17, 18, 19
<223> /mod_base = "2'-hydroxy corresponding nucleoside"

<400> 523
uccggaagug uugauaagat t 21

<210> 524
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 524
tccggaagug uugauaagat t 21

<210> 525
<211> 21
<212> DNA

<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 8, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 11, 13, 14, 15, 16, 17, 18, 19
<223> /mod_base = "2'-hydroxy corresponding nucleoside"

<400> 525
agaugauuag gcagaggugt t 21

<210> 526
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 18, 19
<223> /mod_base = "2'-hydroxy corresponding nucleoside"

<400> 526
aguccaagag uccucuuaut t 21

<210> 527
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 527
tugauaaaac gccgcagact t 21

<210> 528
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 14, 15, 16, 17, 18, 19
<223> /mod_base = "2'-hydroxy corresponding nucleoside"

<400> 528
guccgcggga uucagcgcct t 21

<210> 529

<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 529
ugagaucuuc ugcgacgcgt t 21

<210> 530
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 17, 18, 19
<223> /mod_base = "2'-hydroxy corresponding nucleoside"

<400> 530
aaggucgguc guugacauut t 21

<210> 531
<211> 21

<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 7, 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 11, 12, 13, 14, 15, 16, 17, 18, 19
<223> /mod_base = "2'-hydroxy corresponding nucleoside"

<400> 531
uggcacuagu aaacugagct t 21

<210> 532
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 532
tauugagaga aguccaccat t 21

<210> 533
<211> 21
<212> DNA

<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 533
ucuucugcga cgcggcgaut t 21

<210> 534
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 13, 14, 15, 16, 17, 18, 19
<223> /mod_base = "2'-hydroxy corresponding nucleoside"

<400> 534
gaaccacuga acaaauggct t 21

<210> 535
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 8, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 9, 10, 12, 13, 14, 15, 16, 17, 18, 19
<223> /mod_base = "2'-hydroxy corresponding nucleoside"

<400> 535
aguuccgcag uauggaucgt t 21

<210> 536
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 12, 13, 14, 15, 16, 17, 18, 19
<223> /mod_base = "2'-hydroxy corresponding nucleoside"

<400> 536
gaugauuagg cagaggugat t 21

<210> 537

<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 16, 18, 19
<223> /mod_base = "2'-hydroxy corresponding nucleoside"

<400> 537
aaaauugaga gaaguccact t 21

<210> 538
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 10, 11, 12, 13, 14, 15, 16, 17, 18, 19
<223> /mod_base = "2'-hydroxy corresponding nucleoside"

<400> 538
cccaagaaua uggugaccct t 21

<210> 539
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 539
agaucuucug cgacgcggct t 21

<210> 540
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 540
ucugcgacgc ggcgauugat t 21

<210> 541
<211> 21
<212> DNA

<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 8, 12, 14, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 2, 3, 4, 5, 6, 7, 9, 10, 11, 13, 15, 16, 17, 19
<223> /mod_base = "2’-hydroxy corresponding nucleoside"

<400> 541
aacgccgcag acacauccat t 21

<210> 542
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 15, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 2, 3, 4, 5, 6, 7, 8, 9, 10, 11, 12, 13, 14, 16, 17, 19
<223> /mod_base = "2’-hydroxy corresponding nucleoside"

<400> 542
cuuggaggcu ugaacaguat t 21

<210> 543
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 9, 12, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 10, 11, 13, 14, 16, 17, 18, 19
<223> /mod_base = "2'-hydroxy corresponding nucleoside"

<400> 543
acaaauggca cuaguaaact t 21

<210> 544
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 13, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 14, 15, 17, 18, 19
<223> /mod_base = "2'-hydroxy corresponding nucleoside"

<400> 544
cugaacaaau ggcacuagut t 21

<210> 545
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 545
gagaucuucu gcgacgcggt t 21

<210> 546
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 13, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 14, 15, 16, 17, 19
<223> /mod_base = "2'-hydroxy corresponding nucleoside"

<400> 546

cggaaguguu gauaagauat t 21

<210> 547
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 547
gggacugcga auuuuggcct t 21

<210> 548
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 11, 12, 13, 14, 15, 16, 17, 18, 19
<223> /mod_base = "2'-hydroxy corresponding nucleoside"

<400> 548
augauuaggc agaggugaat t 21

<210> 549
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 8, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 12, 13, 14, 15, 16, 17, 18, 19
<223> /mod_base = "2'-hydroxy corresponding nucleoside"

<400> 549
auggcacuag uaaacugagt t 21

<210> 550
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 16, 17, 19
<223> /mod_base = "2'-hydroxy corresponding nucleoside"

<400> 550
agaggacaaa cgggcaacat t 21

<210> 551
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 8, 15, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 9, 10, 11, 12, 13, 14, 16, 17, 19
<223> /mod_base = "2'-hydroxy corresponding nucleoside"

<400> 551
cacugaacaa auggcacuat t 21

<210> 552
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 6, 12, 14
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 2, 3, 4, 5, 7, 8, 9, 10, 11, 13, 15, 16, 17, 18, 19
<223> /mod_base = "2'-hydroxy corresponding nucleoside"

<400> 552
caauuuaugc cuacagccut t 21

<210> 553
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 11, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 10, 12, 13, 14, 16, 18, 19
<223> /mod_base = "2'-hydroxy corresponding nucleoside"

<400> 553
uaaaacgccg cagacacaut t 21

<210> 554
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base

<222> 21
<223> /mod_base = "5'-phosphorothioate thymidine"

<400> 554
taaaacgccg cagacacaut t 21

<210> 555
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 16, 17, 18, 19
<223> /mod_base = "2'-hydroxy corresponding nucleoside"

<400> 555
ugcagaggug aagcgaagut t 21

<210> 556
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 14, 16
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 1, 2, 3, 4, 5, 6, 7, 8, 9, 10, 11, 12, 13, 15, 17, 18, 19
<223> /mod_base = "2'-hydroxy corresponding nucleoside"

<400> 556
gugaagcgaa gugcacacgt t 21

<210> 557
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 15, 16, 18, 19
<223> /mod_base = "2'-hydroxy corresponding nucleoside"

<400> 557
auugagagaa guccaccact t 21

<210> 558
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 12, 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 13, 14, 15, 17, 19
<223> /mod_base = "2'-hydroxy corresponding nucleoside"

<400> 558
auaaaacgcc gcagacacat t 21

<210> 559
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 9, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 10, 11, 13, 15, 16, 17, 18, 19
<223> /mod_base = "2'-hydroxy corresponding nucleoside"

<400> 559
caaacgggca acauaccuut t 21

<210> 560
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>

<221> modified_base
<222> 4
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 13, 14, 15, 16, 17, 18, 19
<223> /mod_base = "2'-hydroxy corresponding nucleoside"

<400> 560
ccucaagguc ggucguugat t 21

<210> 561
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 13, 14, 15, 17, 18, 19
<223> /mod_base = "2'-hydroxy corresponding nucleoside"

<400> 561
guccaagagu ccucuuaugt t 21

<210> 562
<211> 21
<212> DNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<220>
<221> modified_base
<222> 3
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 16, 17, 18, 19
<223> /mod_base = "2'-hydroxy corresponding nucleoside"

<400> 562
cucaaggucg gucguugact t 21

<210> 563
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 11, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 16, 18, 19
<223> /mod_base = "2'-hydroxy corresponding nucleoside"

<400> 563
cucuuguucc caagaauaut t 21

<210> 564
<211> 21
<212> DNA
<213> Artificial sequence

<220>

<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 12, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 13, 14, 15, 16, 17, 19
<223> /mod_base = "2'-hydroxy corresponding nucleoside"

<400> 564
cccgccugua acacgagcat t 21

<210> 565
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 565
uucugcgacg cggcgauugt t 21

<210> 566
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 17, 18, 19
<223> /mod_base = "2'-hydroxy corresponding nucleoside"

<400> 566
gcuuggaggc uugaacagut t 21

<210> 567
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 567
gaucuucugc gacgcggcgt t 21

<210> 568
<211> 21
<212> DNA
<213> Artificial sequence

<220>
<223> Description of the artificial sequence: antisense strand of dsRNA

<220>

<221> modified_base
<222> 1
<223> /mod_base = "nucleoside: lacks 5'-phosphate group"

<220>
<221> modified_base
<222> 7, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 15, 16, 17, 18, 19
<223> /mod_base = "2'-hydroxy corresponding nucleoside"

<400> 568
acgaaccacu gaacaaaugt t 21

<210> 569
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 17, 18, 19
<223> /mod_base = "2'-hydroxy corresponding nucleoside"

<400> 569
ucguccgcgg gauucagcgt t 21

<210> 570
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 8, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 9, 10, 11, 12, 13, 14, 15, 16, 17, 19
<223> /mod_base = "2'-hydroxy corresponding nucleoside"

<400> 570
ccaagaauau ggugacccat t 21

<210> 571
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 14, 15, 16, 17, 18, 19
<223> /mod_base = "2'-hydroxy corresponding nucleoside"

<400> 571
aguauggauc ggcagaggat t 21

<210> 572

<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 10, 11, 12, 13, 14, 15, 16, 18, 19
<223> /mod_base = "2'-hydroxy corresponding nucleoside"

<400> 572
caaggucggu cguugacaut t 21

<210> 573
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 10, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 13, 15, 16, 17, 18, 19
<223> /mod_base = "2'-hydroxy corresponding nucleoside"

<400> 573
ugagaugauu aggcagaggt t 21

<210> 574
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 13, 15, 16, 17, 18, 19
<223> /mod_base = "2'-hydroxy corresponding nucleoside"

<400> 574
gaagcgaagu gcacacggut t 21

<210> 575
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 8, 18
<223> /mod_base = "2'-O-methyl corresponding nucleoside"

<220>
<221> modified_base
<222> 21
<223> /mod_base = "5'-phosphorothioate thymidine"

<220>

<221> modified_base
<222> 1, 2, 3, 4, 6, 7, 9, 10, 11, 12, 13, 14, 15, 16, 17, 19
<223> /mod_base = "2'-hydroxy corresponding nucleoside"

<400> 575
uccgcaguau ggaucggcat t 21

<210> 576
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 12, 13, 14, 15, 16, 17, 18, 19
<223> /mod_base = "2'-hydroxy corresponding nucleoside"

<400> 576
accacgaguc uagacucugt t 21

<210> 577
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 17
<223> /mod_base = "2'-O-methyl corresponding nucleoside"

<220>
<221> modified_base

<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 16, 18, 19
<223> /mod_base = "2'-hydroxy corresponding nucleoside"

<400> 577
agcuuggagg cuugaacagt t 21

<210> 578
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 10, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 11, 13, 14, 15, 16, 17, 18, 19
<223> /mod_base = "2'-hydroxy corresponding nucleoside"

<400> 578
auuuaugccu acagccucct t 21

<210> 579
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 9, 11

<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 10, 12, 13, 14, 15, 16, 17, 18, 19
<223> /mod_base = "2'-hydroxy corresponding nucleoside"

<400> 579
uuuaugccua cagccuccut t 21

<210> 580
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 17, 18, 19
<223> /mod_base = "2'-hydroxy corresponding nucleoside"

<400> 580
aaauugagag aaguccacct t 21

<210> 581
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 12, 13, 14, 15, 16, 17, 18, 19
<223> /mod_base = "2'-hydroxy corresponding nucleoside"

<400> 581
gcagagguga aaaaguugct t 21

<210> 582
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 582
tcgccuguaa cacgagcagt t 21

<210> 583
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>

<221> modified_base
<222> 7, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 15, 16, 18, 19
<223> /mod_base = "2'-hydroxy corresponding nucleoside"

<400> 583
acugaacaaa uggcacuagt t 21

<210> 584
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 7, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 8, 9, 10, 11, 12, 13, 14, 15, 16, 18, 19
<223> /mod_base = "2'-hydroxy corresponding nucleoside"

<400> 584
ccgcaguaug gaucggcagt t 21

<210> 585
<211> 21
<212> DNA
<213> Artificial sequence

<220>
<223> Description of the artificial sequence: antisense strand of dsRNA

<220>
<221> modified_base

<222> 1
<223> /mod_base = "nucleoside: lacks 5'-phosphate group"

<220>
<221> modified_base
<222> 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 16, 18, 19
<223> /mod_base = "2'-hydroxy corresponding nucleoside"

<400> 585
ggugaagcga agugcacact t 21

<210> 586
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 14, 15, 16, 17, 18, 19
<223> /mod_base = "2'-hydroxy corresponding nucleoside"

<400> 586
gcccgccugu aacacgagct t 21

<210> 587
<211> 21
<212> DNA
<213> Artificial sequence

<220>

<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 11, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 14, 15, 17, 18, 19
<223> /mod_base = "2'-hydroxy corresponding nucleoside"

<400> 587
gaaguguuga uaagauaggt t 21

<210> 588
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 588
ugcgacgcgg cgauugagat t 21

<210> 589
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 11
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 12, 13, 14, 15, 16, 17, 18, 19
<223> /mod_base = "2'-hydroxy corresponding nucleoside"

<400> 589
ugagaaggca cagacgggct t 21

<210> 590
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 12, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 13, 14, 15, 16, 18, 19
<223> /mod_base = "2'-hydroxy corresponding nucleoside"

<400> 590
ggaaguguug auaagauagt t 21

<210> 591
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 14, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 13, 15, 16, 18, 19
<223> /mod_base = "2'-hydroxy corresponding nucleoside"

<400> 591
gaggacaaac gggcaacaut t 21

<210> 592
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4, 12, 15, 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 13, 14, 16, 18, 19
<223> /mod_base = "2'-hydroxy corresponding nucleoside"

<400> 592
ggacaaacgg gcaacauact t 21

<210> 593

<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 13, 14, 15, 16, 17, 18, 19
<223> /mod_base = "2'-hydroxy corresponding nucleoside"

<400> 593
gugcagaggu gaagcgaagt t 21

<210> 594
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 16, 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 17, 19
<223> /mod_base = "2'-hydroxy corresponding nucleoside"

<400> 594
aggugaagcg aagugcacat t 21

<210> 595
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 595
cgggacugcg aauuuuggct t 21

<210> 596
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 10, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 13, 14, 15, 17, 18, 19
<223> /mod_base = "2'-hydroxy corresponding nucleoside"

<400> 596

cgccuguaac acgagcaggt t 21

<210> 597
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 11, 13, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 2, 3, 4, 5, 6, 7, 8, 9, 10, 12, 14, 15, 16, 17, 19
<223> /mod_base = "2’-hydroxy corresponding nucleoside"

<400> 597
agugcgaauc cacacuccat t 21

<210> 598
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 6
<223> /mod_base = "2’-O-methyl corresponding nucleoside"

<220>
<221> modified_base
<222> 21
<223> /mod_base = "5’-phosphorothioate thymidine"

<220>
<221> modified_base

<222> 1, 2, 3, 4, 5, 7, 8, 9, 10, 11, 12, 13, 14, 15, 16, 17, 18, 19
<223> /mod_base = "2'-hydroxy corresponding nucleoside"

<400> 598
ugccucaagg ucggucguut t 21

<210> 599
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 11, 12, 13, 14, 15, 16, 17, 18, 19
<223> /mod_base = "2'-hydroxy corresponding nucleoside"

<400> 599
ccacgagucu agacucugut t 21

<210> 600
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19

<223> /mod_base = "2'-hydroxy corresponding nucleoside"

<400> 600
cuucugcgac gcggcgauut t 21

<210> 601
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 11, 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 12, 13, 15, 17, 18, 19
<223> /mod_base = "2'-hydroxy corresponding nucleoside"

<400> 601
gacaaacggg caacauacct t 21

<210> 602
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6, 9
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 10, 11, 12, 13, 14, 15, 16, 17, 18, 19
<223> /mod_base = "2'-hydroxy corresponding nucleoside"

<400> 602
uuccgcagua uggaucggct t 21

<210> 603
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 12, 13, 15, 16, 17, 18, 19
<223> /mod_base = "2'-hydroxy corresponding nucleoside"

<400> 603
aguguugaua agauagggct t 21

<210> 604
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 8, 11, 14
<223> /mod_base = "2'-O-methyl corresponding nucleoside"

<220>

<221> modified_base
<222> 21
<223> /mod_base = "5’-phosphorothioate thymidine"

<220>
<221> modified_base
<222> 2, 3, 4, 5, 6, 7, 9, 10, 12, 13, 15, 16, 17, 18, 19
<223> /mod_base = "2’-hydroxy corresponding nucleoside"

<400> 604
caaauggcac uaguaaacut t 21

<210> 605
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 2, 18
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 3, 4, 5, 6, 7, 8, 9, 10, 11, 12, 13, 14, 15, 16, 17, 19
<223> /mod_base = "2’-hydroxy corresponding nucleoside"

<400> 605
ucaaggucgg ucguugacat t 21

<210> 606
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base

<222> 2
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 12, 13, 14, 15, 16, 17, 18, 19
<223> /mod_base = "2'-hydroxy corresponding nucleoside"

<400> 606
cuagaaaauu gagagaagut t 21

<210> 607
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 16, 17, 18, 19
<223> /mod_base = "2'-hydroxy corresponding nucleoside"

<400> 607
gacaaaggac gucccgcgct t 21

<210> 608
<211> 21
<212> DNA
<213> Artificial sequence

<220>
<223> Description of the artificial sequence: antisense strand of dsRNA

<220>
<221> modified_base
<222> 1

<223> /mod_base = "nucleoside: lacks 5'-phosphate group"

<220>
<221> modified_base
<222> 5, 8, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 12, 13, 14, 15, 17, 18, 19
<223> /mod_base = "2'-hydroxy corresponding nucleoside"

<400> 608
aguccaccac gagucuagat t 21

<210> 609
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 9, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 10, 11, 13, 14, 15, 16, 17, 18, 19
<223> /mod_base = "2'-hydroxy corresponding nucleoside"

<400> 609
gaguuccgca guauggauct t 21

<210> 610
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 5, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 6, 7, 8, 9, 10, 11, 12, 13, 14, 16, 17, 18, 19
<223> /mod_base = "2'-hydroxy corresponding nucleoside"

<400> 610
gcaguaugga ucggcagagt t 21

<210> 611
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3, 6, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 7, 8, 9, 10, 11, 12, 13, 14, 15, 17, 18, 19
<223> /mod_base = "2'-hydroxy corresponding nucleoside"

<400> 611
cgcaguaugg aucggcagat t 21

<210> 612
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 4, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 5, 6, 7, 8, 9, 10, 11, 12, 13, 15, 16, 17, 18, 19
<223> /mod_base = "2'-hydroxy corresponding nucleoside"

<400> 612
caguauggau cggcagaggt t 21

<210> 613
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 10, 11, 12, 13, 14, 15, 16, 17, 18, 19
<223> /mod_base = "2'-hydroxy corresponding nucleoside"

<400> 613
accgcguaaa gagaggugct t 21

<210> 614

<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 21
<223> /mod_base = "5’-phosphorothioate thymidine"

<220>
<221> modified_base
<222> 1..19
<223> /mod_base = "2’-hydroxy corresponding nucleoside"

<400> 614
gcgaggcgag ggaguucuut t 21

<210> 615
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 21
<223> /mod_base = "5’-phosphorothioate thymidine"

<220>
<221> modified_base
<222> 1..19
<223> /mod_base = "2’-hydroxy corresponding nucleoside"

<400> 615
cugcgaggcg agggaguuct t 21

<210> 616
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 17, 18, 19
<223> /mod_base = "2'-hydroxy corresponding nucleoside"

<400> 616
gcccuagaaa auugagagat t 21

<210> 617
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 3
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 7, 8, 9, 10, 11, 12, 13, 14, 15, 16, 17, 18, 19
<223> /mod_base = "2'-hydroxy corresponding nucleoside"

<400> 617
cguaaagaga ggugcgccct t 21

<210> 618
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 7, 10
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 8, 9, 11, 12, 13, 14, 15, 16, 17, 18, 19
<223> /mod_base = "2'-hydroxy corresponding nucleoside"

<400> 618
guuccgcagu auggaucggt t 21

<210> 619
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 619
ucugcgaggc gagggaguut t 21

<210> 620
<211> 21
<212> DNA
<213> Artificial sequence

<220>

<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2, 12
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 13, 14, 15, 16, 17, 18, 19
<223> /mod_base = "2'-hydroxy corresponding nucleoside"

<400> 620
guauggaucg gcagaggagt t 21

<210> 621
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 13, 15, 16, 17, 18, 19
<223> /mod_base = "2'-hydroxy corresponding nucleoside"

<400> 621
gagugcgaau ccacacucct t 21

<210> 622
<211> 21

<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 3, 4, 5, 6, 7, 8, 9, 10, 11, 12, 13, 14, 15, 16, 17, 18, 19
<223> /mod_base = "2'-hydroxy corresponding nucleoside"

<400> 622
guaaagagag gugcgcccgt t 21

<210> 623
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 623
gcgacgcggc gauugagact t 21

<210> 624
<211> 21
<212> DNA

<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 10, 13
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 11, 12, 14, 15, 16, 17, 18, 19
<223> /mod_base = "2'-hydroxy corresponding nucleoside"

<400> 624
ggaguuccgc aguauggaut t 21

<210> 625
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 13, 14, 15, 16, 17, 18, 19
<223> /mod_base = "2'-hydroxy corresponding nucleoside"

<400> 625
ccgcguaaag agaggugcgt t 21

<210> 626
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 17, 18, 19
<223> /mod_base = "2'-hydroxy corresponding nucleoside"

<400> 626
gccucaaggu cggucguugt t 21

<210> 627
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 12, 15
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 2, 3, 4, 5, 6, 7, 8, 9, 10, 11, 13, 14, 16, 17, 18, 19
<223> /mod_base = "2'-hydroxy corresponding nucleoside"

<400> 627
uaggaguucc gcaguauggt t 21

<210> 628
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 18
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 16, 17, 19
<223> /mod_base = "2'-hydroxy corresponding nucleoside"

<400> 628
gggucguccg cgggauucat t 21

<210> 629
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 629

ugcgaggcga gggaguucut t 21

<210> 630
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 4
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 5, 6, 7, 8, 9, 10, 11, 12, 13, 14, 15, 16, 17, 18, 19
<223> /mod_base = "2'-hydroxy corresponding nucleoside"

<400> 630
gcguaaagag aggugcgcct t 21

<210> 631
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5
<223> /mod_base = "2'-O-methyl corresponding nucleoside"

<220>
<221> modified_base
<222> 21
<223> /mod_base = "5'-phosphorothioate thymidine"

<220>
<221> modified_base

<222> 1, 2, 3, 4, 6, 7, 8, 9, 10, 11, 12, 13, 14, 15, 16, 17, 18, 19
<223> /mod_base = "2'-hydroxy corresponding nucleoside"

<400> 631
cgcguaaaga gaggugcgct t 21

<210> 632
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 17, 18, 19
<223> /mod_base = "2'-hydroxy corresponding nucleoside"

<400> 632
gucguccgcg ggauucagct t 21

<210> 633
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 2
<223> /mod_base = "2'-O-methyl corresponding nucleoside"

<220>
<221> modified_base
<222> 21

<223> /mod_base = "5'-phosphorothioate thymidine"

<220>
<221> modified_base
<222> 1, 3, 4, 5, 6, 7, 8, 9, 10, 11, 12, 13, 14, 15, 16, 17, 18, 19
<223> /mod_base = "2'-hydroxy corresponding nucleoside"

<400> 633
gcagagguga agcgaagugt t 21

<210> 634
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 21
<223> /mod_base = "5'-phosphorothioate thymidine"

<220>
<221> modified_base
<222> 1..19
<223> /mod_base = "2'-hydroxy corresponding nucleoside"

<400> 634
cugcgacgcg gcgauugagt t 21

<210> 635
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 6
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 7, 8, 9, 10, 11, 12, 13, 14, 15, 16, 17, 18, 19
<223> /mod_base = "2'-hydroxy corresponding nucleoside"

<400> 635
ggauucagcg ccgacgggat t 21

<210> 636
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 17
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 16, 18, 19
<223> /mod_base = "2'-hydroxy corresponding nucleoside"

<400> 636
ggucguccgc gggauucagt t 21

<210> 637
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 1, 4, 12
<223> /mod_base = "2'-O-methyl corresponding nucleoside"

<220>

<221> modified_base
<222> 21
<223> /mod_base = "5'-phosphorothioate thymidine"

<220>
<221> modified_base
<222> 2, 3, 5, 6, 7, 8, 9, 10, 11, 13, 14, 15, 16, 17, 18, 19
<223> /mod_base = "2'-hydroxy corresponding nucleoside"

<400> 637
caccacgagu cuagacucut t 21

<210> 638
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 11, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 12, 13, 15, 16, 17, 18, 19
<223> /mod_base = "2'-hydroxy corresponding nucleoside"

<400> 638
aggaguuccg caguauggat t 21

<210> 639
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base

<222> 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 15, 16, 17, 18, 19
<223> /mod_base = "2'-hydroxy corresponding nucleoside"

<400> 639
cguccgcggg auucagcgct t 21

<210> 640
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 14, 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 15, 17, 18, 19
<223> /mod_base = "2'-hydroxy corresponding nucleoside"

<400> 640
aggagugcga auccacacut t 21

<210> 641
<211> 21
<212> DNA
<213> Artificial sequence

<220>
<223> Description of the artificial sequence: antisense strand of dsRNA

<220>
<221> modified_base
<222> 1

<223> /mod_base = "nucleoside: lacks 5’-phosphate group"

<220>
<221> modified_base
<222> 6, 9, 15
<223> /mod_base = "2’-O-methyl corresponding nucleoside"

<220>
<221> modified_base
<222> 21
<223> /mod_base = "5’-phosphorothioate thymidine"

<220>
<221> modified_base
<222> 1, 2, 3, 4, 5, 7, 8, 10, 11, 12, 13, 14, 16, 17, 18, 19
<223> /mod_base = "2’-hydroxy corresponding nucleoside"

<400> 641
gccuguaaca cgagcagggt t 21

<210> 642
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 21
<223> /mod_base = "5’-phosphorothioate thymidine"

<220>
<221> modified_base
<222> 1..19
<223> /mod_base = "2’-hydroxy corresponding nucleoside"

<400> 642
cgggucgucc gcgggauuct t 21

<210> 643
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5’-phosphate group"

<220>
<221> modified_base
<222> 3, 7, 12, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 4, 5, 6, 8, 9, 10, 11, 13, 15, 16, 17, 18, 19
<223> /mod_base = "2'-hydroxy corresponding nucleoside"

<400> 643
gccacccaag gcacagcuut t 21

<210> 644
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 5, 8, 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 9, 10, 11, 12, 13, 15, 16, 17, 18, 19
<223> /mod_base = "2'-hydroxy corresponding nucleoside"

<400> 644
ccuguaacac gagcagggct t 21

<210> 645
<211> 21
<212> DNA
<213> Artificial sequence

<220>
<223> Description of the artificial sequence: antisense strand of dsRNA

<220>

<221> modified_base
<222> 1
<223> /mod_base = "nucleoside: lacks 5'-phosphate group"

<220>
<221> modified_base
<222> 5
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 6, 7, 8, 9, 10, 11, 12, 13, 14, 15, 16, 17, 18, 19
<223> /mod_base = "2'-hydroxy corresponding nucleoside"

<400> 645
gauucagcgc cgacgggact t 21

<210> 646
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 14
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 15, 16, 17, 18, 19
<223> /mod_base = "2'-hydroxy corresponding nucleoside"

<400> 646
ccggaagugu ugauaagaut t 21

<210> 647
<211> 21
<212> DNA
<213> Artificial sequence

<220>
<223> Description of the artificial sequence: antisense strand of dsRNA

<220>
<221> modified_base
<222> 1
<223> /mod_base = "nucleoside: lacks 5'-phosphate group"

<220>
<221> modified_base
<222> 16
<223> /mod_base = "2'-O-methyl corresponding nucleoside"

<220>
<221> modified_base
<222> 21
<223> /mod_base = "5'-phosphorothioate thymidine"

<220>
<221> modified_base
<222> 1, 2, 3, 4, 5, 6, 7, 8, 9, 10, 11, 12, 13, 14, 15, 17, 18, 19
<223> /mod_base = "2'-hydroxy corresponding nucleoside"

<400> 647
uuccggaagu guugauaagt t 21

<210> 648
<211> 21
<212> DNA
<213> Artificial sequence

<220>
<223> Description of the artificial sequence: antisense strand of dsRNA

<220>
<221> modified_base
<222> 3, 5, 7, 9, 11, 13, 15, 17, 19
<223> /mod_base = "2'-O-methyl corresponding nucleoside"

<220>
<221> modified_base
<222> 2, 4, 6, 8, 10, 12, 14, 16, 18
<223> /mod_base = "2'-deoxy-2'-fluoro corresponding nucleoside"

<220>
<221> modified_base
<222> 21
<223> /mod_base = "5'-phosphorothioate thymidine"

<400> 648
tuccggaagu guugauaagt t 21

## Description

### BACKGROUND OF THE INVENTION

This invention relates to double-stranded ribonucleic acids (dsRNAs), and their use in mediating RNA interference to inhibit the expression of genes, necessary for replication and pathogenesis of Hepatitis B Virus, in particular in the inhibition of viral polymerase, surface antigen, e-antigen and the X protein. Furthermore, the use of said dsRNAs to treat or prevent chronic liver diseases/disorders, inflammations, fibrotic conditions and proliferative disorders, like cancers, as consequence of Hepatitis B Virus infection, is part of this invention.

The Hepatitis B Virus is a strict hepatotrophic, double-stranded DNA containing virus. Although DNA is the genetic material, the replication cycle involves a reverse transcription step to copy a pregenomic RNA into DNA. In order to accomplish this essential step, the viral-encoded polymerase possesses reverse transcriptase activity. Hepatitis B virus is classified as one member of the Hepadnaviruses and belongs to the family of Hepadnaviridae. The primary infection of adult humans with Hepatitis B Virus causes an acute hepatitis with symptoms of organ inflammation, fever, jaundice and increased liver transaminases in blood. About 95% of acute hepatitis resolve without treatment. Those patients, that are not able to overcome the virus infection, suffer a chronic disease progression over many years with increased risk of developing cirrhotic liver or liver cancer. Perinatal transmission from Hepatitis B virus-infected mothers to newborns also leads to chronic hepatitis. The treatment options for chronic Hepatitis B Virus infection are limited and lead only in some cases to complete and lasting remission. Additional clinical and therapeutical complications arise in Hepatitis B Virus patients co-infected with Hepatitis C, Hepatitis D or Human Immunodeficiency Virus.

The Hepatitis B Virus is transmitted via blood or blood products, sperm, vaginal secrets, or saliva. Drug abuse and sexual intercourse are dangerous activities and support spreading the virus. Contact of damaged, mucoid epithelia with contaminated body fluids may be sufficient for infection. Incubation time is between 40 to 200 days. The risk for infection is proportional to the number of transmitted Hepatitis B Virus particles. Babies are often infected perinatally by their Hepatitis B Virus carrying mother, a major health problem in endemic areas.

About 2 billion people are infected with Hepatitis B Virus and 400 million are chronic carriers. Areas with high prevalence are Africa and South-East Asia, with local accumulation of 20-80% infected persons.

Based on sequence homology, Hepatitis B Viruses are classified into genotypes A-H, with genotypes A-D being the most important ones. Genotype A is frequent in North-Western Europe, USA, South and Central America. Genotype B and C are dominant in China, Japan, Indonesia and other countries in East Asia. Genotype D is found in Southern Europe, Northern Africa and South Africa. Disease progression and response to pharmaceutical treatment differ among genotypes.

Infectious Hepatitis B Virus particles have a diameter of about 42 nm. The outer membrane bilayer contains the large, middle and small surface protein. The cognate hepatocellular receptor for virus surface protein binding and internalization is unknown. Many copies of core protein form a spherical nucleocapsid structure inside the virus particle. Each nucleocapsid carries partial double-stranded DNA as genetic material, together with viral polymerase.

Upon uptake by hepatocytes, the nucleocapsid is transferred to the nucleus and DNA is released. There, the DNA strand synthesis is completed and gaps repaired to give the covalently closed circular (ccc) supercoiled DNA of 3.2kb. The cccDNA serves as template for transcription of four major viral mRNAs, which are 3.5, 2.4, 2.1 and 0.7 kb long. All mRNAs are 5'-capped and polyadenylated at the 3'-end. There is sequence overlap at the 3'-end between all four mRNAs.

The 3.5 kb mRNA serves as template for core protein and polymerase production. In addition, the same transcript serves as a pre-genomic replication intermediate and allows the viral polymerase to initiate the reverse transcription into DNA. Core protein is needed for nucleocapsid formation. In addition, sequential processing activities transforms some core protein into the secretable e-antigen. The abundance of e-antigen in blood correlates with Hepatitis B Virus replication in liver and serves as important diagnostic marker for monitoring the disease progression.

The 2.4 and 2.1 kb mRNAs carry the open reading frames pre-SI, pre-S2 and S2 for expression of viral large, medium and small surface antigen. The s-antigen is associated with infectious, complete particles. In addition, blood of infected patients also contain non-infectious particles derived from s-antigen alone, free of genomic DNA or polymerase. The function of these particles is not fully understood. The complete and lasting depletion of detectable s-antigen in blood is considered as reliable indicator for Hepatitis B Virus clearance and thus, successful cure.

The 0.7 kb mRNA encodes the X protein. This gene product is important for efficient transcription of viral genes and also acts as a transactivator on host gene expression. The latter activity seems to be important for hepatocyte transformation during development of liver cancer.

Recombinant Hepatitis B Virus s-antigen is used for vaccination. The injection of three doses of formulated s-antigen at day 1, at 4 weeks and at 6 months usually induces a sufficient titer of neutralizing antibodies. Vaccinated patients are protected for 10 years or longer. However, the vaccines are no substitute for therapy.

Patients with acute Hepatitis B Virus infection are not treated due to the high, natural remission rate.

However, those patients with detectable s-antigen, e-antigen or viral DNA in the blood for more than 6 months are considered chronically infected. Nucleoside analogs as inhibitors of reverse transcriptase activity are the first treatment option for many patients. Lamivudine, Tenofovir, or Entecavir suppress Hepatitis B Virus replication, sometimes to undetectable levels. Improvement of liver function and reduction of liver inflammation are the most important benefits. However, only few patients achieve complete and lasting remission after the end of treatment. Furthermore, the Hepatitis B Virus develops drug resistance with increasing duration of treatment. This is especially difficult for patients co-infected with Hepatitis B and Human Immunodeficiency Virus. Both viruses are susceptible to nucleoside analogue drugs and may co-develop resistance.

The second treatment option is the administration of interferon-alpha. Here, patients receive high doses of interferon-alpha over a period of 6 months. Depending on the virus genotype, up to 50% of chronic infection are curable. However, the Asian genotype B gives very poor response rates. Co-infection with Hepatitis D or Human Immunodeficiency Virus renders interferon-alpha therapy completely ineffective. Patients with strong liver damage and heavy fibrotic conditions are not qualified for interferon-alpha therapy.

Despite significant advances in the field of Hepatitis B Virus treatment, there remains a need for an agent that can selectively and efficiently silence the gene expression of the virus, blocks replication and subsequently reduces viral burden in chronically infected patients.

Double-stranded RNA molecules (dsRNA) have been shown to block gene expression in a highly conserved regulatory mechanism known as RNA interference (RNAi). The invention provides double-stranded ribonucleic acid molecules (dsRNAs), as well as compositions and methods for inhibiting the expression of the Hepatitis B Virus gene, in particular the expression of the Hepatitis B Virus gene, in a cell, tissue or mammal using such dsRNA. The invention also provides compositions and methods for treating or preventing pathological conditions and diseases caused by the infection of the Hepatitis B Virus such as in chronic liver diseases/disorders, inflammations, fibrotic conditions and proliferative disorders, like cancers.
US 2003/206887 discloses methods and reagents useful in modulating hepatitis B virus (HBV) gene expression in a variety of applications, including use in therapeutic, diagnostic, target validation, and genomic discovery applications.
WO 2011/073218 discloses a double-stranded ribonucleic acid (dsRNA) for inhibiting the expression of a IL-18 gene.
Zhang et al. BMC Microbiol (2010) 10: 214 discloses that RNA Interference inhibits Hepatitis B Virus of different genotypes *in vitro* and *in vivo.*

## SUMMARY OF THE INVENTION

The invention provides a double-stranded ribonucleic acid molecule capable of inhibiting the expression of a Hepatitis B Virus gene in vitro by at least 60%, preferably by at least 70% and most preferably by at least 80%, wherein said double-stranded ribonucleic acid molecule comprises a sense strand comprising in order nucleotides 1-19 of SEQ ID NOs: 5 or 13 and an antisense strand at least partially complementary to the sense strand, and wherein the antisense strand sequence and the sense strand sequence are each less than 30 nucleotides in length, and wherein at least one of the nucleotides in each of the sense strand and in the antisense strand are modified nucleotides.
The invention also provides a pharmaceutical composition comprising:

(a) at least one double-stranded ribonucleic acid molecule of the invention; or
(b) at least one nucleic acid sequence encoding a sense strand or an antisense strand comprising a double-stranded ribonucleic acid molecule of the invention.

The invention also provides a pharmaceutical composition comprising:

(a) at least one first double-stranded ribonucleic acid molecule comprising a double stranded ribonucleic acid molecule of the invention and at least one second double-stranded ribonucleic acid molecule capable of inhibiting the expression of a Hepatitis B Virus gene wherein the sequences of the first and second double-stranded ribonucleic acid molecules are different; or
(b) at least one nucleic acid sequence encoding a sense strand or an antisense strand of a first double-stranded ribonucleic acid molecule of the invention and at least one second nucleic acid sequence encoding a sense strand or an antisense strand of a double-stranded ribonucleic acid molecule capable of inhibiting the expression of a Hepatitis B Virus gene wherein the sense strand or antisense strand sequences encoded by the first and second nucleic acids are different.

The invention provides double-stranded ribonucleic acid (dsRNA) molecules able to selectively and efficiently decrease the expression of Hepatitis B Virus gene. The use of Hepatitis B Virus RNAi provides a method for the therapeutic and/or prophylactic treatment of diseases/disorders which are associated with chronic liver diseases/disorders, inflammations,

fibrotic conditions and proliferative disorders, like cancers, such method comprises administration of dsRNA targeting Hepatitis B Virus to a human being or animal.

In one preferred embodiment the described dsRNA molecule is capable of inhibiting the expression of a Hepatitis B Virus gene by at least 60%, preferably by at least 70%, most preferably by at least 80%.

In one embodiment, the invention provides double-stranded ribonucleic acid (dsRNA) molecules for inhibiting the expression of a Hepatitis B Virus gene, in particular the expression of the genes related to replication or pathogenesis of Hepatitis B Virus. The dsRNA comprises at least two sequences that are complementary to each other. The dsRNA comprises a sense strand comprising a first sequence and an antisense strand comprising a second sequence, see sequences provided in the sequence listing and also the specific dsRNA pairs in the appended Table 1 and Table 2. In one embodiment the sense strand comprises a sequence which has an identity of at least 90% to at least a portion of an Hepatitis B Virus mRNA . Said sequence is located in a region of complementarity of the sense strand to the antisense strand, preferably within nucleotides 2-7 of the 5'terminus of the antisense strand. In one preferred embodiment the dsRNA specifically targets the Hepatitis B Virus gene that encodes core protein, viral polymerase, surface antigen, e-antigen or the X protein. Furthermore, it is preferred that the dsRNA specifically targets a consensus sequence which has a highly conserved nucleic acid sequence among the Hepatitis B Virus genomic sequences of genotype A, B, C and D. Preferably, the consensus sequence is at least 13 contiguous nucleotides in length, more preferably at least 17 contiguous nucleotides, and most preferably at least 19 contiguous nucleotides. Preferred highly conserved nucleic acid sequences are listed in Table 5.

In one embodiment, the antisense strand comprises a nucleotide sequence which is substantially complementary to at least part of an mRNA encoding said Hepatitis B Virus gene, and the region of complementarity is most preferably less than 30 nucleotides in length. Furthermore, it is preferred that the length of the herein described inventive dsRNA molecules (duplex length) is in the range of about 16 to 30 nucleotides, in particular in the range of about 18 to 28 nucleotides. Particularly useful in context of this invention are duplex lengths of about 19, 20, 21, 22, 23 or 24 nucleotides. Most preferred are duplex stretches of 19, 21 or 23 nucleotides. The dsRNA, upon delivery to a cell infected by a Hepatitis B Virus, inhibits the expression of a Hepatitis B Virus gene *in vitro* by at least 60%, preferably by at least 70%, and most preferably by 80%.

## BRIEF DESCRIPTION OF THE DRAWINGS

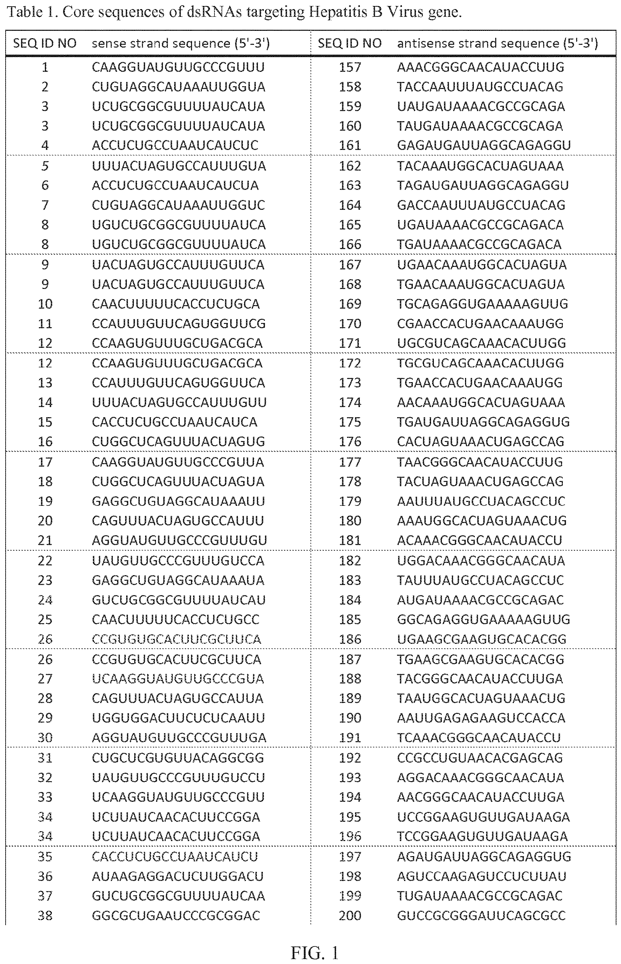

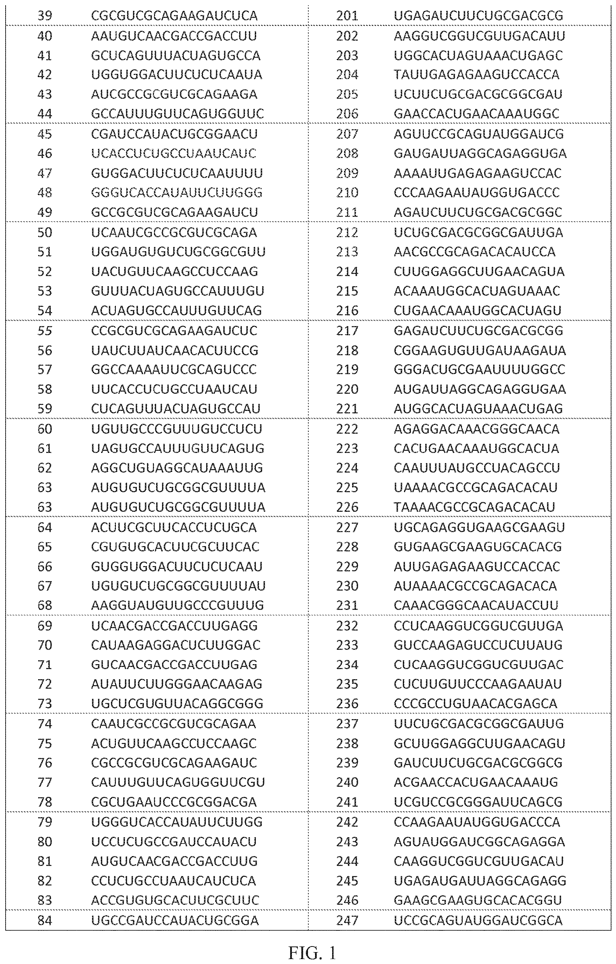

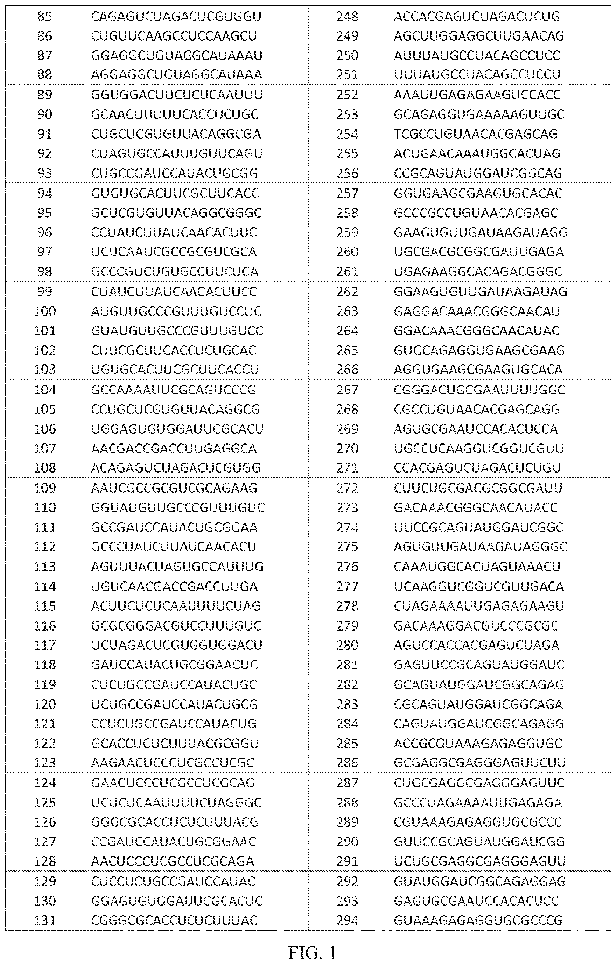

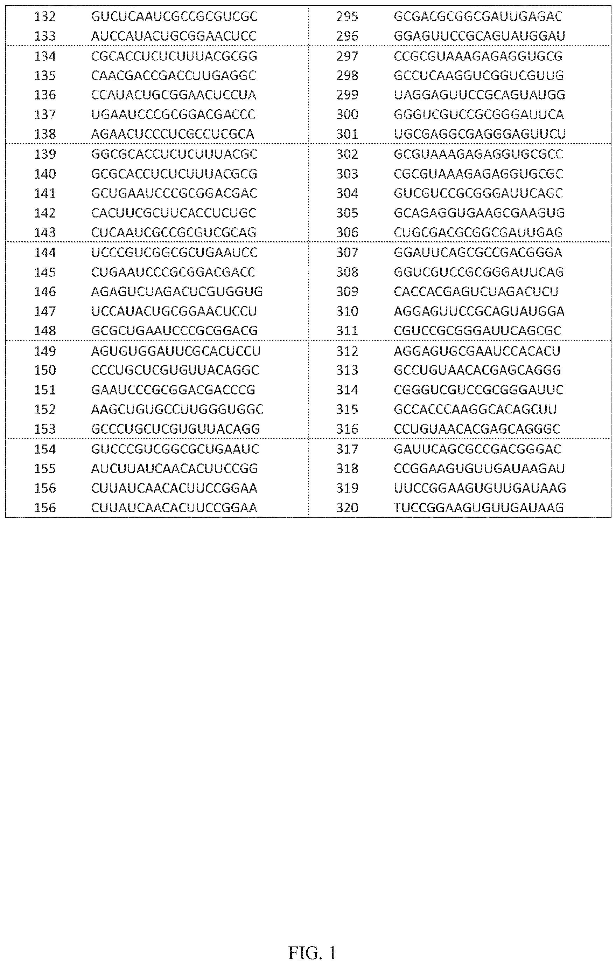

- FIG. 1. Table 1. Core sequences of dsRNAs targeting Hepatitis B Virus gene. Letters in capitals represent RNA nucleotides.

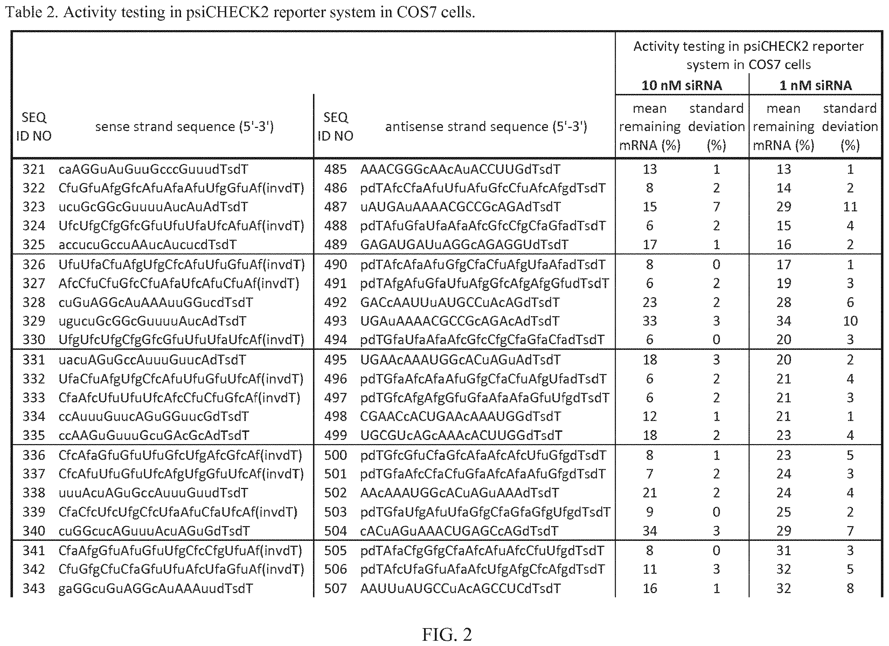

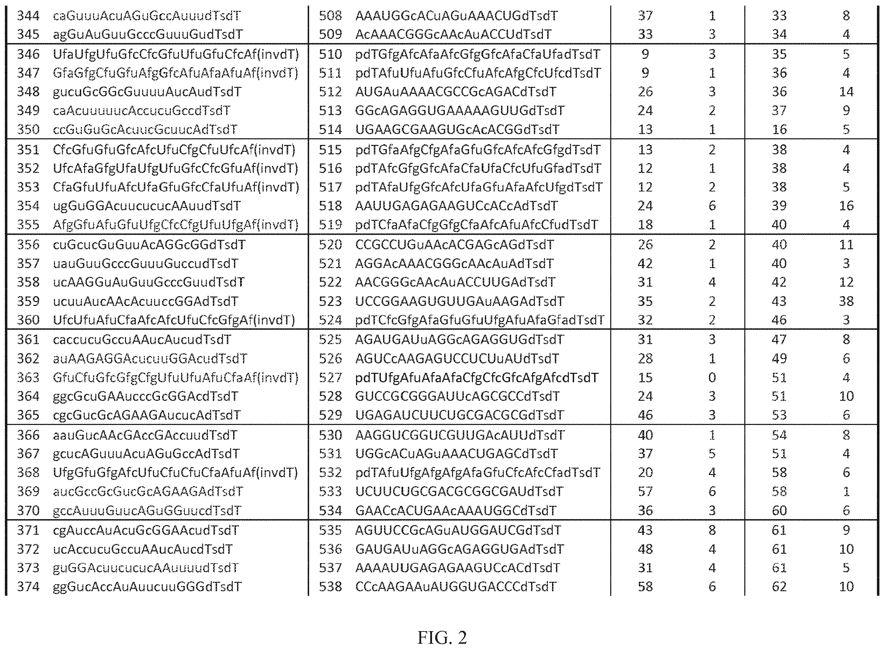

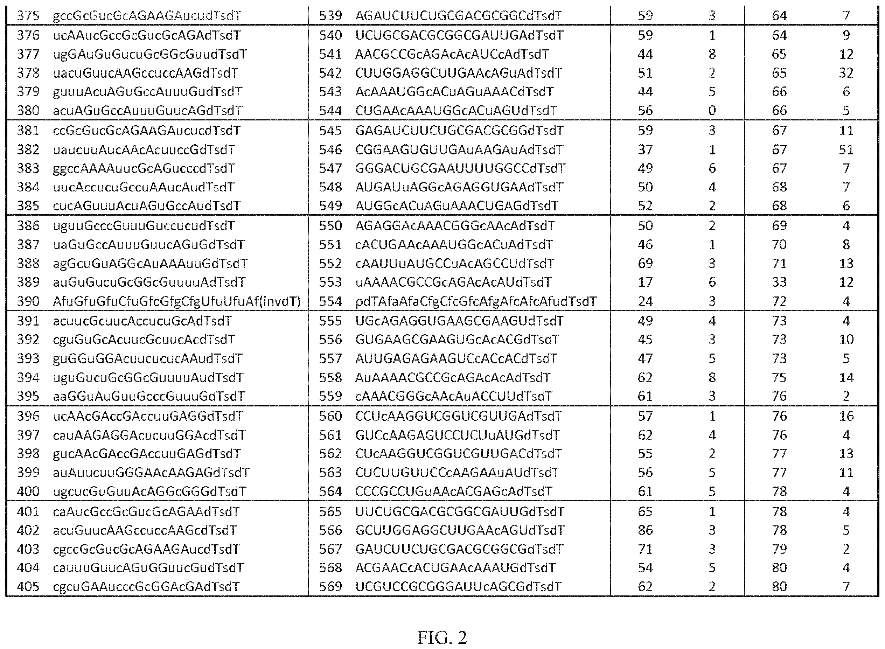

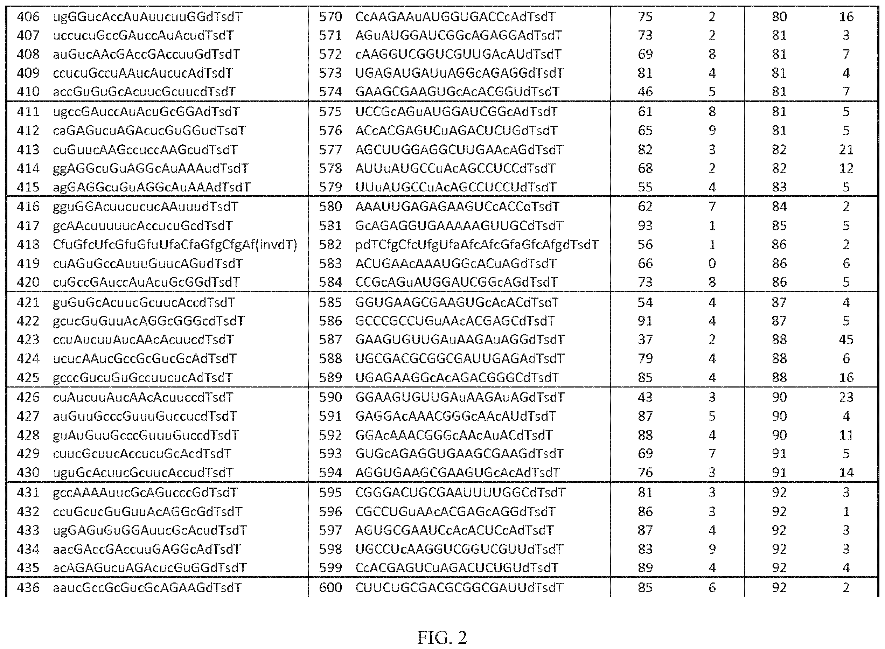

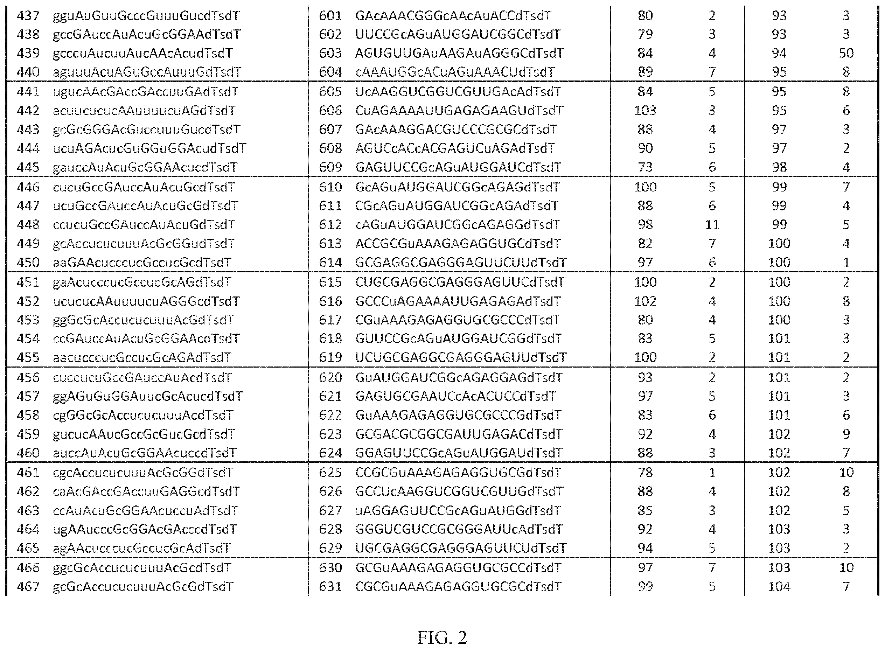

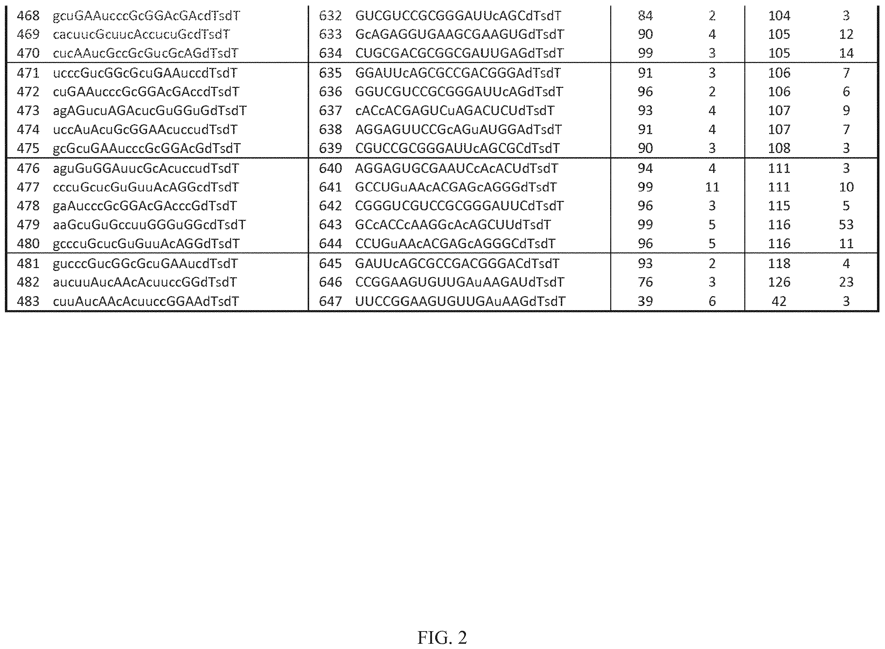

- FIG. 2 Table 2. Characterization of dsRNAs targeting Hepatitis B Virus : Activity testing with single dose. Letters in capitals represent RNA nucleotides, lower case letters "c", "g", "a" and "u" represent 2' O-methyl-modified nucleotides, "s" represents phosphorothioate, "dT" represents deoxythymidine, upper case letters A, C, G, U followed by "f" indicates a 2'-fluoro nucleotide. Lower case "p" indicates a 5'-phosphate. (invdT) represents an inverted deoxythimidine (3'-3'-linked).

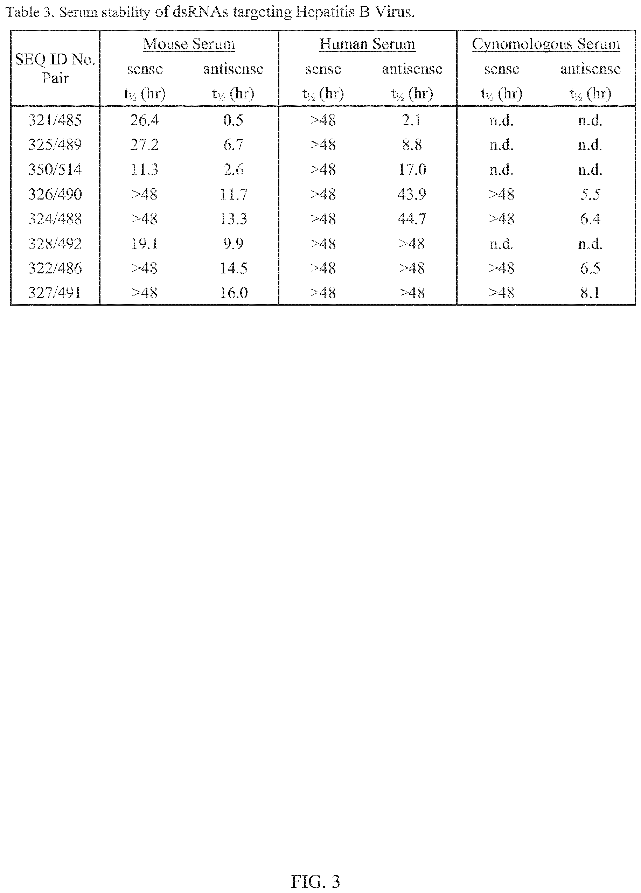

- FIG. 3. Table 3. Characterization of dsRNAs targeting Hepatitis B Virus: Stability. t½ = half-life of a strand as defined in examples.

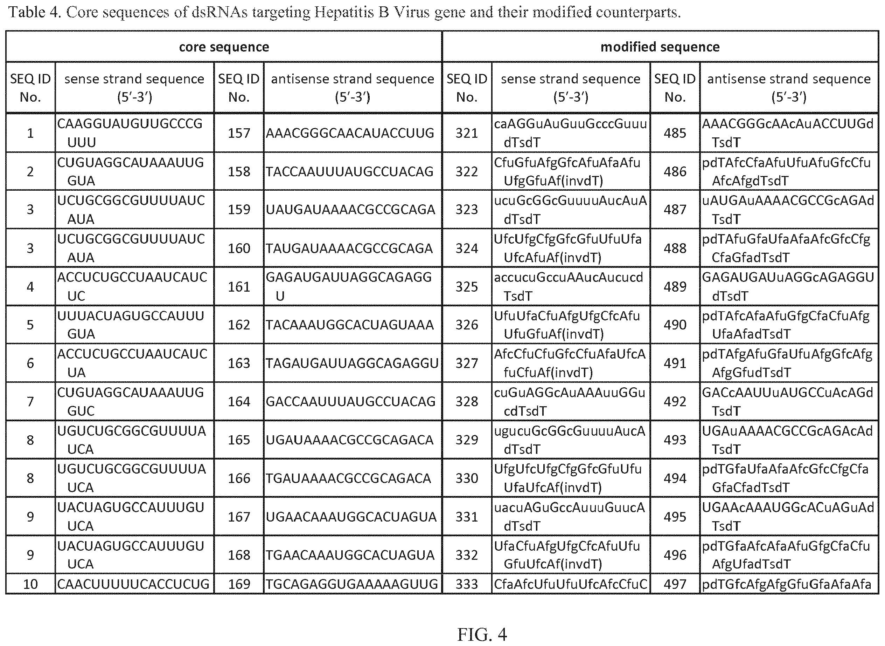

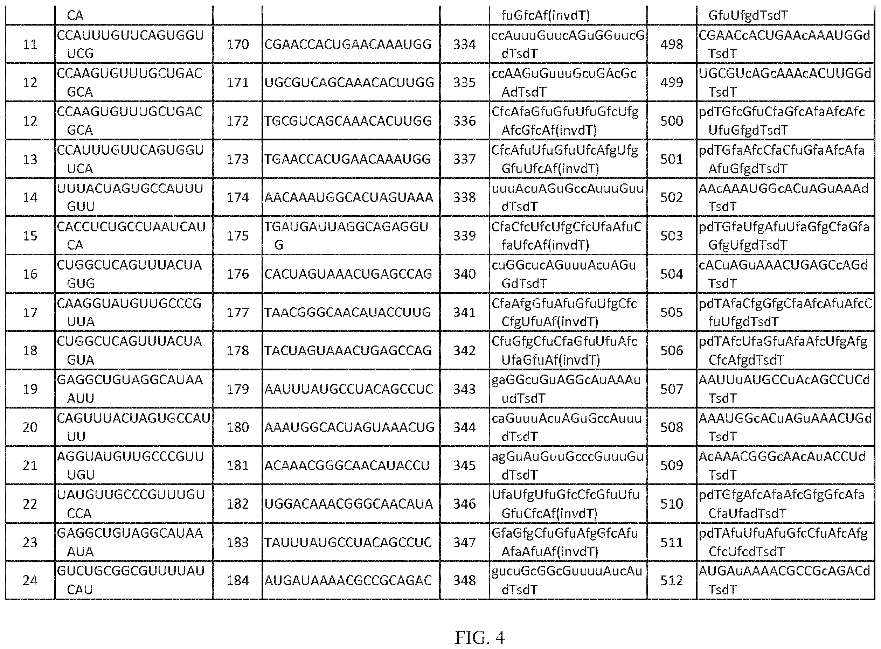

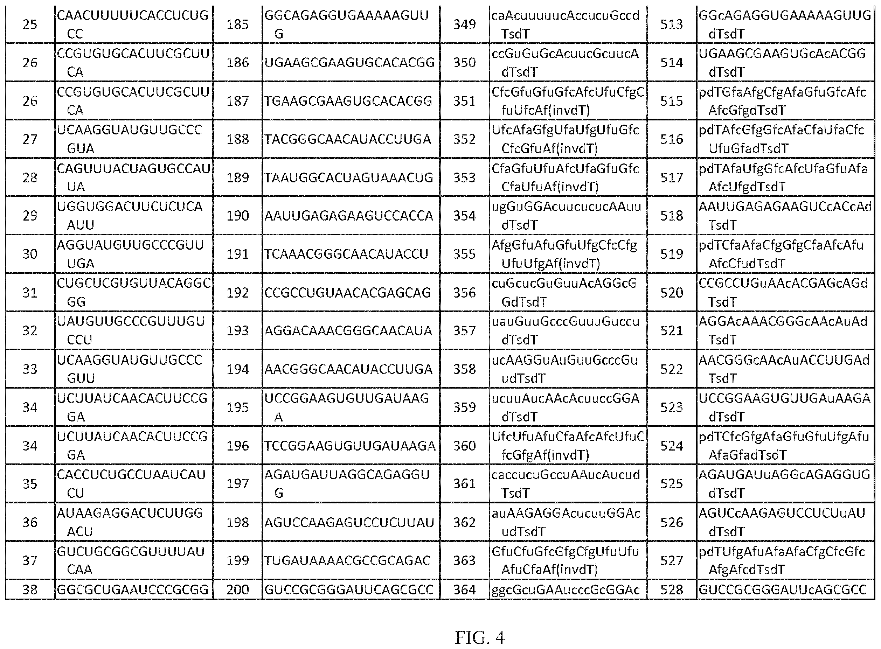

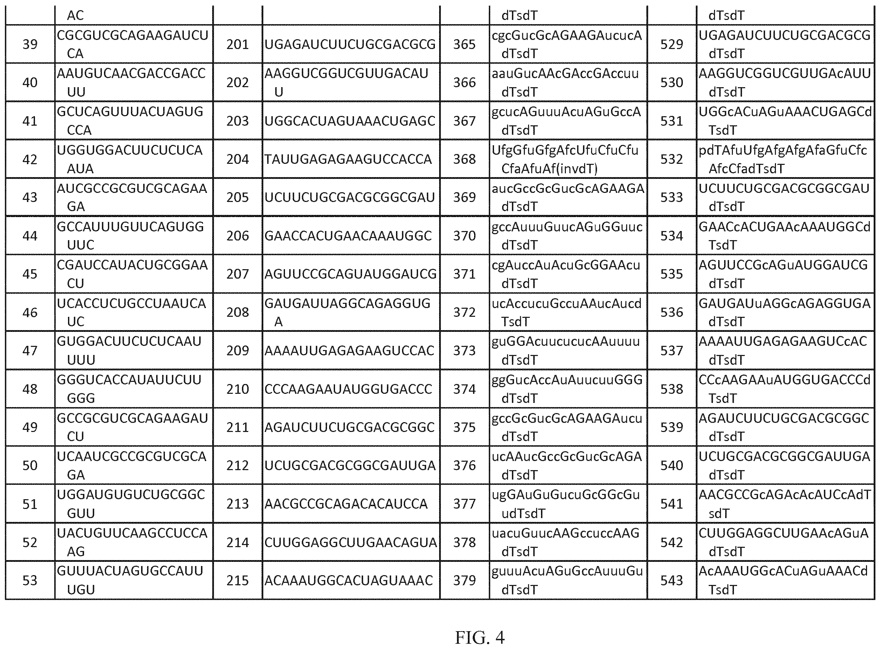

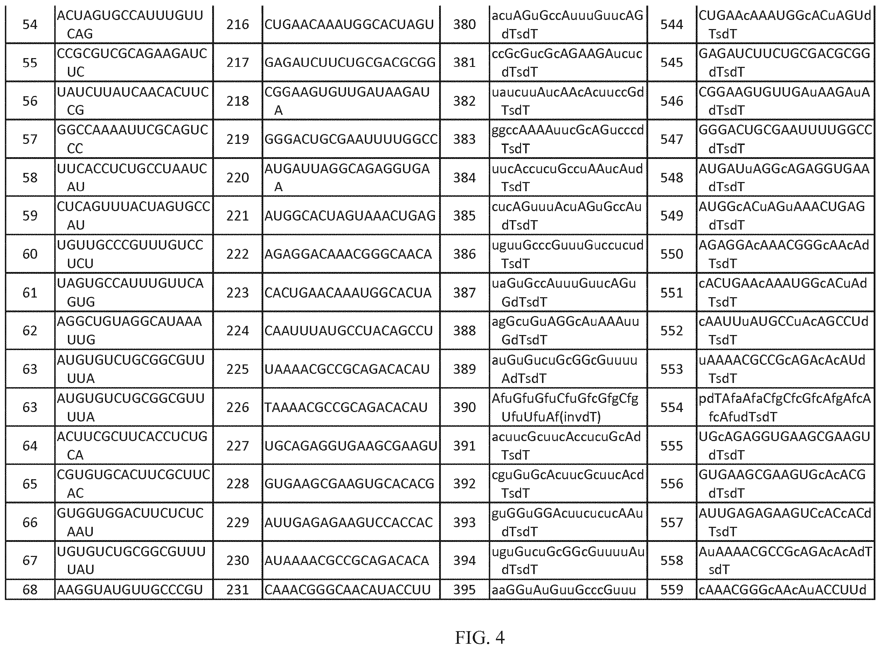

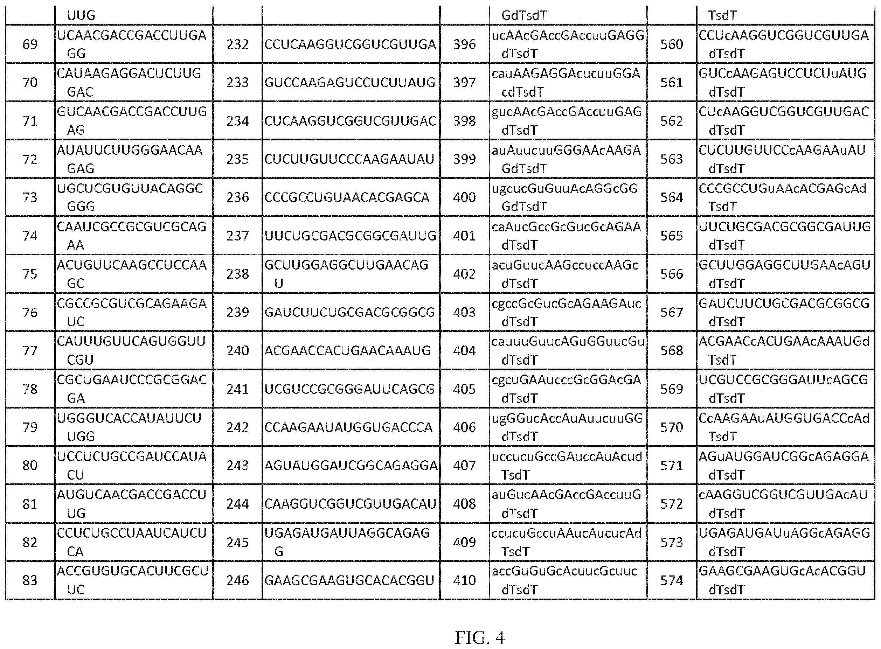

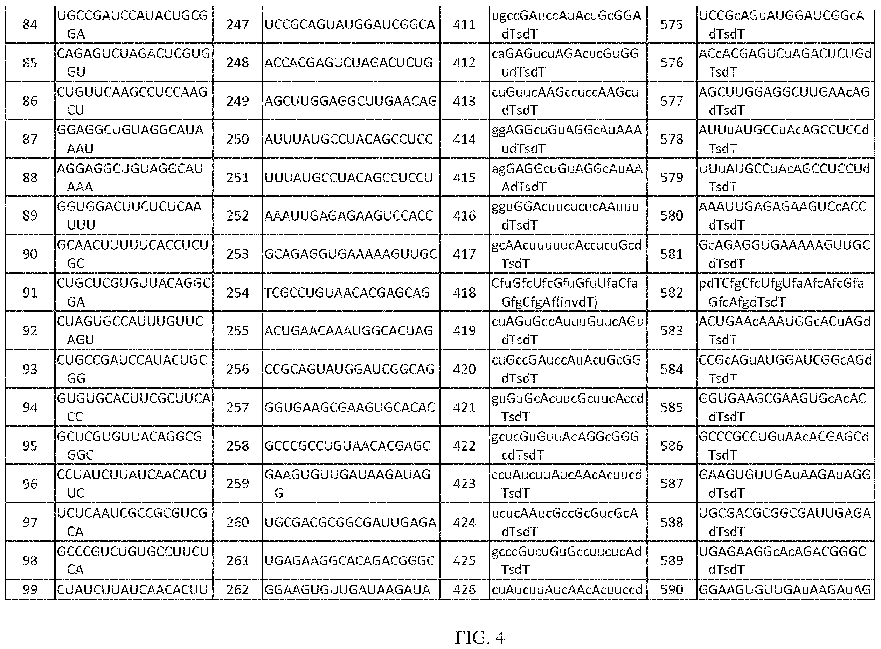

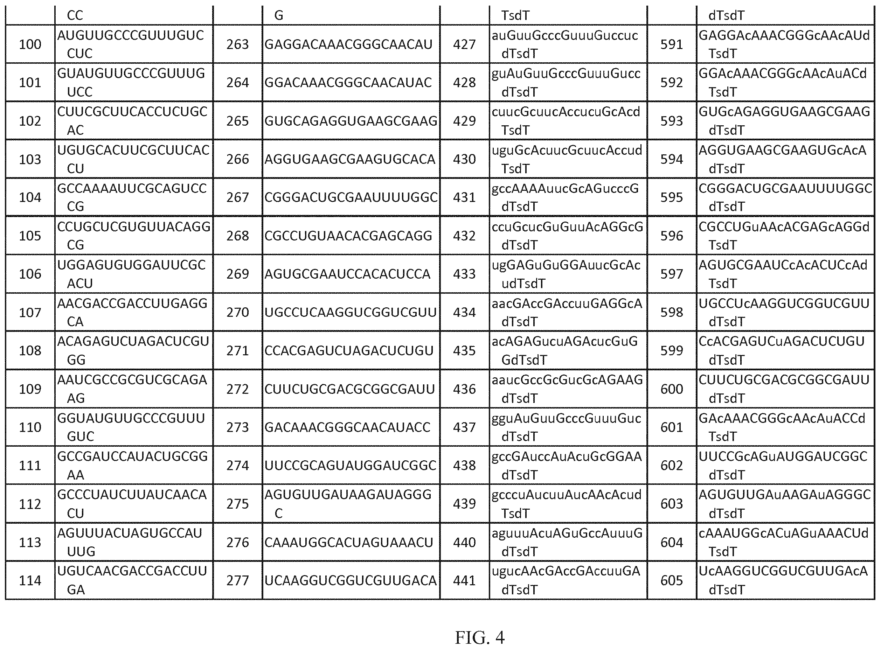

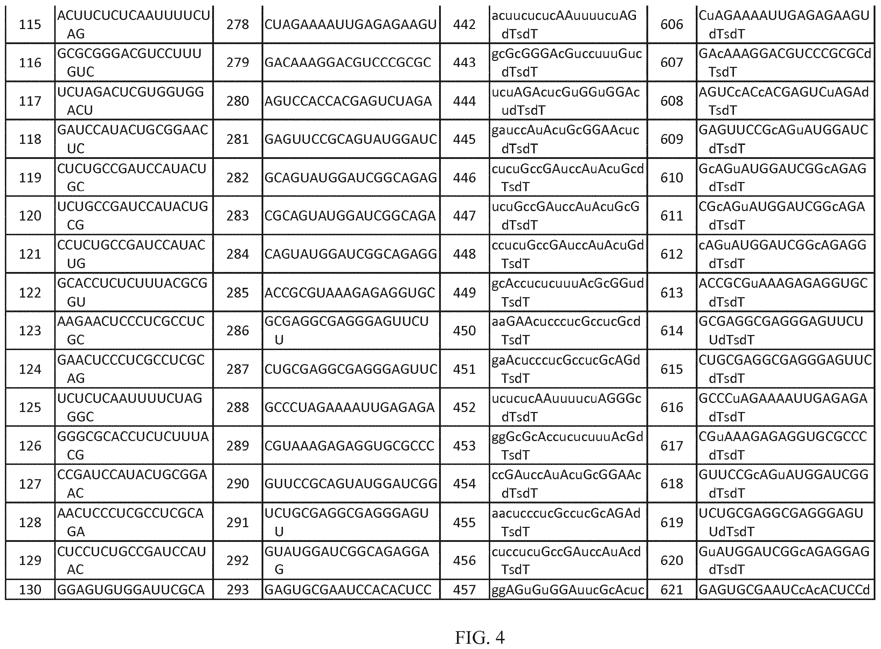

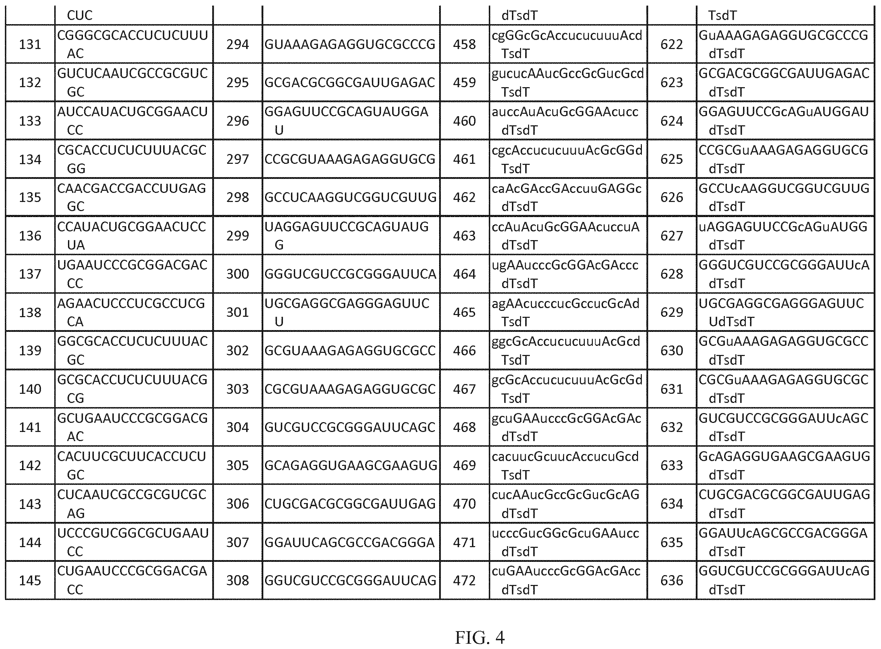

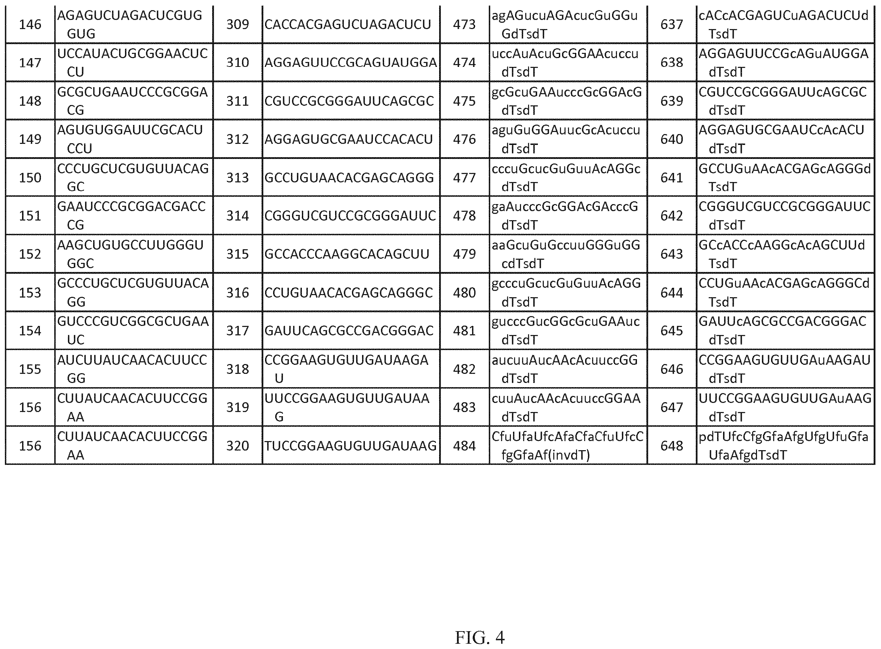

- FIG. 4. Table 4. Core sequences of dsRNAs targeting Hepatitis B Virus gene and their modified counterparts. Letters in capitals represent RNA nucleotides, lower case letters "c", "g", "a" and "u" represent 2' O-methyl-modified nucleotides, "s" represents phosphorothioate, "dT" represents deoxythymidine, upper case letters A, C, G, U followed by "f" indicates a 2'-fluoro nucleotide. Lower case "p" indicates a 5'-phosphate. (invdT) represents an inverted deoxythimidine (3'-3'-linked).

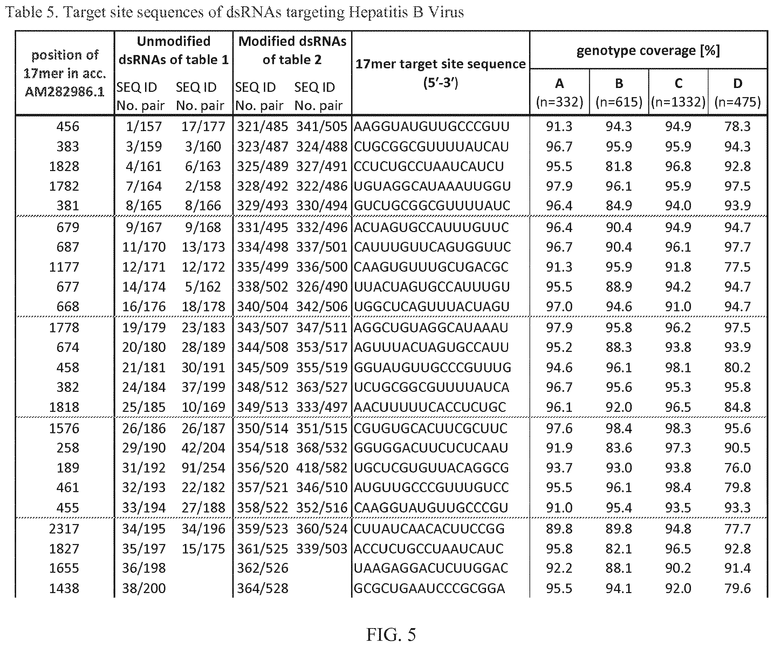

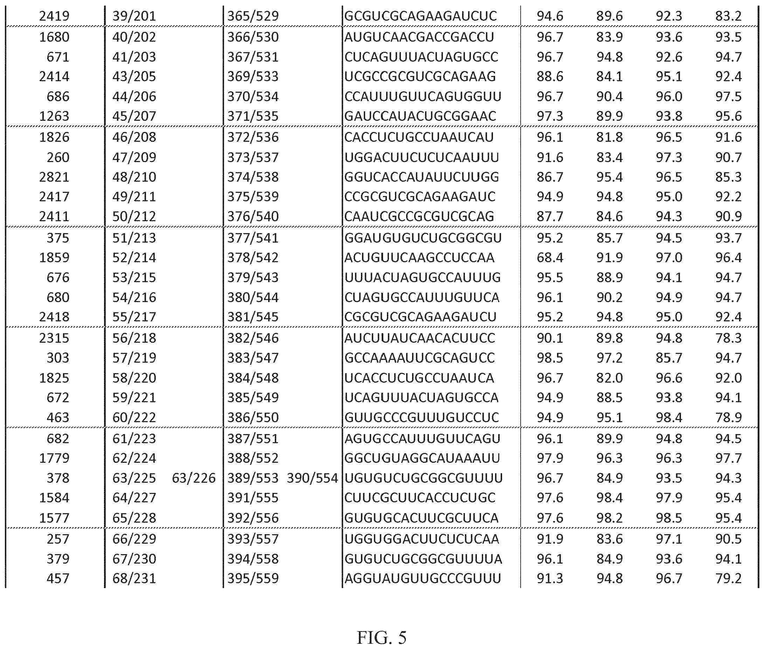

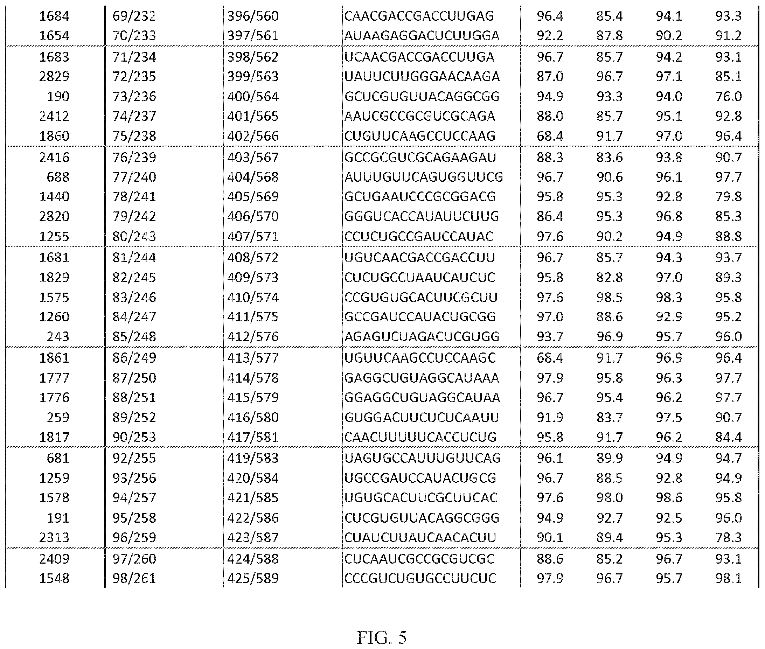

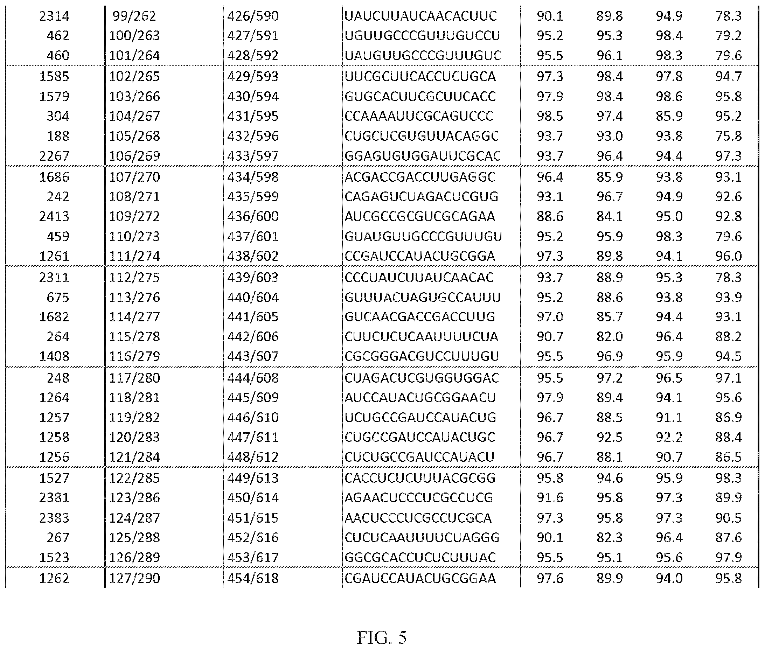

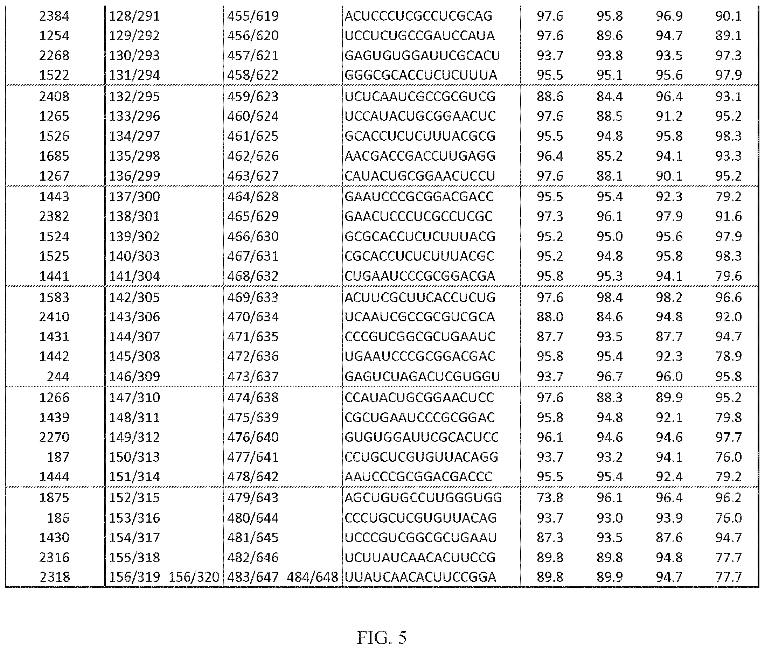

- FIG. 5. Table 5. Target site sequences of dsRNAs targeting Hepatitis B Virus and their coverage rate with respect to Hepatitis B Virus genotypes A, B, C and D. n = number of available HBV sequences of each genotype

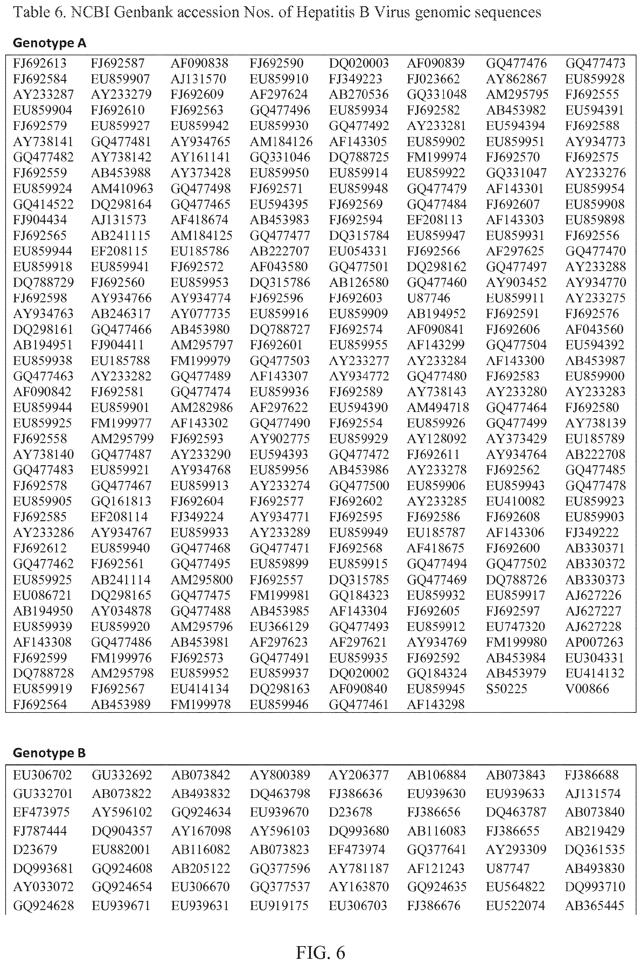

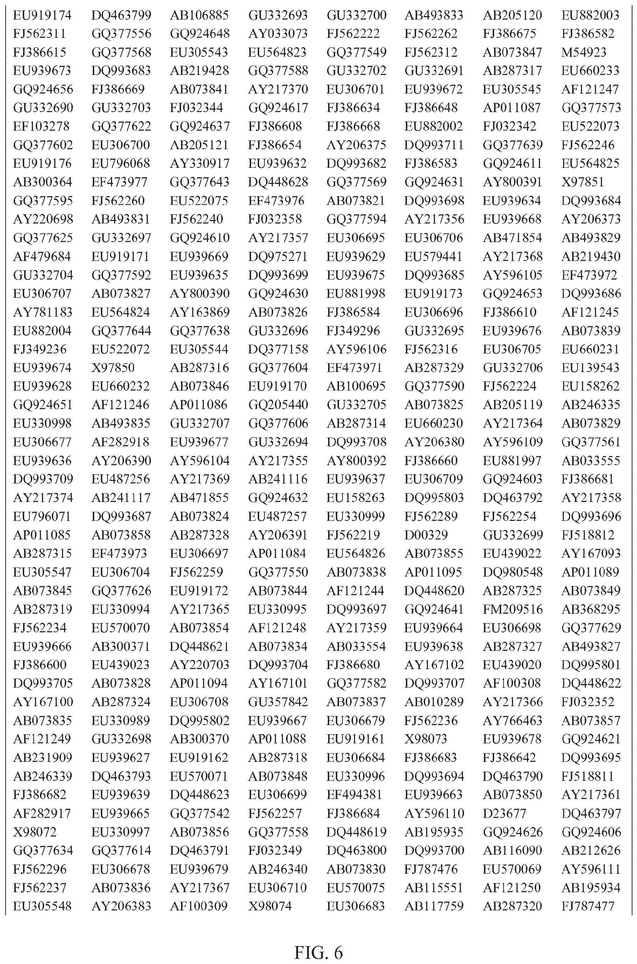

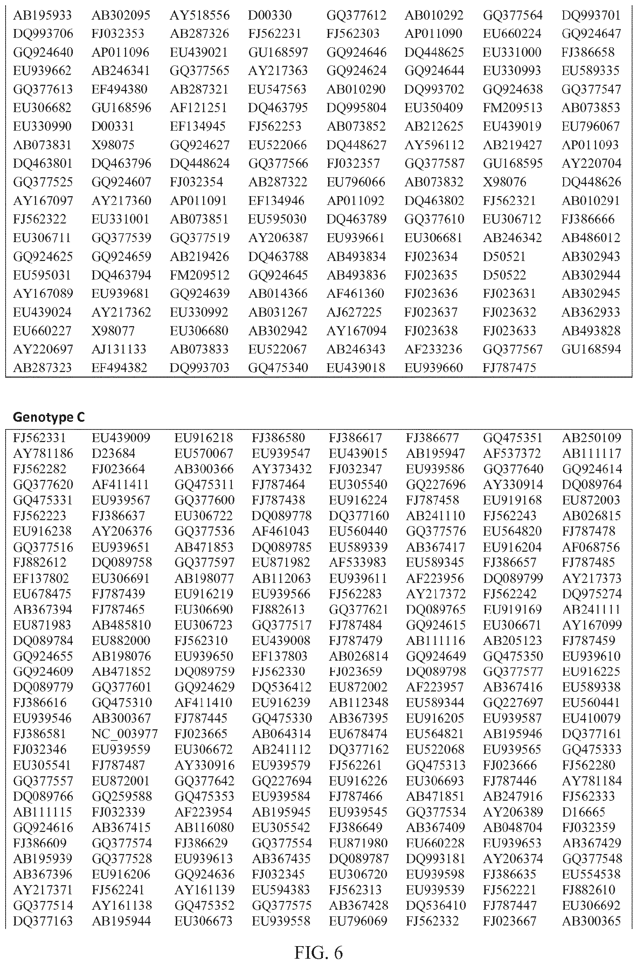

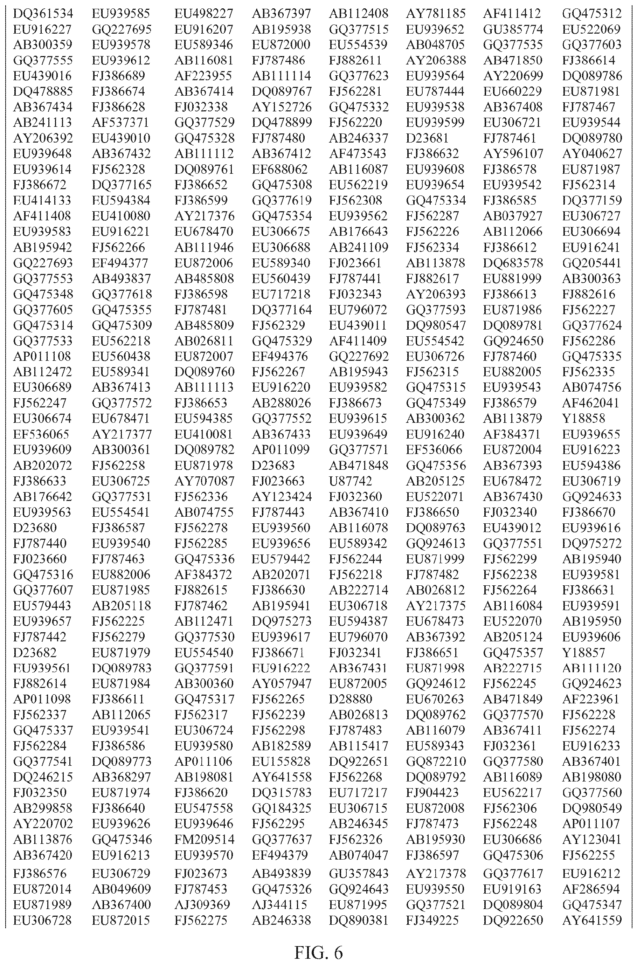

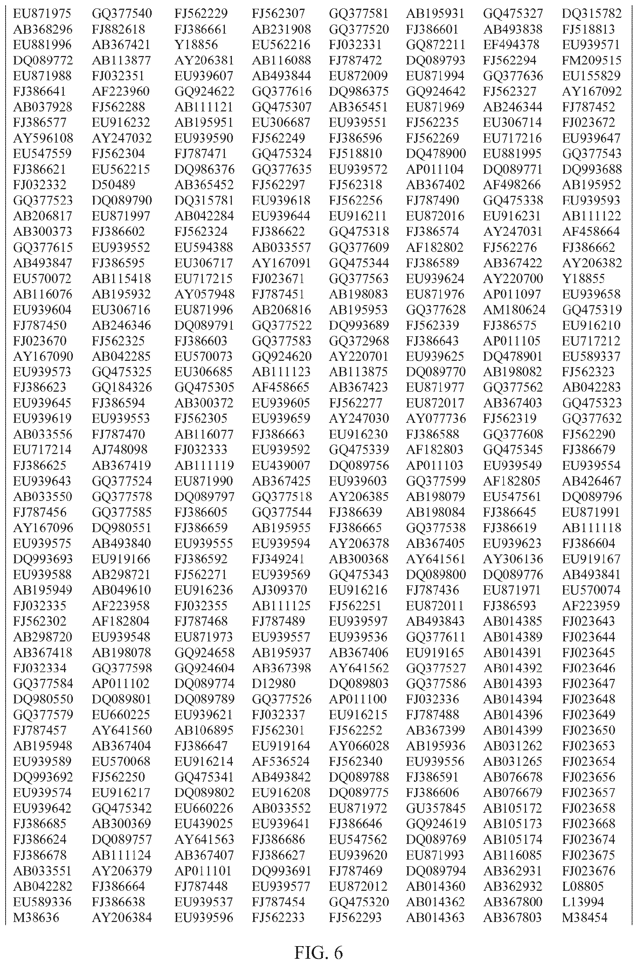

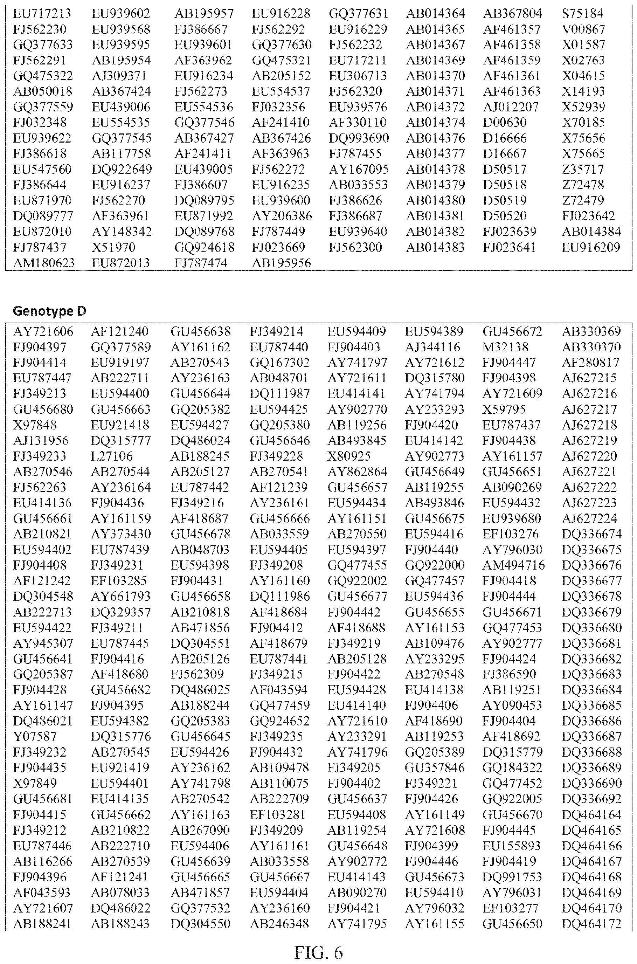

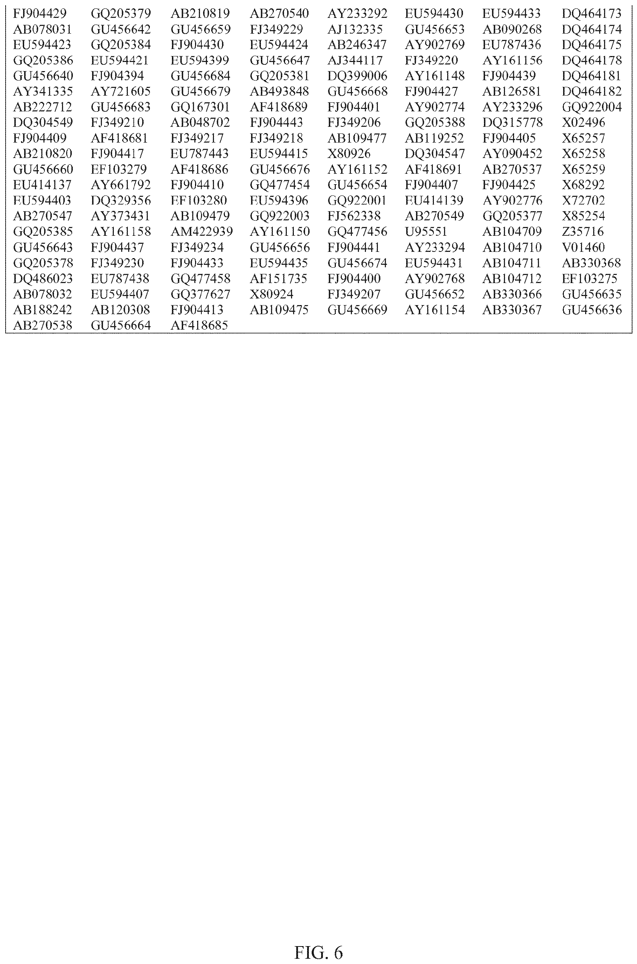

- FIG. 6. Table 6. NCBI Genbank accession Nos. of Hepatitis B Virus genomic sequences.

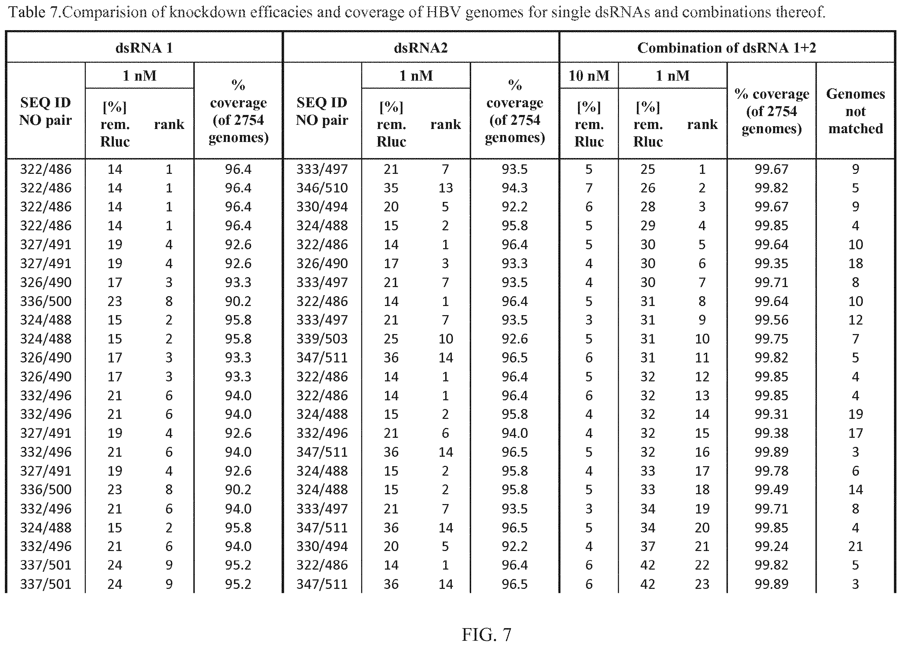

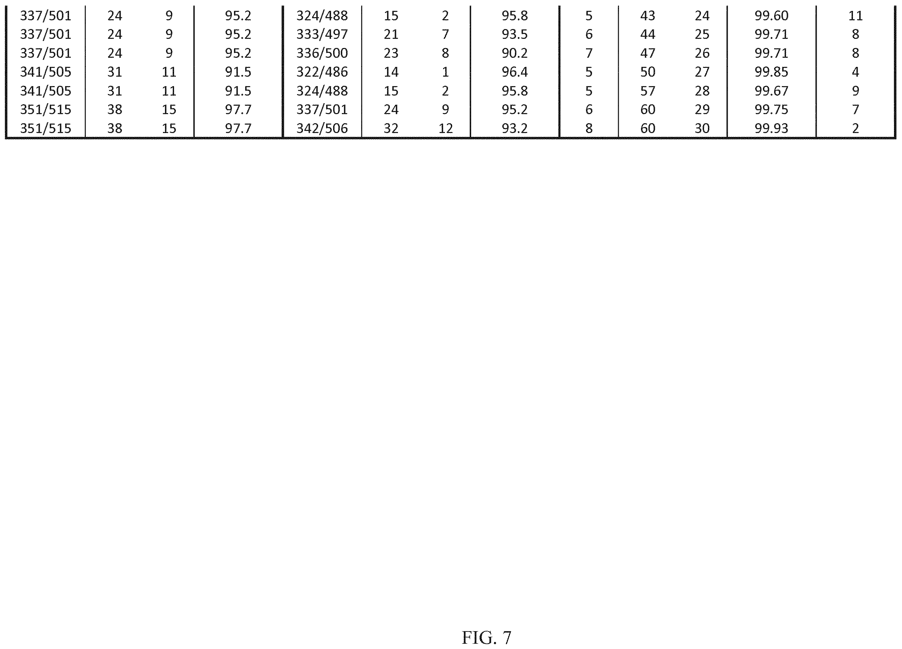

- FIG. 7. Table 7. Comparision of knockdown efficacies and coverage of HBV genomes for single dsRNAs and combinations thereof. Activity testing for combinations of two dsRNAs was done at final concentrations of 10 nM and at 1 nM with the best performing dsRNAs according to Table 2 and compared with respective data.

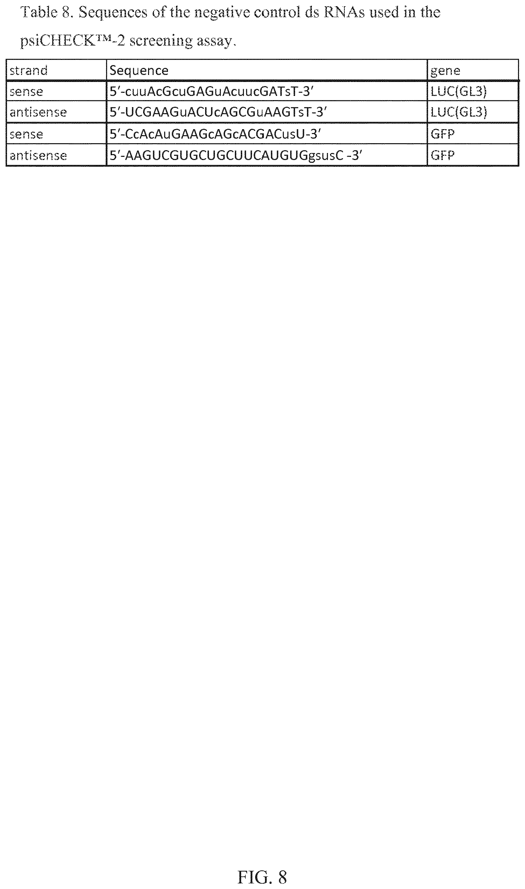

- FIG. 8. Table 8. Sequences of the negative control ds RNAs used in the psiCHECK™-2 screening assay.

## DETAILED DESCRIPTION OF THE INVENTION

Appended Table 1 relates to preferred molecules to be used as dsRNA in accordance with this disclosure. Also modified dsRNA molecules are provided herein and are in particular disclosed in appended Table 2, providing illustrative examples of modified dsRNA molecules of the present disclosure. As pointed out herein above, Table 2 provides for

illustrative examples of modified dsRNAs of this disclosure (whereby the corresponding sense strand and antisense strand is provided in this Table). The relation of the unmodified preferred molecules shown in Table 1 to the modified dsRNAs of Table 2 is illustrated in Table 4. Yet, the illustrative modifications of these constituents of the inventive dsRNAs are provided herein as examples of modifications.

Table 3 provides for selective biological, clinical and pharmaceutical relevant parameters of certain dsRNA molecules of this disclosure.

Some of the preferred dsRNA molecules are provided in the appended Table 1 and, inter alia and preferably, wherein the sense strand is selected from the group consisting of the nucleic acid sequences depicted in SEQ ID NOs: 1, 2, 3, 4, 5, 6, 7, and 26. The antisense strand is selected from the group consisting of the nucleic acid sequences depicted in SEQ ID NOs: 157, 158, 160, 161, 162, 163, 164, and 186. Accordingly, the inventive dsRNA molecule may, inter alia, comprise the sequence pairs selected from the group consisting of SEQ ID NOs: 1/157, 2/158, 3/160, 4/161, 5/162, 6/163, 7/164, and 26/186. In the context of specific dsRNA molecules provided herein, pairs of SEQ ID NOs relate to corresponding sense and antisense strands sequences (5' to 3') as also shown in the Tables.

In one embodiment the dsRNA molecules comprise an antisense strand with a 3' overhang of 1-5 nucleotides in length, preferably 1-2 nucleotides in length. Preferably said overhang of the antisense strand comprises uracil or nucleotides which are complementary to the mRNA encoding a protein necessary for replication or pathogenesis of Hepatitis B Virus, in particular core protein, viral polymerase, surface antigen, e-antigen and X protein. In another preferred embodiment, said dsRNA molecules comprise a sense strand with a 3' overhang of 1-5 nucleotides in length, preferably 1-2 nucleotides in length. Preferably said overhang of the sense strand comprises uracil or nucleotides which are identical to the mRNA encoding a protein necessary for replication or pathogenesis of Hepatitis B Virus.

In another preferred embodiment, the dsRNA molecules comprise a sense strand with a 3' overhang of 1-5 nucleotides in length, preferably 1-2 nucleotides in length, and an antisense strand with a 3' overhang of 1-5 nucleotides in length, preferably 1-2 nucleotides in length. Preferably said overhang of the sense strand comprises uracil or nucleotides which are at least 90% identical to the pregenomic RNA and/or the mRNA encoding the protein necessary for replication or pathogenesis of Hepatitis B Virus and said overhang of the antisense strand comprises uracil or nucleotides which are at least 90% complementary to the mRNA encoding the protein necessary for replication or pathogenesis of Hepatitis B Virus.

The dsRNA molecules of the invention may be comprised of naturally occurring nucleotides or may be comprised of at least one modified nucleotide, such as a 2'-O-methyl modified nucleotide, inverted deoxythymidine, a nucleotide comprising a 5'-phosphorothioate group, and a terminal nucleotide linked to a cholesteryl derivative or dodecanoic acid bisdecylamide group. 2' modified nucleotides may have the additional advantage that certain immunostimulatory factors or cytokines are suppressed when the inventive dsRNA molecules are employed *in vivo,* for example in a medical setting. Alternatively and non-limiting, the modified nucleotide may be chosen from the group of: a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide. In one preferred embodiment the dsRNA molecules comprises at least one of the following modified nucleotides: a 2'-O-methyl modified nucleotide, a nucleotide comprising a 5'-phosphorothioate group and a deoxythymidine. Preferred dsRNA molecules comprising modified nucleotides are given in Table 2. In another preferred embodiment one of those deoxythymidine nucleotides at the 3' of both strand is an inverted deoxythymidine.

In a preferred embodiment the inventive dsRNA molecules comprise modified nucleotides as detailed in the sequences given in Table 2. In one preferred embodiment the inventive dsRNA molecule comprises sequence pairs selected from the group consisting of SEQ ID NOs: 1/157, 2/158, 3/160, 4/161, 5/162, 6/163, 7/164, and 26/186, and comprises overhangs at the antisense and/or sense strand of 1-2 deoxythymidines.

In one preferred embodiment the inventive dsRNA molecule comprises sequence pairs selected from the group consisting of SEQ ID NOs: 1/157, 2/158, 3/160, 4/161, 5/162, 6/163, 7/164, and 26/186, and comprise modifications as detailed in Table 2. Preferred dsRNA molecules comprising modified nucleotides are listed in Table 2-4, with the most preferred dsRNA molecules depicted in SEQ ID Nos: 321/485, 322/486, 324/488, 325/489, 326/490, 327/491, 328/492, and 350/514.

In another embodiment, the inventive dsRNAs comprise modified nucleotides on positions different from those disclosed in Table 2. In one preferred embodiment two deoxythymidine nucleotides are found at the 3' of both strands of the dsRNA molecule. Preferably said deoxythymidine nucleotides form an overhang.

In one embodiment the dsRNA molecules of the disclosure comprise a sense and an antisense strand wherein both strands have a half-life of at least 0.9 h. In one preferred embodiment the dsRNA molecules of the disclosure comprise a sense and an antisense strand wherein both strands have a half-life of at least 48 h, preferably in human serum.

In another embodiment, a nucleic acid sequence encoding a sense strand and/or an antisense strand comprised in the dsRNAs as defined herein are provided.

The disclosure also provides for cells comprising at least one of the dsRNAs of the invention. The cell is preferably a mammalian cell, such as a human cell. Furthermore, tissues and/or non-human organisms comprising the

herein defined dsRNA molecules are an embodiment of this disclosure, whereby said non-human organisms are particularly useful for research purposes or as research tools, for example in drug testing.

Furthermore, the disclosure relates to a method for inhibiting the expression of a Hepatitis B Virus gene, in particular a Hepatitis B Virus gene that encodes core protein, viral polymerase, surface antigen, e-antigen or the X protein, in a cell, tissue or organism comprising the following steps:

(a) introducing into the cell, tissue or organism a double-stranded ribonucleic acid (dsRNA) as defined herein; and
(b) maintaining said cell, tissue or organism produced in step (a) for a time sufficient to obtain degradation of the mRNA transcript of a Hepatitis B Virus gene, thereby inhibiting expression of a Hepatitis B Virus gene in a given cell.

The invention also relates to pharmaceutical compositions comprising at least one kind of the inventive dsRNAs. These pharmaceutical compositions are particularly useful in the inhibition of the expression of a Hepatitis B Virus gene in a cell, a tissue or an organism.

Preferably said at least one kind of the inventive double-stranded ribonucleic acid molecules target the region of a pregenomic RNA and/or a mRNA encoding a protein necessary for replication or pathogenesis of Hepatitis B Virus gene. More preferably said target region of the inventive double-stranded ribonucleic acid molecules comprises a consensus sequence which is highly conserved among the Hepatitis B Virus genomic sequences of genotype A, B, C and D, and said consensus sequence is at least 13 contiguous nucleotides in length, preferably at least 17 contiguous nucleotides, most preferably at least 19 contiguous nucleotides. Preferred highly conserved nucleic acid sequences are listed in Table 5. The pharmaceutical compositions may be used to treat patients who are infected with any genotype of Hepatitis B Virus or co-infected with different genotypes of Hepatitis B Virus.

In case the pharmaceutical composition comprises at least two kinds of inventive double- stranded ribonucleic acid molecules, it is preferable that the targets of said double-stranded ribonucleic acid molecules are different from each other. The inventive pharmaceutical compositions may be used to treat the patients and to prevent the Hepatitis B Virus from developing resistance to the pharmaceutical compositions. In a preferred embodiment the inventive pharmaceutical compositions comprise the combination of dsRNA molecules as detailed in the sequences given in Table 7. In one preferred embodiment the inventive pharmaceutical compositions comprise combinations of dsRNA pairs selected from the group consisting of SEQ ID NOs: 322/486 and 333/497, 322/486 and 346/510, 322/486 and 330/494, and, 322/486 and 324/488.

The pharmaceutical compositions described above may also comprise (a) pharmaceutically acceptable carrier(s), diluent(s) and/or excipient(s).

In another embodiment, the disclosure provides methods for treating, preventing or managing chronic liver diseases/disorders, inflammations, fibrotic conditions and/or proliferative disorders like cancers which are associated with Hepatitis B Virus, said method comprising administering to a subject in need of such treatment, prevention or management a therapeutically or prophylactically effective amount of one or more of the dsRNAs of the invention. Preferably, said subject is a mammal, most preferably a human patient.

In one embodiment, the disclosure provides a method for treating a subject having a pathological condition mediated by the infection of a Hepatitis B Virus. Such conditions comprise disorders associated with chronic liver diseases/disorders, inflammations, fibrotic conditions and/or proliferative disorders like cancers, as described above. In this embodiment, the dsRNA acts as a therapeutic agent for controlling the expression of a Hepatitis B Virus gene. The method comprises administering a pharmaceutical composition of the invention to the patient (e.g., human), such that expression of a Hepatitis B Virus gene is silenced. Because of their high specificity, the dsRNAs of the invention specifically target mRNAs of a Hepatitis B Virus gene. In one preferred embodiment the described dsRNAs specifically decrease Hepatitis B Virus mRNA levels and do not directly affect the expression and/or mRNA levels of off- target genes in the cell.

In one preferred embodiment the described dsRNA decrease Hepatitis B Virus mRNA levels in the liver by at least 60%, preferably by at least 70%, most preferably by at least 80% *in vivo.* In another embodiment the described dsRNAs decrease Hepatitis B Virus mRNA levels *in vivo* for at least 4 days. In another preferred embodiment, the dsRNAs of the invention are used for the preparation of a pharmaceutical composition for the treatment of chronic liver diseases/disorders, inflammations, fibrotic conditions and proliferative disorders like cancers. Such diseases to be treated with said pharmaceutical composition comprise but are not limited to: chronic hepatitis (CH), hepatic cirrhosis (HC) and hepatocellular carcinoma (HCC).

In another embodiment, the disclosure provides vectors for inhibiting the expression of a Hepatitis B Virus gene in a cell, in particular a Hepatitis B Virus gene comprising a regulatory sequence operably linked to a nucleotide sequence that encodes at least one strand of the dsRNA molecules of the invention.

In another embodiment, the disclosure provides a cell comprising a vector for inhibiting the expression of a Hepatitis B Virus gene in a cell. Said vector comprises a regulatory sequence operably linked to a nucleotide sequence that encodes at least one strand of the dsRNA molecule of the invention. Yet, it is preferred that said vector comprises, besides said regulatory sequence a sequence that encodes at least one "sense strand" of the inventive dsRNA and at

least one "antisense strand" of said dsRNA. It is also envisaged that the claimed cell comprises two or more vectors comprising, besides said regulatory sequences, the herein defined sequence(s) that encode(s) at least one strand of the dsRNA molecules of the invention.

In one embodiment, the method comprises administering a composition comprising a dsRNA, wherein the dsRNA comprises a nucleotide sequence which is complementary to at least a part of an RNA transcript of a Hepatitis B Virus gene of the mammal to be treated. As pointed out above, also vectors and cells comprising nucleic acid molecules that encode for at least one strand of the herein defined dsRNA molecules can be used as pharmaceutical compositions and may, therefore, also be employed in the herein disclosed methods of treating a subject in need of medical intervention. It is also of note that these embodiments relating to pharmaceutical compositions and to corresponding methods of treating a (human) subject also relate to approaches like gene therapy approaches.

Hepatitis B Virus specific dsRNA molecules as provided herein or nucleic acid molecules encoding individual strands of these inventive dsRNA molecules may also be inserted into vectors and used as gene therapy vectors for human patients. Gene therapy vectors can be delivered to a subject by, for example, intravenous injection, local administration (see U.S. Patent 5,328,470) or by stereotactic injection (see e.g., Chen et al. Proc. Natl. Acad. Sci. USA (1994) 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

In another aspect of the disclosure, Hepatitis B Virus specific dsRNA molecules that modulate Hepatitis B Virus gene expression activity are expressed from transcription units inserted into DNA or RNA vectors (see, e.g., Skillern A et al., International PCT Publication No. WO 00/22113). These transgenes can be introduced as a linear construct, a circular plasmid, or a viral vector, which can be incorporated and inherited as a transgene integrated into the host genome. The transgene can also be constructed to permit it to be inherited as an extrachromosomal plasmid (Gassmann et al., Proc. Natl. Acad. Sci. USA (1995) 92:1292).

The individual strands of a dsRNA can be transcribed by promoters on two separate expression vectors and co-transfected into a target cell. Alternatively each individual strand of the dsRNA can be transcribed by promoters both of which are located on the same expression plasmid. In a preferred embodiment, a dsRNA is expressed as an inverted repeat joined by a linker polynucleotide sequence such that the dsRNA has a stem and loop structure.

The recombinant dsRNA expression vectors are preferably DNA plasmids or viral vectors. dsRNA expressing viral vectors can be constructed based on, but not limited to, adeno- associated virus (for a review, see Muzyczka et al., Curr. Topics Micro. Immunol. (1992) 158:97-129); adenovirus (see, for example, Berkner et al., BioTechniques (1998) 6:616; Rosenfeld et al. Science (1991) 252:431-434; and Rosenfeld et al. Cell (1992) 68:143-155); or alphavirus as well as others known in the art. Retroviruses have been used to introduce a variety of genes into many different cell types, including epithelial cells, *in vitro* and/or *in vivo* (see, e.g., Danos and Mulligan, Proc. Natl. Acad. Sci. USA (1998) 85:6460-6464). Recombinant retroviral vectors capable of transducing and expressing genes inserted into the genome of a cell can be produced by transfecting the recombinant retroviral genome into suitable packaging cell lines such as PA317 and Psi-CRIP (Comette et al., Human Gene Therapy (1991) 2:5-10; Cone et al., Proc. Natl. Acad. Sci. USA (1984) 81:6349). Recombinant adenoviral vectors can be used to infect a wide variety of cells and tissues in susceptible hosts (e.g., rat, hamster, dog, and chimpanzee) (Hsu et al., J. Infectious Disease, (1992) 166:769), and also have the advantage of not requiring mitotically active cells for infection.

The promoter driving dsRNA expression in either a DNA plasmid or viral vector of the disclosure may be a eukaryotic RNA polymerase I (e.g. ribosomal RNA promoter), RNA polymerase II (e.g. CMV early promoter or actin promoter or U1 snRNA promoter) or preferably RNA polymerase III promoter (e.g. U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the pancreas (see, e.g. the insulin regulatory sequence for pancreas (Bucchini et al., Proc. Natl. Acad. Sci. USA (1986) 83:2511-2515).

In addition, expression of the transgene can be precisely regulated, for example, by using an inducible regulatory sequence and expression systems such as a regulatory sequence that is sensitive to certain physiological regulators, e.g., circulating glucose levels, or hormones (Docherty et al., FASEB J. (1994) 8:20-24). Such inducible expression systems, suitable for the control of transgene expression in cells or in mammals include regulation by ecdysone, by estrogen, progesterone, tetracycline, chemical inducers of dimerization, and isopropyl-beta-D1 - thiogalactopyranoside (IPTG). A person skilled in the art would be able to choose the appropriate regulatory/promoter sequence based on the intended use of the dsRNA transgene.

Preferably, recombinant vectors capable of expressing dsRNA molecules are delivered as described below, and persist in target cells. Alternatively, viral vectors can be used that provide for transient expression of dsRNA molecules. Such vectors can be repeatedly administered as necessary. Once expressed, the dsRNAs bind to target RNA and modulate its function or expression. Delivery of dsRNA expressing vectors can be systemic, such as by intravenous or intramuscular administration, by administration to target cells ex-planted from the patient followed by reintroduction into

the patient, or by any other means that allows for introduction into a desired target cell.

dsRNA expression DNA plasmids are typically transfected into target cells as a complex with cationic lipid carriers (e.g. Oligofectamine) or non-cationic lipid-based carriers (e.g. Transit-TKO™). Multiple lipid transfections for dsRNA-mediated knockdowns targeting different regions of a single Hepatitis B Virus gene or multiple Hepatitis B Virus genes over a period of a week or more are also contemplated by the disclosure. Successful introduction of the vectors of the disclosure into host cells can be monitored using various known methods. For example, transient transfection can be signaled with a reporter, such as a fluorescent marker, such as Green Fluorescent Protein (GFP). Stable transfection of ex *vivo* cells can be ensured using markers that provide the transfected cell with resistance to specific environmental factors (e.g., antibiotics and drugs), such as hygromycin B resistance.

The following detailed description discloses how to make and use the dsRNA and compositions containing dsRNA to inhibit the expression of a target Hepatitis B Virus gene, as well as compositions and methods for treating diseases and disorders caused by the infection of said Hepatitis B Virus.

Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A", "U" and "T" or "dT" respectively, each generally stand for a nucleotide that contains guanine, cytosine, adenine, uracil and deoxythymidine as a base, respectively. However, the term "ribonucleotide" or "nucleotide" can also refer to a modified nucleotide, as further detailed below, or a surrogate replacement moiety. Sequences comprising such replacement moieties are embodiments of the invention. As detailed below, the herein described dsRNA molecules may also comprise "overhangs", i.e. unpaired, overhanging nucleotides which are not directly involved in the RNA double helical structure normally formed by the herein defined pair of "sense strand" and "antisense strand". Often, such an overhanging stretch comprises the deoxythymidine nucleotide, in most embodiments, two deoxythymidines in the 3' end. Such overhangs will be described and illustrated below.

The term "Hepatitis B Virus gene" as used herein relates to the genes necessary for replication and pathogenesis of Hepatitis B Virus, in particular to the genes that encode core protein, viral polymerase, surface antigen, e-antigen and the X protein and the genes that encode the functional fragments of the same. The term "Hepatitis B Virus gene/sequence" does not only relate to (the) wild-type sequence(s) but also to mutations and alterations which may be comprised in said gene/sequence. Accordingly, the present disclosure is not limited to the specific dsRNA molecules provided herein. The disclosure also relates to dsRNA molecules that comprise an antisense strand that is at least 85% complementary to the corresponding nucleotide stretch of an RNA transcript of a Hepatitis B Virus gene that comprises such mutations/alterations.

As used herein, the term "consensus sequence" refers to at least 13 contiguous nucleotides, preferably at least 17 contiguous nucleotides, most preferably at least 19 contiguous nucleotides, which is highly conserved among the Hepatitis B Virus genomic sequences of genotype A, B, C and D.

As used herein, "target sequence" refers to a contiguous portion of the nucleotide sequence of an mRNA molecule formed during the transcription of a Hepatitis B Virus gene, including mRNA that is a product of RNA processing of a primary transcription product.

As used herein, the term "strand comprising a sequence" refers to an oligonucleotide comprising a chain of nucleotides that is described by the sequence referred to using the standard nucleotide nomenclature. However, as detailed herein, such a "strand comprising a sequence" may also comprise modifications, like modified nucleotides.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence. "Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled.

Sequences referred to as "fully complementary" comprise base-pairing of the oligonucleotide or polynucleotide comprising the first nucleotide sequence to the oligonucleotide or polynucleotide comprising the second nucleotide sequence over the entire length of the first and second nucleotide sequence.

However, where a first sequence is referred to as "substantially complementary" with respect to a second sequence herein, the two sequences can be fully complementary, or they may form one or more, but preferably not more than 13 mismatched base pairs upon hybridization.

The terms "complementary", "fully complementary" and "substantially complementary" herein may be used with respect to the base matching between the sense strand and the antisense strand of a dsRNA, or between the antisense strand of a dsRNA and a target sequence, as will be understood from the context of their use.

The term "double-stranded RNA", "dsRNA molecule", or "dsRNA", as used herein, refers to a ribonucleic acid molecule, or complex of ribonucleic acid molecules, having a duplex structure comprising two anti-parallel and substantially complementary nucleic acid strands. The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5' end of the respective other strand forming the duplex structure, the connecting RNA chain is referred to as a "hairpin loop". Where the two strands are connected covalently by means other than an uninterrupted chain of nucleotides between the 3'-end of one strand and the 5' end of the respective other strand forming the duplex structure, the connecting structure is referred to as a "linker". The RNA strands may have the same or a different number of nucleotides. In addition to the duplex structure, a dsRNA may comprise one or more nucleotide overhangs. The nucleotides in said "overhangs" may comprise between 0 and 5 nucleotides, whereby "0" means no additional nucleotide(s) that form(s) an "overhang" and whereas "5" means five additional nucleotides on the individual strands of the dsRNA duplex. These optional "overhangs" are located in the 3' end of the individual strands. As will be detailed below, also dsRNA molecules which comprise only an "overhang" in one of the two strands may be useful and even advantageous in context of this invention. The "overhang" comprises preferably between 0 and 2 nucleotides. Most preferably two "dT" (deoxythymidine) nucleotides are found at the 3' end of both strands of the dsRNA. Also two "U"(uracil) nucleotides can be used as overhangs at the 3' end of both strands of the dsRNA. Accordingly, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. For example the antisense strand comprises 23 nucleotides and the sense strand comprises 21 nucleotides, forming a two nucleotide overhang at the 3' end of the antisense strand. Preferably, the two nucleotide overhang is fully complementary to the mRNA of the target gene. "Blunt" or "blunt end" means that there are no unpaired nucleotides at that end of the dsRNA, i.e., no nucleotide overhang. A "blunt ended" dsRNA is a dsRNA that is double-stranded over its entire length, i.e., no nucleotide overhang at either end of the molecule.

The term "antisense strand" refers to the strand of a dsRNA which includes a region that is substantially complementary to a target sequence. As used herein, the term "region of complementarity" refers to the region on the antisense strand that is substantially complementary to a sequence, for example a target sequence. Where the region of complementarity is not fully complementary to the target sequence, the mismatches are most tolerated outside nucleotides 2-7 of the 5' terminus of the antisense strand

The term "sense strand," as used herein, refers to the strand of a dsRNA that includes a region that is substantially complementary to a region of the antisense strand. "Substantially complementary" means preferably at least 85% of the overlapping nucleotides in sense and antisense strand are complementary.

"Introducing into a cell", when referring to a dsRNA, means facilitating uptake or absorption into the cell, as is understood by those skilled in the art. Absorption or uptake of dsRNA can occur through unaided diffusive or active cellular processes, or by auxiliary agents or devices. The meaning of this term is not limited to cells *in vitro;* a dsRNA may also be "introduced into a cell", wherein the cell is part of a living organism. In such instance, introduction into the cell will include the delivery to the organism. For example, for *in vivo* delivery, dsRNA can be injected into a tissue site or administered systemically. It is, for example envisaged that the dsRNA molecules of this invention be administered to a subject in need of medical intervention. Such an administration may comprise the injection of the dsRNA of the invention or the vector or a cell of this disclosure into a diseased site in said subject, for example into liver tissue/cells or into cancerous tissues/cells, like liver cancer tissue. In addition, the injection is preferably in close proximity to the diseased tissue envisaged. *In vitro* introduction into a cell includes methods known in the art such as electroporation and lipofection.

As used herein, "chronic liver diseases/disorders" refers to the functional abnormality of liver lasting more than six months which can be caused by the infection of virus. One example of the chronic liver diseases/disorders is chronic hepatitis (CH).

The term "inflammation" as used herein refers to the biologic response of body tissue to injury, irritation, or disease which can be caused by harmful stimuli, for example, pathogens, damaged cells, or irritants. Inflammation is typically characterized by pain and swelling. Inflammation is intended to encompass both acute responses, in which inflammatory processes are active (e.g., neutrophils and leukocytes), and chronic responses, which are marked by slow progress, a shift in the type of cell present at the site of inflammation, and the formation of connective tissue. One example of an inflammation-caused disease is fibrosis.

The term "fibrotic conditions" as used herein refers to the functional problem of organs which can be caused by growth of fibrous tissue. One such example of such kind of disease is hepatic cirrhosis (HC).

The term "proliferating" and "proliferation" as used herein refer to cells undergoing mitosis. Throughout this application, the term "proliferative disorder" refers to any disease/disorder marked by unwanted or aberrant proliferation of tissue. As used herein, the term "proliferative disorder" also refers to conditions in which the unregulated and/or abnormal growth of cells can lead to the development of an unwanted condition or disease, which can be cancerous or non-cancerous.

Cancers to be treated comprise, but are again not limited to liver cancer, whereby said liver cancer may, inter alia, be selected from the group consisting of hepatocellular carcinoma (HCC), hepatoblastoma, a mixed liver cancer, a cancer derived from mesenchymal tissue, a liver sarcoma or a cholangiocarcinoma.

The terms "silence", "inhibit the expression of" and "knock down", in as far as they refer to a Hepatitis B Virus gene, herein refer to the at least partial suppression of the expression of a Hepatitis B Virus gene, as manifested by a reduction of the amount of mRNA transcribed from a Hepatitis B Virus gene which may be isolated from a first cell or group of cells in which a Hepatitis B Virus gene is transcribed and which has or have been treated such that the expression of a Hepatitis B Virus gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \times 100\%$$

Alternatively, the degree of inhibition may be given in terms of a reduction of a parameter that is functionally linked to the Hepatitis B Virus gene transcription, e.g. the amount of protein encoded by a Hepatitis B Virus gene which is secreted by a cell, or the number of cells displaying a certain phenotype.

As illustrated in the appended examples and in the appended Tables provided herein, the inventive dsRNA molecules are capable of inhibiting the expression of a Hepatitis B Virus by at least about 60%, preferably by at least 70%, most preferably by at least 80% in *in vitro* assays, i.e. *in vitro.* The term "*in vitro*" as used herein includes but is not limited to cell culture assays. The person skilled in the art can readily determine such an inhibition rate and related effects, in particular in light of the assays provided herein.

The term "off target" as used herein refers to all non-target mRNAs of the transcriptome that are predicted by *in silico* methods to hybridize to the described dsRNAs based on sequence complementarity. The dsRNAs of the present invention preferably do specifically inhibit the expression of Hepatitis B Virus gene, i.e. do not inhibit the expression of any off-target.

The term "half-life" as used herein is a measure of stability of a compound or molecule and can be assessed by methods known to a person skilled in the art, especially in light of the assays provided herein.

The term "non-immunostimulatory" as used herein refers to the absence of any induction of an immune response by the invented dsRNA molecules. Methods to determine immune responses are well known to a person skilled in the art, for example by assessing the release of cytokines, as described in the examples section.

The terms "treat", "treatment", and the like, mean in context of this invention the relief from or alleviation of a disorder related to Hepatitis B Virus infection, like chronic liver diseases/disorders, inflammations, fibrotic conditions and proliferative disorders, like cancers.

As used herein, a "pharmaceutical composition" comprises a pharmacologically effective amount of at least one kind of dsRNAs and a pharmaceutically acceptable carrier. However, such a "pharmaceutical composition" may also comprise individual strands of such dsRNA molecules or the herein described vector(s) comprising a regulatory sequence operably linked to a nucleotide sequence that encodes at least one strand of a sense or an antisense strand comprised in the dsRNAs of this invention. It is also envisaged that cells, tissues or isolated organs that express or comprise the herein defined dsRNAs may be used as "pharmaceutical compositions". As used herein, "pharmacologically effective amount," "therapeutically effective amount," or simply "effective amount" refers to that amount of an RNA effective to produce the intended pharmacological, therapeutic or preventive result.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives as known to persons skilled in the art.

It is in particular envisaged that the pharmaceutically acceptable carrier allows for the systemic administration of the dsRNAs of the invention or vectors or cells of this disclosure. Whereas also the enteric administration is envisaged the parenteral administration and also transdermal or transmucosal (e.g. insufflation, buccal, vaginal, anal) administration as well as inhalation of the drug are feasible ways of administering to a patient in need of medical intervention the compounds of this invention. When parenteral administration is employed, this can comprise the direct injection of the compounds of this invention into the diseased tissue or at least in close proximity. However, also intravenous, intraarterial, subcutaneous, intramuscular, intraperitoneal, intradermal, intrathecal and other administrations of the compounds of this invention are within the skill of the artisan, for example the attending physician.

For intramuscular, subcutaneous and intravenous use, the pharmaceutical compositions of the invention will

generally be provided in sterile aqueous solutions or suspensions, buffered to an appropriate pH and isotonicity. In a preferred embodiment, the carrier consists exclusively of an aqueous buffer. In this context, "exclusively" means no auxiliary agents or encapsulating substances are present which might affect or mediate uptake of dsRNA in the cells that express a Hepatitis B Virus gene. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate. The pharmaceutical compositions useful according to the invention also include encapsulated formulations to protect the dsRNA against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions and bi-specific antibodies can also be used as pharmaceutically acceptable carriers. These can be prepared according to methods known to those skilled in the art, for example, as described in PCT publication WO91/06309 and WO2011/003780.

As used herein, a "transformed cell" is a cell into which at least one vector has been introduced from which a dsRNA molecule or at least one strand of such a dsRNA molecule may be expressed. Such a vector is preferably a vector comprising a regulatory sequence operably linked to nucleotide sequence that encodes at least one sense strand or antisense strand of a dsRNA of the present invention.

It can be reasonably expected that shorter dsRNAs comprising one of the sequences in Table 1 and 4 minus only a few nucleotides on one or both ends may be similarly effective as compared to the dsRNAs described above.

In one preferred embodiment the inventive dsRNA molecules comprise nucleotides 1-19 of the sequences given in Table 1.

As pointed out above, in most embodiments of this invention, the dsRNA molecules provided herein comprise a duplex length (i.e. without "overhangs") of about 16 to about 30 nucleotides. Particular useful dsRNA duplex lengths are about 19 to about 25 nucleotides. Most preferred are duplex structures with a length of 19 nucleotides. In the inventive dsRNA molecules, the antisense strand is at least partially complementary to the sense strand.

The dsRNA of the invention can contain one or more mismatches to the target sequence. In a preferred embodiment, the dsRNA of the invention contains no more than 13 mismatches. If the antisense strand of the dsRNA contains mismatches to a target sequence, it is preferable that the area of mismatch not be located within nucleotides 2-7 of the 5' terminus of the antisense strand. In another embodiment it is preferable that the area of mismatch not be located within nucleotides 2-9 of the 5' terminus of the antisense strand.

As mentioned above, at least one end/strand of the dsRNA may have a single-stranded nucleotide overhang of 1 to 5, preferably 1 or 2 nucleotides. dsRNAs having at least one nucleotide overhang have unexpectedly superior inhibitory properties than their blunt-ended counterparts. Moreover, the present inventors have discovered that the presence of only one nucleotide overhang strengthens the interference activity of the dsRNA, without affecting its overall stability. dsRNA having only one overhang has proven particularly stable and effective *in vivo,* as well as in a variety of cells, cell culture mediums, blood, and serum. Preferably, the single-stranded overhang is located at the 3'-terminal end of the antisense strand or, alternatively, at the 3'-terminal end of the sense strand. The dsRNA may also have a blunt end, preferably located at the 5'-end of the antisense strand. Preferably, the antisense strand of the dsRNA has a nucleotide overhang at the 3'-end, and the 5'-end is blunt. In another embodiment, one or more of the nucleotides in the overhang is replaced with a nucleoside thiophosphate.

The dsRNA of the present invention may also be chemically modified to enhance stability. The nucleic acids of the invention may be synthesized and/or modified by methods well established in the art. Chemical modifications may include, but are not limited to 2' modifications, introduction of non-natural bases, covalent attachment to a ligand, and replacement of phosphate linkages with thiophosphate linkages, inverted deoxythymidines. In this embodiment, the integrity of the duplex structure is strengthened by at least one, and preferably two, chemical linkages. Chemical linking may be achieved by any of a variety of well-known techniques, for example by introducing covalent, ionic or hydrogen bonds; hydrophobic interactions, van der Waals or stacking interactions; by means of metal-ion coordination, or through use of purine analogues. Preferably, the chemical groups that can be used to modify the dsRNA include, without limitation, methylene blue; bifunctional groups, preferably bis-(2-chloroethyl)amine; N-acetyl-N'-(p-glyoxylbenzoyl)cystamine; 4-thiouracil; and psoralen. In one preferred embodiment, the linker is a hexa-ethylene glycol linker. In this case, the dsRNA are produced by solid phase synthesis and the hexa-ethylene glycol linker is incorporated according to standard methods (e.g., Williams DJ and Hall KB, Biochem. (1996) 35:14665-14670). In a particular embodiment, the 5'-end of the antisense strand and the 3'-end of the sense strand are chemically linked via a hexaethylene glycol linker. In another embodiment, at least one nucleotide of the dsRNA comprises a phosphorothioate or phosphorodithioate groups. The chemical bond at the ends of the dsRNA is preferably formed by triple-helix bonds.

In certain embodiments, a chemical bond may be formed by means of one or several bonding groups, wherein such bonding groups are preferably poly-(oxyphosphinicooxy-1,3-propandiol) and/or polyethylene glycol chains. In other embodiments, a chemical bond may also be formed by means of purine analogs introduced into the double-stranded

structure instead of purines. In further embodiments, a chemical bond may be formed by azabenzene units introduced into the double-stranded structure. In still further embodiments, a chemical bond may be formed by branched nucleotide analogs instead of nucleotides introduced into the double- stranded structure. In certain embodiments, a chemical bond may be induced by ultraviolet light.

In yet another embodiment, the nucleotides at one or both of the two single strands may be modified to prevent or inhibit the activation of cellular enzymes, for example certain nucleases. Techniques for inhibiting the activation of cellular enzymes are known in the art including, but not limited to, 2'-amino modifications, 2'-amino sugar modifications, 2'-F sugar modifications, 2'-F modifications, 2'-alkyl sugar modifications, uncharged backbone modifications, morpholino modifications, 2'-O-methyl modifications, and phosphoramidate (see, e.g., Wagner, Nat. Med. (1995) 1:1116-8). Thus, at least one 2'-hydroxyl group of the nucleotides on a dsRNA is replaced by a chemical group, preferably by a 2'-amino or a 2'-methyl group. Also, at least one nucleotide may be modified to form a locked nucleotide. Such locked nucleotide contains a methylene bridge that connects the 2'-oxygen of ribose with the 4'- carbon of ribose. Introduction of a locked nucleotide into an oligonucleotide improves the affinity for complementary sequences and increases the melting temperature by several degrees.

Modifications of dsRNA molecules provided herein may positively influence their stability *in vivo* as well as *in vitro* and also improve their delivery to the (diseased) target side. Furthermore, such structural and chemical modifications may positively influence physiological reactions towards the dsRNA molecules upon administration, e.g. the cytokine release which is preferably suppressed. Such chemical and structural modifications are known in the art and are, inter alia, illustrated in Nawrot Current Topics in Med Chem, (2006) 6:913-925.

Conjugating a ligand to a dsRNA can enhance its cellular absorption as well as targeting to a particular tissue. In certain instances, a hydrophobic ligand is conjugated to the dsRNA to facilitate direct permeation of the cellular membrane. Alternatively, the ligand conjugated to the dsRNA is a substrate for receptor-mediated endocytosis. These approaches have been used to facilitate cell permeation of antisense oligonucleotides. For example, cholesterol has been conjugated to various antisense oligonucleotides resulting in compounds that are substantially more active compared to their non-conjugated analogs (See Manoharan M, Antisense & Nucleic Acid Drug Development (2002) 12:103). Other lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-mediated endocytosis. dsRNA compounds bearing folic acid would be efficiently transported into the cell via the folate-receptor-mediated endocytosis. Attachment of folic acid to the 3'-terminus of an oligonucleotide results in increased cellular uptake of the oligonucleotide (Li S, Deshmukh HM, and Huang L, Pharm. Res. (1998) 15:1540). Other ligands that have been conjugated to oligonucleotides include polyethylene glycols, carbohydrate clusters, cross-linking agents, porphyrin conjugates, and delivery peptides.

In certain instances, conjugation of a cationic ligand to oligonucleotides often results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed throughout the oligonucleotide. See Manoharan M, Antisense & Nucleic Acid Drug Development (2002) 12:103 and references therein.

The ligand-conjugated dsRNA of the invention may be synthesized by the use of a dsRNA that bears a pendant reactive functionality, such as that derived from the attachment of a linking molecule onto the dsRNA. This reactive oligonucleotide may be reacted directly with commercially-available ligands, ligands that are synthesized bearing any of a variety of protecting groups, or ligands that have a linking moiety attached thereto. The methods of the disclosure facilitate the synthesis of ligand-conjugated dsRNA by the use of, in some preferred embodiments, nucleoside monomers that have been appropriately conjugated with ligands and that may further be attached to a solid-support material. Such ligand-nucleoside conjugates, optionally attached to a solid-support material, are prepared according to some preferred embodiments of the methods of the disclosure via reaction of a selected serum-binding ligand with a linking moiety located on the 5' position of a nucleoside or oligonucleotide. In certain instances, a dsRNA bearing an aralkyl ligand attached to the 3'-terminus of the dsRNA is prepared by first covalently attaching a monomer building block to a controlled-pore-glass support via a long-chain aminoalkyl group. Then, nucleotides are bonded via standard solid-phase synthesis techniques to the monomer building-block bound to the solid support. The monomer building block may be a nucleoside or other organic compound that is compatible with solid-phase synthesis.

The dsRNA used in the conjugates of the invention may be conveniently and routinely made through the well-known technique of solid-phase synthesis. It is also known to use similar techniques to prepare other oligonucleotides, such as the phosphorothioates and alkylated derivatives.

Teachings regarding the synthesis of particular modified oligonucleotides may be found in the following U.S. patents: U.S. Pat. No. 5,218,105, drawn to polyamine conjugated oligonucleotides; U.S. Pat. Nos. 5,541,307, drawn to oligonucleotides having modified backbones; U.S. Pat. No. 5,521,302, drawn to processes for preparing oligonucleotides having chiral phosphorus linkages; U.S. Pat. No. 5,539,082, drawn to peptide nucleic acids; U.S. Pat. No. 5,554,746, drawn to oligonucleotides having β-lactam backbones; U.S. Pat. No. 5,571,902, drawn to methods and materials for the

synthesis of oligonucleotides; U.S. Pat. No. 5,578,718, drawn to nucleosides having alkylthio groups, wherein such groups may be used as linkers to other moieties attached at any of a variety of positions of the nucleoside; U.S. Pat. No 5,587,361 drawn to oligonucleotides having phosphorothioate linkages of high chiral purity; U.S. Pat. No. 5,506,351, drawn to processes for the preparation of 2'-O-alkyl guanosine and related compounds, including 2,6-diaminopurine compounds; U.S. Pat. No. 5,587,469, drawn to oligonucleotides having N-2 substituted purines; U.S. Pat. No. 5,587,470, drawn to oligonucleotides having 3-deazapurines; U.S. Pat. No. 5,608,046, both drawn to conjugated 4'-desmethyl nucleoside analogs; U.S. Pat. No. 5,610,289, drawn to backbone-modified oligonucleotide analogs; U.S. Pat. No 6,262,241 drawn to, inter alia, methods of synthesizing 2'-fluoro-oligonucleotides.

In the ligand-conjugated dsRNA and ligand-molecule bearing sequence-specific linked nucleosides of the invention, the oligonucleotides and oligonucleosides may be assembled on a suitable oligonucleotide synthesizer utilizing standard nucleotide or nucleoside precursors, or nucleotide or nucleoside conjugate precursors that already bear the linking moiety, ligand-nucleotide or nucleoside-conjugate precursors that already bear the ligand molecule, or non-nucleoside ligand-bearing building blocks.

When using nucleotide-conjugate precursors that already bear a linking moiety, the synthesis of the sequence-specific linked nucleosides is typically completed, and the ligand molecule is then reacted with the linking moiety to form the ligand-conjugated oligonucleotide. Oligonucleotide conjugates bearing a variety of molecules such as steroids, vitamins, lipids and reporter molecules, has previously been described (see Manoharan et al., PCT Application WO 93/07883). In a preferred embodiment, the oligonucleotides or linked nucleosides of the invention are synthesized by an automated synthesizer using phosphoramidites derived from ligand-nucleoside conjugates in addition to commercially available phosphoramidites.

The incorporation of a 2'-O-methyl, 2'-O-ethyl, 2'-O-propyl, 2'-O-allyl, 2'-O-aminoalkyl or 2'-deoxy-2'-fluoro group in nucleosides of an oligonucleotide confers enhanced hybridization properties to the oligonucleotide. Further, oligonucleotides containing phosphorothioate backbones have enhanced nuclease stability. Thus, functionalized, linked nucleosides of the invention can be augmented to include either or both a phosphorothioate backbone or a 2'-O-methyl, 2'-O-ethyl, 2'-O-propyl, 2'-O-aminoalkyl, 2'-O-allyl or 2'-deoxy-2'-fluoro group.

In some preferred embodiments, functionalized nucleoside sequences of the invention possessing an amino group at the 5'-terminus are prepared using a DNA synthesizer, and then reacted with an active ester derivative of a selected ligand. Active ester derivatives are well known to those skilled in the art. Representative active esters include N-hydrosuccinimide esters, tetrafluorophenolic esters, pentafluorophenolic esters and pentachlorophenolic esters. The reaction of the amino group and the active ester produces an oligonucleotide in which the selected ligand is attached to the 5'-position through a linking group. The amino group at the 5'- terminus can be prepared utilizing a 5'-Amino-Modifier C6 reagent. In a preferred embodiment, ligand molecules may be conjugated to oligonucleotides at the 5'-position by the use of a ligand-nucleoside phosphoramidite wherein the ligand is linked to the 5'-hydroxy group directly or indirectly via a linker. Such ligand-nucleoside phosphoramidites are typically used at the end of an automated synthesis procedure to provide a ligand-conjugated oligonucleotide bearing the ligand at the 5'-terminus.

In one preferred embodiment of the methods of the disclosure, the preparation of ligand conjugated oligonucleotides commences with the selection of appropriate precursor molecules upon which to construct the ligand molecule. Typically, the precursor is an appropriately- protected derivative of the commonly-used nucleosides. For example, the synthetic precursors for the synthesis of the ligand-conjugated oligonucleotides of the disclosure include, but are not limited to, 2'-aminoalkoxy-5'-ODMT-nucleosides, 2'-6-aminoalkylamino-5'-ODMT-nucleosides, 5'-6-aminoalkoxy-2'-deoxy-nucleosides, 5'-6-aminoalkoxy-2-protected-nucleosides, 3'-6- aminoalkoxy-5'-ODMT-nucleosides, and 3'-aminoalkylamino-5'-ODMT-nucleosides that may be protected in the nucleobase portion of the molecule. Methods for the synthesis of such amino-linked protected nucleoside precursors are known to those of ordinary skill in the art.

In many cases, protecting groups are used during the preparation of the compounds of the invention. As used herein, the term "protected" means that the indicated moiety has a protecting group appended thereon. In some preferred embodiments of the invention, compounds contain one or more protecting groups. A wide variety of protecting groups can be employed in the methods of the disclosure. In general, protecting groups render chemical functionalities inert to specific reaction conditions, and can be appended to and removed from such functionalities in a molecule without substantially damaging the remainder of the molecule.

Protecting groups in general and hydroxyl protecting groups in particular are well known in the art (Greene and Wuts, Protective Groups in Organic Synthesis, Chapter 2, 2d ed., John Wiley & Sons, New York, 1991). Amino-protecting groups stable to acid treatment are selectively removed with base treatment, and are used to make reactive amino groups selectively available for substitution. Examples of such groups are the Fmoc and various substituted sulfonylethyl carbamates exemplified by the Nsc group.

Additional amino-protecting groups include, but are not limited to, carbamate protecting groups, such as 2-trimethylsilylethoxycarbonyl (Teoc), 1-methyl-1-(4-biphenylyl)-ethoxycarbonyl (Bpoc), t-butoxycarbonyl (BOC), allyloxycarbonyl (Alloc), 9-fluorenyl-methyloxycarbonyl (Fmoc), and benzyloxycarbonyl (Cbz); amide protecting groups, such as formyl, acetyl, trihaloacetyl, benzoyl, and nitrophenylacetyl; sulfonamide protecting groups, such as 2-nitrobenzenesulfonyl; and imine and cyclic imide protecting groups, such as phthalimido and dithiasuccinoyl. Equivalents of these amino-protecting groups are also encompassed by the compounds of the invention and methods of the disclosure.

Many solid supports are commercially available and one of ordinary skill in the art can readily select a solid support to be used in the solid-phase synthesis steps. In certain embodiments, a universal support is used. A universal support, well known in the art, allows for the preparation of oligonucleotides having unusual or modified nucleotides located at the 3'-terminus of the oligonucleotide. In addition, it has been reported that the oligonucleotide can be cleaved from the universal support under milder reaction conditions when the oligonucleotide is bonded to the solid support via a syn-1,2-acetoxyphosphate group which more readily undergoes basic hydrolysis. See Guzaev AI, and Manoharan MJ. Am. Chem. Soc. (2003) 125:2380.

The nucleosides are linked by phosphorus-containing or non-phosphorus-containing covalent internucleoside linkages. For the purposes of identification, such conjugated nucleosides can be characterized as ligand-bearing nucleosides or ligand-nucleoside conjugates. The linked nucleosides having an aralkyl ligand conjugated to a nucleoside within their sequence will demonstrate enhanced dsRNA activity when compared to like dsRNA compounds that are not conjugated.

The aralkyl-ligand-conjugated oligonucleotides of the disclosure also include conjugates of oligonucleotides and linked nucleosides wherein the ligand is attached directly to the nucleoside or nucleotide without the intermediacy of a linker group. The ligand may preferably be attached, via linking groups, at a carboxyl, amino or oxo group of the ligand. Typical linking groups may be ester, amide or carbamate groups.

Specific examples of preferred modified oligonucleotides envisioned for use in the ligand-conjugated oligonucleotides of the disclosure include oligonucleotides containing modified backbones or non-natural internucleoside linkages. As defined here, oligonucleotides having modified backbones or internucleoside linkages include those that retain a phosphorus atom in the backbone and those that do not have a phosphorus atom in the backbone. For the purposes of the invention, modified oligonucleotides that do not have a phosphorus atom in their intersugar backbone can also be considered to be oligonucleosides.

Specific oligonucleotide chemical modifications are described below. It is not necessary for all positions in a given compound to be uniformly modified. Conversely, more than one modifications may be incorporated in a single dsRNA compound or even in a single nucleotide thereof.

Preferred modified internucleoside linkages or backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free-acid forms are also included.

Representative United States Patents relating to the preparation of the above phosphorus-atom-containing linkages include, but are not limited to, U.S. Pat. Nos. 4,469,863, 5,023,243, 5,264,423, 5,321,131, 5,399,676, 5,405,939, 5,453,496, 5,455,233, and 5,466,677.

Preferred modified internucleoside linkages or backbones that do not include a phosphorus atom therein (i.e., oligonucleosides) have backbones that are formed by short chain alkyl or cycloalkyl intersugar linkages, mixed heteroatom and alkyl or cycloalkyl intersugar linkages, or one or more short chain heteroatomic or heterocyclic intersugar linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Representative United States patents relating to the preparation of the above oligonucleosides include, but are not limited to, U.S. Pat. Nos. 5,034,506, 5,214,134, 5,216,141, 5,264,562, 5,466,677, 5,470,967, 5,489,677, 5,602,240, and 5,663,312.

In other preferred oligonucleotide mimetics, both the sugar and the internucleoside linkage, i.e., the backbone, of the nucleoside units are replaced with novel groups. The nucleobase units are maintained for hybridization with an appropriate nucleic acid target compound. One such oligonucleotide, an oligonucleotide mimetic, that has been shown to have excellent hybridization properties, is referred to as a peptide nucleic acid (PNA). In PNA compounds, the sugar-backbone of an oligonucleotide is replaced with an amide-containing backbone, in particular an aminoethylglycine backbone. The nucleobases are retained and are bound directly or indirectly to atoms of the amide portion of the backbone. Teaching of PNA compounds can be found for example in U.S. Pat. No. 5,539,082.

Some preferred embodiments of the invention employ oligonucleotides with phosphorothioate linkages and oligonucleosides with heteroatom backbones, and in particular $-CH_2-NH-O-CH_2-$, $-CH_2-N(CH_3)-O-CH_2-$[known as a methylene (methylimino) or MMI backbone], $-CH_2-O-N(CH_3)-CH_2-$, $-CH_2-N(CH_3)-N(CH_3)-CH_2-$, and -O-

$N(CH_3)-CH_2-CH_2-$ [wherein the native phosphodiester backbone is represented as $-O-P-O-CH_2-$] of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

The oligonucleotides employed in the ligand-conjugated oligonucleotides of the invention may additionally or alternatively comprise nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C), and uracil (U). Modified nucleobases include other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2- thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8- hydroxyl and other 8-substituted adenines and guanines, 5-halo particularly 5-bromo, 5- trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine.

Further nucleobases include those disclosed in U.S. Pat. No. 3,687,808, or otherwise known in the art or commercially available. Certain of these nucleobases are particularly useful for increasing the binding affinity of the oligonucleotides of the invention. These include 5-substituted pyrimidines, 6- azapyrimidines and N-2, N-6 and O-6 substituted purines, including 2-aminopropyladenine, 5-propynyluracil and 5-propynylcytosine. 5-Methylcytosine substitutions have been shown to increase nucleic acid duplex stability by 0.6-1.2°C and are presently preferred base substitutions, even more particularly when combined with 2'-methoxyethyl sugar modifications.

Representative United States patents relating to the preparation of certain of the above-noted modified nucleobases as well as other modified nucleobases include, but are not limited to, the above noted U.S. Pat. No. 3,687,808, as well as U.S. Pat. Nos. 5,134,066, 5,459,255, 5,552,540, 5,594,121, and 5,596,091.

In certain embodiments, the oligonucleotides employed in the ligand-conjugated oligonucleotides of the disclosure may additionally or alternatively comprise one or more substituted sugar moieties. Preferred oligonucleotides comprise one of the following at the 2' position: OH; F; O-, S-, or N-alkyl, O-, S-, or N-alkenyl, or O, S- or N-alkynyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other preferred oligonucleotides comprise one of the following at the 2' position: C1 to C10 lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-$CH_2CH_2OCH_3$, also known as 2'-O-(2-methoxyethyl) or 2'-MOE), i.e., an alkoxyalkoxy group. A further preferred modification includes 2'-dimethylaminooxyethoxy, i.e., a $O(CH_2)_2ON(CH_3)_2$ group, also known as 2'-DMAOE, as described in U.S. Pat. No. 6,127,533.

Other preferred modifications include 2'-methoxy (2'-O-$CH_3$), 2'-aminopropoxy (2'-$OCH_2CH_2CH_2NH_2$) and 2'-fluoro (2'-F). Similar modifications may also be made at other positions on the oligonucleotide, particularly the 3' position of the sugar on the 3' terminal nucleotide or in 2'-5' linked oligonucleotides.

As used herein, the term ..sugar substituent group" or "2'-substituent group" includes groups attached to the 2'-position of the ribofuranosyl moiety with or without an oxygen atom. Sugar substituent groups include, but are not limited to, fluoro, O-alkyl, O-alkylamino, O-alkylalkoxy, protected O-alkylamino, O-alkylaminoalkyl, O-alkyl imidazole and polyethers of the formula (O-alkyl)m, wherein m is 1 to about 10. Preferred among these polyethers are linear and cyclic polyethylene glycols (PEGs), and (PEG)-containing groups, such as crown ethers and, inter alia, those which are disclosed by Delgardo et. al. (Critical Reviews in Therapeutic Drug Carrier Systems (1992) 9:249). Further sugar modifications are disclosed by Cook (Anti-fibrosis Drug Design, (1991) 6:585-607). Fluoro, O-alky1, O-alkylamino, O-alkyl imidazole, O-alkylaminoalkyl, and alkyl amino substitution is described in U.S. Patent 6,166,197, entitled "Oligomeric Compounds having Pyrimidine Nucleotide(s) with 2' and 5' Substitutions,".

Additional sugar substituent groups amenable to the invention include 2'-SR and 2'-$NR_2$ groups, wherein each R is, independently, hydrogen, a protecting group or substituted or unsubstituted alkyl, alkenyl, or alkynyl. 2'-SR Nucleosides are disclosed in U.S. Pat. No. 5,670,633. The incorporation of 2'-SR monomer synthons is disclosed by Hamm et al. (J. Org. Chem., (1997) 62:3415-3420). 2'-NR nucleosides are disclosed by Thomson JB, J. Org. Chem., (1996) 61:6273-6281; and Polushin et al., Tetrahedron Lett., (1996) 37:3227-3230. Further representative 2'-substituent groups amenable to the invention include those having one of formula I or II:

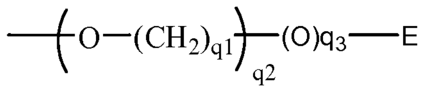

I

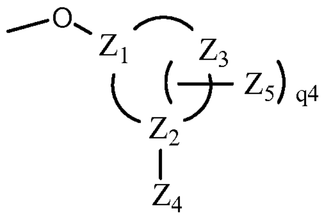

II

wherein

E is $C_1$-$C_{10}$ alkyl, N(Q3)(Q4) or N=C(Q3)(Q4); each Q3 and Q4 is, independently, H, $C_1$-$C_{10}$ alkyl, dialkylaminoalkyl, a nitrogen protecting group, a tethered or untethered conjugate group, a linker to a solid support; or Q3 and Q4, together, form a nitrogen protecting group or a ring structure optionally including at least one additional heteroatom selected from N and O;
q1 is an integer from 1 to 10;
q2 is an integer from 1 to 10;
q3 is 0 or 1;
q4 is 0, 1 or 2;
each Z1, Z2, and Z3 is, independently, $C_4$-$C_7$ cycloalkyl, $C_5$-$C_{14}$ aryl or $C_3$-$C_{15}$ heterocyclyl, wherein the heteroatom in said heterocyclyl group is selected from oxygen, nitrogen and sulfur;
Z4 is OM1, SM1, or $N(M1)_2$; each M1 is, independently, H, $C_1$-$C_8$ alkyl, $C_1$-$C_8$ haloalkyl, C(=NH)N(H)M2, C(=O)N(H)M2 or OC(=O)N(H)M2; M2 is H or $C_1$-$C_8$ alkyl; and
Z5 is $C_1$-$C_{10}$ alkyl, $C_1$-$C_{10}$ haloalkyl, $C_2$-$C_{10}$ alkenyl, $C_2$-$C_{10}$ alkynyl, $C_6$-$C_{14}$ aryl, N(Q3)(Q4), OQ3, halo, SQ3 or CN.

Representative 2'-O-sugar substituent groups of formula I are disclosed in U.S. Pat. No. 6,172,209, entitled "Capped 2'-Oxyethoxy Oligonucleotides,". Representative cyclic 2'-O-sugar substituent groups of formula II are disclosed in U.S. Patent 6,271,358, entitled "RNA Targeted 2'-Modified Oligonucleotides that are Conformationally Preorganized,".

Sugars having O-substitutions on the ribosyl ring are also amenable to the invention. Representative substitutions for ring O include, but are not limited to, S, $CH_2$, CHF, and $CF_2$.

Oligonucleotides may also have sugar mimetics, such as cyclobutyl moieties, in place of the pentofuranosyl sugar. Representative United States patents relating to the preparation of such modified sugars include, but are not limited to, U.S. Pat. Nos. 5,359,044, 5,466,786, 5,519,134, 5,591,722, 5,597,909, 5,646,265, and 5,700,920.

Additional modifications may also be made at other positions on the oligonucleotide, particularly the 3' position of the sugar on the 3' terminal nucleotide. For example, one additional modification of the ligand-conjugated oligonucleotides of the disclosure involves chemically linking to the oligonucleotide one or more additional non-ligand moieties or conjugates which enhance the activity, cellular distribution or cellular uptake of the oligonucleotide. Such moieties include but are not limited to lipid moieties, such as a cholesterol moiety (Letsinger et al., Proc. Natl. Acad. Sci. USA, (1989) 86:6553), cholic acid (Manoharan et al., Bioorg. Med. Chem. Lett., (1994) 4:1053), a thioether, e.g., hexyl-S-tritylthiol (Manoharan et al., Ann. N.Y. Acad. Sci., (1992) 660:306; Manoharan et al., Bioorg. Med. Chem. Let., (1993) 3:2765), a thiocholesterol (Oberhauser et al., Nucl. Acids Res., (1992) 20:533), an aliphatic chain, e.g., dodecandiol or undecyl residues (Saison-Behmoaras et al., EMBO J., (1991) 10:111; Kabanov et al., FEBS Lett., (1990) 259:327; Svinarchuk et al., Biochimie, (1993) 75:49), a phospholipid, e.g., di-hexadecyl-rac-glycerol or triethylammonium 1,2-di-O-hexadecyl-rac-glycero-3-H-phosphonate (Manoharan et al., Tetrahedron Lett., (1995) 36:3651; Shea et al., Nucl. Acids Res., (1990) 18:3777), a polyamine or a polyethylene glycol chain (Manoharan et al., Nucleosides & Nucleotides, (1995) 14:969), or adamantane acetic acid (Manoharan et al., Tetrahedron Lett., (1995) 36:3651), a palmityl moiety (Mishra et al., Biochim. Biophys. Acta, (1995) 1264:229), or an octadecylamine or hexylamino-carbonyl-oxycholesterol moiety (Crooke et al., J. Pharmacol. Exp. Ther., (1996) 277:923).

The invention also includes compositions employing oligonucleotides that are substantially chirally pure with regard to particular positions within the oligonucleotides. Examples of substantially chirally pure oligonucleotides include, but are not limited to, those having phosphorothioate linkages that are at least 75% Sp or Rp (Cook et al., U.S. Pat. No. 5,587,361) and those having substantially chirally pure (Sp or Rp) alkylphosphonate, phosphoramidate or phosphotriester linkages (Cook, U.S. Pat. Nos. 5,212,295 and 5,521,302).

In certain instances, the oligonucleotide may be modified by a non-ligand group. A number of non-ligand molecules have been conjugated to oligonucleotides in order to enhance the activity, cellular distribution or cellular uptake of the oligonucleotide, and procedures for performing such conjugations are available in the scientific literature. Such non-ligand moieties have included lipid moieties, such as cholesterol (Letsinger et al., Proc. Natl. Acad. Sci. USA,

(1989, 86:6553), cholic acid (Manoharan et al., Bioorg. Med. Chem. Lett., (1994, 4:1053), a thioether, e.g., hexyl-S-tritylthiol (Manoharan et al., Ann. N.Y. Acad. Sci., (1992, 660:306; Manoharan et al., Bioorg. Med. Chem. Let., (1993, 3:2765), a thiocholesterol (Oberhauser et al., Nucl. Acids Res., (1992, 20:533), an aliphatic chain, e.g., dodecandiol or undecyl residues (Saison-Behmoaras et al., EMBO J., (1991) 10:111; Kabanov et al., FEBS Lett., (1990) 259:327; Svinarchuk et al., Biochimie, (1993) 75:49), a phospholipid, e.g., di-hexadecyl-rac-glycerol or triethylammonium 1,2-di-O-hexadecyl-rac-glycero-3-H-phosphonate (Manoharan et al., Tetrahedron Lett., (1995) 36:3651; Shea et al., Nucl. Acids Res., (1990) 18:3777), a polyamine or a polyethylene glycol chain (Manoharan et al., Nucleosides & Nucleotides, (1995) 14:969), or adamantane acetic acid (Manoharan et al., Tetrahedron Lett., (1995) 36:3651), a palmityl moiety (Mishra et al., Biochim. Biophys. Acta, (1995) 1264:229), or an octadecylamine or hexylamino-carbonyl-oxycholesterol moiety (Crooke et al., J. Pharmacol. Exp. Ther., (1996) 277:923). Typical conjugation protocols involve the synthesis of oligonucleotides bearing an aminolinker at one or more positions of the sequence. The amino group is then reacted with the molecule being conjugated using appropriate coupling or activating reagents. The conjugation reaction may be performed either with the oligonucleotide still bound to the solid support or following cleavage of the oligonucleotide in solution phase. Purification of the oligonucleotide conjugate by HPLC typically affords the pure conjugate.

Alternatively, the molecule being conjugated may be converted into a building block, such as a phosphoramidite, via an alcohol group present in the molecule or by attachment of a linker bearing an alcohol group that may be phosphorylated.

Importantly, each of these approaches may be used for the synthesis of ligand conjugated oligonucleotides. Amino linked oligonucleotides may be coupled directly with ligand via the use of coupling reagents or following activation of the ligand as an NHS or pentfluorophenolate ester. Ligand phosphoramidites may be synthesized via the attachment of an aminohexanol linker to one of the carboxyl groups followed by phosphitylation of the terminal alcohol functionality. Other linkers, such as cysteamine, may also be utilized for conjugation to a chloroacetyl linker present on a synthesized oligonucleotide.

The person skilled in the art is readily aware of methods to introduce the molecules of this invention into cells, tissues or organisms. Corresponding examples have also been provided in the detailed description of the invention above. For example, the nucleic acid molecules or the vectors of this invention, encoding for at least one strand of the inventive dsRNAs may be introduced into cells or tissues by methods known in the art, like transfections etc.

Also for the introduction of dsRNA molecules, means and methods have been provided. For example, targeted delivery by glycosylated and folate-modified molecules, including the use of polymeric carriers with ligands, such as galactose and lactose or the attachment of folic acid to various macromolecules allows the binding of molecules to be delivered to folate receptors. Targeted delivery by peptides and proteins other than antibodies, for example, including RGD-modified nanoparticles to deliver siRNA *in vivo* or multicomponent (nonviral) delivery systems including short cyclodextrins, adamantine-PEG are known. Yet, also the targeted delivery using antibodies or antibody fragments, including (monovalent) Fab-fragments of an antibody (or other fragments of such an antibody) or single-chain antibodies are envisaged. Injection approaches for target directed delivery comprise, inter alia, hydrodynamic i.v. injection. Also cholesterol conjugates of dsRNA may be used for targeted delivery, whereby the conjugation to lipohilic groups enhances cell uptake and improve pharmacokinetics and tissue biodistribution of oligonucleotides. Also cationic delivery systems are known, whereby synthetic vectors with net positive (cationic) charge to facilitate the complex formation with the polyanionic nucleic acid and interaction with the negatively charged cell membrane. Such cationic delivery systems comprise also cationic liposomal delivery systems, cationic polymer and peptide delivery systems. Other delivery systems for the cellular uptake of dsRNA/siRNA are aptamer-ds/siRNA. Also gene therapy approaches can be used to deliver the inventive dsRNA molecules or nucleic acid molecules encoding the same. Such systems comprise the use of non-pathogenic virus, modified viral vectors, as well as deliveries with nanoparticles or liposomes. Other delivery methods for the cellular uptake of dsRNA are extracorporeal, for example ex vivo treatments of cells, organs or tissues. Certain of these technologies are described and summarized in publications, like Akhtar, Journal of Clinical Investigation (2007) 117:3623-3632, Nguyen et al., Current Opinion in Molecular Therapeutics (2008) 10:158-167, Zamboni, Clin Cancer Res (2005) 11:8230-8234 or Ikeda et al., Pharmaceutical Research (2006) 23:1631-1640.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. In case of conflict, the present specification, including definitions, will control. In addition, the materials, methods, and examples are illustrative only and not intended to be limiting.

The above provided embodiments and items of the present invention are now illustrated with the following, non-limiting examples.

EXAMPLES

*Identification of dsRNAs for therapeutic use.* dsRNA design was carried out to identify dsRNAs specifically

targeting Hepatitis B Virus genotypes A, B, C and D for therapeutic use.

First, the known Hepatitis B Virus genomic sequences were downloaded from NCBI Genbank (accessions listed in Table.6). The genotype information was either extracted form NCBI Genbank files or determined by computer aided comparison with reference genomes (accessions listed in Table. 6).

The Hepatitis B Virus genomic sequences of genotype A-D were examined by computer analysis to identify optimal target regions for RNAi agents, namely highly conserved 17 nucleotide long sequence stretches that were identical in at least 90% of all sequences.

In identifying RNAi agents, the selection was limited to 17mer sequences having at least two mismatches to any sequence in the human RefSeq database (release 41), which we assumed to represent the comprehensive human transcriptome, by using a proprietary algorithm.

All 17mer sequences containing four or more consecutive G's (poly-G sequences) were further excluded from the synthesis.

Sequences of 19 nucleotides length were defined that harbor the selected 17mers in position 2 to 18.

These 19mer sequences yield RNA interference (RNAi) agents cross-reactive to Hepatitis B Virus genomic sequences of genotype A-D and formed the basis for the synthesis of the RNAi agents in appended Tables 1 and 2.

*dsRNA synthesis.* Oligoribonucleotides were synthesized according to the phosphoramidite technology on solid phase. Depending on the scale either an ABI 394 synthesizer (Applied Biosystems) or an AKTA oligopilot 100 (GE Healthcare, Freiburg, Germany) was used. Syntheses were performed on a solid support made of controlled pore glass (CPG, 520Å, with a loading of 75 μmol/g, obtained from Prime Synthesis, Aston, PA, USA). All 2'-modified RNA phosphoramidites as well as ancillary reagents were purchased from SAFC (Hamburg, Germany). Specifically, the following 2'-O-Methyl phosphoramidites were used: (5'-O-dimethoxytrityl-$N^6$-(benzoyl)-2'-O-methyl-adenosine-3'-O-(2-cyanoethyl-N,N-diisopropylamino) phosphoramidite, 5'-O-dimethoxytrityl-$N^4$-(acetyl)-2'-O-methyl-cytidine-3'-O-(2-cyanoethyl-N,N-diisopropylamino) phosphoramidite, (5'-O-dimethoxy-trityl-$N^2$-(isobutyryl)-2'-O-methyl-guanosine-3'-O-(2-cyanoethyl-N,N-diisopropylamino) phosphoramidite, and 5'-O-dimethoxy-trityl-2'-O-methyl-uridine-3'-O-(2-cyanoethyl-N,N-diisopropylamino) phosphoramidite. The 2'-Deoxy-2'-fluoro-phosphoramidites carried the same protecting groups as the 2'-0-methyl RNA amidites. All amidites were dissolved in anhydrous acetonitrile (100 mM) and molecular sieves (3Å) were added. To generate the 5'-phosphate the 2-[2-(4,4'-Dimethoxytrityloxy) ethylsulfonyl]ethyl-(2-cyanoethyl)-(N,N-diisopropyl)-phosphoramidite from Glen Research (Sterling, Virginia, USA) was used. In order to introduce the C-6 aminolinker at the 5'-end of the oligomers the 6-(Trifluoroacetylamino)-hexyl-(2-cyanoethyl)-(N,N-diisopropyl)-phosphoramidite from Thermo Fisher Scientific (Milwaukee, Wisconsin, USA) was employed. The 5'-modifications were introduced without any modification of the synthesis cycle. 5-Ethyl thiotetrazole (ETT, 500 mM in acetonitrile) was used as activator solution. Coupling times were 6 min. In order to introduce phosphorothioate linkages, a 50 mM solution of 3-((Dimethylamino-methylidene)amino)-3H-1,2,4-dithiazole-3-thione (DDTT, obtained from AM Chemicals, Oceanside, CA, USA) in anhydrous Acetonitrile/pyridine (1:1 v/v) was employed.

*Cleavage and deprotection of support bound oligomer.* After finalization of the solid phase synthesis, cyanoethyl protecting groups were removed by a 30 min treatment with 20% Diethyl amine in ACN without cleaving the oligonucleotides from the support. Subsequently, the dried solid support was transferred to a 15 mL tube and treated with concentrated aqueous ammonia (Aldrich) for 18 h at 40°C. After centrifugation the supernatant was transferred to a new tube and the CPG was washed with aqueous ammonia. The combined solutions were evaporated and the solid residue was reconstituted in buffer A (see below).

*Purification of oligoribonucleotides.* Crude oligomers were purified by anionic exchange HPLC using a column packed with Source Q15 (GE Helthcare) and an AKTA Explorer system (GE Helthcare). Buffer A was 10 mM sodium perchlorate, 20 mM Tris, 1 mM EDTA, pH 7.4 (Fluka, Buchs, Switzerland) and contained 20% Acetonitrile and buffer B was the same as buffer A with the exception of 500 mM sodium perchlorate. A gradient of 22%B to 42%B within 32 column volumes (CV) was employed. UV traces at 280 nm were recorded Appropriate fractions were pooled and precipitated with 3M NaOAc, pH=5.2 and 70% Ethanol. Finally, the pellet was washed with 70% Ethanol. Alternatively, desalting was carried out using Sephadex HiTrap columns (GE Helthcare) according to the manufacturer's recommendation.

*Annealing of oligoribonucleotides to generate siRNA.* Complementary strands were mixed by combining equimolar RNA solutions. The mixture was lyophilized and reconstituted with an appropriate volume of annealing buffer (100 mM NaCl, 20 mM sodium phosphate, pH 6.8) to achieve the desired concentration. This solution was placed into a water bath at 80°C which was cooled to RT within 3 h.

In Vitro *screening of HBV mRNA-targeting dsRNA.* The psiCHECK™-2 vector (Promega) contains two reporter genes for monitoring RNAi activity: a synthetic version of the renilla luciferase (hRluc) gene and a synthetic firefly luciferase gene (hluc+). Measurement of firefly luciferase activity permits determination of changes unrelated to the RNAi activity of tested dsRNA. Renilla and firefly luciferase activities were measured using the Dual-Glo® Luciferase Assay System (Promega). HBV target sites of interest were inserted into the psiCHECK™-2 vector, after cloning into the multiple cloning region located 3' of the synthetic renilla luciferase gene's translational stop codon and the polyA

tail. Cell line COS-7 was transfected with the vector, and subsequently treated with dsRNA-lipofectamine 2000 lipoplexes targeting the HBV sequences. The RNAi effect conferred by the dsRNA towards the cloned HBV target site was determined by measuring activity of the renilla luciferase fusion gene.

*Generation of psiCHECK Vectors Containing Target Sequences.* In order to test the activity of the HBV dsRNAs, a Dual-Luciferase HBV reporter was constructed. Regions 84 to 805, 1075 to 1992, 2165 to 2530, and 2718 to 2940 of Hepatitis B Virus genomic sequence accession number EU554538.1 (genotype C) were joined *in silico*. Two mutations were inserted intentionally (128 A→T, 598 T→C, positions relative to EU554538.1). One was needed to remove an internal XhoI site. The second mutation led to removal of a single mismatch to a dsRNA. This HBV target construct was extended by adding restriction sites at both the 5' and 3' end. The artificial DNA sequence was chemically synthesized by Geneart (Regensburg, Germany) and cloned into the XhoI/NotI site of psiCHECK™-2 Dual-Luciferase vector.

*Transfection and Luciferase Quantification.* Cos-7 cells (DSMZ, Braunschweig, Germany, cat. No. ACC-60) were seeded at a density of $2.25 \times 10^4$ cells/well in 96-well plates. Plasmid transfection was carried out at a concentration of 50 ng/well with 0.5 μL/well Lipofectamine 2000 (Invitrogen GmbH, Karlsruhe, Germany, cat. No. 11668-019) as described by the manufacturer. 4 h after vector transfection, the medium was discarded and fresh medium was added. After this period, the dsRNAs were added to the cells in a concentration of 10 nM or 1 nM using Lipofectamine 2000 as described above. In order to optimize the HBV genotype coverage and to minimize development of resistance against dsRNAs, two different dsRNAs can be used simultaneously in combination. For demonstrating the feasibility of such approach, pairs of two different dsRNAs were selected among the most efficient dsRNAs with additional bias towards optimized genotype coverage.

The dsRNAs were added to the cells in a concentration of 5 nM or 0.5 nM for each dsRNA, resulting in 10 nM or 1 nM total dsRNA concentration, using Lipofectamine 2000 as described above. The cells were lysed 48 hours later using luciferase reagents as described by the manufacturer. Renilla luciferase protein levels were normalized to firefly luciferase levels to consider transfection efficiency. For each dsRNA four individual data points were collected. At least one dsRNA unrelated to all target sites was used as a control to determine the relative renilla luciferase protein levels in cells treated with dsRNA (Table 8). For comparison of silencing activity under full-match conditions, dsRNAs with full match to the renilla open reading frame were synthesized and tested in parallel to the HBV dsRNAs.

Inhibition data are given in appended Table 2.

*Stability of dsRNAs.* Stability of dsRNAs targeting human Hepatitis B Virus was determined in *in vitro* assays with any one of human, cynomolgous monkey or mouse serum by measuring the half-life of each single strand.

Measurements were carried out in triplicates for each time point, using 3 μL 50 μM dsRNA sample mixed with 30 μL human serum (Sigma), cynomolgous monkey serum (Sigma) or mouse serum (Sigma). Mixtures were incubated for either 0 min, 30min, 1h, 3h, 6h, 24h, or 48h at 37°C. As control for unspecific degradation dsRNA was incubated with 30 μL 1× PBS pH 6.8 for 48h. Reactions were stopped by the addition of 4 μL proteinase K (20 mg/ml), 25 μL of "Tissue and Cell Lysis Solution" (Epicentre) and 38 μL Millipore water for 30 min at 65°C. Samples were afterwards spin filtered through a 0.2 μm 96 well filter plate at 1400 rpm for 8 min, washed with 55 μL Millipore water twice and spin filtered again.

For separation of single strands and analysis of remaining full length product (FLP), samples were run through an ion exchange Dionex Summit HPLC under denaturing conditions using as eluent A 20mM $Na_3P0^4$ in 10% ACN pH=11 and for eluent B 1M NaBr in eluent A.

The following gradient was applied:

| Time (min) | %A | %B |
|---|---|---|
| -1.0 | 75 | 25 |
| 1.00 | 75 | 25 |
| 19.0 | 38 | 62 |
| 19.5 | 0 | 100 |
| 21.5 | 0 | 100 |
| 22.0 | 75 | 25 |
| 24.0 | 75 | 25 |

For every injection, the chromatograms were integrated automatically by the Dionex Chromeleon 6.60 HPLC software, and were adjusted manually if necessary. All peak areas were corrected to the internal standard (IS) peak and normalized to the incubation at t = 0 min. The area under the peak and resulting remaining FLP was calculated for each single strand and triplicate separately. Half-life (t½) of a strand was defined by the average time point (h) for triplicates at which half of the FLP was degraded. Results are given in appended Table 3.

## Claims

1. A double-stranded ribonucleic acid molecule capable of inhibiting the expression of a Hepatitis B Virus gene in vitro by at least 60%, preferably by at least 70% and most preferably by at least 80%, wherein said double-stranded ribonucleic acid molecule comprises a sense strand comprising in order nucleotides 1-19 of SEQ ID NOs: 5 or 13

and an antisense strand at least partially complementary to the sense strand, and wherein the antisense strand sequence and the sense strand sequence are each less than 30 nucleotides in length, and wherein at least one of the nucleotides in each of the sense strand and in the antisense strand are modified nucleotides.

2. The double-stranded ribonucleic acid molecule of claim 1, wherein said antisense strand comprises in order nucleotides 1-19 of SEQ ID Nos: 162 or 173.

3. The double-stranded ribonucleic acid molecule of claim 2, wherein said double-stranded ribonucleic acid molecule comprises sequence pairs selected from the group consisting of SEQ ID NOs: 5/162 or 13/173.

4. The double-stranded ribonucleic acid molecule of claim 1, wherein the antisense strand further comprises a 3' overhang of 1-5 nucleotides in length.

5. The double-stranded ribonucleic acid molecule of claim 4, wherein the overhang of the antisense strand comprises uracil or nucleotides which are complementary to the pregenomic RNA and/or the mRNA encoding the protein necessary for replication or pathogenesis of Hepatitis B Virus.

6. The double-stranded ribonucleic acid molecule of claim 1, wherein the sense strand further comprises a 3' overhang of 1-5 nucleotides in length.

7. The double-stranded ribonucleic acid molecule of claim 6, wherein the overhang of the sense strand comprises uracil or nucleotides which are identical to the pregenomic RNA and/or the mRNA encoding the protein necessary for replication or pathogenesis of Hepatitis B Virus.

8. The double-stranded ribonucleic acid molecule of claim 1, wherein the double-stranded ribonucleic acid molecule comprises at least one modified nucleotide selected from the group consisting of: 2'-O-methyl modified nucleotide, nucleotide comprising a 5'-phosphorothioate group, terminal nucleotide linked to a cholesteryl derivative, terminal nucleotide linked to a dodecanoic acid bisdecylamide group, 2'-deoxy-2'-fluoro modified nucleotide, 2'-deoxy-modified nucleotide, locked nucleotide, abasic nucleotide, deoxythymidine, inverted deoxythymidine, 2'-amino-modified nucleotide, 2'-alkyl-modified nucleotide, morpholino nucleotide, phosphoramidate, and non-natural base comprising nucleotide.

9. The double-stranded ribonucleic acid molecule of claim 8, wherein said double-stranded ribonucleic acid molecule contains a 2'-O-methyl modified nucleotide, a nucleotide comprising a 5'-phosphorothioate group, and a deoxythymidine.

10. The double-stranded ribonucleic acid molecule of claim 9, wherein said sense strand or said antisense strand comprises an overhang of 1-2 deoxythymidines.

11. The double-stranded ribonucleic acid molecule of claim 10, wherein the double-stranded ribonucleic acid molecule comprises the sequence pairs selected from the group consisting of SEQ ID NOs: 326/490 and 337/501.

12. The double-stranded ribonucleic acid molecule of claim 1, wherein said double-stranded ribonucleic acid molecule comprises a nucleic acid sequence encoding said sense strand or said antisense strand.

13. A pharmaceutical composition comprising:

(a) at least one double-stranded ribonucleic acid molecule as defined in any of claims 1-12; or
(b) at least one nucleic acid sequence encoding a sense strand and an antisense strand comprising the double-stranded ribonucleic acid molecule as defined in any of claims 1-12.

14. A pharmaceutical composition comprising:

(a) at least one first double-stranded ribonucleic acid molecule comprising the double-stranded ribonucleic acid molecule as defined in any of claims 1-12 and at least one second double-stranded ribonucleic acid molecule capable of inhibiting the expression of a Hepatitis B Virus gene wherein the sequences of the first and second double-stranded ribonucleic acid molecules are different; or
(b) at least one nucleic acid sequence encoding a sense strand and an antisense strand of a first double-stranded ribonucleic acid molecule as defined in any of claims 1-12 and at least one second nucleic acid sequence encoding a sense strand or an antisense strand of a double-stranded ribonucleic acid molecule capable of inhibiting the expression of a Hepatitis B Virus gene wherein the sense strand or antisense strand sequences encoded by the first and second nucleic acids are different.

**15.** The pharmaceutical composition of claim 14, further comprising a pharmaceutically acceptable carrier, stablilizer, and/or diluent.

**Patentansprüche**

**1.** Doppelstrang-Ribonukleinsäuremolekül, das zur Hemmung der Expression eines Hepatitis-B-Virusgens in vitro um mindestens 60 %, vorzugsweise um mindestens 70 % und am meisten bevorzugt um mindestens 80 % fähig ist, wobei das Doppelstrang-Ribonukleinsäuremolekül einen Sense-Strang, der in dieser Reihenfolge die Nukleotide 1-19 der SEQ ID Nr.: 5 oder 13 umfasst, und einen Antisense-Strang umfasst, der zumindest teilweise komplementär zum Sense-Strang ist, und wobei die Sequenz des Antisense-Strangs und die Sequenz des Sense-Strangs jeweils eine Länge von weniger als 30 Nukleotiden aufweisen und wobei mindestens eines der Nukleotide sowohl im Sense-Strang als auch im Antisense-Strang modifizierte Nukleotide sind.

**2.** Doppelstrang-Ribonukleinsäuremolekül nach Anspruch 1, wobei der Antisense-Strang in dieser Reihenfolge die Nukleotide 1-19 der SEQ ID Nr.: 162 oder 173 umfasst.

**3.** Doppelstrang-Ribonukleinsäuremolekül nach Anspruch 2, wobei das Doppelstrang-Ribonukleinsäuremolekül Sequenzpaare umfasst, die aus der aus SEQ ID Nr.: 5/162 oder 13/173 bestehenden Gruppe ausgewählt sind.

**4.** Doppelstrang-Ribonukleinsäuremolekül nach Anspruch 1, wobei der Antisense-Strang weiterhin einen 3'-Überhang mit einer Länge von 1-5 Nukleotiden umfasst.

**5.** Doppelstrang-Ribonukleinsäuremolekül nach Anspruch 4, wobei der Überhang des Antisense-Strangs Uracil oder Nukleotide umfasst, die komplementär zur prägenomischen RNA und/oder der mRNA sind, die das zur Replikation oder Pathogenese des Hepatitis-B-Virus erforderliche Protein codieren.

**6.** Doppelstrang-Ribonukleinsäuremolekül nach Anspruch 1, wobei der Sense-Strang weiterhin einen 3'-Überhang mit einer Länge von 1-5 Nukleotiden umfasst.

**7.** Doppelstrang-Ribonukleinsäuremolekül nach Anspruch 6, wobei der Überhang des Sense-Strangs Uracil oder Nukleotide umfasst, die identisch mit der prägenomischen RNA und/oder der mRNA sind, die das zur Replikation oder Pathogenese des Hepatitis-B-Virus erforderliche Protein codieren.

**8.** Doppelstrang-Ribonukleinsäuremolekül nach Anspruch 1, wobei das Doppelstrang-Ribonukleinsäuremolekül mindestens ein modifiziertes Nukleotid umfasst, das ausgewählt ist aus der Gruppe bestehend aus: einem 2'-O-Methyl-modifizierten Nukleotid, einem eine 5'-Phosphorothioatgruppe umfassenden Nukleotid, einem an ein Cholesterylderivat gebundenen terminalen Nukleotid, einem an eine Dodecansäurebisdecylamid-Gruppe gebundenen terminalen Nukleotid, einem 2'-Deoxy-2'-fluor-modifizierten Nukleotid, einem 2'-Deoxy-modifizierten Nukleotid, einem verbrückten ("locked") Nukleotid, einem abasischen Nukleotid, einem Deoxythymidin, einem invertierten Deoxythymidin, einem 2'-Aminomodifizierten Nukleotid, einem 2'-Alkyl-modifizierten Nukleotid, einem Morpholinonukleotid, Phosphoramidat und einem eine unnatürliche Base umfassenden Nukleotid.

**9.** Doppelstrang-Ribonukleinsäuremolekül nach Anspruch 8, wobei das Doppelstrang-Ribonukleinsäuremolekül ein 2'-O-Methyl-modifiziertes Nukleotid, ein eine 5'-Phosphorothioat-Gruppe umfassendes Nukleotid und ein Deoxythymidin enthält.

**10.** Doppelstrang-Ribonukleinsäuremolekül nach Anspruch 9, wobei der Sense-Strang oder der Antisense-Strang einen Überhang von 1-2 Deoxythymidinen umfasst.

**11.** Doppelstrang-Ribonukleinsäuremolekül nach Anspruch 10, wobei das Doppelstrang-Ribonukleinsäuremolekül die Sequenzpaare umfasst, die aus der aus SEQ ID Nr.: 326/490 und 337/501 bestehenden Gruppe ausgewählt sind.

**12.** Doppelstrang-Ribonukleinsäuremolekül nach Anspruch 1, wobei das Doppelstrang-Ribonukleinsäuremolekül eine Nukleinsäuresequenz umfasst, die den Sense-Strang oder den Antisense-Strang codiert.

**13.** Pharmazeutische Verbindung, die Folgendes umfasst:

(a) mindestens ein Doppelstrang-Ribonukleinsäuremolekül gemäß der Definition in einem der Ansprüche 1-12 oder
(b) mindestens eine Nukleinsäuresequenz, die einen Sense-Strang und einen Antisense-Strang codiert, umfassend das Doppelstrang-Ribonukleinsäuremolekül gemäß der Definition in einem der Ansprüche 1-12.

**14.** Pharmazeutische Verbindung, umfassend:

(a) mindestens ein erstes Doppelstrang-Ribonukleinsäuremolekül, welches das Doppelstrang-Ribonukleinsäuremolekül gemäß der Definition in einem der Ansprüche 1-12 umfasst, und mindestens ein zweites Doppelstrang-Ribonukleinsäuremolekül, das dazu fähig ist, die Expression eines Hepatitis-B-Virusgens zu hemmen, wobei die Sequenzen des ersten und des zweiten Doppelstrang-Ribonukleinsäuremoleküls verschieden sind; oder
(b) mindestens eine Nukleinsäuresequenz, die einen Sense-Strang und einen Antisense-Strang eines ersten Doppelstrang-Ribonukleinsäuremoleküls gemäß der Definition in einem der Ansprüche 1-12 codiert, und mindestens eine zweite Nukleinsäuresequenz, die einen Sense-Strang oder einen Antisense-Strang eines Doppelstrang-Ribonukleinsäuremoleküls codiert, das dazu fähig ist, die Expression eines Hepatitis-B-Virusgens zu hemmen, wobei der Sense-Strang oder der Antisense-Strang, die von der ersten und der zweiten Nukleinsäure codiert werden, verschieden sind.

**15.** Pharmazeutische Zusammensetzung nach Anspruch 14, die weiterhin einen pharmazeutisch annehmbaren Träger, Stabilisator und/oder ein pharmazeutisch annehmbares Verdünnungsmittel umfasst.

## Revendications

**1.** Molécule d'acide ribonucléique double brin susceptible d'inhiber l'expression d'un gène du virus de l'hépatite B in vitro à raison d'au moins 60 %, de préférence d'au moins 70 % et tout spécialement d'au moins 80 %, ladite molécule d'acide ribonucléique double brin comprenant un brin sens comprenant dans l'ordre les nucléotides 1 à 19 de SEQ ID NO: 5 ou 13 et un brin antisens au moins partiellement complémentaire du brin sens, et la séquence du brin antisens et la séquence du brin sens ayant chacune une longueur inférieure à 30 nucléotides, et au moins l'un des nucléotides de chacun du brin sens et du brin antisens étant un nucléotide modifié.

**2.** Molécule d'acide ribonucléique double brin selon la revendication 1, dans laquelle le brin antisens comprend dans l'ordre les nucléotides 1 à 19 de SEQ ID NO: 162 ou 173.

**3.** Molécule d'acide ribonucléique double brin selon la revendication 2, ladite molécule d'acide ribonucléique double brin comprenant des paires de séquences choisies dans le groupe consistant en SEQ ID NO: 5/162 ou 13/173.

**4.** Molécule d'acide ribonucléique double brin selon la revendication 1, dans laquelle le brin antisens comprend en outre un surplomb en 3' ayant une longueur de 1 à 5 nucléotides.

**5.** Molécule d'acide ribonucléique double brin selon la revendication 4, dans laquelle le surplomb du brin antisens comprend un uracile ou des nucléotides qui sont complémentaires de l'ARN prégénomique et/ou de l'ARNm codant pour la protéine nécessaire à la réplication ou à la pathogenèse du virus de l'hépatite B.

**6.** Molécule d'acide ribonucléique double brin selon la revendication 1, dans laquelle le brin sens comprend en outre un surplomb en 3' ayant une longueur de 1 à 5 nucléotides.

**7.** Molécule d'acide ribonucléique double brin selon la revendication 6, dans laquelle le surplomb du brin sens comprend un uracile ou des nucléotides qui sont identiques à l'ARN prégénomique et/ou à l'ARNm codant pour la protéine nécessaire à la réplication ou à la pathogenèse du virus de l'hépatite B.

**8.** Molécule d'acide ribonucléique double brin selon la revendication 1, la molécule d'acide ribonucléique double brin comprenant au moins un nucléotide modifié choisi dans le groupe consistant en : un nucléotide modifié par un 2'-O-méthyle, un nucléotide comprenant un groupe 5'-phosphorothioate, un nucléotide terminal lié à un dérivé de cholestéryle, un nucléotide terminal lié à un groupe bisdécylamide d'acide dodécanoïque, un nucléotide modifié par un 2'-désoxo-2'-fluoro, un nucléotide modifié par un 2'-désoxy, un nucléotide verrouillé, un nucléotide abasique, une désoxythymidine, une désoxythymidine inversée, un nucléotide modifié par un 2'-amino, un nucléotide modifié par un 2'-alkyle, un morpholino nucléotide, un phosphoramidate, et un nucléotide comprenant une base non-naturelle.

**9.** Molécule d'acide ribonucléique double brin selon la revendication 8, ladite molécule d'acide ribonucléique double brin contenant un nucléotide modifié par un 2'-O-méthyle, un nucléotide comprenant un groupe 5'-phosphorothioate et une désoxythymidine.

**10.** Molécule d'acide ribonucléique double brin selon la revendication 9, dans laquelle ledit brin sens ou ledit brin antisens comprend un surplomb de 1 à 2 désoxythymidines.

**11.** Molécule d'acide ribonucléique double brin selon la revendication 10, la molécule d'acide ribonucléique double brin comprenant les paires de séquences choisies dans le groupe consistant en les SEQ ID NO: 326/490 et 337/501.

**12.** Molécule d'acide ribonucléique double brin selon la revendication 1, ladite molécule d'acide ribonucléique double brin comprenant une séquence d'acide nucléique codant pour ledit brin sens ou ledit brin antisens.

**13.** Composition pharmaceutique comprenant :

(a) au moins une molécule d'acide ribonucléique double brin telle que définie dans l'une quelconque des revendications 1 à 12 ; ou
(b) au moins une séquence d'acide nucléique codant pour un brin sens et un brin antisens comprenant la molécule d'acide ribonucléique double brin telle que définie dans l'une quelconque des revendications 1 à 12.

**14.** Composition pharmaceutique comprenant :

(a) au moins une première molécule d'acide ribonucléique double brin comprenant la molécule d'acide ribonucléique double brin telle que définie dans l'une quelconque des revendications 1 à 12 et au moins une seconde molécule d'acide ribonucléique double brin susceptible d'inhiber l'expression du gène du virus de l'hépatite B, les séquences de la première et de la seconde molécules d'acide ribonucléique double brin étant différentes ; ou
(b) au moins une séquence d'acide nucléique codant pour un brin sens et un brin antisens d'une première molécule d'acide ribonucléique double brin telle que définie dans l'une quelconque des revendications 1 à 12 et au moins une seconde séquence d'acide nucléique codant pour un brin sens ou un brin antisens d'une molécule d'acide ribonucléique double brin susceptible d'inhiber l'expression du gène du virus de l'hépatite B, la séquence du brin sens ou celle du brin antisens codées par le premier et le second acides nucléiques étant différentes.

**15.** Composition pharmaceutique selon la revendication 14, comprenant en outre un véhicule, un stabilisant et/ou un diluant pharmaceutiquement acceptables.

Table 1. Core sequences of dsRNAs targeting Hepatitis B Virus gene.

| SEQ ID NO | sense strand sequence (5'-3') | SEQ ID NO | antisense strand sequence (5'-3') |
|---|---|---|---|
| 1 | CAAGGUAUGUUGCCCGUUU | 157 | AAACGGGCAACAUACCUUG |
| 2 | CUGUAGGCAUAAAUUGGUA | 158 | TACCAAUUUAUGCCUACAG |
| 3 | UCUGCGGCGUUUUAUCAUA | 159 | UAUGAUAAAACGCCGCAGA |
| 3 | UCUGCGGCGUUUUAUCAUA | 160 | TAUGAUAAAACGCCGCAGA |
| 4 | ACCUCUGCCUAAUCAUCUC | 161 | GAGAUGAUUAGGCAGAGGU |
| 5 | UUUACUAGUGCCAUUUGUA | 162 | TACAAAUGGCACUAGUAAA |
| 6 | ACCUCUGCCUAAUCAUCUA | 163 | TAGAUGAUUAGGCAGAGGU |
| 7 | CUGUAGGCAUAAAUUGGUC | 164 | GACCAAUUUAUGCCUACAG |
| 8 | UGUCUGCGGCGUUUUAUCA | 165 | UGAUAAAACGCCGCAGACA |
| 8 | UGUCUGCGGCGUUUUAUCA | 166 | TGAUAAAACGCCGCAGACA |
| 9 | UACUAGUGCCAUUUGUUCA | 167 | UGAACAAAUGGCACUAGUA |
| 9 | UACUAGUGCCAUUUGUUCA | 168 | TGAACAAAUGGCACUAGUA |
| 10 | CAACUUUUUCACCUCUGCA | 169 | TGCAGAGGUGAAAAAGUUG |
| 11 | CCAUUUGUUCAGUGGUUCG | 170 | CGAACCACUGAACAAAUGG |
| 12 | CCAAGUGUUUGCUGACGCA | 171 | UGCGUCAGCAAACACUUGG |
| 12 | CCAAGUGUUUGCUGACGCA | 172 | TGCGUCAGCAAACACUUGG |
| 13 | CCAUUUGUUCAGUGGUUCA | 173 | TGAACCACUGAACAAAUGG |
| 14 | UUUACUAGUGCCAUUUGUU | 174 | AACAAAUGGCACUAGUAAA |
| 15 | CACCUCUGCCUAAUCAUCA | 175 | TGAUGAUUAGGCAGAGGUG |
| 16 | CUGGCUCAGUUUACUAGUG | 176 | CACUAGUAAACUGAGCCAG |
| 17 | CAAGGUAUGUUGCCCGUUA | 177 | TAACGGGCAACAUACCUUG |
| 18 | CUGGCUCAGUUUACUAGUA | 178 | TACUAGUAAACUGAGCCAG |
| 19 | GAGGCUGUAGGCAUAAAUU | 179 | AAUUUAUGCCUACAGCCUC |
| 20 | CAGUUUACUAGUGCCAUUU | 180 | AAAUGGCACUAGUAAACUG |
| 21 | AGGUAUGUUGCCCGUUUGU | 181 | ACAAACGGGCAACAUACCU |
| 22 | UAUGUUGCCCGUUUGUCCA | 182 | UGGACAAACGGGCAACAUA |
| 23 | GAGGCUGUAGGCAUAAAUA | 183 | TAUUUAUGCCUACAGCCUC |
| 24 | GUCUGCGGCGUUUUAUCAU | 184 | AUGAUAAAACGCCGCAGAC |
| 25 | CAACUUUUUCACCUCUGCC | 185 | GGCAGAGGUGAAAAAGUUG |
| 26 | CCGUGUGCACUUCGCUUCA | 186 | UGAAGCGAAGUGCACACGG |
| 26 | CCGUGUGCACUUCGCUUCA | 187 | TGAAGCGAAGUGCACACGG |
| 27 | UCAAGGUAUGUUGCCCGUA | 188 | TACGGGCAACAUACCUUGA |
| 28 | CAGUUUACUAGUGCCAUUA | 189 | TAAUGGCACUAGUAAACUG |
| 29 | UGGUGGACUUCUCUCAAUU | 190 | AAUUGAGAGAAGUCCACCA |
| 30 | AGGUAUGUUGCCCGUUUGA | 191 | TCAAACGGGCAACAUACCU |
| 31 | CUGCUCGUGUUACAGGCGG | 192 | CCGCCUGUAACACGAGCAG |
| 32 | UAUGUUGCCCGUUUGUCCU | 193 | AGGACAAACGGGCAACAUA |
| 33 | UCAAGGUAUGUUGCCCGUU | 194 | AACGGGCAACAUACCUUGA |
| 34 | UCUUAUCAACACUUCCGGA | 195 | UCCGGAAGUGUUGAUAAGA |
| 34 | UCUUAUCAACACUUCCGGA | 196 | TCCGGAAGUGUUGAUAAGA |
| 35 | CACCUCUGCCUAAUCAUCU | 197 | AGAUGAUUAGGCAGAGGUG |
| 36 | AUAAGAGGACUCUUGGACU | 198 | AGUCCAAGAGUCCUCUUAU |
| 37 | GUCUGCGGCGUUUUAUCAA | 199 | TUGAUAAAACGCCGCAGAC |
| 38 | GGCGCUGAAUCCCGCGGAC | 200 | GUCCGCGGGAUUCAGCGCC |

FIG. 1

| | | | |
|---|---|---|---|
| 39 | CGCGUCGCAGAAGAUCUCA | 201 | UGAGAUCUUCUGCGACGCG |
| 40 | AAUGUCAACGACCGACCUU | 202 | AAGGUCGGUCGUUGACAUU |
| 41 | GCUCAGUUUACUAGUGCCA | 203 | UGGCACUAGUAAACUGAGC |
| 42 | UGGUGGACUUCUCUCAAUA | 204 | TAUUGAGAGAAGUCCACCA |
| 43 | AUCGCCGCGUCGCAGAAGA | 205 | UCUUCUGCGACGCGGCGAU |
| 44 | GCCAUUUGUUCAGUGGUUC | 206 | GAACCACUGAACAAAUGGC |
| 45 | CGAUCCAUACUGCGGAACU | 207 | AGUUCCGCAGUAUGGAUCG |
| 46 | UCACCUCUGCCUAAUCAUC | 208 | GAUGAUUAGGCAGAGGUGA |
| 47 | GUGGACUUCUCUCAAUUUU | 209 | AAAAUUGAGAGAAGUCCAC |
| 48 | GGGUCACCAUAUUCUUGGG | 210 | CCCAAGAAUAUGGUGACCC |
| 49 | GCCGCGUCGCAGAAGAUCU | 211 | AGAUCUUCUGCGACGCGGC |
| 50 | UCAAUCGCCGCGUCGCAGA | 212 | UCUGCGACGCGGCGAUUGA |
| 51 | UGGAUGUGUCUGCGGCGUU | 213 | AACGCCGCAGACACAUCCA |
| 52 | UACUGUUCAAGCCUCCAAG | 214 | CUUGGAGGCUUGAACAGUA |
| 53 | GUUUACUAGUGCCAUUUGU | 215 | ACAAAUGGCACUAGUAAAC |
| 54 | ACUAGUGCCAUUUGUUCAG | 216 | CUGAACAAAUGGCACUAGU |
| 55 | CCGCGUCGCAGAAGAUCUC | 217 | GAGAUCUUCUGCGACGCGG |
| 56 | UAUCUUAUCAACACUUCCG | 218 | CGGAAGUGUUGAUAAGAUA |
| 57 | GGCCAAAAUUCGCAGUCCC | 219 | GGGACUGCGAAUUUUGGCC |
| 58 | UUCACCUCUGCCUAAUCAU | 220 | AUGAUUAGGCAGAGGUGAA |
| 59 | CUCAGUUUACUAGUGCCAU | 221 | AUGGCACUAGUAAACUGAG |
| 60 | UGUUGCCCGUUUGUCCUCU | 222 | AGAGGACAAACGGGCAACA |
| 61 | UAGUGCCAUUUGUUCAGUG | 223 | CACUGAACAAAUGGCACUA |
| 62 | AGGCUGUAGGCAUAAAUUG | 224 | CAAUUUAUGCCUACAGCCU |
| 63 | AUGUGUCUGCGGCGUUUUA | 225 | UAAAACGCCGCAGACACAU |
| 63 | AUGUGUCUGCGGCGUUUUA | 226 | TAAAACGCCGCAGACACAU |
| 64 | ACUUCGCUUCACCUCUGCA | 227 | UGCAGAGGUGAAGCGAAGU |
| 65 | CGUGUGCACUUCGCUUCAC | 228 | GUGAAGCGAAGUGCACACG |
| 66 | GUGGUGGACUUCUCUCAAU | 229 | AUUGAGAGAAGUCCACCAC |
| 67 | UGUGUCUGCGGCGUUUUAU | 230 | AUAAAACGCCGCAGACACA |
| 68 | AAGGUAUGUUGCCCGUUUG | 231 | CAAACGGGCAACAUACCUU |
| 69 | UCAACGACCGACCUUGAGG | 232 | CCUCAAGGUCGGUCGUUGA |
| 70 | CAUAAGAGGACUCUUGGAC | 233 | GUCCAAGAGUCCUCUUAUG |
| 71 | GUCAACGACCGACCUUGAG | 234 | CUCAAGGUCGGUCGUUGAC |
| 72 | AUAUUCUUGGGAACAAGAG | 235 | CUCUUGUUCCCAAGAAUAU |
| 73 | UGCUCGUGUUACAGGCGGG | 236 | CCCGCCUGUAACACGAGCA |
| 74 | CAAUCGCCGCGUCGCAGAA | 237 | UUCUGCGACGCGGCGAUUG |
| 75 | ACUGUUCAAGCCUCCAAGC | 238 | GCUUGGAGGCUUGAACAGU |
| 76 | CGCCGCGUCGCAGAAGAUC | 239 | GAUCUUCUGCGACGCGGCG |
| 77 | CAUUUGUUCAGUGGUUCGU | 240 | ACGAACCACUGAACAAAUG |
| 78 | CGCUGAAUCCCGCGGACGA | 241 | UCGUCCGCGGGAUUCAGCG |
| 79 | UGGGUCACCAUAUUCUUGG | 242 | CCAAGAAUAUGGUGACCCA |
| 80 | UCCUCUGCCGAUCCAUACU | 243 | AGUAUGGAUCGGCAGAGGA |
| 81 | AUGUCAACGACCGACCUUG | 244 | CAAGGUCGGUCGUUGACAU |
| 82 | CCUCUGCCUAAUCAUCUCA | 245 | UGAGAUGAUUAGGCAGAGG |
| 83 | ACCGUGUGCACUUCGCUUC | 246 | GAAGCGAAGUGCACACGGU |
| 84 | UGCCGAUCCAUACUGCGGA | 247 | UCCGCAGUAUGGAUCGGCA |

FIG. 1

| | | | |
|---|---|---|---|
| 85 | CAGAGUCUAGACUCGUGGU | 248 | ACCACGAGUCUAGACUCUG |
| 86 | CUGUUCAAGCCUCCAAGCU | 249 | AGCUUGGAGGCUUGAACAG |
| 87 | GGAGGCUGUAGGCAUAAAU | 250 | AUUUAUGCCUACAGCCUCC |
| 88 | AGGAGGCUGUAGGCAUAAA | 251 | UUUAUGCCUACAGCCUCCU |
| 89 | GGUGGACUUCUCUCAAUUU | 252 | AAAUUGAGAGAAGUCCACC |
| 90 | GCAACUUUUUCACCUCUGC | 253 | GCAGAGGUGAAAAAGUUGC |
| 91 | CUGCUCGUGUUACAGGCGA | 254 | TCGCCUGUAACACGAGCAG |
| 92 | CUAGUGCCAUUUGUUCAGU | 255 | ACUGAACAAAUGGCACUAG |
| 93 | CUGCCGAUCCAUACUGCGG | 256 | CCGCAGUAUGGAUCGGCAG |
| 94 | GUGUGCACUUCGCUUCACC | 257 | GGUGAAGCGAAGUGCACAC |
| 95 | GCUCGUGUUACAGGCGGGC | 258 | GCCCGCCUGUAACACGAGC |
| 96 | CCUAUCUUAUCAACACUUC | 259 | GAAGUGUUGAUAAGAUAGG |
| 97 | UCUCAAUCGCCGCGUCGCA | 260 | UGCGACGCGGCGAUUGAGA |
| 98 | GCCCGUCUGUGCCUUCUCA | 261 | UGAGAAGGCACAGACGGGC |
| 99 | CUAUCUUAUCAACACUUCC | 262 | GGAAGUGUUGAUAAGAUAG |
| 100 | AUGUUGCCCGUUUGUCCUC | 263 | GAGGACAAACGGGCAACAU |
| 101 | GUAUGUUGCCCGUUUGUCC | 264 | GGACAAACGGGCAACAUAC |
| 102 | CUUCGCUUCACCUCUGCAC | 265 | GUGCAGAGGUGAAGCGAAG |
| 103 | UGUGCACUUCGCUUCACCU | 266 | AGGUGAAGCGAAGUGCACA |
| 104 | GCCAAAAUUCGCAGUCCCG | 267 | CGGGACUGCGAAUUUUGGC |
| 105 | CCUGCUCGUGUUACAGGCG | 268 | CGCCUGUAACACGAGCAGG |
| 106 | UGGAGUGUGGAUUCGCACU | 269 | AGUGCGAAUCCACACUCCA |
| 107 | AACGACCGACCUUGAGGCA | 270 | UGCCUCAAGGUCGGUCGUU |
| 108 | ACAGAGUCUAGACUCGUGG | 271 | CCACGAGUCUAGACUCUGU |
| 109 | AAUCGCCGCGUCGCAGAAG | 272 | CUUCUGCGACGCGGCGAUU |
| 110 | GGUAUGUUGCCCGUUUGUC | 273 | GACAAACGGGCAACAUACC |
| 111 | GCCGAUCCAUACUGCGGAA | 274 | UUCCGCAGUAUGGAUCGGC |
| 112 | GCCCUAUCUUAUCAACACU | 275 | AGUGUUGAUAAGAUAGGGC |
| 113 | AGUUUACUAGUGCCAUUUG | 276 | CAAAUGGCACUAGUAAACU |
| 114 | UGUCAACGACCGACCUUGA | 277 | UCAAGGUCGGUCGUUGACA |
| 115 | ACUUCUCUCAAUUUUCUAG | 278 | CUAGAAAAUUGAGAGAAGU |
| 116 | GCGCGGGACGUCCUUUGUC | 279 | GACAAAGGACGUCCCGCGC |
| 117 | UCUAGACUCGUGGUGGACU | 280 | AGUCCACCACGAGUCUAGA |
| 118 | GAUCCAUACUGCGGAACUC | 281 | GAGUUCCGCAGUAUGGAUC |
| 119 | CUCUGCCGAUCCAUACUGC | 282 | GCAGUAUGGAUCGGCAGAG |
| 120 | UCUGCCGAUCCAUACUGCG | 283 | CGCAGUAUGGAUCGGCAGA |
| 121 | CCUCUGCCGAUCCAUACUG | 284 | CAGUAUGGAUCGGCAGAGG |
| 122 | GCACCUCUCUUUACGCGGU | 285 | ACCGCGUAAAGAGAGGUGC |
| 123 | AAGAACUCCCUCGCCUCGC | 286 | GCGAGGCGAGGGAGUUCUU |
| 124 | GAACUCCCUCGCCUCGCAG | 287 | CUGCGAGGCGAGGGAGUUC |
| 125 | UCUCUCAAUUUUCUAGGGC | 288 | GCCCUAGAAAAUUGAGAGA |
| 126 | GGGCGCACCUCUCUUUACG | 289 | CGUAAAGAGAGGUGCGCCC |
| 127 | CCGAUCCAUACUGCGGAAC | 290 | GUUCCGCAGUAUGGAUCGG |
| 128 | AACUCCCUCGCCUCGCAGA | 291 | UCUGCGAGGCGAGGGAGUU |
| 129 | CUCCUCUGCCGAUCCAUAC | 292 | GUAUGGAUCGGCAGAGGAG |
| 130 | GGAGUGUGGAUUCGCACUC | 293 | GAGUGCGAAUCCACACUCC |
| 131 | CGGGCGCACCUCUCUUUAC | 294 | GUAAAGAGAGGUGCGCCCG |

FIG. 1

| | | | |
|---|---|---|---|
| 132 | GUCUCAAUCGCCGCGUCGC | 295 | GCGACGCGGCGAUUGAGAC |
| 133 | AUCCAUACUGCGGAACUCC | 296 | GGAGUUCCGCAGUAUGGAU |
| 134 | CGCACCUCUCUUUACGCGG | 297 | CCGCGUAAAGAGAGGUGCG |
| 135 | CAACGACCGACCUUGAGGC | 298 | GCCUCAAGGUCGGUCGUUG |
| 136 | CCAUACUGCGGAACUCCUA | 299 | UAGGAGUUCCGCAGUAUGG |
| 137 | UGAAUCCCGCGGACGACCC | 300 | GGGUCGUCCGCGGGAUUCA |
| 138 | AGAACUCCCUCGCCUCGCA | 301 | UGCGAGGCGAGGGAGUUCU |
| 139 | GGCGCACCUCUCUUUACGC | 302 | GCGUAAAGAGAGGUGCGCC |
| 140 | GCGCACCUCUCUUUACGCG | 303 | CGCGUAAAGAGAGGUGCGC |
| 141 | GCUGAAUCCCGCGGACGAC | 304 | GUCGUCCGCGGGAUUCAGC |
| 142 | CACUUCGCUUCACCUCUGC | 305 | GCAGAGGUGAAGCGAAGUG |
| 143 | CUCAAUCGCCGCGUCGCAG | 306 | CUGCGACGCGGCGAUUGAG |
| 144 | UCCCGUCGGCGCUGAAUCC | 307 | GGAUUCAGCGCCGACGGGA |
| 145 | CUGAAUCCCGCGGACGACC | 308 | GGUCGUCCGCGGGAUUCAG |
| 146 | AGAGUCUAGACUCGUGGUG | 309 | CACCACGAGUCUAGACUCU |
| 147 | UCCAUACUGCGGAACUCCU | 310 | AGGAGUUCCGCAGUAUGGA |
| 148 | GCGCUGAAUCCCGCGGACG | 311 | CGUCCGCGGGAUUCAGCGC |
| 149 | AGUGUGGAUUCGCACUCCU | 312 | AGGAGUGCGAAUCCACACU |
| 150 | CCCUGCUCGUGUUACAGGC | 313 | GCCUGUAACACGAGCAGGG |
| 151 | GAAUCCCGCGGACGACCCG | 314 | CGGGUCGUCCGCGGGAUUC |
| 152 | AAGCUGUGCCUUGGGUGGC | 315 | GCCACCCAAGGCACAGCUU |
| 153 | GCCCUGCUCGUGUUACAGG | 316 | CCUGUAACACGAGCAGGGC |
| 154 | GUCCCGUCGGCGCUGAAUC | 317 | GAUUCAGCGCCGACGGGAC |
| 155 | AUCUUAUCAACACUUCCGG | 318 | CCGGAAGUGUUGAUAAGAU |
| 156 | CUUAUCAACACUUCCGGAA | 319 | UUCCGGAAGUGUUGAUAAG |
| 156 | CUUAUCAACACUUCCGGAA | 320 | TUCCGGAAGUGUUGAUAAG |

FIG. 1

Table 2. Activity testing in psiCHECK2 reporter system in COS7 cells.

| SEQ ID NO | sense strand sequence (5'-3') | SEQ ID NO | antisense strand sequence (5'-3') | Activity testing in psiCHECK2 reporter system in COS7 cells: 10 nM siRNA, mean remaining mRNA (%) | 10 nM siRNA, standard deviation (%) | 1 nM siRNA, mean remaining mRNA (%) | 1 nM siRNA, standard deviation (%) |
|---|---|---|---|---|---|---|---|
| 321 | caAGGuAuGuuGcccGuuudTsdT | 485 | AAACGGGcAAcAuACCUUGdTsdT | 13 | 1 | 13 | 1 |
| 322 | CfuGfuAfgGfcAfuAfaAfuUfgGfuAf(invdT) | 486 | pdTAfcCfaAfuUfuAfuGfcCfuAfcAfgdTsdT | 8 | 2 | 14 | 2 |
| 323 | ucuGcGGcGuuuuAucAuAdTsdT | 487 | uAUGAuAAAACGCCGcAGAdTsdT | 15 | 7 | 29 | 11 |
| 324 | UfcUfgCfgGfcGfuUfuUfaUfcAfuAf(invdT) | 488 | pdTAfuGfaUfaAfaAfcGfcCfgCfaGfadTsdT | 6 | 2 | 15 | 4 |
| 325 | accucuGccuAAucAucucdTsdT | 489 | GAGAUGAUuAGGcAGAGGUdTsdT | 17 | 1 | 16 | 2 |
| 326 | UfuUfaCfuAfgUfgCfcAfuUfuGfuAf(invdT) | 490 | pdTAfcAfaAfuGfgCfaCfuAfgUfaAfadTsdT | 8 | 0 | 17 | 1 |
| 327 | AfcCfuCfuGfcCfuAfaUfcAfuCfuAf(invdT) | 491 | pdTAfgAfuGfaUfuAfgGfcAfgAfgGfudTsdT | 6 | 2 | 19 | 3 |
| 328 | cuGuAGGcAuAAAuuGGucdTsdT | 492 | GACcAAUUuAUGCCuAcAGdTsdT | 23 | 2 | 28 | 6 |
| 329 | ugucuGcGGcGuuuuAucAdTsdT | 493 | UGAuAAAACGCCGcAGAcAdTsdT | 33 | 3 | 34 | 10 |
| 330 | UfgUfcUfgCfgGfcGfuUfuUfaUfcAf(invdT) | 494 | pdTGfaUfaAfaAfcGfcCfgCfaGfaCfadTsdT | 6 | 0 | 20 | 3 |
| 331 | uacuAGuGccAuuuGuucAdTsdT | 495 | UGAAcAAAUGGcACuAGuAdTsdT | 18 | 3 | 20 | 2 |
| 332 | UfaCfuAfgUfgCfcAfuUfuGfuUfcAf(invdT) | 496 | pdTGfaAfcAfaAfuGfgCfaCfuAfgUfadTsdT | 6 | 2 | 21 | 4 |
| 333 | CfaAfcUfuUfuUfcAfcCfuCfuGfcAf(invdT) | 497 | pdTGfcAfgAfgGfuGfaAfaAfaGfuUfgdTsdT | 6 | 2 | 21 | 3 |
| 334 | ccAuuuGuucAGuGGuucGdTsdT | 498 | CGAACcACUGAAcAAAUGGdTsdT | 12 | 1 | 21 | 1 |
| 335 | ccAAGuGuuuGcuGAcGcAdTsdT | 499 | UGCGUcAGcAAAcACUUGGdTsdT | 18 | 2 | 23 | 4 |
| 336 | CfcAfaGfuGfuUfuGfcUfgAfcGfcAf(invdT) | 500 | pdTGfcGfuCfaGfcAfaAfcAfcUfuGfgdTsdT | 8 | 1 | 23 | 5 |
| 337 | CfcAfuUfuGfuUfcAfgUfgGfuUfcAf(invdT) | 501 | pdTGfaAfcCfaCfuGfaAfcAfaAfuGfgdTsdT | 7 | 2 | 24 | 3 |
| 338 | uuuAcuAGuGccAuuuGuudTsdT | 502 | AAcAAAUGGcACuAGuAAAdTsdT | 21 | 2 | 24 | 4 |
| 339 | CfaCfcUfcUfgCfcUfaAfuCfaUfcAf(invdT) | 503 | pdTGfaUfgAfuUfaGfgCfaGfaGfgUfgdTsdT | 9 | 0 | 25 | 2 |
| 340 | cuGGcucAGuuuAcuAGuGdTsdT | 504 | cACuAGuAAACUGAGCcAGdTsdT | 34 | 3 | 29 | 7 |
| 341 | CfaAfgGfuAfuGfuUfgCfcCfgUfuAf(invdT) | 505 | pdTAfaCfgGfgCfaAfcAfuAfcCfuUfgdTsdT | 8 | 0 | 31 | 3 |
| 342 | CfuGfgCfuCfaGfuUfuAfcUfaGfuAf(invdT) | 506 | pdTAfcUfaGfuAfaAfcUfgAfgCfcAfgdTsdT | 11 | 3 | 32 | 5 |
| 343 | gaGGcuGuAGGcAuAAAuudTsdT | 507 | AAUUuAUGCCuAcAGCCUCdTsdT | 16 | 1 | 32 | 8 |

FIG. 2

| | | | | | | | |
|---|---|---|---|---|---|---|---|
| 344 | caGuuuAcuAGuGccAuuudTsdT | 508 | AAAUGGcACuAGuAAACUGdTsdT | 37 | 1 | 33 | 8 |
| 345 | agGuAuGuuGcccGuuuGudTsdT | 509 | AcAAACGGGcAAcAuACCUdTsdT | 33 | 3 | 34 | 4 |
| 346 | UfaUfgUfuGfcCfcGfuUfuGfuCfcAf(invdT) | 510 | pdTGfgAfcAfaAfcGfgGfcAfaCfaUfadTsdT | 9 | 3 | 35 | 5 |
| 347 | GfaGfgCfuGfuAfgGfcAfuAfaAfuAf(invdT) | 511 | pdTAfuUfuAfuGfcCfuAfcAfgCfcUfcdTsdT | 9 | 1 | 36 | 4 |
| 348 | gucuGcGGcGuuuuAucAudTsdT | 512 | AUGAuAAAACGCCGcAGACdTsdT | 26 | 3 | 36 | 14 |
| 349 | caAcuuuuucAccucuGccdTsdT | 513 | GGcAGAGGUGAAAAAGUUGdTsdT | 24 | 2 | 37 | 9 |
| 350 | ccGuGuGcAcuucGcuucAdTsdT | 514 | UGAAGCGAAGUGcAcACGGdTsdT | 13 | 1 | 16 | 5 |
| 351 | CfcGfuGfuGfcAfcUfuCfgCfuUfcAf(invdT) | 515 | pdTGfaAfgCfgAfaGfuGfcAfcAfcGfgdTsdT | 13 | 2 | 38 | 4 |
| 352 | UfcAfaGfgUfaUfgUfuGfcCfcGfuAf(invdT) | 516 | pdTAfcGfgGfcAfaCfaUfaCfcUfuGfadTsdT | 12 | 1 | 38 | 4 |
| 353 | CfaGfuUfuAfcUfaGfuGfcCfaUfuAf(invdT) | 517 | pdTAfaUfgGfcAfcUfaGfuAfaAfcUfgdTsdT | 12 | 2 | 38 | 5 |
| 354 | ugGuGGAcuucucucAAuudTsdT | 518 | AAUUGAGAGAAGUCcACcAdTsdT | 24 | 6 | 39 | 16 |
| 355 | AfgGfuAfuGfuUfgCfcCfgUfuUfgAf(invdT) | 519 | pdTCfaAfaCfgGfgCfaAfcAfuAfcCfudTsdT | 18 | 1 | 40 | 4 |
| 356 | cuGcucGuGuuAcAGGcGGdTsdT | 520 | CCGCCUGuAAcACGAGcAGdTsdT | 26 | 2 | 40 | 11 |
| 357 | uauGuuGcccGuuuGuccudTsdT | 521 | AGGAcAAACGGGcAAcAuAdTsdT | 42 | 1 | 40 | 3 |
| 358 | ucAAGGuAuGuuGcccGuudTsdT | 522 | AACGGGcAAcAuACCUUGAdTsdT | 31 | 4 | 42 | 12 |
| 359 | ucuuAucAAcAcuuccGGAdTsdT | 523 | UCCGGAAGUGUUGAuAAGAdTsdT | 35 | 2 | 43 | 38 |
| 360 | UfcUfuAfuCfaAfcAfcUfuCfcGfgAf(invdT) | 524 | pdTCfcGfgAfaGfuGfuUfgAfuAfaGfadTsdT | 32 | 2 | 46 | 3 |
| 361 | caccucuGccuAAucAucudTsdT | 525 | AGAUGAUuAGGcAGAGGUGdTsdT | 31 | 3 | 47 | 8 |
| 362 | auAAGAGGAcucuuGGAcudTsdT | 526 | AGUCcAAGAGUCCUCUuAUdTsdT | 28 | 1 | 49 | 6 |
| 363 | GfuCfuGfcGfgCfgUfuUfuAfuCfaAf(invdT) | 527 | pdTUfgAfuAfaAfaCfgCfcGfcAfgAfcdTsdT | 15 | 0 | 51 | 4 |
| 364 | ggcGcuGAAucccGcGGAcdTsdT | 528 | GUCCGCGGGAUUcAGCGCCdTsdT | 24 | 3 | 51 | 10 |
| 365 | cgcGucGcAGAAGAucucAdTsdT | 529 | UGAGAUCUUCUGCGACGCGdTsdT | 46 | 3 | 53 | 6 |
| 366 | aauGucAAcGAccGAccuudTsdT | 530 | AAGGUCGGUCGUUGAcAUUdTsdT | 40 | 1 | 54 | 8 |
| 367 | gcucAGuuuAcuAGuGccAdTsdT | 531 | UGGcACuAGuAAACUGAGCdTsdT | 37 | 5 | 51 | 4 |
| 368 | UfgGfuGfgAfcUfuCfuCfuCfaAfuAf(invdT) | 532 | pdTAfuUfgAfgAfgAfaGfuCfcAfcCfadTsdT | 20 | 4 | 58 | 6 |
| 369 | aucGccGcGucGcAGAAGAdTsdT | 533 | UCUUCUGCGACGCGGCGAUdTsdT | 57 | 6 | 58 | 1 |
| 370 | gccAuuuGuucAGuGGuucdTsdT | 534 | GAACcACUGAAcAAAUGGCdTsdT | 36 | 3 | 60 | 6 |
| 371 | cgAuccAuAcuGcGGAAcudTsdT | 535 | AGUUCCGcAGuAUGGAUCGdTsdT | 43 | 8 | 61 | 9 |
| 372 | ucAccucuGccuAAucAucdTsdT | 536 | GAUGAUuAGGcAGAGGUGAdTsdT | 48 | 4 | 61 | 10 |
| 373 | guGGAcuucucucAAuuuudTsdT | 537 | AAAAUUGAGAGAAGUCcACdTsdT | 31 | 4 | 61 | 5 |
| 374 | ggGucAccAuAuucuuGGGdTsdT | 538 | CCcAAGAAuAUGGUGACCCdTsdT | 58 | 6 | 62 | 10 |

FIG. 2

| | | | | | | | |
|---|---|---|---|---|---|---|---|
| 375 | gccGcGucGcAGAAGAucudTsdT | 539 | AGAUCUUCUGCGACGCGGCdTsdT | 59 | 3 | 64 | 7 |
| 376 | ucAAucGccGcGucGcAGAdTsdT | 540 | UCUGCGACGCGGCGAUUGAdTsdT | 59 | 1 | 64 | 9 |
| 377 | ugGAuGuGucuGcGGcGuudTsdT | 541 | AACGCCGcAGAcAcAUCcAdTsdT | 44 | 8 | 65 | 12 |
| 378 | uacuGuucAAGccuccAAGdTsdT | 542 | CUUGGAGGCUUGAAcAGuAdTsdT | 51 | 2 | 65 | 32 |
| 379 | guuuAcuAGuGccAuuuGudTsdT | 543 | AcAAAUGGcACuAGuAAACdTsdT | 44 | 5 | 66 | 6 |
| 380 | acuAGuGccAuuuGuucAGdTsdT | 544 | CUGAAcAAAUGGcACuAGUdTsdT | 56 | 0 | 66 | 5 |
| 381 | ccGcGucGcAGAAGAucucdTsdT | 545 | GAGAUCUUCUGCGACGCGGdTsdT | 59 | 3 | 67 | 11 |
| 382 | uaucuuAucAAcAcuuccGdTsdT | 546 | CGGAAGUGUUGAuAAGAuAdTsdT | 37 | 1 | 67 | 51 |
| 383 | ggccAAAAuucGcAGucccdTsdT | 547 | GGGACUGCGAAUUUUGGCCdTsdT | 49 | 6 | 67 | 7 |
| 384 | uucAccucuGccuAAucAudTsdT | 548 | AUGAUuAGGcAGAGGUGAAdTsdT | 50 | 4 | 68 | 7 |
| 385 | cucAGuuuAcuAGuGccAudTsdT | 549 | AUGGcACuAGuAAACUGAGdTsdT | 52 | 2 | 68 | 6 |
| 386 | uguuGcccGuuuGuccucudTsdT | 550 | AGAGGAcAAACGGGcAAcAdTsdT | 50 | 2 | 69 | 4 |
| 387 | uaGuGccAuuuGuucAGuGdTsdT | 551 | cACUGAAcAAAUGGcACuAdTsdT | 46 | 1 | 70 | 8 |
| 388 | agGcuGuAGGcAuAAAuuGdTsdT | 552 | cAAUUuAUGCCuAcAGCCUdTsdT | 69 | 3 | 71 | 13 |
| 389 | auGuGucuGcGGcGuuuuAdTsdT | 553 | uAAAACGCCGcAGAcAcAUdTsdT | 17 | 6 | 33 | 12 |
| 390 | AfuGfuGfuCfuGfcGfgCfgUfuUfuAf(invdT) | 554 | pdTAfaAfaCfgCfcGfcAfgAfcAfcAfudTsdT | 24 | 3 | 72 | 4 |
| 391 | acuucGcuucAccucuGcAdTsdT | 555 | UGcAGAGGUGAAGCGAAGUdTsdT | 49 | 4 | 73 | 4 |
| 392 | cguGuGcAcuucGcuucAcdTsdT | 556 | GUGAAGCGAAGUGcAcACGdTsdT | 45 | 3 | 73 | 10 |
| 393 | guGGuGGAcuucucucAAudTsdT | 557 | AUUGAGAGAAGUCcACcACdTsdT | 47 | 5 | 73 | 5 |
| 394 | uguGucuGcGGcGuuuuAudTsdT | 558 | AuAAAACGCCGcAGAcAcAdTsdT | 62 | 8 | 75 | 14 |
| 395 | aaGGuAuGuuGcccGuuuGdTsdT | 559 | cAAACGGGcAAcAuACCUUdTsdT | 61 | 3 | 76 | 2 |
| 396 | ucAAcGAccGAccuuGAGGdTsdT | 560 | CCUcAAGGUCGGUCGUUGAdTsdT | 57 | 1 | 76 | 16 |
| 397 | cauAAGAGGAcucuuGGAcdTsdT | 561 | GUCcAAGAGUCCUCUuAUGdTsdT | 62 | 4 | 76 | 4 |
| 398 | gucAAcGAccGAccuuGAGdTsdT | 562 | CUcAAGGUCGGUCGUUGACdTsdT | 55 | 2 | 77 | 13 |
| 399 | auAuucuuGGGAAcAAGAGdTsdT | 563 | CUCUUGUUCCcAAGAAuAUdTsdT | 56 | 5 | 77 | 11 |
| 400 | ugcucGuGuuAcAGGcGGGdTsdT | 564 | CCCGCCUGuAAcACGAGcAdTsdT | 61 | 5 | 78 | 4 |
| 401 | caAucGccGcGucGcAGAAdTsdT | 565 | UUCUGCGACGCGGCGAUUGdTsdT | 65 | 1 | 78 | 4 |
| 402 | acuGuucAAGccuccAAGcdTsdT | 566 | GCUUGGAGGCUUGAAcAGUdTsdT | 86 | 3 | 78 | 5 |
| 403 | cgccGcGucGcAGAAGAucdTsdT | 567 | GAUCUUCUGCGACGCGGCGdTsdT | 71 | 3 | 79 | 2 |
| 404 | cauuuGuucAGuGGuucGudTsdT | 568 | ACGAACcACUGAAcAAAUGdTsdT | 54 | 5 | 80 | 4 |
| 405 | cgcuGAAucccGcGGAcGAdTsdT | 569 | UCGUCCGCGGGAUUcAGCGdTsdT | 62 | 2 | 80 | 7 |

FIG. 2

| | | | | | | | |
|---|---|---|---|---|---|---|---|
| 406 | ugGGucAccAuAuucuuGGdTsdT | 570 | CcAAGAAuAUGGUGACCcAdTsdT | 75 | 2 | 80 | 16 |
| 407 | uccucuGccGAuccAuAcudTsdT | 571 | AGuAUGGAUCGGcAGAGGAdTsdT | 73 | 2 | 81 | 3 |
| 408 | auGucAAcGAccGAccuuGdTsdT | 572 | cAAGGUCGGUCGUUGAcAUdTsdT | 69 | 8 | 81 | 7 |
| 409 | ccucuGccuAAucAucucAdTsdT | 573 | UGAGAUGAUuAGGcAGAGGdTsdT | 81 | 4 | 81 | 4 |
| 410 | accGuGuGcAcuucGcuucdTsdT | 574 | GAAGCGAAGUGcAcACGGUdTsdT | 46 | 5 | 81 | 7 |
| 411 | ugccGAuccAuAcuGcGGAdTsdT | 575 | UCCGcAGuAUGGAUCGGcAdTsdT | 61 | 8 | 81 | 5 |
| 412 | caGAGucuAGAcucGuGGudTsdT | 576 | ACcACGAGUCuAGACUCUGdTsdT | 65 | 9 | 81 | 5 |
| 413 | cuGuucAAGccuccAAGcudTsdT | 577 | AGCUUGGAGGCUUGAAcAGdTsdT | 82 | 3 | 82 | 21 |
| 414 | ggAGGcuGuAGGcAuAAAudTsdT | 578 | AUUuAUGCCuAcAGCCUCCdTsdT | 68 | 2 | 82 | 12 |
| 415 | agGAGGcuGuAGGcAuAAAdTsdT | 579 | UUuAUGCCuAcAGCCUCCUdTsdT | 55 | 4 | 83 | 5 |
| 416 | gguGGAcuucucucAAuuudTsdT | 580 | AAAUUGAGAGAAGUCcACCdTsdT | 62 | 7 | 84 | 2 |
| 417 | gcAAcuuuuucAccucuGcdTsdT | 581 | GcAGAGGUGAAAAAGUUGCdTsdT | 93 | 1 | 85 | 5 |
| 418 | CfuGfcUfcGfuGfuUfaCfaGfgCfgAf(invdT) | 582 | pdTCfgCfcUfgUfaAfcAfcGfaGfcAfgdTsdT | 56 | 1 | 86 | 2 |
| 419 | cuAGuGccAuuuGuucAGudTsdT | 583 | ACUGAAcAAAUGGcACuAGdTsdT | 66 | 0 | 86 | 6 |
| 420 | cuGccGAuccAuAcuGcGGdTsdT | 584 | CCGcAGuAUGGAUCGGcAGdTsdT | 73 | 8 | 86 | 5 |
| 421 | guGuGcAcuucGcuucAccdTsdT | 585 | GGUGAAGCGAAGUGcAcACdTsdT | 54 | 4 | 87 | 4 |
| 422 | gcucGuGuuAcAGGcGGGcdTsdT | 586 | GCCCGCCUGuAAcACGAGCdTsdT | 91 | 4 | 87 | 5 |
| 423 | ccuAucuuAucAAcAcuucdTsdT | 587 | GAAGUGUUGAuAAGAuAGGdTsdT | 37 | 2 | 88 | 45 |
| 424 | ucucAAucGccGcGucGcAdTsdT | 588 | UGCGACGCGGCGAUUGAGAdTsdT | 79 | 4 | 88 | 6 |
| 425 | gcccGucuGuGccuucucAdTsdT | 589 | UGAGAAGGcAcAGACGGGCdTsdT | 85 | 4 | 88 | 16 |
| 426 | cuAucuuAucAAcAcuuccdTsdT | 590 | GGAAGUGUUGAuAAGAuAGdTsdT | 43 | 3 | 90 | 23 |
| 427 | auGuuGcccGuuuGuccucdTsdT | 591 | GAGGAcAAACGGGcAAcAUdTsdT | 87 | 5 | 90 | 4 |
| 428 | guAuGuuGcccGuuuGuccdTsdT | 592 | GGAcAAACGGGcAAcAuACdTsdT | 88 | 4 | 90 | 11 |
| 429 | cuucGcuucAccucuGcAcdTsdT | 593 | GUGcAGAGGUGAAGCGAAGdTsdT | 69 | 7 | 91 | 5 |
| 430 | uguGcAcuucGcuucAccudTsdT | 594 | AGGUGAAGCGAAGUGcAcAdTsdT | 76 | 3 | 91 | 14 |
| 431 | gccAAAAuucGcAGucccGdTsdT | 595 | CGGGACUGCGAAUUUUGGCdTsdT | 81 | 3 | 92 | 3 |
| 432 | ccuGcucGuGuuAcAGGcGdTsdT | 596 | CGCCUGuAAcACGAGcAGGdTsdT | 86 | 3 | 92 | 1 |
| 433 | ugGAGuGuGGAuucGcAcudTsdT | 597 | AGUGCGAAUCcAcACUCcAdTsdT | 87 | 4 | 92 | 3 |
| 434 | aacGAccGAccuuGAGGcAdTsdT | 598 | UGCCUcAAGGUCGGUCGUUdTsdT | 83 | 9 | 92 | 3 |
| 435 | acAGAGucuAGAcucGuGGdTsdT | 599 | CcACGAGUCuAGACUCUGUdTsdT | 89 | 4 | 92 | 4 |
| 436 | aaucGccGcGucGcAGAAGdTsdT | 600 | CUUCUGCGACGCGGCGAUUdTsdT | 85 | 6 | 92 | 2 |

FIG. 2

| | | | | | | | |
|---|---|---|---|---|---|---|---|
| 437 | gguAuGuuGcccGuuuGucdTsdT | 601 | GAcAAACGGGcAAcAuACCdTsdT | 80 | 2 | 93 | 3 |
| 438 | gccGAuccAuAcuGcGGAAdTsdT | 602 | UUCCGcAGuAUGGAUCGGCdTsdT | 79 | 3 | 93 | 3 |
| 439 | gcccuAucuuAucAAcAcudTsdT | 603 | AGUGUUGAuAAGAuAGGGCdTsdT | 84 | 4 | 94 | 50 |
| 440 | aguuuAcuAGuGccAuuuGdTsdT | 604 | cAAAUGGcACuAGuAAACUdTsdT | 89 | 7 | 95 | 8 |
| 441 | ugucAAcGAccGAccuuGAdTsdT | 605 | UcAAGGUCGGUCGUUGAcAdTsdT | 84 | 5 | 95 | 8 |
| 442 | acuucucucAAuuuucuAGdTsdT | 606 | CuAGAAAAUUGAGAGAAGUdTsdT | 103 | 3 | 95 | 6 |
| 443 | gcGcGGGAcGuccuuuGucdTsdT | 607 | GAcAAAGGACGUCCCGCGCdTsdT | 88 | 4 | 97 | 3 |
| 444 | ucuAGAcucGuGGuGGAcudTsdT | 608 | AGUCcACcACGAGUCuAGAdTsdT | 90 | 5 | 97 | 2 |
| 445 | gauccAuAcuGcGGAAcucdTsdT | 609 | GAGUUCCGcAGuAUGGAUCdTsdT | 73 | 6 | 98 | 4 |
| 446 | cucuGccGAuccAuAcuGcdTsdT | 610 | GcAGuAUGGAUCGGcAGAGdTsdT | 100 | 5 | 99 | 7 |
| 447 | ucuGccGAuccAuAcuGcGdTsdT | 611 | CGcAGuAUGGAUCGGcAGAdTsdT | 88 | 6 | 99 | 4 |
| 448 | ccucuGccGAuccAuAcuGdTsdT | 612 | cAGuAUGGAUCGGcAGAGGdTsdT | 98 | 11 | 99 | 5 |
| 449 | gcAccucucuuuAcGcGGudTsdT | 613 | ACCGCGuAAAGAGAGGUGCdTsdT | 82 | 7 | 100 | 4 |
| 450 | aaGAAcucccucGccucGcdTsdT | 614 | GCGAGGCGAGGGAGUUCUUdTsdT | 97 | 6 | 100 | 1 |
| 451 | gaAcucccucGccucGcAGdTsdT | 615 | CUGCGAGGCGAGGGAGUUCdTsdT | 100 | 2 | 100 | 2 |
| 452 | ucucucAAuuuucuAGGGcdTsdT | 616 | GCCCuAGAAAAUUGAGAGAdTsdT | 102 | 4 | 100 | 8 |
| 453 | ggGcGcAccucucuuuAcGdTsdT | 617 | CGuAAAGAGAGGUGCGCCCdTsdT | 80 | 4 | 100 | 3 |
| 454 | ccGAuccAuAcuGcGGAAcdTsdT | 618 | GUUCCGcAGuAUGGAUCGGdTsdT | 83 | 5 | 101 | 3 |
| 455 | aacucccucGccucGcAGAdTsdT | 619 | UCUGCGAGGCGAGGGAGUUdTsdT | 100 | 2 | 101 | 2 |
| 456 | cuccucuGccGAuccAuAcdTsdT | 620 | GuAUGGAUCGGcAGAGGAGdTsdT | 93 | 2 | 101 | 2 |
| 457 | ggAGuGuGGAuucGcAcucdTsdT | 621 | GAGUGCGAAUCcAcACUCCdTsdT | 97 | 5 | 101 | 3 |
| 458 | cgGGcGcAccucucuuuAcdTsdT | 622 | GuAAAGAGAGGUGCGCCCGdTsdT | 83 | 6 | 101 | 6 |
| 459 | gucucAAucGccGcGucGcdTsdT | 623 | GCGACGCGGCGAUUGAGACdTsdT | 92 | 4 | 102 | 9 |
| 460 | auccAuAcuGcGGAAcuccdTsdT | 624 | GGAGUUCCGcAGuAUGGAUdTsdT | 88 | 3 | 102 | 7 |
| 461 | cgcAccucucuuuAcGcGGdTsdT | 625 | CCGCGuAAAGAGAGGUGCGdTsdT | 78 | 1 | 102 | 10 |
| 462 | caAcGAccGAccuuGAGGcdTsdT | 626 | GCCUcAAGGUCGGUCGUUGdTsdT | 88 | 4 | 102 | 8 |
| 463 | ccAuAcuGcGGAAcuccuAdTsdT | 627 | uAGGAGUUCCGcAGuAUGGdTsdT | 85 | 3 | 102 | 5 |
| 464 | ugAAucccGcGGAcGAcccdTsdT | 628 | GGGUCGUCCGCGGGAUUcAdTsdT | 92 | 4 | 103 | 3 |
| 465 | agAAcucccucGccucGcAdTsdT | 629 | UGCGAGGCGAGGGAGUUCUdTsdT | 94 | 5 | 103 | 2 |
| 466 | ggcGcAccucucuuuAcGcdTsdT | 630 | GCGuAAAGAGAGGUGCGCCdTsdT | 97 | 7 | 103 | 10 |
| 467 | gcGcAccucucuuuAcGcGdTsdT | 631 | CGCGuAAAGAGAGGUGCGCdTsdT | 99 | 5 | 104 | 7 |

FIG. 2

| | | | | | | | |
|---|---|---|---|---|---|---|---|
| 468 | gcuGAAucccGcGGAcGAcdTsdT | 632 | GUCGUCCGCGGGAUUcAGCdTsdT | 84 | 2 | 104 | 3 |
| 469 | cacuucGcuucAccucuGcdTsdT | 633 | GcAGAGGUGAAGCGAAGUGdTsdT | 90 | 4 | 105 | 12 |
| 470 | cucAAucGccGcGucGcAGdTsdT | 634 | CUGCGACGCGGCGAUUGAGdTsdT | 99 | 3 | 105 | 14 |
| 471 | ucccGucGGcGcuGAAuccdTsdT | 635 | GGAUUcAGCGCCGACGGGAdTsdT | 91 | 3 | 106 | 7 |
| 472 | cuGAAucccGcGGAcGAccdTsdT | 636 | GGUCGUCCGCGGGAUUcAGdTsdT | 96 | 2 | 106 | 6 |
| 473 | agAGucuAGAcucGuGGuGdTsdT | 637 | cACcACGAGUCuAGACUCUdTsdT | 93 | 4 | 107 | 9 |
| 474 | uccAuAcuGcGGAAcuccudTsdT | 638 | AGGAGUUCCGcAGuAUGGAdTsdT | 91 | 4 | 107 | 7 |
| 475 | gcGcuGAAucccGcGGAcGdTsdT | 639 | CGUCCGCGGGAUUcAGCGCdTsdT | 90 | 3 | 108 | 3 |
| 476 | aguGuGGAuucGcAcuccudTsdT | 640 | AGGAGUGCGAAUCcAcACUdTsdT | 94 | 4 | 111 | 3 |
| 477 | cccuGcucGuGuuAcAGGcdTsdT | 641 | GCCUGuAAcACGAGcAGGGdTsdT | 99 | 11 | 111 | 10 |
| 478 | gaAucccGcGGAcGAcccGdTsdT | 642 | CGGGUCGUCCGCGGGAUUCdTsdT | 96 | 3 | 115 | 5 |
| 479 | aaGcuGuGccuuGGGuGGcdTsdT | 643 | GCcACCcAAGGcAcAGCUUdTsdT | 99 | 5 | 116 | 53 |
| 480 | gcccuGcucGuGuuAcAGGdTsdT | 644 | CCUGuAAcACGAGcAGGGCdTsdT | 96 | 5 | 116 | 11 |
| 481 | gucccGucGGcGcuGAAucdTsdT | 645 | GAUUcAGCGCCGACGGGACdTsdT | 93 | 2 | 118 | 4 |
| 482 | aucuuAucAAcAcuuccGGdTsdT | 646 | CCGGAAGUGUUGAuAAGAUdTsdT | 76 | 3 | 126 | 23 |
| 483 | cuuAucAAcAcuuccGGAAdTsdT | 647 | UUCCGGAAGUGUUGAuAAGdTsdT | 39 | 6 | 42 | 3 |

FIG. 2

Table 3. Serum stability of dsRNAs targeting Hepatitis B Virus.

| SEQ ID No. Pair | Mouse Serum | | Human Serum | | Cynomologous Serum | |
|---|---|---|---|---|---|---|
| | sense $t_{½}$ (hr) | antisense $t_{½}$ (hr) | sense $t_{½}$ (hr) | antisense $t_{½}$ (hr) | sense $t_{½}$ (hr) | antisense $t_{½}$ (hr) |
| 321/485 | 26.4 | 0.5 | >48 | 2.1 | n.d. | n.d. |
| 325/489 | 27.2 | 6.7 | >48 | 8.8 | n.d. | n.d. |
| 350/514 | 11.3 | 2.6 | >48 | 17.0 | n.d. | n.d. |
| 326/490 | >48 | 11.7 | >48 | 43.9 | >48 | 5.5 |
| 324/488 | >48 | 13.3 | >48 | 44.7 | >48 | 6.4 |
| 328/492 | 19.1 | 9.9 | >48 | >48 | n.d. | n.d. |
| 322/486 | >48 | 14.5 | >48 | >48 | >48 | 6.5 |
| 327/491 | >48 | 16.0 | >48 | >48 | >48 | 8.1 |

FIG. 3

Table 4. Core sequences of dsRNAs targeting Hepatitis B Virus gene and their modified counterparts.

| core sequence | | | | modified sequence | | | |
|---|---|---|---|---|---|---|---|
| SEQ ID No. | sense strand sequence (5′-3′) | SEQ ID No. | antisense strand sequence (5′-3′) | SEQ ID No. | sense strand sequence (5′-3′) | SEQ ID No. | antisense strand sequence (5′-3′) |
| 1 | CAAGGUAUGUUGCCCGUUU | 157 | AAACGGGCAACAUACCUUG | 321 | caAGGuAuGuuGcccGuuudTsdT | 485 | AAACGGGcAAcAuACCUUGdTsdT |
| 2 | CUGUAGGCAUAAAUUGGUA | 158 | TACCAAUUUAUGCCUACAG | 322 | CfuGfuAfgGfcAfuAfaAfuUfgGfuAf(invdT) | 486 | pdTAfcCfaAfuUfuAfuGfcCfuAfcAfgdTsdT |
| 3 | UCUGCGGCGUUUUAUCAUA | 159 | UAUGAUAAAACGCCGCAGA | 323 | ucuGcGGcGuuuuAucAuAdTsdT | 487 | uAUGAuAAAACGCCGcAGAdTsdT |
| 3 | UCUGCGGCGUUUUAUCAUA | 160 | TAUGAUAAAACGCCGCAGA | 324 | UfcUfgCfgGfcGfuUfuUfaUfcAfuAf(invdT) | 488 | pdTAfuGfaUfaAfaAfcGfcCfgCfaGfadTsdT |
| 4 | ACCUCUGCCUAAUCAUCUC | 161 | GAGAUGAUUAGGCAGAGGU | 325 | accucuGccuAAucAucucdTsdT | 489 | GAGAUGAUuAGGcAGAGGUdTsdT |
| 5 | UUUACUAGUGCCAUUUGUA | 162 | TACAAAUGGCACUAGUAAA | 326 | UfuUfaCfuAfgUfgCfcAfuUfuGfuAf(invdT) | 490 | pdTAfcAfaAfuGfgCfaCfuAfgUfaAfadTsdT |
| 6 | ACCUCUGCCUAAUCAUCUA | 163 | TAGAUGAUUAGGCAGAGGU | 327 | AfcCfuCfuGfcCfuAfaUfcAfuCfuAf(invdT) | 491 | pdTAfgAfuGfaUfuAfgGfcAfgAfgGfudTsdT |
| 7 | CUGUAGGCAUAAAUUGGUC | 164 | GACCAAUUUAUGCCUACAG | 328 | cuGuAGGcAuAAAuuGGucdTsdT | 492 | GACcAAUUuAUGCCuAcAGdTsdT |
| 8 | UGUCUGCGGCGUUUUAUCA | 165 | UGAUAAAACGCCGCAGACA | 329 | ugucuGcGGcGuuuuAucAdTsdT | 493 | UGAuAAAACGCCGcAGAcAdTsdT |
| 8 | UGUCUGCGGCGUUUUAUCA | 166 | TGAUAAAACGCCGCAGACA | 330 | UfgUfcUfgCfgGfcGfuUfuUfaUfcAf(invdT) | 494 | pdTGfaUfaAfaAfcGfcCfgCfaGfaCfadTsdT |
| 9 | UACUAGUGCCAUUUGUUCA | 167 | UGAACAAAUGGCACUAGUA | 331 | uacuAGuGccAuuuGuucAdTsdT | 495 | UGAAcAAAUGGcACuAGuAdTsdT |
| 9 | UACUAGUGCCAUUUGUUCA | 168 | TGAACAAAUGGCACUAGUA | 332 | UfaCfuAfgUfgCfcAfuUfuGfuUfcAf(invdT) | 496 | pdTGfaAfcAfaAfuGfgCfaCfuAfgUfadTsdT |
| 10 | CAACUUUUUCACCUCUG | 169 | TGCAGAGGUGAAAAAGUUG | 333 | CfaAfcUfuUfuUfcAfcCfuC | 497 | pdTGfcAfgAfgGfuGfaAfaAfa |

FIG. 4

| | | | | | | | |
|---|---|---|---|---|---|---|---|
| | CA | | | | fuGfcAf(invdT) | | GfuUfgdTsdT |
| 11 | CCAUUUGUUCAGUGGUUCG | 170 | CGAACCACUGAACAAAUGG | 334 | ccAuuuGuucAGuGGuucGdTsdT | 498 | CGAACcACUGAAcAAAUGGdTsdT |
| 12 | CCAAGUGUUUGCUGACGCA | 171 | UGCGUCAGCAAACACUUGG | 335 | ccAAGuGuuuGcuGAcGcAdTsdT | 499 | UGCGUcAGcAAAcACUUGGdTsdT |
| 12 | CCAAGUGUUUGCUGACGCA | 172 | TGCGUCAGCAAACACUUGG | 336 | CfcAfaGfuGfuUfuGfcUfgAfcGfcAf(invdT) | 500 | pdTGfcGfuCfaGfcAfaAfcAfcUfuGfgdTsdT |
| 13 | CCAUUUGUUCAGUGGUUCA | 173 | TGAACCACUGAACAAAUGG | 337 | CfcAfuUfuGfuUfcAfgUfgGfuUfcAf(invdT) | 501 | pdTGfaAfcCfaCfuGfaAfcAfaAfuGfgdTsdT |
| 14 | UUUACUAGUGCCAUUUGUU | 174 | AACAAAUGGCACUAGUAAA | 338 | uuuAcuAGuGccAuuuGuudTsdT | 502 | AAcAAAUGGcACuAGuAAAdTsdT |
| 15 | CACCUCUGCCUAAUCAUCA | 175 | TGAUGAUUAGGCAGAGGUG | 339 | CfaCfcUfcUfgCfcUfaAfuCfaUfcAf(invdT) | 503 | pdTGfaUfgAfuUfaGfgCfaGfaGfgUfgdTsdT |
| 16 | CUGGCUCAGUUUACUAGUG | 176 | CACUAGUAAACUGAGCCAG | 340 | cuGGcucAGuuuAcuAGuGdTsdT | 504 | cACuAGuAAACUGAGCcAGdTsdT |
| 17 | CAAGGUAUGUUGCCCGUUA | 177 | TAACGGGCAACAUACCUUG | 341 | CfaAfgGfuAfuGfuUfgCfcCfgUfuAf(invdT) | 505 | pdTAfaCfgGfgCfaAfcAfuAfcCfuUfgdTsdT |
| 18 | CUGGCUCAGUUUACUAGUA | 178 | TACUAGUAAACUGAGCCAG | 342 | CfuGfgCfuCfaGfuUfuAfcUfaGfuAf(invdT) | 506 | pdTAfcUfaGfuAfaAfcUfgAfgCfcAfgdTsdT |
| 19 | GAGGCUGUAGGCAUAAAUU | 179 | AAUUUAUGCCUACAGCCUC | 343 | gaGGcuGuAGGcAuAAAuudTsdT | 507 | AAUUuAUGCCuAcAGCCUCdTsdT |
| 20 | CAGUUUACUAGUGCCAUUU | 180 | AAAUGGCACUAGUAAACUG | 344 | caGuuuAcuAGuGccAuuudTsdT | 508 | AAAUGGcACuAGuAAACUGdTsdT |
| 21 | AGGUAUGUUGCCCGUUUGU | 181 | ACAAACGGGCAACAUACCU | 345 | agGuAuGuuGcccGuuuGudTsdT | 509 | AcAAACGGGcAAcAuACCUdTsdT |
| 22 | UAUGUUGCCCGUUUGUCCA | 182 | UGGACAAACGGGCAACAUA | 346 | UfaUfgUfuGfcCfcGfuUfuGfuCfcAf(invdT) | 510 | pdTGfgAfcAfaAfcGfgGfcAfaCfaUfadTsdT |
| 23 | GAGGCUGUAGGCAUAAAUA | 183 | TAUUUAUGCCUACAGCCUC | 347 | GfaGfgCfuGfuAfgGfcAfuAfaAfuAf(invdT) | 511 | pdTAfuUfuAfuGfcCfuAfcAfgCfcUfcdTsdT |
| 24 | GUCUGCGGCGUUUUAUCAU | 184 | AUGAUAAAACGCCGCAGAC | 348 | gucuGcGGcGuuuuAucAudTsdT | 512 | AUGAuAAAACGCCGcAGACdTsdT |

FIG. 4

| | | | | | | | |
|---|---|---|---|---|---|---|---|
| 25 | CAACUUUUUCACCUCUGCC | 185 | GGCAGAGGUGAAAAAGUUG | 349 | caAcuuuuucAccucuGccdTsdT | 513 | GGcAGAGGUGAAAAAGUUGdTsdT |
| 26 | CCGUGUGCACUUCGCUUCA | 186 | UGAAGCGAAGUGCACACGG | 350 | ccGuGuGcAcuucGcuucAdTsdT | 514 | UGAAGCGAAGUGcAcACGGdTsdT |
| 26 | CCGUGUGCACUUCGCUUCA | 187 | TGAAGCGAAGUGCACACGG | 351 | CfcGfuGfuGfcAfcUfuCfgCfuUfcAf(invdT) | 515 | pdTGfaAfgCfgAfaGfuGfcAfcAfcGfgdTsdT |
| 27 | UCAAGGUAUGUUGCCCGUA | 188 | TACGGGCAACAUACCUUGA | 352 | UfcAfaGfgUfaUfgUfuGfcCfcGfuAf(invdT) | 516 | pdTAfcGfgGfcAfaCfaUfaCfcUfuGfadTsdT |
| 28 | CAGUUUACUAGUGCCAUUA | 189 | TAAUGGCACUAGUAAACUG | 353 | CfaGfuUfuAfcUfaGfuGfcCfaUfuAf(invdT) | 517 | pdTAfaUfgGfcAfcUfaGfuAfaAfcUfgdTsdT |
| 29 | UGGUGGACUUCUCUCAAUU | 190 | AAUUGAGAGAAGUCCACCA | 354 | ugGuGGAcuucucucAAuudTsdT | 518 | AAUUGAGAGAAGUCcACcAdTsdT |
| 30 | AGGUAUGUUGCCCGUUUGA | 191 | TCAAACGGGCAACAUACCU | 355 | AfgGfuAfuGfuUfgCfcCfgUfuUfgAf(invdT) | 519 | pdTCfaAfaCfgGfgCfaAfcAfuAfcCfudTsdT |
| 31 | CUGCUCGUGUUACAGGCGG | 192 | CCGCCUGUAACACGAGCAG | 356 | cuGcucGuGuuAcAGGcGGdTsdT | 520 | CCGCCUGuAAcACGAGcAGdTsdT |
| 32 | UAUGUUGCCCGUUUGUCCU | 193 | AGGACAAACGGGCAACAUA | 357 | uauGuuGcccGuuuGuccudTsdT | 521 | AGGAcAAACGGGcAAcAuAdTsdT |
| 33 | UCAAGGUAUGUUGCCCGUU | 194 | AACGGGCAACAUACCUUGA | 358 | ucAAGGuAuGuuGcccGuudTsdT | 522 | AACGGGcAAcAuACCUUGAdTsdT |
| 34 | UCUUAUCAACACUUCCGGA | 195 | UCCGGAAGUGUUGAUAAGA | 359 | ucuuAucAAcAcuuccGGAdTsdT | 523 | UCCGGAAGUGUUGAuAAGAdTsdT |
| 34 | UCUUAUCAACACUUCCGGA | 196 | TCCGGAAGUGUUGAUAAGA | 360 | UfcUfuAfuCfaAfcAfcUfuCfcGfgAf(invdT) | 524 | pdTCfcGfgAfaGfuGfuUfgAfuAfaGfadTsdT |
| 35 | CACCUCUGCCUAAUCAUCU | 197 | AGAUGAUUAGGCAGAGGUG | 361 | caccucuGccuAAucAucudTsdT | 525 | AGAUGAUuAGGcAGAGGUGdTsdT |
| 36 | AUAAGAGGACUCUUGGACU | 198 | AGUCCAAGAGUCCUCUUAU | 362 | auAAGAGGAcucuuGGAcudTsdT | 526 | AGUCcAAGAGUCCUCUuAUdTsdT |
| 37 | GUCUGCGGCGUUUUAUCAA | 199 | TUGAUAAAACGCCGCAGAC | 363 | GfuCfuGfcGfgCfgUfuUfuAfuCfaAf(invdT) | 527 | pdTUfgAfuAfaAfaCfgCfcGfcAfgAfcdTsdT |
| 38 | GGCGCUGAAUCCCGCGG | 200 | GUCCGCGGGAUUCAGCGCC | 364 | ggcGcuGAAucccGcGGAc | 528 | GUCCGCGGGAUUcAGCGCC |

FIG. 4

| | | | | | | | |
|---|---|---|---|---|---|---|---|
| | AC | | | | dTsdT | | dTsdT |
| 39 | CGCGUCGCAGAAGAUCUCA | 201 | UGAGAUCUUCUGCGACGCG | 365 | cgcGucGcAGAAGAucucAdTsdT | 529 | UGAGAUCUUCUGCGACGCGdTsdT |
| 40 | AAUGUCAACGACCGACCUU | 202 | AAGGUCGGUCGUUGACAUU | 366 | aauGucAAcGAccGAccuudTsdT | 530 | AAGGUCGGUCGUUGAcAUUdTsdT |
| 41 | GCUCAGUUUACUAGUGCCA | 203 | UGGCACUAGUAAACUGAGC | 367 | gcucAGuuuAcuAGuGccAdTsdT | 531 | UGGcACuAGuAAACUGAGCdTsdT |
| 42 | UGGUGGACUUCUCUCAAUA | 204 | TAUUGAGAGAAGUCCACCA | 368 | UfgGfuGfgAfcUfuCfuCfuCfaAfuAf(invdT) | 532 | pdTAfuUfgAfgAfgAfaGfuCfcAfcCfadTsdT |
| 43 | AUCGCCGCGUCGCAGAAGA | 205 | UCUUCUGCGACGCGGCGAU | 369 | aucGccGcGucGcAGAAGAdTsdT | 533 | UCUUCUGCGACGCGGCGAUdTsdT |
| 44 | GCCAUUUGUUCAGUGGUUC | 206 | GAACCACUGAACAAAUGGC | 370 | gccAuuuGuucAGuGGuucdTsdT | 534 | GAACcACUGAAcAAAUGGCdTsdT |
| 45 | CGAUCCAUACUGCGGAACU | 207 | AGUUCCGCAGUAUGGAUCG | 371 | cgAuccAuAcuGcGGAAcudTsdT | 535 | AGUUCCGcAGuAUGGAUCGdTsdT |
| 46 | UCACCUCUGCCUAAUCAUC | 208 | GAUGAUUAGGCAGAGGUGA | 372 | ucAccucuGccuAAucAucdTsdT | 536 | GAUGAUuAGGcAGAGGUGAdTsdT |
| 47 | GUGGACUUCUCUCAAUUUU | 209 | AAAAUUGAGAGAAGUCCAC | 373 | guGGAcuucucucAAuuuudTsdT | 537 | AAAAUUGAGAGAAGUCcACdTsdT |
| 48 | GGGUCACCAUAUUCUUGGG | 210 | CCCAAGAAUAUGGUGACCC | 374 | ggGucAccAuAuucuuGGGdTsdT | 538 | CCcAAGAAuAUGGUGACCCdTsdT |
| 49 | GCCGCGUCGCAGAAGAUCU | 211 | AGAUCUUCUGCGACGCGGC | 375 | gccGcGucGcAGAAGAucudTsdT | 539 | AGAUCUUCUGCGACGCGGCdTsdT |
| 50 | UCAAUCGCCGCGUCGCAGA | 212 | UCUGCGACGCGGCGAUUGA | 376 | ucAAucGccGcGucGcAGAdTsdT | 540 | UCUGCGACGCGGCGAUUGAdTsdT |
| 51 | UGGAUGUGUCUGCGGCGUU | 213 | AACGCCGCAGACACAUCCA | 377 | ugGAuGuGucuGcGGcGuudTsdT | 541 | AACGCCGcAGAcAcAUCcAdTsdT |
| 52 | UACUGUUCAAGCCUCCAAG | 214 | CUUGGAGGCUUGAACAGUA | 378 | uacuGuucAAGccuccAAGdTsdT | 542 | CUUGGAGGCUUGAAcAGuAdTsdT |
| 53 | GUUUACUAGUGCCAUUUGU | 215 | ACAAAUGGCACUAGUAAAC | 379 | guuuAcuAGuGccAuuuGudTsdT | 543 | AcAAAUGGcACuAGuAAACdTsdT |

FIG. 4

| | | | | | | | |
|---|---|---|---|---|---|---|---|
| 54 | ACUAGUGCCAUUUGUUCAG | 216 | CUGAACAAAUGGCACUAGU | 380 | acuAGuGccAuuuGuucAGdTsdT | 544 | CUGAAcAAAUGGcACuAGUdTsdT |
| 55 | CCGCGUCGCAGAAGAUCUC | 217 | GAGAUCUUCUGCGACGCGG | 381 | ccGcGucGcAGAAGAucucdTsdT | 545 | GAGAUCUUCUGCGACGCGGdTsdT |
| 56 | UAUCUUAUCAACACUUCCG | 218 | CGGAAGUGUUGAUAAGAUA | 382 | uaucuuAucAAcAcuuccGdTsdT | 546 | CGGAAGUGUUGAuAAGAuAdTsdT |
| 57 | GGCCAAAAUUCGCAGUCCC | 219 | GGGACUGCGAAUUUUGGCC | 383 | ggccAAAAuucGcAGucccdTsdT | 547 | GGGACUGCGAAUUUUGGCCdTsdT |
| 58 | UUCACCUCUGCCUAAUCAU | 220 | AUGAUUAGGCAGAGGUGAA | 384 | uucAccucuGccuAAucAudTsdT | 548 | AUGAUuAGGcAGAGGUGAAdTsdT |
| 59 | CUCAGUUUACUAGUGCCAU | 221 | AUGGCACUAGUAAACUGAG | 385 | cucAGuuuAcuAGuGccAudTsdT | 549 | AUGGcACuAGuAAACUGAGdTsdT |
| 60 | UGUUGCCCGUUUGUCCUCU | 222 | AGAGGACAAACGGGCAACA | 386 | uguuGcccGuuuGuccucudTsdT | 550 | AGAGGAcAAACGGGcAAcAdTsdT |
| 61 | UAGUGCCAUUUGUUCAGUG | 223 | CACUGAACAAAUGGCACUA | 387 | uaGuGccAuuuGuucAGuGdTsdT | 551 | cACUGAAcAAAUGGcACuAdTsdT |
| 62 | AGGCUGUAGGCAUAAAUUG | 224 | CAAUUUAUGCCUACAGCCU | 388 | agGcuGuAGGcAuAAAuuGdTsdT | 552 | cAAUUuAUGCCuAcAGCCUdTsdT |
| 63 | AUGUGUCUGCGGCGUUUUA | 225 | UAAAACGCCGCAGACACAU | 389 | auGuGucuGcGGcGuuuuAdTsdT | 553 | uAAAACGCCGcAGAcAcAUdTsdT |
| 63 | AUGUGUCUGCGGCGUUUUA | 226 | TAAAACGCCGCAGACACAU | 390 | AfuGfuGfuCfuGfcGfgCfgUfuUfuAf(invdT) | 554 | pdTAfaAfaCfgCfcGfcAfgAfcAfcAfudTsdT |
| 64 | ACUUCGCUUCACCUCUGCA | 227 | UGCAGAGGUGAAGCGAAGU | 391 | acuucGcuucAccucuGcAdTsdT | 555 | UGcAGAGGUGAAGCGAAGUdTsdT |
| 65 | CGUGUGCACUUCGCUUCAC | 228 | GUGAAGCGAAGUGCACACG | 392 | cguGuGcAcuucGcuucAcdTsdT | 556 | GUGAAGCGAAGUGcAcACGdTsdT |
| 66 | GUGGUGGACUUCUCUCAAU | 229 | AUUGAGAGAAGUCCACCAC | 393 | guGGuGGAcuucucucAAudTsdT | 557 | AUUGAGAGAAGUCcACcACdTsdT |
| 67 | UGUGUCUGCGGCGUUUUAU | 230 | AUAAAACGCCGCAGACACA | 394 | uguGucuGcGGcGuuuuAudTsdT | 558 | AuAAAACGCCGcAGAcAcAdTsdT |
| 68 | AAGGUAUGUUGCCCGU | 231 | CAAACGGGCAACAUACCUU | 395 | aaGGuAuGuuGcccGuuu | 559 | cAAACGGGcAAcAuACCUUd |

FIG. 4

| | | | | | | | |
|---|---|---|---|---|---|---|---|
| | UUG | | | | GdTsdT | | TsdT |
| 69 | UCAACGACCGACCUUGA GG | 232 | CCUCAAGGUCGGUCGUUGA | 396 | ucAAcGAccGAccuuGAGG dTsdT | 560 | CCUcAAGGUCGGUCGUUGA dTsdT |
| 70 | CAUAAGAGGACUCUUG GAC | 233 | GUCCAAGAGUCCUCUUAUG | 397 | cauAAGAGGAcucuuGGA cdTsdT | 561 | GUCcAAGAGUCCUCUuAUG dTsdT |
| 71 | GUCAACGACCGACCUUG AG | 234 | CUCAAGGUCGGUCGUUGAC | 398 | gucAAcGAccGAccuuGAG dTsdT | 562 | CUcAAGGUCGGUCGUUGAC dTsdT |
| 72 | AUAUUCUUGGGAACAA GAG | 235 | CUCUUGUUCCCAAGAAUAU | 399 | auAuucuuGGGAAcAAGA GdTsdT | 563 | CUCUUGUUCCcAAGAAuAU dTsdT |
| 73 | UGCUCGUGUUACAGGC GGG | 236 | CCCGCCUGUAACACGAGCA | 400 | ugcucGuGuuAcAGGcGG GdTsdT | 564 | CCCGCCUGuAAcACGAGcAd TsdT |
| 74 | CAAUCGCCGCGUCGCAG AA | 237 | UUCUGCGACGCGGCGAUUG | 401 | caAucGccGcGucGcAGAA dTsdT | 565 | UUCUGCGACGCGGCGAUUG dTsdT |
| 75 | ACUGUUCAAGCCUCCAA GC | 238 | GCUUGGAGGCUUGAACAG U | 402 | acuGuucAAGccuccAAGc dTsdT | 566 | GCUUGGAGGCUUGAAcAGU dTsdT |
| 76 | CGCCGCGUCGCAGAAGA UC | 239 | GAUCUUCUGCGACGCGGCG | 403 | cgccGcGucGcAGAAGAuc dTsdT | 567 | GAUCUUCUGCGACGCGGCG dTsdT |
| 77 | CAUUUGUUCAGUGGUU CGU | 240 | ACGAACCACUGAACAAAUG | 404 | cauuuGuucAGuGGuucGu dTsdT | 568 | ACGAACcACUGAAcAAAUGd TsdT |
| 78 | CGCUGAAUCCCGCGGAC GA | 241 | UCGUCCGCGGGAUUCAGCG | 405 | cgcuGAAucccGcGGAcGA dTsdT | 569 | UCGUCCGCGGGAUUcAGCG dTsdT |
| 79 | UGGGUCACCAUAUUCU UGG | 242 | CCAAGAAUAUGGUGACCCA | 406 | ugGGucAccAuAuucuuGG dTsdT | 570 | CcAAGAAuAUGGUGACCcAd TsdT |
| 80 | UCCUCUGCCGAUCCAUA CU | 243 | AGUAUGGAUCGGCAGAGGA | 407 | uccucuGccGAuccAuAcud TsdT | 571 | AGuAUGGAUCGGcAGAGGA dTsdT |
| 81 | AUGUCAACGACCGACCU UG | 244 | CAAGGUCGGUCGUUGACAU | 408 | auGucAAcGAccGAccuuG dTsdT | 572 | cAAGGUCGGUCGUUGAcAU dTsdT |
| 82 | CCUCUGCCUAAUCAUCU CA | 245 | UGAGAUGAUUAGGCAGAG G | 409 | ccucuGccuAAucAucucAd TsdT | 573 | UGAGAUGAUuAGGcAGAGG dTsdT |
| 83 | ACCGUGUGCACUUCGCU UC | 246 | GAAGCGAAGUGCACACGGU | 410 | accGuGuGcAcuucGcuuc dTsdT | 574 | GAAGCGAAGUGcAcACGGU dTsdT |

FIG. 4

| | | | | | | | |
|---|---|---|---|---|---|---|---|
| 84 | UGCCGAUCCAUACUGCGGA | 247 | UCCGCAGUAUGGAUCGGCA | 411 | ugccGAuccAuAcuGcGGAdTsdT | 575 | UCCGcAGuAUGGAUCGGcAdTsdT |
| 85 | CAGAGUCUAGACUCGUGGU | 248 | ACCACGAGUCUAGACUCUG | 412 | caGAGucuAGAcucGuGGudTsdT | 576 | ACcACGAGUCuAGACUCUGdTsdT |
| 86 | CUGUUCAAGCCUCCAAGCU | 249 | AGCUUGGAGGCUUGAACAG | 413 | cuGuucAAGccuccAAGcudTsdT | 577 | AGCUUGGAGGCUUGAAcAGdTsdT |
| 87 | GGAGGCUGUAGGCAUAAAU | 250 | AUUUAUGCCUACAGCCUCC | 414 | ggAGGcuGuAGGcAuAAAudTsdT | 578 | AUUuAUGCCuAcAGCCUCCdTsdT |
| 88 | AGGAGGCUGUAGGCAUAAA | 251 | UUUAUGCCUACAGCCUCCU | 415 | agGAGGcuGuAGGcAuAAAdTsdT | 579 | UUuAUGCCuAcAGCCUCCUdTsdT |
| 89 | GGUGGACUUCUCUCAAUUU | 252 | AAAUUGAGAGAAGUCCACC | 416 | gguGGAcuucucucAAuuudTsdT | 580 | AAAUUGAGAGAAGUCcACCdTsdT |
| 90 | GCAACUUUUUCACCUCUGC | 253 | GCAGAGGUGAAAAAGUUGC | 417 | gcAAcuuuuucAccucuGcdTsdT | 581 | GcAGAGGUGAAAAAGUUGCdTsdT |
| 91 | CUGCUCGUGUUACAGGCGA | 254 | TCGCCUGUAACACGAGCAG | 418 | CfuGfcUfcGfuGfuUfaCfaGfgCfgAf(invdT) | 582 | pdTCfgCfcUfgUfaAfcAfcGfaGfcAfgdTsdT |
| 92 | CUAGUGCCAUUUGUUCAGU | 255 | ACUGAACAAAUGGCACUAG | 419 | cuAGuGccAuuuGuucAGudTsdT | 583 | ACUGAAcAAAUGGcACuAGdTsdT |
| 93 | CUGCCGAUCCAUACUGCGG | 256 | CCGCAGUAUGGAUCGGCAG | 420 | cuGccGAuccAuAcuGcGGdTsdT | 584 | CCGcAGuAUGGAUCGGcAGdTsdT |
| 94 | GUGUGCACUUCGCUUCACC | 257 | GGUGAAGCGAAGUGCACAC | 421 | guGuGcAcuucGcuucAccdTsdT | 585 | GGUGAAGCGAAGUGcAcACdTsdT |
| 95 | GCUCGUGUUACAGGCGGGC | 258 | GCCCGCCUGUAACACGAGC | 422 | gcucGuGuuAcAGGcGGGcdTsdT | 586 | GCCCGCCUGuAAcACGAGCdTsdT |
| 96 | CCUAUCUUAUCAACACUUC | 259 | GAAGUGUUGAUAAGAUAGG | 423 | ccuAucuuAucAAcAcuucdTsdT | 587 | GAAGUGUUGAuAAGAuAGGdTsdT |
| 97 | UCUCAAUCGCCGCGUCGCA | 260 | UGCGACGCGGCGAUUGAGA | 424 | ucucAAucGccGcGucGcAdTsdT | 588 | UGCGACGCGGCGAUUGAGAdTsdT |
| 98 | GCCCGUCUGUGCCUUCUCA | 261 | UGAGAAGGCACAGACGGGC | 425 | gcccGucuGuGccuucucAdTsdT | 589 | UGAGAAGGcAcAGACGGGCdTsdT |
| 99 | CUAUCUUAUCAACACUU | 262 | GGAAGUGUUGAUAAGAUA | 426 | cuAucuuAucAAcAcuuccd | 590 | GGAAGUGUUGAuAAGAuAG |

FIG. 4

| | | | | | | | |
|---|---|---|---|---|---|---|---|
| | CC | | G | | TsdT | | dTsdT |
| 100 | AUGUUGCCCGUUUGUC CUC | 263 | GAGGACAAACGGGCAACAU | 427 | auGuuGcccGuuuGuccuc dTsdT | 591 | GAGGAcAAACGGGcAAcAUd TsdT |
| 101 | GUAUGUUGCCCGUUUG UCC | 264 | GGACAAACGGGCAACAUAC | 428 | guAuGuuGcccGuuuGucc dTsdT | 592 | GGAcAAACGGGcAAcAuACd TsdT |
| 102 | CUUCGCUUCACCUCUGC AC | 265 | GUGCAGAGGUGAAGCGAAG | 429 | cuucGcuucAccucuGcAcd TsdT | 593 | GUGcAGAGGUGAAGCGAAG dTsdT |
| 103 | UGUGCACUUCGCUUCAC CU | 266 | AGGUGAAGCGAAGUGCACA | 430 | uguGcAcuucGcuucAccud TsdT | 594 | AGGUGAAGCGAAGUGcAcA dTsdT |
| 104 | GCCAAAAUUCGCAGUCC CG | 267 | CGGGACUGCGAAUUUUGGC | 431 | gccAAAAuucGcAGucccG dTsdT | 595 | CGGGACUGCGAAUUUUGGC dTsdT |
| 105 | CCUGCUCGUGUUACAGG CG | 268 | CGCCUGUAACACGAGCAGG | 432 | ccuGcucGuGuuAcAGGcG dTsdT | 596 | CGCCUGuAAcACGAGcAGGd TsdT |
| 106 | UGGAGUGUGGAUUCGC ACU | 269 | AGUGCGAAUCCACACUCCA | 433 | ugGAGuGuGGAuucGcAc udTsdT | 597 | AGUGCGAAUCcAcACUCcAd TsdT |
| 107 | AACGACCGACCUUGAGG CA | 270 | UGCCUCAAGGUCGGUCGUU | 434 | aacGAccGAccuuGAGGcA dTsdT | 598 | UGCCUcAAGGUCGGUCGUU dTsdT |
| 108 | ACAGAGUCUAGACUCGU GG | 271 | CCACGAGUCUAGACUCUGU | 435 | acAGAGucuAGAcucGuG GdTsdT | 599 | CcACGAGUCuAGACUCUGU dTsdT |
| 109 | AAUCGCCGCGUCGCAGA AG | 272 | CUUCUGCGACGCGGCGAUU | 436 | aaucGccGcGucGcAGAAG dTsdT | 600 | CUUCUGCGACGCGGCGAUU dTsdT |
| 110 | GGUAUGUUGCCCGUUU GUC | 273 | GACAAACGGGCAACAUACC | 437 | gguAuGuuGcccGuuuGuc dTsdT | 601 | GAcAAACGGGcAAcAuACCd TsdT |
| 111 | GCCGAUCCAUACUGCGG AA | 274 | UUCCGCAGUAUGGAUCGGC | 438 | gccGAuccAuAcuGcGGAA dTsdT | 602 | UUCCGcAGuAUGGAUCGGC dTsdT |
| 112 | GCCCUAUCUUAUCAACA CU | 275 | AGUGUUGAUAAGAUAGGG C | 439 | gcccuAucuuAucAAcAcud TsdT | 603 | AGUGUUGAuAAGAuAGGGC dTsdT |
| 113 | AGUUUACUAGUGCCAU UUG | 276 | CAAAUGGCACUAGUAAACU | 440 | aguuuAcuAGuGccAuuuG dTsdT | 604 | cAAAUGGcACuAGuAAACUd TsdT |
| 114 | UGUCAACGACCGACCUU GA | 277 | UCAAGGUCGGUCGUUGACA | 441 | ugucAAcGAccGAccuuGA dTsdT | 605 | UcAAGGUCGGUCGUUGAcA dTsdT |

FIG. 4

| | | | | | | | |
|---|---|---|---|---|---|---|---|
| 115 | ACUUCUCUCAAUUUUCUAG | 278 | CUAGAAAAUUGAGAGAAGU | 442 | acuucucucAAuuuucuAGdTsdT | 606 | CuAGAAAAUUGAGAGAAGUdTsdT |
| 116 | GCGCGGGACGUCCUUUGUC | 279 | GACAAAGGACGUCCCGCGC | 443 | gcGcGGGAcGuccuuuGucdTsdT | 607 | GAcAAAGGACGUCCCGCGCdTsdT |
| 117 | UCUAGACUCGUGGUGGACU | 280 | AGUCCACCACGAGUCUAGA | 444 | ucuAGAcucGuGGuGGAcudTsdT | 608 | AGUCcACcACGAGUCuAGAdTsdT |
| 118 | GAUCCAUACUGCGGAACUC | 281 | GAGUUCCGCAGUAUGGAUC | 445 | gauccAuAcuGcGGAAcucdTsdT | 609 | GAGUUCCGcAGuAUGGAUCdTsdT |
| 119 | CUCUGCCGAUCCAUACUGC | 282 | GCAGUAUGGAUCGGCAGAG | 446 | cucuGccGAuccAuAcuGcdTsdT | 610 | GcAGuAUGGAUCGGcAGAGdTsdT |
| 120 | UCUGCCGAUCCAUACUGCG | 283 | CGCAGUAUGGAUCGGCAGA | 447 | ucuGccGAuccAuAcuGcGdTsdT | 611 | CGcAGuAUGGAUCGGcAGAdTsdT |
| 121 | CCUCUGCCGAUCCAUACUG | 284 | CAGUAUGGAUCGGCAGAGG | 448 | ccucuGccGAuccAuAcuGdTsdT | 612 | cAGuAUGGAUCGGcAGAGGdTsdT |
| 122 | GCACCUCUCUUUACGCGGU | 285 | ACCGCGUAAAGAGAGGUGC | 449 | gcAccucucuuuAcGcGGudTsdT | 613 | ACCGCGuAAAGAGAGGUGCdTsdT |
| 123 | AAGAACUCCCUCGCCUCGC | 286 | GCGAGGCGAGGGAGUUCUU | 450 | aaGAAcucccucGccucGcdTsdT | 614 | GCGAGGCGAGGGAGUUCUUdTsdT |
| 124 | GAACUCCCUCGCCUCGCAG | 287 | CUGCGAGGCGAGGGAGUUC | 451 | gaAcucccucGccucGcAGdTsdT | 615 | CUGCGAGGCGAGGGAGUUCdTsdT |
| 125 | UCUCUCAAUUUUCUAGGGC | 288 | GCCCUAGAAAAUUGAGAGA | 452 | ucucucAAuuuucuAGGGcdTsdT | 616 | GCCCuAGAAAAUUGAGAGAdTsdT |
| 126 | GGGCGCACCUCUCUUUACG | 289 | CGUAAAGAGAGGUGCGCCC | 453 | ggGcGcAccucucuuuAcGdTsdT | 617 | CGuAAAGAGAGGUGCGCCCdTsdT |
| 127 | CCGAUCCAUACUGCGGAAC | 290 | GUUCCGCAGUAUGGAUCGG | 454 | ccGAuccAuAcuGcGGAAcdTsdT | 618 | GUUCCGcAGuAUGGAUCGGdTsdT |
| 128 | AACUCCCUCGCCUCGCAGA | 291 | UCUGCGAGGCGAGGGAGUU | 455 | aacucccucGccucGcAGAdTsdT | 619 | UCUGCGAGGCGAGGGAGUUdTsdT |
| 129 | CUCCUCUGCCGAUCCAUAC | 292 | GUAUGGAUCGGCAGAGGAG | 456 | cuccucuGccGAuccAuAcdTsdT | 620 | GuAUGGAUCGGcAGAGGAGdTsdT |
| 130 | GGAGUGUGGAUUCGCA | 293 | GAGUGCGAAUCCACACUCC | 457 | ggAGuGuGGAuucGcAcuc | 621 | GAGUGCGAAUCcAcACUCCd |

FIG. 4

| | | | | | | | |
|---|---|---|---|---|---|---|---|
| | CUC | | | | dTsdT | | TsdT |
| 131 | CGGGCGCACCUCUCUUU AC | 294 | GUAAAGAGAGGUGCGCCCG | 458 | cgGGcGcAccucucuuuAcd TsdT | 622 | GuAAAGAGAGGUGCGCCCG dTsdT |
| 132 | GUCUCAAUCGCCGCGUC GC | 295 | GCGACGCGGCGAUUGAGAC | 459 | gucucAAucGccGcGucGcd TsdT | 623 | GCGACGCGGCGAUUGAGAC dTsdT |
| 133 | AUCCAUACUGCGGAACU CC | 296 | GGAGUUCCGCAGUAUGGA U | 460 | auccAuAcuGcGGAAcucc dTsdT | 624 | GGAGUUCCGcAGuAUGGAU dTsdT |
| 134 | CGCACCUCUCUUUACGC GG | 297 | CCGCGUAAAGAGAGGUGCG | 461 | cgcAccucucuuuAcGcGGd TsdT | 625 | CCGCGuAAAGAGAGGUGCG dTsdT |
| 135 | CAACGACCGACCUUGAG GC | 298 | GCCUCAAGGUCGGUCGUUG | 462 | caAcGAccGAccuuGAGGc dTsdT | 626 | GCCUcAAGGUCGGUCGUUG dTsdT |
| 136 | CCAUACUGCGGAACUCC UA | 299 | UAGGAGUUCCGCAGUAUG G | 463 | ccAuAcuGcGGAAcuccuA dTsdT | 627 | uAGGAGUUCCGcAGuAUGG dTsdT |
| 137 | UGAAUCCCGCGGACGAC CC | 300 | GGGUCGUCCGCGGGAUUCA | 464 | ugAAucccGcGGAcGAccc dTsdT | 628 | GGGUCGUCCGCGGGAUUcA dTsdT |
| 138 | AGAACUCCCUCGCCUCG CA | 301 | UGCGAGGCGAGGGAGUUC U | 465 | agAAcucccucGccucGcAd TsdT | 629 | UGCGAGGCGAGGGAGUUC UdTsdT |
| 139 | GGCGCACCUCUCUUUAC GC | 302 | GCGUAAAGAGAGGUGCGCC | 466 | ggcGcAccucucuuuAcGcd TsdT | 630 | GCGuAAAGAGAGGUGCGCC dTsdT |
| 140 | GCGCACCUCUCUUUACG CG | 303 | CGCGUAAAGAGAGGUGCGC | 467 | gcGcAccucucuuuAcGcGd TsdT | 631 | CGCGuAAAGAGAGGUGCGC dTsdT |
| 141 | GCUGAAUCCCGCGGACG AC | 304 | GUCGUCCGCGGGAUUCAGC | 468 | gcuGAAucccGcGGAcGAc dTsdT | 632 | GUCGUCCGCGGGAUUcAGC dTsdT |
| 142 | CACUUCGCUUCACCUCU GC | 305 | GCAGAGGUGAAGCGAAGUG | 469 | cacuucGcuucAccucuGcd TsdT | 633 | GcAGAGGUGAAGCGAAGUG dTsdT |
| 143 | CUCAAUCGCCGCGUCGC AG | 306 | CUGCGACGCGGCGAUUGAG | 470 | cucAAucGccGcGucGcAG dTsdT | 634 | CUGCGACGCGGCGAUUGAG dTsdT |
| 144 | UCCCGUCGGCGCUGAAU CC | 307 | GGAUUCAGCGCCGACGGGA | 471 | ucccGucGGcGcuGAAucc dTsdT | 635 | GGAUUcAGCGCCGACGGGA dTsdT |
| 145 | CUGAAUCCCGCGGACGA CC | 308 | GGUCGUCCGCGGGAUUCAG | 472 | cuGAAucccGcGGAcGAcc dTsdT | 636 | GGUCGUCCGCGGGAUUcAG dTsdT |

FIG. 4

| | | | | | | | |
|---|---|---|---|---|---|---|---|
| 146 | AGAGUCUAGACUCGUGGUG | 309 | CACCACGAGUCUAGACUCU | 473 | agAGucuAGAcucGuGGuGdTsdT | 637 | cACcACGAGUCuAGACUCUdTsdT |
| 147 | UCCAUACUGCGGAACUCCU | 310 | AGGAGUUCCGCAGUAUGGA | 474 | uccAuAcuGcGGAAcuccudTsdT | 638 | AGGAGUUCCGcAGuAUGGAdTsdT |
| 148 | GCGCUGAAUCCCGCGGACG | 311 | CGUCCGCGGGAUUCAGCGC | 475 | gcGcuGAAucccGcGGAcGdTsdT | 639 | CGUCCGCGGGAUUcAGCGCdTsdT |
| 149 | AGUGUGGAUUCGCACUCCU | 312 | AGGAGUGCGAAUCCACACU | 476 | aguGuGGAuucGcAcuccudTsdT | 640 | AGGAGUGCGAAUCcAcACUdTsdT |
| 150 | CCCUGCUCGUGUUACAGGC | 313 | GCCUGUAACACGAGCAGGG | 477 | cccuGcucGuGuuAcAGGcdTsdT | 641 | GCCUGuAAcACGAGcAGGGdTsdT |
| 151 | GAAUCCCGCGGACGACCCG | 314 | CGGGUCGUCCGCGGGAUUC | 478 | gaAucccGcGGAcGAcccGdTsdT | 642 | CGGGUCGUCCGCGGGAUUCdTsdT |
| 152 | AAGCUGUGCCUUGGGUGGC | 315 | GCCACCCAAGGCACAGCUU | 479 | aaGcuGuGccuuGGGuGGcdTsdT | 643 | GCcACCcAAGGcAcAGCUUdTsdT |
| 153 | GCCCUGCUCGUGUUACAGG | 316 | CCUGUAACACGAGCAGGGC | 480 | gcccuGcucGuGuuAcAGGdTsdT | 644 | CCUGuAAcACGAGcAGGGCdTsdT |
| 154 | GUCCCGUCGGCGCUGAAUC | 317 | GAUUCAGCGCCGACGGGAC | 481 | gucccGucGGcGcuGAAucdTsdT | 645 | GAUUcAGCGCCGACGGGACdTsdT |
| 155 | AUCUUAUCAACACUUCCGG | 318 | CCGGAAGUGUUGAUAAGAU | 482 | aucuuAucAAcAcuuccGGdTsdT | 646 | CCGGAAGUGUUGAuAAGAUdTsdT |
| 156 | CUUAUCAACACUUCCGGAA | 319 | UUCCGGAAGUGUUGAUAAG | 483 | cuuAucAAcAcuuccGGAAdTsdT | 647 | UUCCGGAAGUGUUGAuAAGdTsdT |
| 156 | CUUAUCAACACUUCCGGAA | 320 | TUCCGGAAGUGUUGAUAAG | 484 | CfuUfaUfcAfaCfaCfuUfcCfgGfaAf(invdT) | 648 | pdTUfcCfgGfaAfgUfgUfuGfaUfaAfgdTsdT |

FIG. 4

Table 5. Target site sequences of dsRNAs targeting Hepatitis B Virus

| position of 17mer in acc. AM282986.1 | Unmodified dsRNAs of table 1 | | Modified dsRNAs of table 2 | | 17mer target site sequence (5'-3') | genotype coverage [%] | | | |
|---|---|---|---|---|---|---|---|---|---|
| | SEQ ID No. pair | SEQ ID No. pair | SEQ ID No. pair | SEQ ID No. pair | | A (n=332) | B (n=615) | C (n=1332) | D (n=475) |
| 456 | 1/157 | 17/177 | 321/485 | 341/505 | AAGGUAUGUUGCCCGUU | 91.3 | 94.3 | 94.9 | 78.3 |
| 383 | 3/159 | 3/160 | 323/487 | 324/488 | CUGCGGCGUUUUAUCAU | 96.7 | 95.9 | 95.9 | 94.3 |
| 1828 | 4/161 | 6/163 | 325/489 | 327/491 | CCUCUGCCUAAUCAUCU | 95.5 | 81.8 | 96.8 | 92.8 |
| 1782 | 7/164 | 2/158 | 328/492 | 322/486 | UGUAGGCAUAAAUUGGU | 97.9 | 96.1 | 95.9 | 97.5 |
| 381 | 8/165 | 8/166 | 329/493 | 330/494 | GUCUGCGGCGUUUUAUC | 96.4 | 84.9 | 94.0 | 93.9 |
| 679 | 9/167 | 9/168 | 331/495 | 332/496 | ACUAGUGCCAUUUGUUC | 96.4 | 90.4 | 94.9 | 94.7 |
| 687 | 11/170 | 13/173 | 334/498 | 337/501 | CAUUUGUUCAGUGGUUC | 96.7 | 90.4 | 96.1 | 97.7 |
| 1177 | 12/171 | 12/172 | 335/499 | 336/500 | CAAGUGUUUGCUGACGC | 91.3 | 95.9 | 91.8 | 77.5 |
| 677 | 14/174 | 5/162 | 338/502 | 326/490 | UUACUAGUGCCAUUUGU | 95.5 | 88.9 | 94.2 | 94.7 |
| 668 | 16/176 | 18/178 | 340/504 | 342/506 | UGGCUCAGUUUACUAGU | 97.0 | 94.6 | 91.0 | 94.7 |
| 1778 | 19/179 | 23/183 | 343/507 | 347/511 | AGGCUGUAGGCAUAAAU | 97.9 | 95.8 | 96.2 | 97.5 |
| 674 | 20/180 | 28/189 | 344/508 | 353/517 | AGUUUACUAGUGCCAUU | 95.2 | 88.3 | 93.8 | 93.9 |
| 458 | 21/181 | 30/191 | 345/509 | 355/519 | GGUAUGUUGCCCGUUUG | 94.6 | 96.1 | 98.1 | 80.2 |
| 382 | 24/184 | 37/199 | 348/512 | 363/527 | UCUGCGGCGUUUUAUCA | 96.7 | 95.6 | 95.3 | 95.8 |
| 1818 | 25/185 | 10/169 | 349/513 | 333/497 | AACUUUUUCACCUCUGC | 96.1 | 92.0 | 96.5 | 84.8 |
| 1576 | 26/186 | 26/187 | 350/514 | 351/515 | CGUGUGCACUUCGCUUC | 97.6 | 98.4 | 98.3 | 95.6 |
| 258 | 29/190 | 42/204 | 354/518 | 368/532 | GGUGGACUUCUCUCAAU | 91.9 | 83.6 | 97.3 | 90.5 |
| 189 | 31/192 | 91/254 | 356/520 | 418/582 | UGCUCGUGUUACAGGCG | 93.7 | 93.0 | 93.8 | 76.0 |
| 461 | 32/193 | 22/182 | 357/521 | 346/510 | AUGUUGCCCGUUUGUCC | 95.5 | 96.1 | 98.4 | 79.8 |
| 455 | 33/194 | 27/188 | 358/522 | 352/516 | CAAGGUAUGUUGCCCGU | 91.0 | 95.4 | 93.5 | 93.3 |
| 2317 | 34/195 | 34/196 | 359/523 | 360/524 | CUUAUCAACACUUCCGG | 89.8 | 89.8 | 94.8 | 77.7 |
| 1827 | 35/197 | 15/175 | 361/525 | 339/503 | ACCUCUGCCUAAUCAUC | 95.8 | 82.1 | 96.5 | 92.8 |
| 1655 | 36/198 | | 362/526 | | UAAGAGGACUCUUGGAC | 92.2 | 88.1 | 90.2 | 91.4 |
| 1438 | 38/200 | | 364/528 | | GCGCUGAAUCCCGCGGA | 95.5 | 94.1 | 92.0 | 79.6 |

FIG. 5

| | | | | | | | | |
|---|---|---|---|---|---|---|---|---|
| 2419 | 39/201 | 365/529 | GCGUCGCAGAAGAUCUC | 94.6 | 89.6 | 92.3 | 83.2 |
| 1680 | 40/202 | 366/530 | AUGUCAACGACCGACCU | 96.7 | 83.9 | 93.6 | 93.5 |
| 671 | 41/203 | 367/531 | CUCAGUUUACUAGUGCC | 96.7 | 94.8 | 92.6 | 94.7 |
| 2414 | 43/205 | 369/533 | UCGCCGCGUCGCAGAAG | 88.6 | 84.1 | 95.1 | 92.4 |
| 686 | 44/206 | 370/534 | CCAUUUGUUCAGUGGUU | 96.7 | 90.4 | 96.0 | 97.5 |
| 1263 | 45/207 | 371/535 | GAUCCAUACUGCGGAAC | 97.3 | 89.9 | 93.8 | 95.6 |
| 1826 | 46/208 | 372/536 | CACCUCUGCCUAAUCAU | 96.1 | 81.8 | 96.5 | 91.6 |
| 260 | 47/209 | 373/537 | UGGACUUCUCUCAAUUU | 91.6 | 83.4 | 97.3 | 90.7 |
| 2821 | 48/210 | 374/538 | GGUCACCAUAUUCUUGG | 86.7 | 95.4 | 96.5 | 85.3 |
| 2417 | 49/211 | 375/539 | CCGCGUCGCAGAAGAUC | 94.9 | 94.8 | 95.0 | 92.2 |
| 2411 | 50/212 | 376/540 | CAAUCGCCGCGUCGCAG | 87.7 | 84.6 | 94.3 | 90.9 |
| 375 | 51/213 | 377/541 | GGAUGUGUCUGCGGCGU | 95.2 | 85.7 | 94.5 | 93.7 |
| 1859 | 52/214 | 378/542 | ACUGUUCAAGCCUCCAA | 68.4 | 91.9 | 97.0 | 96.4 |
| 676 | 53/215 | 379/543 | UUUACUAGUGCCAUUUG | 95.5 | 88.9 | 94.1 | 94.7 |
| 680 | 54/216 | 380/544 | CUAGUGCCAUUUGUUCA | 96.1 | 90.2 | 94.9 | 94.7 |
| 2418 | 55/217 | 381/545 | CGCGUCGCAGAAGAUCU | 95.2 | 94.8 | 95.0 | 92.4 |
| 2315 | 56/218 | 382/546 | AUCUUAUCAACACUUCC | 90.1 | 89.8 | 94.8 | 78.3 |
| 303 | 57/219 | 383/547 | GCCAAAAUUCGCAGUCC | 98.5 | 97.2 | 85.7 | 94.7 |
| 1825 | 58/220 | 384/548 | UCACCUCUGCCUAAUCA | 96.7 | 82.0 | 96.6 | 92.0 |
| 672 | 59/221 | 385/549 | UCAGUUUACUAGUGCCA | 94.9 | 88.5 | 93.8 | 94.1 |
| 463 | 60/222 | 386/550 | GUUGCCCGUUUGUCCUC | 94.9 | 95.1 | 98.4 | 78.9 |
| 682 | 61/223 | 387/551 | AGUGCCAUUUGUUCAGU | 96.1 | 89.9 | 94.8 | 94.5 |
| 1779 | 62/224 | 388/552 | GGCUGUAGGCAUAAAUU | 97.9 | 96.3 | 96.3 | 97.7 |
| 378 | 63/225 63/226 | 389/553 390/554 | UGUGUCUGCGGCGUUUU | 96.7 | 84.9 | 93.5 | 94.3 |
| 1584 | 64/227 | 391/555 | CUUCGCUUCACCUCUGC | 97.6 | 98.4 | 97.9 | 95.4 |
| 1577 | 65/228 | 392/556 | GUGUGCACUUCGCUUCA | 97.6 | 98.2 | 98.5 | 95.4 |
| 257 | 66/229 | 393/557 | UGGUGGACUUCUCUCAA | 91.9 | 83.6 | 97.1 | 90.5 |
| 379 | 67/230 | 394/558 | GUGUCUGCGGCGUUUUA | 96.1 | 84.9 | 93.6 | 94.1 |
| 457 | 68/231 | 395/559 | AGGUAUGUUGCCCGUUU | 91.3 | 94.8 | 96.7 | 79.2 |

FIG. 5

| | | | | | | | |
|---|---|---|---|---|---|---|---|
| 1684 | 69/232 | 396/560 | CAACGACCGACCUUGAG | 96.4 | 85.4 | 94.1 | 93.3 |
| 1654 | 70/233 | 397/561 | AUAAGAGGACUCUUGGA | 92.2 | 87.8 | 90.2 | 91.2 |
| 1683 | 71/234 | 398/562 | UCAACGACCGACCUUGA | 96.7 | 85.7 | 94.2 | 93.1 |
| 2829 | 72/235 | 399/563 | UAUUCUUGGGAACAAGA | 87.0 | 96.7 | 97.1 | 85.1 |
| 190 | 73/236 | 400/564 | GCUCGUGUUACAGGCGG | 94.9 | 93.3 | 94.0 | 76.0 |
| 2412 | 74/237 | 401/565 | AAUCGCCGCGUCGCAGA | 88.0 | 85.7 | 95.1 | 92.8 |
| 1860 | 75/238 | 402/566 | CUGUUCAAGCCUCCAAG | 68.4 | 91.7 | 97.0 | 96.4 |
| 2416 | 76/239 | 403/567 | GCCGCGUCGCAGAAGAU | 88.3 | 83.6 | 93.8 | 90.7 |
| 688 | 77/240 | 404/568 | AUUUGUUCAGUGGUUCG | 96.7 | 90.6 | 96.1 | 97.7 |
| 1440 | 78/241 | 405/569 | GCUGAAUCCCGCGGACG | 95.8 | 95.3 | 92.8 | 79.8 |
| 2820 | 79/242 | 406/570 | GGGUCACCAUAUUCUUG | 86.4 | 95.3 | 96.8 | 85.3 |
| 1255 | 80/243 | 407/571 | CCUCUGCCGAUCCAUAC | 97.6 | 90.2 | 94.9 | 88.8 |
| 1681 | 81/244 | 408/572 | UGUCAACGACCGACCUU | 96.7 | 85.7 | 94.3 | 93.7 |
| 1829 | 82/245 | 409/573 | CUCUGCCUAAUCAUCUC | 95.8 | 82.8 | 97.0 | 89.3 |
| 1575 | 83/246 | 410/574 | CCGUGUGCACUUCGCUU | 97.6 | 98.5 | 98.3 | 95.8 |
| 1260 | 84/247 | 411/575 | GCCGAUCCAUACUGCGG | 97.0 | 88.6 | 92.9 | 95.2 |
| 243 | 85/248 | 412/576 | AGAGUCUAGACUCGUGG | 93.7 | 96.9 | 95.7 | 96.0 |
| 1861 | 86/249 | 413/577 | UGUUCAAGCCUCCAAGC | 68.4 | 91.7 | 96.9 | 96.4 |
| 1777 | 87/250 | 414/578 | GAGGCUGUAGGCAUAAA | 97.9 | 95.8 | 96.3 | 97.7 |
| 1776 | 88/251 | 415/579 | GGAGGCUGUAGGCAUAA | 96.7 | 95.4 | 96.2 | 97.7 |
| 259 | 89/252 | 416/580 | GUGGACUUCUCUCAAUU | 91.9 | 83.7 | 97.5 | 90.7 |
| 1817 | 90/253 | 417/581 | CAACUUUUUCACCUCUG | 95.8 | 91.7 | 96.2 | 84.4 |
| 681 | 92/255 | 419/583 | UAGUGCCAUUUGUUCAG | 96.1 | 89.9 | 94.9 | 94.7 |
| 1259 | 93/256 | 420/584 | UGCCGAUCCAUACUGCG | 96.7 | 88.5 | 92.8 | 94.9 |
| 1578 | 94/257 | 421/585 | UGUGCACUUCGCUUCAC | 97.6 | 98.0 | 98.6 | 95.8 |
| 191 | 95/258 | 422/586 | CUCGUGUUACAGGCGGG | 94.9 | 92.7 | 92.5 | 96.0 |
| 2313 | 96/259 | 423/587 | CUAUCUUAUCAACACUU | 90.1 | 89.4 | 95.3 | 78.3 |
| 2409 | 97/260 | 424/588 | CUCAAUCGCCGCGUCGC | 88.6 | 85.2 | 96.7 | 93.1 |
| 1548 | 98/261 | 425/589 | CCCGUCUGUGCCUUCUC | 97.9 | 96.7 | 95.7 | 98.1 |

FIG. 5

| | | | | | | | |
|---|---|---|---|---|---|---|---|
| 2314 | 99/262 | 426/590 | UAUCUUAUCAACACUUC | 90.1 | 89.8 | 94.9 | 78.3 |
| 462 | 100/263 | 427/591 | UGUUGCCCGUUUGUCCU | 95.2 | 95.3 | 98.4 | 79.2 |
| 460 | 101/264 | 428/592 | UAUGUUGCCCGUUUGUC | 95.5 | 96.1 | 98.3 | 79.6 |
| 1585 | 102/265 | 429/593 | UUCGCUUCACCUCUGCA | 97.3 | 98.4 | 97.8 | 94.7 |
| 1579 | 103/266 | 430/594 | GUGCACUUCGCUUCACC | 97.9 | 98.4 | 98.6 | 95.8 |
| 304 | 104/267 | 431/595 | CCAAAAUUCGCAGUCCC | 98.5 | 97.4 | 85.9 | 95.2 |
| 188 | 105/268 | 432/596 | CUGCUCGUGUUACAGGC | 93.7 | 93.0 | 93.8 | 75.8 |
| 2267 | 106/269 | 433/597 | GGAGUGUGGAUUCGCAC | 93.7 | 96.4 | 94.4 | 97.3 |
| 1686 | 107/270 | 434/598 | ACGACCGACCUUGAGGC | 96.4 | 85.9 | 93.8 | 93.1 |
| 242 | 108/271 | 435/599 | CAGAGUCUAGACUCGUG | 93.1 | 96.7 | 94.9 | 92.6 |
| 2413 | 109/272 | 436/600 | AUCGCCGCGUCGCAGAA | 88.6 | 84.1 | 95.0 | 92.8 |
| 459 | 110/273 | 437/601 | GUAUGUUGCCCGUUUGU | 95.2 | 95.9 | 98.3 | 79.6 |
| 1261 | 111/274 | 438/602 | CCGAUCCAUACUGCGGA | 97.3 | 89.8 | 94.1 | 96.0 |
| 2311 | 112/275 | 439/603 | CCCUAUCUUAUCAACAC | 93.7 | 88.9 | 95.3 | 78.3 |
| 675 | 113/276 | 440/604 | GUUUACUAGUGCCAUUU | 95.2 | 88.6 | 93.8 | 93.9 |
| 1682 | 114/277 | 441/605 | GUCAACGACCGACCUUG | 97.0 | 85.7 | 94.4 | 93.1 |
| 264 | 115/278 | 442/606 | CUUCUCUCAAUUUUCUA | 90.7 | 82.0 | 96.4 | 88.2 |
| 1408 | 116/279 | 443/607 | CGCGGGACGUCCUUUGU | 95.5 | 96.9 | 95.9 | 94.5 |
| 248 | 117/280 | 444/608 | CUAGACUCGUGGUGGAC | 95.5 | 97.2 | 96.5 | 97.1 |
| 1264 | 118/281 | 445/609 | AUCCAUACUGCGGAACU | 97.9 | 89.4 | 94.1 | 95.6 |
| 1257 | 119/282 | 446/610 | UCUGCCGAUCCAUACUG | 96.7 | 88.5 | 91.1 | 86.9 |
| 1258 | 120/283 | 447/611 | CUGCCGAUCCAUACUGC | 96.7 | 92.5 | 92.2 | 88.4 |
| 1256 | 121/284 | 448/612 | CUCUGCCGAUCCAUACU | 96.7 | 88.1 | 90.7 | 86.5 |
| 1527 | 122/285 | 449/613 | CACCUCUCUUUACGCGG | 95.8 | 94.6 | 95.9 | 98.3 |
| 2381 | 123/286 | 450/614 | AGAACUCCCUCGCCUCG | 91.6 | 95.8 | 97.3 | 89.9 |
| 2383 | 124/287 | 451/615 | AACUCCCUCGCCUCGCA | 97.3 | 95.8 | 97.3 | 90.5 |
| 267 | 125/288 | 452/616 | CUCUCAAUUUUCUAGGG | 90.1 | 82.3 | 96.4 | 87.6 |
| 1523 | 126/289 | 453/617 | GGCGCACCUCUCUUUAC | 95.5 | 95.1 | 95.6 | 97.9 |
| 1262 | 127/290 | 454/618 | CGAUCCAUACUGCGGAA | 97.6 | 89.9 | 94.0 | 95.8 |

FIG. 5

| | | | | | | | |
|---|---|---|---|---|---|---|---|
| 2384 | 128/291 | 455/619 | ACUCCCUCGCCUCGCAG | 97.6 | 95.8 | 96.9 | 90.1 |
| 1254 | 129/292 | 456/620 | UCCUCUGCCGAUCCAUA | 97.6 | 89.6 | 94.7 | 89.1 |
| 2268 | 130/293 | 457/621 | GAGUGUGGAUUCGCACU | 93.7 | 93.8 | 93.5 | 97.3 |
| 1522 | 131/294 | 458/622 | GGGCGCACCUCUCUUUA | 95.5 | 95.1 | 95.6 | 97.9 |
| 2408 | 132/295 | 459/623 | UCUCAAUCGCCGCGUCG | 88.6 | 84.4 | 96.4 | 93.1 |
| 1265 | 133/296 | 460/624 | UCCAUACUGCGGAACUC | 97.6 | 88.5 | 91.2 | 95.2 |
| 1526 | 134/297 | 461/625 | GCACCUCUCUUUACGCG | 95.5 | 94.8 | 95.8 | 98.3 |
| 1685 | 135/298 | 462/626 | AACGACCGACCUUGAGG | 96.4 | 85.2 | 94.1 | 93.3 |
| 1267 | 136/299 | 463/627 | CAUACUGCGGAACUCCU | 97.6 | 88.1 | 90.1 | 95.2 |
| 1443 | 137/300 | 464/628 | GAAUCCCGCGGACGACC | 95.5 | 95.4 | 92.3 | 79.2 |
| 2382 | 138/301 | 465/629 | GAACUCCCUCGCCUCGC | 97.3 | 96.1 | 97.9 | 91.6 |
| 1524 | 139/302 | 466/630 | GCGCACCUCUCUUUACG | 95.2 | 95.0 | 95.6 | 97.9 |
| 1525 | 140/303 | 467/631 | CGCACCUCUCUUUACGC | 95.2 | 94.8 | 95.8 | 98.3 |
| 1441 | 141/304 | 468/632 | CUGAAUCCCGCGGACGA | 95.8 | 95.3 | 94.1 | 79.6 |
| 1583 | 142/305 | 469/633 | ACUUCGCUUCACCUCUG | 97.6 | 98.4 | 98.2 | 96.6 |
| 2410 | 143/306 | 470/634 | UCAAUCGCCGCGUCGCA | 88.0 | 84.6 | 94.8 | 92.0 |
| 1431 | 144/307 | 471/635 | CCCGUCGGCGCUGAAUC | 87.7 | 93.5 | 87.7 | 94.7 |
| 1442 | 145/308 | 472/636 | UGAAUCCCGCGGACGAC | 95.8 | 95.4 | 92.3 | 78.9 |
| 244 | 146/309 | 473/637 | GAGUCUAGACUCGUGGU | 93.7 | 96.7 | 96.0 | 95.8 |
| 1266 | 147/310 | 474/638 | CCAUACUGCGGAACUCC | 97.6 | 88.3 | 89.9 | 95.2 |
| 1439 | 148/311 | 475/639 | CGCUGAAUCCCGCGGAC | 95.8 | 94.8 | 92.1 | 79.8 |
| 2270 | 149/312 | 476/640 | GUGUGGAUUCGCACUCC | 96.1 | 94.6 | 94.6 | 97.7 |
| 187 | 150/313 | 477/641 | CCUGCUCGUGUUACAGG | 93.7 | 93.2 | 94.1 | 76.0 |
| 1444 | 151/314 | 478/642 | AAUCCCGCGGACGACCC | 95.5 | 95.4 | 92.4 | 79.2 |
| 1875 | 152/315 | 479/643 | AGCUGUGCCUUGGGUGG | 73.8 | 96.1 | 96.4 | 96.2 |
| 186 | 153/316 | 480/644 | CCCUGCUCGUGUUACAG | 93.7 | 93.0 | 93.9 | 76.0 |
| 1430 | 154/317 | 481/645 | UCCCGUCGGCGCUGAAU | 87.3 | 93.5 | 87.6 | 94.7 |
| 2316 | 155/318 | 482/646 | UCUUAUCAACACUUCCG | 89.8 | 89.8 | 94.8 | 77.7 |
| 2318 | 156/319 156/320 | 483/647 484/648 | UUAUCAACACUUCCGGA | 89.8 | 89.9 | 94.7 | 77.7 |

FIG. 5

Table 6. NCBI Genbank accession Nos. of Hepatitis B Virus genomic sequences

**Genotype A**

| | | | | | | | |
|---|---|---|---|---|---|---|---|
| FJ692613 | FJ692587 | AF090838 | FJ692590 | DQ020003 | AF090839 | GQ477476 | GQ477473 |
| FJ692584 | EU859907 | AJ131570 | EU859910 | FJ349223 | FJ023662 | AY862867 | EU859928 |
| AY233287 | AY233279 | FJ692609 | AF297624 | AB270536 | GQ331048 | AM295795 | FJ692555 |
| EU859904 | FJ692610 | FJ692563 | GQ477496 | EU859934 | FJ692582 | AB453982 | EU594391 |
| FJ692579 | EU859927 | EU859942 | EU859930 | GQ477492 | AY233281 | EU594394 | FJ692588 |
| AY738141 | GQ477481 | AY934765 | AM184126 | AF143305 | EU859902 | EU859951 | AY934773 |
| GQ477482 | AY738142 | AY161141 | GQ331046 | DQ788725 | FM199974 | FJ692570 | FJ692575 |
| FJ692559 | AB453988 | AY373428 | EU859950 | EU859914 | EU859922 | GQ331047 | AY233276 |
| EU859924 | AM410963 | GQ477498 | FJ692571 | EU859948 | GQ477479 | AF143301 | EU859954 |
| GQ414522 | DQ298164 | GQ477465 | EU594395 | FJ692569 | GQ477484 | FJ692607 | EU859908 |
| FJ904434 | AJ131573 | AF418674 | AB453983 | FJ692594 | EF208113 | AF143303 | EU859898 |
| FJ692565 | AB241115 | AM184125 | GQ477477 | DQ315784 | EU859947 | EU859931 | FJ692556 |
| EU859944 | EF208115 | EU185786 | AB222707 | EU054331 | FJ692566 | AF297625 | GQ477470 |
| EU859918 | EU859941 | FJ692572 | AF043580 | GQ477501 | DQ298162 | GQ477497 | AY233288 |
| DQ788729 | FJ692560 | EU859953 | DQ315786 | AB126580 | GQ477460 | AY903452 | AY934770 |
| FJ692598 | AY934766 | AY934774 | FJ692596 | FJ692603 | U87746 | EU859911 | AY233275 |
| AY934763 | AB246317 | AY077735 | EU859916 | EU859909 | AB194952 | FJ692591 | FJ692576 |
| DQ298161 | GQ477466 | AB453980 | DQ788727 | FJ692574 | AF090841 | FJ692606 | AF043560 |
| AB194951 | FJ904411 | AM295797 | FJ692601 | EU859955 | AF143299 | GQ477504 | EU594392 |
| EU859938 | EU185788 | FM199979 | GQ477503 | AY233277 | AY233284 | AF143300 | AB453987 |
| GQ477463 | AY233282 | GQ477489 | AF143307 | AY934772 | GQ477480 | FJ692583 | EU859900 |
| AF090842 | FJ692581 | GQ477474 | EU859936 | FJ692589 | AY738143 | AY233280 | AY233283 |
| EU859944 | EU859901 | AM282986 | AF297622 | EU594390 | AM494718 | GQ477464 | FJ692580 |
| EU859925 | FM199977 | AF143302 | GQ477490 | FJ692554 | EU859926 | GQ477499 | AY738139 |
| FJ692558 | AM295799 | FJ692593 | AY902775 | EU859929 | AY128092 | AY373429 | EU185789 |
| AY738140 | GQ477487 | AY233290 | EU594393 | GQ477472 | FJ692611 | AY934764 | AB222708 |
| GQ477483 | EU859921 | AY934768 | EU859956 | AB453986 | AY233278 | FJ692562 | GQ477485 |
| FJ692578 | GQ477467 | EU859913 | AY233274 | GQ477500 | EU859906 | EU859943 | GQ477478 |
| EU859905 | GQ161813 | FJ692604 | FJ692577 | FJ692602 | AY233285 | EU410082 | EU859923 |
| FJ692585 | EF208114 | FJ349224 | AY934771 | FJ692595 | FJ692586 | FJ692608 | EU859903 |
| AY233286 | AY934767 | EU859933 | AY233289 | EU859949 | EU185787 | AF143306 | FJ349222 |
| FJ692612 | EU859940 | GQ477468 | GQ477471 | FJ692568 | AF418675 | FJ692600 | AB330371 |
| GQ477462 | FJ692561 | GQ477495 | EU859899 | EU859915 | GQ477494 | GQ477502 | AB330372 |
| EU859925 | AB241114 | AM295800 | FJ692557 | DQ315785 | GQ477469 | DQ788726 | AB330373 |
| EU086721 | DQ298165 | GQ477475 | FM199981 | GQ184323 | EU859932 | EU859917 | AJ627226 |
| AB194950 | AY034878 | GQ477488 | AB453985 | AF143304 | FJ692605 | FJ692597 | AJ627227 |
| EU859939 | EU859920 | AM295796 | EU366129 | GQ477493 | EU859912 | EU747320 | AJ627228 |
| AF143308 | GQ477486 | AB453981 | AF297623 | AF297621 | AY934769 | FM199980 | AP007263 |
| FJ692599 | FM199976 | FJ692573 | GQ477491 | EU859935 | FJ692592 | AB453984 | EU304331 |
| DQ788728 | AM295798 | EU859952 | EU859937 | DQ020002 | GQ184324 | AB453979 | EU414132 |
| EU859919 | FJ692567 | EU414134 | DQ298163 | AF090840 | EU859945 | S50225 | V00866 |
| FJ692564 | AB453989 | FM199978 | EU859946 | GQ477461 | AF143298 | | |

**Genotype B**

| | | | | | | | |
|---|---|---|---|---|---|---|---|
| EU306702 | GU332692 | AB073842 | AY800389 | AY206377 | AB106884 | AB073843 | FJ386688 |
| GU332701 | AB073822 | AB493832 | DQ463798 | FJ386636 | EU939630 | EU939633 | AJ131574 |
| EF473975 | AY596102 | GQ924634 | EU939670 | D23678 | FJ386656 | DQ463787 | AB073840 |
| FJ787444 | DQ904357 | AY167098 | AY596103 | DQ993680 | AB116083 | FJ386655 | AB219429 |
| D23679 | EU882001 | AB116082 | AB073823 | EF473974 | GQ377641 | AY293309 | DQ361535 |
| DQ993681 | GQ924608 | AB205122 | GQ377596 | AY781187 | AF121243 | U87747 | AB493830 |
| AY033072 | GQ924654 | EU306670 | GQ377537 | AY163870 | GQ924635 | EU564822 | DQ993710 |
| GQ924628 | EU939671 | EU939631 | EU919175 | EU306703 | FJ386676 | EU522074 | AB365445 |

FIG. 6

| | | | | | | | |
|---|---|---|---|---|---|---|---|
| EU919174 | DQ463799 | AB106885 | GU332693 | GU332700 | AB493833 | AB205120 | EU882003 |
| FJ562311 | GQ377556 | GQ924648 | AY033073 | FJ562222 | FJ562262 | FJ386675 | FJ386582 |
| FJ386615 | GQ377568 | EU305543 | EU564823 | GQ377549 | FJ562312 | AB073847 | M54923 |
| EU939673 | DQ993683 | AB219428 | GQ377588 | GU332702 | GU332691 | AB287317 | EU660233 |
| GQ924656 | FJ386669 | AB073841 | AY217370 | EU306701 | EU939672 | EU305545 | AF121247 |
| GU332690 | GU332703 | FJ032344 | GQ924617 | FJ386634 | FJ386648 | AP011087 | GQ377573 |
| EF103278 | GQ377622 | GQ924637 | FJ386608 | FJ386668 | EU882002 | FJ032342 | EU522073 |
| GQ377602 | EU306700 | AB205121 | FJ386654 | AY206375 | DQ993711 | GQ377639 | FJ562246 |
| EU919176 | EU796068 | AY330917 | EU939632 | DQ993682 | FJ386583 | GQ924611 | EU564825 |
| AB300364 | EF473977 | GQ377643 | DQ448628 | GQ377569 | GQ924631 | AY800391 | X97851 |
| GQ377595 | FJ562260 | EU522075 | EF473976 | AB073821 | DQ993698 | EU939634 | DQ993684 |
| AY220698 | AB493831 | FJ562240 | FJ032358 | GQ377594 | AY217356 | EU939668 | AY206373 |
| GQ377625 | GU332697 | GQ924610 | AY217357 | EU306695 | EU306706 | AB471854 | AB493829 |
| AF479684 | EU919171 | EU939669 | DQ975271 | EU939629 | EU579441 | AY217368 | AB219430 |
| GU332704 | GQ377592 | EU939635 | DQ993699 | EU939675 | DQ993685 | AY596105 | EF473972 |
| EU306707 | AB073827 | AY800390 | GQ924630 | EU881998 | EU919173 | GQ924653 | DQ993686 |
| AY781183 | EU564824 | AY163869 | AB073826 | FJ386584 | EU306696 | FJ386610 | AF121245 |
| EU882004 | GQ377644 | GQ377638 | GU332696 | FJ349296 | GU332695 | EU939676 | AB073839 |
| FJ349236 | EU522072 | EU305544 | DQ377158 | AY596106 | FJ562316 | EU306705 | EU660231 |
| EU939674 | X97850 | AB287316 | GQ377604 | EF473971 | AB287329 | GU332706 | EU139543 |
| EU939628 | EU660232 | AB073846 | EU919170 | AB100695 | GQ377590 | FJ562224 | EU158262 |
| GQ924651 | AF121246 | AP011086 | GQ205440 | GU332705 | AB073825 | AB205119 | AB246335 |
| EU330998 | AB493835 | GU332707 | GQ377606 | AB287314 | EU660230 | AY217364 | AB073829 |
| EU306677 | AF282918 | EU939677 | GU332694 | DQ993708 | AY206380 | AY596109 | GQ377561 |
| EU939636 | AY206390 | AY596104 | AY217355 | AY800392 | FJ386660 | EU881997 | AB033555 |
| DQ993709 | EU487256 | AY217369 | AB241116 | EU939637 | EU306709 | GQ924603 | FJ386681 |
| AY217374 | AB241117 | AB471855 | GQ924632 | EU158263 | DQ995803 | DQ463792 | AY217358 |
| EU796071 | DQ993687 | AB073824 | EU487257 | EU330999 | FJ562289 | FJ562254 | DQ993696 |
| AP011085 | AB073858 | AB287328 | AY206391 | FJ562219 | D00329 | GU332699 | FJ518812 |
| AB287315 | EF473973 | EU306697 | AP011084 | EU564826 | AB073855 | EU439022 | AY167093 |
| EU305547 | EU306704 | FJ562259 | GQ377550 | AB073838 | AP011095 | DQ980548 | AP011089 |
| AB073845 | GQ377626 | EU919172 | AB073844 | AF121244 | DQ448620 | AB287325 | AB073849 |
| AB287319 | EU330994 | AY217365 | EU330995 | DQ993697 | GQ924641 | FM209516 | AB368295 |
| FJ562234 | EU570070 | AB073854 | AF121248 | AY217359 | EU939664 | EU306698 | GQ377629 |
| EU939666 | AB300371 | DQ448621 | AB073834 | AB033554 | EU939638 | AB287327 | AB493827 |
| FJ386600 | EU439023 | AY220703 | DQ993704 | FJ386680 | AY167102 | EU439020 | DQ995801 |
| DQ993705 | AB073828 | AP011094 | AY167101 | GQ377582 | DQ993707 | AF100308 | DQ448622 |
| AY167100 | AB287324 | EU306708 | GU357842 | AB073837 | AB010289 | AY217366 | FJ032352 |
| AB073835 | EU330989 | DQ995802 | EU939667 | EU306679 | FJ562236 | AY766463 | AB073857 |
| AF121249 | GU332698 | AB300370 | AP011088 | EU919161 | X98073 | EU939678 | GQ924621 |
| AB231909 | EU939627 | EU919162 | AB287318 | EU306684 | FJ386683 | FJ386642 | DQ993695 |
| AB246339 | DQ463793 | EU570071 | AB073848 | EU330996 | DQ993694 | DQ463790 | FJ518811 |
| FJ386682 | EU939639 | DQ448623 | EU306699 | EF494381 | EU939663 | AB073850 | AY217361 |
| AF282917 | EU939665 | GQ377542 | FJ562257 | FJ386684 | AY596110 | D23677 | DQ463797 |
| X98072 | EU330997 | AB073856 | GQ377558 | DQ448619 | AB195935 | GQ924626 | GQ924606 |
| GQ377634 | GQ377614 | DQ463791 | FJ032349 | DQ463800 | DQ993700 | AB116090 | AB212626 |
| FJ562296 | EU306678 | EU939679 | AB246340 | AB073830 | FJ787476 | EU570069 | AY596111 |
| FJ562237 | AB073836 | AY217367 | EU306710 | EU570075 | AB115551 | AF121250 | AB195934 |
| EU305548 | AY206383 | AF100309 | X98074 | EU306683 | AB117759 | AB287320 | FJ787477 |

FIG. 6

| | | | | | | | |
|---|---|---|---|---|---|---|---|
| AB195933 | AB302095 | AY518556 | D00330 | GQ377612 | AB010292 | GQ377564 | DQ993701 |
| DQ993706 | FJ032353 | AB287326 | FJ562231 | FJ562303 | AP011090 | EU660224 | GQ924647 |
| GQ924640 | AP011096 | EU439021 | GU168597 | GQ924646 | DQ448625 | EU331000 | FJ386658 |
| EU939662 | AB246341 | GQ377565 | AY217363 | GQ924624 | GQ924644 | EU330993 | EU589335 |
| GQ377613 | EF494380 | AB287321 | EU547563 | AB010290 | DQ993702 | GQ924638 | GQ377547 |
| EU306682 | GU168596 | AF121251 | DQ463795 | DQ995804 | EU350409 | FM209513 | AB073853 |
| EU330990 | D00331 | EF134945 | FJ562253 | AB073852 | AB212625 | EU439019 | EU796067 |
| AB073831 | X98075 | GQ924627 | EU522066 | DQ448627 | AY596112 | AB219427 | AP011093 |
| DQ463801 | DQ463796 | DQ448624 | GQ377566 | FJ032357 | GQ377587 | GU168595 | AY220704 |
| GQ377525 | GQ924607 | FJ032354 | AB287322 | EU796066 | AB073832 | X98076 | DQ448626 |
| AY167097 | AY217360 | AP011091 | EF134946 | AP011092 | DQ463802 | FJ562321 | AB010291 |
| FJ562322 | EU331001 | AB073851 | EU595030 | DQ463789 | GQ377610 | EU306712 | FJ386666 |
| EU306711 | GQ377539 | GQ377519 | AY206387 | EU939661 | EU306681 | AB246342 | AB486012 |
| GQ924625 | GQ924659 | AB219426 | DQ463788 | AB493834 | FJ023634 | D50521 | AB302943 |
| EU595031 | DQ463794 | FM209512 | GQ924645 | AB493836 | FJ023635 | D50522 | AB302944 |
| AY167089 | EU939681 | GQ924639 | AB014366 | AF461360 | FJ023636 | FJ023631 | AB302945 |
| EU439024 | AY217362 | EU330992 | AB031267 | AJ627225 | FJ023637 | FJ023632 | AB362933 |
| EU660227 | X98077 | EU306680 | AB302942 | AY167094 | FJ023638 | FJ023633 | AB493828 |
| AY220697 | AJ131133 | AB073833 | EU522067 | AB246343 | AF233236 | GQ377567 | GU168594 |
| AB287323 | EF494382 | DQ993703 | GQ475340 | EU439018 | EU939660 | FJ787475 | |

**Genotype C**

| | | | | | | | |
|---|---|---|---|---|---|---|---|
| FJ562331 | EU439009 | EU916218 | FJ386580 | FJ386617 | FJ386677 | GQ475351 | AB250109 |
| AY781186 | D23684 | EU570067 | EU939547 | EU439015 | AB195947 | AF537372 | AB111117 |
| FJ562282 | FJ023664 | AB300366 | AY373432 | FJ032347 | EU939586 | GQ377640 | GQ924614 |
| GQ377620 | AF411411 | GQ475311 | FJ787464 | EU305540 | GQ227696 | AY330914 | DQ089764 |
| GQ475331 | EU939567 | GQ377600 | FJ787438 | EU916224 | FJ787458 | EU919168 | EU872003 |
| FJ562223 | FJ386637 | EU306722 | DQ089778 | DQ377160 | AB241110 | FJ562243 | AB026815 |
| EU916238 | AY206376 | GQ377536 | AF461043 | EU560440 | GQ377576 | EU564820 | FJ787478 |
| GQ377516 | EU939651 | AB471853 | DQ089785 | EU589339 | AB367417 | EU916204 | AF068756 |
| FJ882612 | DQ089758 | GQ377597 | EU871982 | AF533983 | EU589345 | FJ386657 | FJ787485 |
| EF137802 | EU306691 | AB198077 | AB112063 | EU939611 | AF223956 | DQ089799 | AY217373 |
| EU678475 | FJ787439 | EU916219 | EU939566 | FJ562283 | AY217372 | FJ562242 | DQ975274 |
| AB367394 | FJ787465 | EU306690 | FJ882613 | GQ377621 | DQ089765 | EU919169 | AB241111 |
| EU871983 | AB485810 | EU306723 | GQ377517 | FJ787484 | GQ924615 | EU306671 | AY167099 |
| DQ089784 | EU882000 | FJ562310 | EU439008 | FJ787479 | AB111116 | AB205123 | FJ787459 |
| GQ924655 | AB198076 | EU939650 | EF137803 | AB026814 | GQ924649 | GQ475350 | EU939610 |
| GQ924609 | AB471852 | DQ089759 | FJ562330 | FJ023659 | DQ089798 | GQ377577 | EU916225 |
| DQ089779 | GQ377601 | GQ924629 | DQ536412 | EU872002 | AF223957 | AB367416 | EU589338 |
| FJ386616 | GQ475310 | AF411410 | EU916239 | AB112348 | EU589344 | GQ227697 | EU560441 |
| EU939546 | AB300367 | FJ787445 | GQ475330 | AB367395 | EU916205 | EU939587 | EU410079 |
| FJ386581 | NC_003977 | FJ023665 | AB064314 | EU678474 | EU564821 | AB195946 | DQ377161 |
| FJ032346 | EU939559 | EU306672 | AB241112 | DQ377162 | EU522068 | EU939565 | GQ475333 |
| EU305541 | FJ787487 | AY330916 | EU939579 | FJ562261 | GQ475313 | FJ023666 | FJ562280 |
| GQ377557 | EU872001 | GQ377642 | GQ227694 | EU916226 | EU306693 | FJ787446 | AY781184 |
| DQ089766 | GQ259588 | GQ475353 | EU939584 | FJ787466 | AB471851 | AB247916 | FJ562333 |
| AB111115 | FJ032339 | AF223954 | AB195945 | EU939545 | GQ377534 | AY206389 | D16665 |
| GQ924616 | AB367415 | AB116080 | EU305542 | FJ386649 | AB367409 | AB048704 | FJ032359 |
| FJ386609 | GQ377574 | FJ386629 | GQ377554 | EU871980 | EU660228 | EU939653 | AB367429 |
| AB195939 | GQ377528 | EU939613 | AB367435 | DQ089787 | DQ993181 | AY206374 | GQ377548 |
| AB367396 | EU916206 | GQ924636 | FJ032345 | EU306720 | EU939598 | FJ386635 | EU554538 |
| AY217371 | FJ562241 | AY161139 | EU594383 | FJ562313 | EU939539 | FJ562221 | FJ882610 |
| GQ377514 | AY161138 | GQ475352 | GQ377575 | AB367428 | DQ536410 | FJ787447 | EU306692 |
| DQ377163 | AB195944 | EU306673 | EU939558 | EU796069 | FJ562332 | FJ023667 | AB300365 |

FIG. 6

| | | | | | | | |
|---|---|---|---|---|---|---|---|
| DQ361534 | EU939585 | EU498227 | AB367397 | AB112408 | AY781185 | AF411412 | GQ475312 |
| EU916227 | GQ227695 | EU916207 | AB195938 | GQ377515 | EU939652 | GU385774 | EU522069 |
| AB300359 | EU939578 | EU589346 | EU872000 | EU554539 | AB048705 | GQ377535 | GQ377603 |
| GQ377555 | EU939612 | AB116081 | FJ787486 | FJ882611 | AY206388 | AB471850 | FJ386614 |
| EU439016 | FJ386689 | AF223955 | AB111114 | GQ377623 | EU939564 | AY220699 | DQ089786 |
| DQ478885 | FJ386674 | AB367414 | DQ089767 | FJ562281 | EU787444 | EU660229 | EU871981 |
| AB367434 | FJ386628 | FJ032338 | AY152726 | GQ475332 | EU939538 | AB367408 | FJ787467 |
| AB241113 | AF537371 | GQ377529 | DQ478899 | FJ562220 | EU939599 | EU306721 | EU939544 |
| AY206392 | EU439010 | GQ475328 | FJ787480 | AB246337 | D23681 | FJ787461 | DQ089780 |
| EU939648 | AB367432 | AB111112 | AB367412 | AF473543 | FJ386632 | AY596107 | AY040627 |
| EU939614 | FJ562328 | DQ089761 | EF688062 | AB116087 | EU939608 | FJ386578 | EU871987 |
| FJ386672 | DQ377165 | FJ386652 | GQ475308 | EU562219 | EU939654 | EU939542 | FJ562314 |
| EU414133 | EU594384 | FJ386599 | GQ377619 | FJ562308 | GQ475334 | FJ386585 | DQ377159 |
| AF411408 | EU410080 | AY217376 | GQ475354 | EU939562 | FJ562287 | AB037927 | EU306727 |
| EU939583 | EU916221 | EU678470 | EU306675 | AB176643 | FJ562226 | AB112066 | EU306694 |
| AB195942 | FJ562266 | AB111946 | EU306688 | AB241109 | FJ562334 | FJ386612 | EU916241 |
| GQ227693 | EF494377 | EU872006 | EU589340 | FJ023661 | AB113878 | DQ683578 | GQ205441 |
| GQ377553 | AB493837 | AB485808 | EU560439 | FJ787441 | FJ882617 | EU881999 | AB300363 |
| GQ475348 | GQ377618 | FJ386598 | EU717218 | FJ032343 | AY206393 | FJ386613 | FJ882616 |
| GQ377605 | GQ475355 | FJ787481 | DQ377164 | EU796072 | GQ377593 | EU871986 | FJ562227 |
| GQ475314 | GQ475309 | AB485809 | FJ562329 | EU439011 | DQ980547 | DQ089781 | GQ377624 |
| GQ377533 | EU562218 | AB026811 | GQ475329 | AF411409 | EU554542 | GQ924650 | FJ562286 |
| AP011108 | EU560438 | EU872007 | EF494376 | GQ227692 | EU306726 | FJ787460 | GQ475335 |
| AB112472 | EU589341 | DQ089760 | FJ562267 | AB195943 | FJ562315 | EU882005 | FJ562335 |
| EU306689 | AB367413 | AB111113 | EU916220 | EU939582 | GQ475315 | EU939543 | AB074756 |
| FJ562247 | GQ377572 | FJ386653 | AB288026 | FJ386673 | GQ475349 | FJ386579 | AF462041 |
| EU306674 | EU678471 | EU594385 | GQ377552 | EU939615 | AB300362 | AB113879 | Y18858 |
| EF536065 | AY217377 | EU410081 | AB367433 | EU939649 | EU916240 | AF384371 | EU939655 |
| EU939609 | AB300361 | DQ089782 | AP011099 | GQ377571 | EF536066 | EU872004 | EU916223 |
| AB202072 | FJ562258 | EU871978 | D23683 | AB471848 | GQ475356 | AB367393 | EU594386 |
| FJ386633 | EU306725 | AY707087 | FJ023663 | U87742 | AB205125 | EU678472 | EU306719 |
| AB176642 | GQ377531 | FJ562336 | AY123424 | FJ032360 | EU522071 | AB367430 | GQ924633 |
| EU939563 | EU554541 | AB074755 | FJ787443 | AB367410 | FJ386650 | FJ032340 | FJ386670 |
| D23680 | FJ386587 | FJ562278 | EU939560 | AB116078 | DQ089763 | EU439012 | EU939616 |
| FJ787440 | EU939540 | FJ562285 | EU939656 | EU589342 | GQ924613 | GQ377551 | DQ975272 |
| FJ023660 | FJ787463 | GQ475336 | EU579442 | FJ562244 | EU871999 | FJ562299 | AB195940 |
| GQ475316 | EU882006 | AF384372 | AB202071 | FJ562218 | FJ787482 | FJ562238 | EU939581 |
| GQ377607 | EU871985 | FJ882615 | FJ386630 | AB222714 | AB026812 | FJ562264 | FJ386631 |
| EU579443 | AB205118 | FJ787462 | AB195941 | EU306718 | AY217375 | AB116084 | EU939591 |
| EU939657 | FJ562225 | AB112471 | DQ975273 | EU594387 | EU678473 | EU522070 | AB195950 |
| FJ787442 | FJ562279 | GQ377530 | EU939617 | EU796070 | AB367392 | AB205124 | EU939606 |
| D23682 | EU871979 | EU554540 | FJ386671 | FJ032341 | FJ386651 | GQ475357 | Y18857 |
| EU939561 | DQ089783 | GQ377591 | EU916222 | AB367431 | EU871998 | AB222715 | AB111120 |
| FJ882614 | EU871984 | AB300360 | AY057947 | EU872005 | GQ924612 | FJ562245 | GQ924623 |
| AP011098 | FJ386611 | GQ475317 | FJ562265 | D28880 | EU670263 | AB471849 | AF223961 |
| FJ562337 | AB112065 | FJ562317 | FJ562239 | AB026813 | DQ089762 | GQ377570 | FJ562228 |
| GQ475337 | EU939541 | EU306724 | FJ562298 | FJ787483 | AB116079 | AB367411 | FJ562274 |
| FJ562284 | FJ386586 | EU939580 | AB182589 | AB115417 | EU589343 | FJ032361 | EU916233 |
| GQ377541 | DQ089773 | AP011106 | EU155828 | DQ922651 | GQ872210 | GQ377580 | AB367401 |
| DQ246215 | AB368297 | AB198081 | AY641558 | FJ562268 | DQ089792 | AB116089 | AB198080 |
| FJ032350 | EU871974 | FJ386620 | DQ315783 | EU717217 | FJ904423 | EU562217 | GQ377560 |
| AB299858 | FJ386640 | EU547558 | GQ184325 | EU306715 | EU872008 | FJ562306 | DQ980549 |
| AY220702 | EU939626 | EU939646 | FJ562295 | AB246345 | FJ787473 | FJ562248 | AP011107 |
| AB113876 | GQ475346 | FM209514 | GQ377637 | FJ562326 | AB195930 | EU306686 | AY123041 |
| AB367420 | EU916213 | EU939570 | EF494379 | AB074047 | FJ386597 | GQ475306 | FJ562255 |
| FJ386576 | EU306729 | FJ023673 | AB493839 | GU357843 | AY217378 | GQ377617 | EU916212 |
| EU872014 | AB049609 | FJ787453 | GQ475326 | GQ924643 | EU939550 | EU919163 | AF286594 |
| EU871989 | AB367400 | AJ309369 | AJ344115 | EU871995 | GQ377521 | DQ089804 | GQ475347 |
| EU306728 | EU872015 | FJ562275 | AB246338 | DQ890381 | FJ349225 | DQ922650 | AY641559 |

FIG. 6

| | | | | | | | |
|---|---|---|---|---|---|---|---|
| EU871975 | GQ377540 | FJ562229 | FJ562307 | GQ377581 | AB195931 | GQ475327 | DQ315782 |
| AB368296 | FJ882618 | FJ386661 | AB231908 | GQ377520 | FJ386601 | AB493838 | FJ518813 |
| EU881996 | AB367421 | Y18856 | EU562216 | FJ032331 | GQ872211 | EF494378 | EU939571 |
| DQ089772 | AB113877 | AY206381 | AB116088 | FJ787472 | DQ089793 | FJ562294 | FM209515 |
| EU871988 | FJ032351 | EU939607 | AB493844 | EU872009 | EU871994 | GQ377636 | EU155829 |
| FJ386641 | AF223960 | GQ924622 | GQ377616 | DQ986375 | GQ924642 | FJ562327 | AY167092 |
| AB037928 | FJ562288 | AB111121 | GQ475307 | AB365451 | EU871969 | AB246344 | FJ787452 |
| FJ386577 | EU916232 | AB195951 | EU306687 | EU939551 | FJ562235 | EU306714 | FJ023672 |
| AY596108 | AY247032 | EU939590 | FJ562249 | FJ386596 | FJ562269 | EU717216 | EU939647 |
| EU547559 | FJ562304 | FJ787471 | GQ475324 | FJ518810 | DQ478900 | EU881995 | GQ377543 |
| FJ386621 | EU562215 | DQ986376 | GQ377635 | EU939572 | AP011104 | DQ089771 | DQ993688 |
| FJ032332 | D50489 | AB365452 | FJ562297 | FJ562318 | AB367402 | AF498266 | AB195952 |
| GQ377523 | DQ089790 | DQ315781 | EU939618 | FJ562256 | FJ787490 | GQ475338 | EU939593 |
| AB206817 | EU871997 | AB042284 | EU939644 | EU916211 | EU872016 | EU916231 | AB111122 |
| AB300373 | FJ386602 | FJ562324 | FJ386622 | GQ475318 | FJ386574 | AY247031 | AF458664 |
| GQ377615 | EU939552 | EU594388 | AB033557 | GQ377609 | AF182802 | FJ562276 | FJ386662 |
| AB493847 | FJ386595 | EU306717 | AY167091 | GQ475344 | FJ386589 | AB367422 | AY206382 |
| EU570072 | AB115418 | EU717215 | FJ023671 | GQ377563 | EU939624 | AY220700 | Y18855 |
| AB116076 | AB195932 | AY057948 | FJ787451 | AB198083 | EU871976 | AP011097 | EU939658 |
| EU939604 | EU306716 | EU871996 | AB206816 | AB195953 | GQ377628 | AM180624 | GQ475319 |
| FJ787450 | AB246346 | DQ089791 | GQ377522 | DQ993689 | FJ562339 | FJ386575 | EU916210 |
| FJ023670 | FJ562325 | FJ386603 | GQ377583 | GQ372968 | FJ386643 | AP011105 | EU717212 |
| AY167090 | AB042285 | EU570073 | GQ924620 | AY220701 | EU939625 | DQ478901 | EU589337 |
| EU939573 | GQ475325 | EU306685 | AB111123 | AB113875 | DQ089770 | AB198082 | FJ562323 |
| FJ386623 | GQ184326 | GQ475305 | AF458665 | AB367423 | EU871977 | GQ377562 | AB042283 |
| EU939645 | FJ386594 | AB300372 | EU939605 | FJ562277 | EU872017 | AB367403 | GQ475323 |
| EU939619 | EU939553 | FJ562305 | EU939659 | AY247030 | AY077736 | FJ562319 | GQ377632 |
| AB033556 | FJ787470 | AB116077 | FJ386663 | EU916230 | FJ386588 | GQ377608 | FJ562290 |
| EU717214 | AJ748098 | FJ032333 | EU939592 | GQ475339 | AF182803 | GQ475345 | FJ386679 |
| FJ386625 | AB367419 | AB111119 | EU439007 | DQ089756 | AP011103 | EU939549 | EU939554 |
| EU939643 | GQ377524 | EU871990 | AB367425 | EU939603 | GQ377599 | AF182805 | AB426467 |
| AB033550 | GQ377578 | DQ089797 | GQ377518 | AY206385 | AB198079 | EU547561 | DQ089796 |
| FJ787456 | GQ377585 | FJ386605 | GQ377544 | FJ386639 | AB198084 | FJ386645 | EU871991 |
| AY167096 | DQ980551 | FJ386659 | AB195955 | FJ386665 | GQ377538 | FJ386619 | AB111118 |
| EU939575 | AB493840 | EU939555 | EU939594 | AY206378 | AB367405 | EU939623 | FJ386604 |
| DQ993693 | EU919166 | FJ386592 | FJ349241 | AB300368 | AY641561 | AY306136 | EU919167 |
| EU939588 | AB298721 | FJ562271 | EU939569 | GQ475343 | DQ089800 | DQ089776 | AB493841 |
| AB195949 | AB049610 | EU916236 | AJ309370 | EU916216 | FJ787436 | EU871971 | EU570074 |
| FJ032335 | AF223958 | FJ032355 | AB111125 | FJ562251 | EU872011 | FJ386593 | AF223959 |
| FJ562302 | AF182804 | FJ787468 | FJ787489 | EU939597 | AB493843 | AB014385 | FJ023643 |
| AB298720 | EU939548 | EU871973 | EU939557 | EU939536 | GQ377611 | AB014389 | FJ023644 |
| AB367418 | AB198078 | GQ924658 | AB195937 | AB367406 | EU919165 | AB014391 | FJ023645 |
| FJ032334 | GQ377598 | GQ924604 | AB367398 | AY641562 | GQ377527 | AB014392 | FJ023646 |
| GQ377584 | AP011102 | DQ089774 | D12980 | DQ089803 | GQ377586 | AB014393 | FJ023647 |
| DQ980550 | DQ089801 | DQ089789 | GQ377526 | AP011100 | FJ032336 | AB014394 | FJ023648 |
| GQ377579 | EU660225 | EU939621 | FJ032337 | EU916215 | FJ787488 | AB014396 | FJ023649 |
| FJ787457 | AY641560 | AB106895 | FJ562301 | FJ562252 | AB367399 | AB014399 | FJ023650 |
| AB195948 | AB367404 | FJ386647 | EU919164 | AY066028 | AB195936 | AB031262 | FJ023653 |
| EU939589 | EU570068 | EU916214 | AF536524 | FJ562340 | EU939556 | AB031265 | FJ023654 |
| DQ993692 | FJ562250 | GQ475341 | AB493842 | DQ089788 | FJ386591 | AB076678 | FJ023656 |
| EU939574 | EU916217 | DQ089802 | EU916208 | DQ089775 | FJ386606 | AB076679 | FJ023657 |
| EU939642 | GQ475342 | EU660226 | AB033552 | EU871972 | GU357845 | AB105172 | FJ023658 |
| FJ386685 | AB300369 | EU439025 | EU939641 | FJ386646 | GQ924619 | AB105173 | FJ023668 |
| FJ386624 | DQ089757 | AY641563 | FJ386686 | EU547562 | DQ089769 | AB105174 | FJ023674 |
| FJ386678 | AB111124 | AB367407 | FJ386627 | EU939620 | EU871993 | AB116085 | FJ023675 |
| AB033551 | AY206379 | AP011101 | DQ993691 | FJ787469 | DQ089794 | AB362931 | FJ023676 |
| AB042282 | FJ386664 | FJ787448 | EU939577 | EU872012 | AB014360 | AB362932 | L08805 |
| EU589336 | FJ386638 | EU939537 | FJ787454 | GQ475320 | AB014362 | AB367800 | L13994 |
| M38636 | AY206384 | EU939596 | FJ562233 | FJ562293 | AB014363 | AB367803 | M38454 |

FIG. 6

| | | | | | | | |
|---|---|---|---|---|---|---|---|
| EU717213 | EU939602 | AB195957 | EU916228 | GQ377631 | AB014364 | AB367804 | S75184 |
| FJ562230 | EU939568 | FJ386667 | FJ562292 | EU916229 | AB014365 | AF461357 | V00867 |
| GQ377633 | EU939595 | EU939601 | GQ377630 | FJ562232 | AB014367 | AF461358 | X01587 |
| FJ562291 | AB195954 | AF363962 | GQ475321 | EU717211 | AB014369 | AF461359 | X02763 |
| GQ475322 | AJ309371 | EU916234 | AB205152 | EU306713 | AB014370 | AF461361 | X04615 |
| AB050018 | AB367424 | FJ562273 | EU554537 | FJ562320 | AB014371 | AF461363 | X14193 |
| GQ377559 | EU439006 | EU554536 | FJ032356 | EU939576 | AB014372 | AJ012207 | X52939 |
| FJ032348 | EU554535 | GQ377546 | AF241410 | AF330110 | AB014374 | D00630 | X70185 |
| EU939622 | GQ377545 | AB367427 | AB367426 | DQ993690 | AB014376 | D16666 | X75656 |
| FJ386618 | AB117758 | AF241411 | AF363963 | FJ787455 | AB014377 | D16667 | X75665 |
| EU547560 | DQ922649 | EU439005 | FJ562272 | AY167095 | AB014378 | D50517 | Z35717 |
| FJ386644 | EU916237 | FJ386607 | EU916235 | AB033553 | AB014379 | D50518 | Z72478 |
| EU871970 | FJ562270 | DQ089795 | EU939600 | FJ386626 | AB014380 | D50519 | Z72479 |
| DQ089777 | AF363961 | EU871992 | AY206386 | FJ386687 | AB014381 | D50520 | FJ023642 |
| EU872010 | AY148342 | DQ089768 | FJ787449 | EU939640 | AB014382 | FJ023639 | AB014384 |
| FJ787437 | X51970 | GQ924618 | FJ023669 | FJ562300 | AB014383 | FJ023641 | EU916209 |
| AM180623 | EU872013 | FJ787474 | AB195956 | | | | |

**Genotype D**

| | | | | | | | |
|---|---|---|---|---|---|---|---|
| AY721606 | AF121240 | GU456638 | FJ349214 | EU594409 | EU594389 | GU456672 | AB330369 |
| FJ904397 | GQ377589 | AY161162 | EU787440 | FJ904403 | AJ344116 | M32138 | AB330370 |
| FJ904414 | EU919197 | AB270543 | GQ167302 | AY741797 | AY721612 | FJ904447 | AF280817 |
| EU787447 | AB222711 | AY236163 | AB048701 | AY721611 | DQ315780 | FJ904398 | AJ627215 |
| FJ349213 | EU594400 | GU456644 | DQ111987 | EU414141 | AY741794 | AY721609 | AJ627216 |
| GU456680 | GU456663 | GQ205382 | EU594425 | AY902770 | AY233293 | X59795 | AJ627217 |
| X97848 | EU921418 | EU594427 | GQ205380 | AB119256 | FJ904420 | EU787437 | AJ627218 |
| AJ131956 | DQ315777 | DQ486024 | GU456646 | AB493845 | EU414142 | FJ904438 | AJ627219 |
| FJ349233 | L27106 | AB188245 | FJ349228 | X80925 | AY902773 | AY161157 | AJ627220 |
| AB270546 | AB270544 | AB205127 | AB270541 | AY862864 | GU456649 | GU456651 | AJ627221 |
| FJ562263 | AY236164 | EU787442 | AF121239 | GU456657 | AB119255 | AB090269 | AJ627222 |
| EU414136 | FJ904436 | FJ349216 | AY236161 | EU594434 | AB493846 | EU594432 | AJ627223 |
| GU456661 | AY161159 | AF418687 | GU456666 | AY161151 | GU456675 | EU939680 | AJ627224 |
| AB210821 | AY373430 | GU456678 | AB033559 | AB270550 | EU594416 | EF103276 | DQ336674 |
| EU594402 | EU787439 | AB048703 | EU594405 | EU594397 | FJ904440 | AY796030 | DQ336675 |
| FJ904408 | FJ349231 | EU594398 | FJ349208 | GQ477455 | GQ922000 | AM494716 | DQ336676 |
| AF121242 | EF103285 | FJ904431 | AY161160 | GQ922002 | GQ477457 | FJ904418 | DQ336677 |
| DQ304548 | AY661793 | GU456658 | DQ111986 | GU456677 | EU594436 | FJ904444 | DQ336678 |
| AB222713 | DQ329357 | AB210818 | AF418684 | FJ904442 | GU456655 | GU456671 | DQ336679 |
| EU594422 | FJ349211 | AB471856 | FJ904412 | AF418688 | AY161153 | GQ477453 | DQ336680 |
| AY945307 | EU787445 | DQ304551 | AF418679 | FJ349219 | AB109476 | AY902777 | DQ336681 |
| GU456641 | FJ904416 | AB205126 | EU787441 | AB205128 | AY233295 | FJ904424 | DQ336682 |
| GQ205387 | AF418680 | FJ562309 | FJ349215 | FJ904422 | AB270548 | FJ386590 | DQ336683 |
| FJ904428 | GU456682 | DQ486025 | AF043594 | EU594428 | EU414138 | AB119251 | DQ336684 |
| AY161147 | FJ904395 | AB188244 | GQ477459 | EU414140 | FJ904406 | AY090453 | DQ336685 |
| DQ486021 | EU594382 | GQ205383 | GQ924652 | AY721610 | AF418690 | FJ904404 | DQ336686 |
| Y07587 | DQ315776 | GU456645 | FJ349235 | AY233291 | AB119253 | AF418692 | DQ336687 |
| FJ349232 | AB270545 | EU594426 | FJ904432 | AY741796 | GQ205389 | DQ315779 | DQ336688 |
| FJ904435 | EU921419 | AY236162 | AB109478 | FJ349205 | GU357846 | GQ184322 | DQ336689 |
| X97849 | EU594401 | AY741798 | AB110075 | FJ904402 | FJ349221 | GQ477452 | DQ336690 |
| GU456681 | EU414135 | AB270542 | AB222709 | GU456637 | FJ904426 | GQ922005 | DQ336692 |
| FJ904415 | GU456662 | AY161163 | EF103281 | EU594408 | AY161149 | GU456670 | DQ464164 |
| FJ349212 | AB210822 | AB267090 | FJ349209 | AB119254 | AY721608 | FJ904445 | DQ464165 |
| EU787446 | AB222710 | EU594406 | AY161161 | GU456648 | FJ904399 | EU155893 | DQ464166 |
| AB116266 | AB270539 | GU456639 | AB033558 | AY902772 | FJ904446 | FJ904419 | DQ464167 |
| FJ904396 | AF121241 | GU456665 | GU456667 | EU414143 | GU456673 | DQ991753 | DQ464168 |
| AF043593 | AB078033 | AB471857 | EU594404 | AB090270 | EU594410 | AY796031 | DQ464169 |
| AY721607 | DQ486022 | GQ377532 | AY236160 | FJ904421 | AY796032 | EF103277 | DQ464170 |
| AB188241 | AB188243 | DQ304550 | AB246348 | AY741795 | AY161155 | GU456650 | DQ464172 |

FIG. 6

| | | | | | | | |
|---|---|---|---|---|---|---|---|
| FJ904429 | GQ205379 | AB210819 | AB270540 | AY233292 | EU594430 | EU594433 | DQ464173 |
| AB078031 | GU456642 | GU456659 | FJ349229 | AJ132335 | GU456653 | AB090268 | DQ464174 |
| EU594423 | GQ205384 | FJ904430 | EU594424 | AB246347 | AY902769 | EU787436 | DQ464175 |
| GQ205386 | EU594421 | EU594399 | GU456647 | AJ344117 | FJ349220 | AY161156 | DQ464178 |
| GU456640 | FJ904394 | GU456684 | GQ205381 | DQ399006 | AY161148 | FJ904439 | DQ464181 |
| AY341335 | AY721605 | GU456679 | AB493848 | GU456668 | FJ904427 | AB126581 | DQ464182 |
| AB222712 | GU456683 | GQ167301 | AF418689 | FJ904401 | AY902774 | AY233296 | GQ922004 |
| DQ304549 | FJ349210 | AB048702 | FJ904443 | FJ349206 | GQ205388 | DQ315778 | X02496 |
| FJ904409 | AF418681 | FJ349217 | FJ349218 | AB109477 | AB119252 | FJ904405 | X65257 |
| AB210820 | FJ904417 | EU787443 | EU594415 | X80926 | DQ304547 | AY090452 | X65258 |
| GU456660 | EF103279 | AF418686 | GU456676 | AY161152 | AF418691 | AB270537 | X65259 |
| EU414137 | AY661792 | FJ904410 | GQ477454 | GU456654 | FJ904407 | FJ904425 | X68292 |
| EU594403 | DQ329356 | EF103280 | EU594396 | GQ922001 | EU414139 | AY902776 | X72702 |
| AB270547 | AY373431 | AB109479 | GQ922003 | FJ562338 | AB270549 | GQ205377 | X85254 |
| GQ205385 | AY161158 | AM422939 | AY161150 | GQ477456 | U95551 | AB104709 | Z35716 |
| GU456643 | FJ904437 | FJ349234 | GU456656 | FJ904441 | AY233294 | AB104710 | V01460 |
| GQ205378 | FJ349230 | FJ904433 | EU594435 | GU456674 | EU594431 | AB104711 | AB330368 |
| DQ486023 | EU787438 | GQ477458 | AF151735 | FJ904400 | AY902768 | AB104712 | EF103275 |
| AB078032 | EU594407 | GQ377627 | X80924 | FJ349207 | GU456652 | AB330366 | GU456635 |
| AB188242 | AB120308 | FJ904413 | AB109475 | GU456669 | AY161154 | AB330367 | GU456636 |
| AB270538 | GU456664 | AF418685 | | | | | |

FIG. 6

Table 7.Comparision of knockdown efficacies and coverage of HBV genomes for single dsRNAs and combinations thereof.

| dsRNA 1 | | | | dsRNA2 | | | | Combination of dsRNA 1+2 | | | | |
|---|---|---|---|---|---|---|---|---|---|---|---|---|
| SEQ ID NO pair | 1 nM | | % coverage (of 2754 genomes) | SEQ ID NO pair | 1 nM | | % coverage (of 2754 genomes) | 10 nM | 1 nM | | % coverage (of 2754 genomes) | Genomes not matched |
| | [%] rem. Rluc | rank | | | [%] rem. Rluc | rank | | [%] rem. Rluc | [%] rem. Rluc | rank | | |
| 322/486 | 14 | 1 | 96.4 | 333/497 | 21 | 7 | 93.5 | 5 | 25 | 1 | 99.67 | 9 |
| 322/486 | 14 | 1 | 96.4 | 346/510 | 35 | 13 | 94.3 | 7 | 26 | 2 | 99.82 | 5 |
| 322/486 | 14 | 1 | 96.4 | 330/494 | 20 | 5 | 92.2 | 6 | 28 | 3 | 99.67 | 9 |
| 322/486 | 14 | 1 | 96.4 | 324/488 | 15 | 2 | 95.8 | 5 | 29 | 4 | 99.85 | 4 |
| 327/491 | 19 | 4 | 92.6 | 322/486 | 14 | 1 | 96.4 | 5 | 30 | 5 | 99.64 | 10 |
| 327/491 | 19 | 4 | 92.6 | 326/490 | 17 | 3 | 93.3 | 4 | 30 | 6 | 99.35 | 18 |
| 326/490 | 17 | 3 | 93.3 | 333/497 | 21 | 7 | 93.5 | 4 | 30 | 7 | 99.71 | 8 |
| 336/500 | 23 | 8 | 90.2 | 322/486 | 14 | 1 | 96.4 | 5 | 31 | 8 | 99.64 | 10 |
| 324/488 | 15 | 2 | 95.8 | 333/497 | 21 | 7 | 93.5 | 3 | 31 | 9 | 99.56 | 12 |
| 324/488 | 15 | 2 | 95.8 | 339/503 | 25 | 10 | 92.6 | 5 | 31 | 10 | 99.75 | 7 |
| 326/490 | 17 | 3 | 93.3 | 347/511 | 36 | 14 | 96.5 | 6 | 31 | 11 | 99.82 | 5 |
| 326/490 | 17 | 3 | 93.3 | 322/486 | 14 | 1 | 96.4 | 5 | 32 | 12 | 99.85 | 4 |
| 332/496 | 21 | 6 | 94.0 | 322/486 | 14 | 1 | 96.4 | 6 | 32 | 13 | 99.85 | 4 |
| 332/496 | 21 | 6 | 94.0 | 324/488 | 15 | 2 | 95.8 | 4 | 32 | 14 | 99.31 | 19 |
| 327/491 | 19 | 4 | 92.6 | 332/496 | 21 | 6 | 94.0 | 4 | 32 | 15 | 99.38 | 17 |
| 332/496 | 21 | 6 | 94.0 | 347/511 | 36 | 14 | 96.5 | 5 | 32 | 16 | 99.89 | 3 |
| 327/491 | 19 | 4 | 92.6 | 324/488 | 15 | 2 | 95.8 | 4 | 33 | 17 | 99.78 | 6 |
| 336/500 | 23 | 8 | 90.2 | 324/488 | 15 | 2 | 95.8 | 5 | 33 | 18 | 99.49 | 14 |
| 332/496 | 21 | 6 | 94.0 | 333/497 | 21 | 7 | 93.5 | 3 | 34 | 19 | 99.71 | 8 |
| 324/488 | 15 | 2 | 95.8 | 347/511 | 36 | 14 | 96.5 | 5 | 34 | 20 | 99.85 | 4 |
| 332/496 | 21 | 6 | 94.0 | 330/494 | 20 | 5 | 92.2 | 4 | 37 | 21 | 99.24 | 21 |
| 337/501 | 24 | 9 | 95.2 | 322/486 | 14 | 1 | 96.4 | 6 | 42 | 22 | 99.82 | 5 |
| 337/501 | 24 | 9 | 95.2 | 347/511 | 36 | 14 | 96.5 | 6 | 42 | 23 | 99.89 | 3 |

FIG. 7

| | | | | | | | | | | | | |
|---|---|---|---|---|---|---|---|---|---|---|---|---|
| 337/501 | 24 | 9 | 95.2 | 324/488 | 15 | 2 | 95.8 | 5 | 43 | 24 | 99.60 | 11 |
| 337/501 | 24 | 9 | 95.2 | 333/497 | 21 | 7 | 93.5 | 6 | 44 | 25 | 99.71 | 8 |
| 337/501 | 24 | 9 | 95.2 | 336/500 | 23 | 8 | 90.2 | 7 | 47 | 26 | 99.71 | 8 |
| 341/505 | 31 | 11 | 91.5 | 322/486 | 14 | 1 | 96.4 | 5 | 50 | 27 | 99.85 | 4 |
| 341/505 | 31 | 11 | 91.5 | 324/488 | 15 | 2 | 95.8 | 5 | 57 | 28 | 99.67 | 9 |
| 351/515 | 38 | 15 | 97.7 | 337/501 | 24 | 9 | 95.2 | 6 | 60 | 29 | 99.75 | 7 |
| 351/515 | 38 | 15 | 97.7 | 342/506 | 32 | 12 | 93.2 | 8 | 60 | 30 | 99.93 | 2 |

FIG. 7

Table 8. Sequences of the negative control ds RNAs used in the psiCHECK™-2 screening assay.

| strand | Sequence | gene |
|---|---|---|
| sense | 5′-cuuAcGcuGAGuAcuucGATsT-3′ | LUC(GL3) |
| antisense | 5′-UCGAAGuACUcAGCGuAAGTsT-3′ | LUC(GL3) |
| sense | 5′-CcAcAuGAAGcAGcACGACusU-3′ | GFP |
| antisense | 5′-AAGUCGUGCUGCUUCAUGUGgsusC -3′ | GFP |

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003206887 A
- WO 2011073218 A
- US 5328470 A
- WO 0022113 A, Skillern A
- WO 9106309 A
- WO 2011003780 A
- US 5218105 A
- US 5541307 A
- US 5521302 A
- US 5539082 A
- US 5554746 A
- US 5571902 A
- US 5578718 A
- US 5587361 A
- US 5506351 A
- US 5587469 A
- US 5587470 A
- US 5608046 A
- US 5610289 A
- US 6262241 B
- WO 9307883 A, Manoharan
- US 4469863 A
- US 5023243 A
- US 5264423 A
- US 5321131 A
- US 5399676 A
- US 5405939 A
- US 5453496 A
- US 5455233 A
- US 5466677 A
- US 5034506 A
- US 5214134 A
- US 5216141 A
- US 5264562 A
- US 5470967 A
- US 5489677 A
- US 5602240 A
- US 5663312 A
- US 3687808 A
- US 5134066 A
- US 5459255 A
- US 5552540 A
- US 5594121 A
- US 5596091 A
- US 6127533 A
- US 6166197 A
- US 5670633 A
- US 6172209 B
- US 6271358 B
- US 5359044 A
- US 5466786 A
- US 5519134 A
- US 5591722 A
- US 5597909 A
- US 5646265 A
- US 5700920 A
- US 5212295 A, Cook
- EP 11172235
- US 13535454 B


### Non-patent literature cited in the description


- **ZHANG et al.** *BMC Microbiol,* 2010, vol. 10, 214
- **CHEN et al.** *Proc. Natl. Acad. Sci. USA,* 1994, vol. 91, 3054-3057
- **GASSMANN et al.** *Proc. Natl. Acad. Sci. USA,* 1995, vol. 92, 1292
- **MUZYCZKA et al.** *Curr. Topics Micro. Immunol.,* 1992, vol. 158, 97-129
- **BERKNER et al.** *BioTechniques,* 1998, vol. 6, 616
- **ROSENFELD et al.** *Science,* 1991, vol. 252, 431-434
- **ROSENFELD et al.** *Cell,* 1992, vol. 68, 143-155
- **DANOS ; MULLIGAN.** *Proc. Natl. Acad. Sci. USA,* 1998, vol. 85, 6460-6464
- **COMETTE et al.** *Human Gene Therapy,* 1991, vol. 2, 5-10
- **CONE et al.** *Proc. Natl. Acad. Sci. USA,* 1984, vol. 81, 6349
- **HSU et al.** *J. Infectious Disease,* 1992, vol. 166, 769
- **BUCCHINI et al.** *Proc. Natl. Acad. Sci. USA,* 1986, vol. 83, 2511-2515
- **DOCHERTY et al.** *FASEB J.,* 1994, vol. 8, 20-24
- **WILLIAMS DJ ; HALL KB.** *Biochem.,* 1996, vol. 35, 14665-14670
- **WAGNER.** *Nat. Med.,* 1995, vol. 1, 1116-8

- **NAWROT.** *Current Topics in Med Chem,* 2006, vol. 6, 913-925
- **MANOHARAN M.** *Antisense & Nucleic Acid Drug Development,* 2002, vol. 12, 103
- **LI S ; DESHMUKH HM ; HUANG L.** *Pharm. Res.,* 1998, vol. 15, 1540
- **GREENE ; WUTS.** Protective Groups in Organic Synthesis. John Wiley & Sons, 1991
- **GUZAEV AL ; MANOHARAN MJ.** *Am. Chem. Soc.,* 2003, vol. 125, 2380
- **DELGARDO.** *Critical Reviews in Therapeutic Drug Carrier Systems,* 1992, vol. 9, 249
- **COOK.** *Anti-fibrosis Drug Design,* 1991, vol. 6, 585-607
- **HAMM et al.** *J. Org. Chem.,* 1997, vol. 62, 3415-3420
- **THOMSON JB.** *J. Org. Chem.,* 1996, vol. 61, 6273-6281
- **POLUSHIN et al.** *Tetrahedron Lett.,* 1996, vol. 37, 3227-3230
- **LETSINGER et al.** *Proc. Natl. Acad. Sci. USA,* 1989, vol. 86, 6553
- **MANOHARAN et al.** *Bioorg. Med. Chem. Lett.,* 1994, vol. 4, 1053
- **MANOHARAN et al.** *Ann. N.Y. Acad. Sci.,* 1992, vol. 660, 306
- **MANOHARAN et al.** *Bioorg. Med. Chem. Let.,* 1993, vol. 3, 2765
- **OBERHAUSER et al.** *Nucl. Acids Res.,* 1992, vol. 20, 533
- **BEHMOARAS et al.** *EMBO J.,* 1991, vol. 10, 111
- **KABANOV et al.** *FEBS Lett.,* 1990, vol. 259, 327
- **SVINARCHUK et al.** *Biochimie,* 1993, vol. 75, 49
- **MANOHARAN et al.** *Tetrahedron Lett.,* 1995, vol. 36, 3651
- **SHEA et al.** *Nucl. Acids Res.,* 1990, vol. 18, 3777
- **MANOHARAN et al.** *Nucleosides & Nucleotides,* 1995, vol. 14, 969
- **MISHRA et al.** *Biochim. Biophys. Acta,* 1995, vol. 1264, 229
- **CROOKE et al.** *J. Pharmacol. Exp. Ther.,* 1996, vol. 277, 923
- **SAISON-BEHMOARAS et al.** *EMBO J.,* 1991, vol. 10, 111
- **AKHTAR.** *Journal of Clinical Investigation,* 2007, vol. 117, 3623-3632
- **NGUYEN et al.** *Current Opinion in Molecular Therapeutics,* 2008, vol. 10, 158-167
- **ZAMBONI.** *Clin Cancer Res,* 2005, vol. 11, 8230-8234
- **IKEDA et al.** *Pharmaceutical Research,* 2006, vol. 23, 1631-1640